# EUROPEAN PATENT APPLICATION

(11) **EP 1 479 780 A1**
(43) Date of publication of application: **24.11.2004**
(21) Application number: 03447119.3
(22) Date of filing: 22.05.2003
(51) Int. Cl.: C12Q 1/68

(54) **A novel design of probes for saccharomyces cerevisiae which minimizes cross-hybridization, the obtained probes and diagnostic uses thereof**

(71) Applicant: INSTITUT PASTEUR, 75015 Paris (FR); UNIVERSITE PIERRE ET MARIE CURIE, 75252 Paris Cédex 05 (FR)
(72) Inventor: Talla, Emmanuel, 93100 Montreuil (FR); Tekaia, Fredj, 77600 Bussy Saint Georges (FR); Dujon, Bernard, 91190 Gif sur Yvette (FR)
(74) Representative: Van Malderen, Joelle

(57) **Abstract**

The present invention is related to nucleotide sequences which are each specific for a *Saccharomyces cerevisiae* CDS and which could used as probes in diagnostic kits. The present invention also concerns a method for the design of said probes, as well as a method for detecting and/or possibly quantifying a target nucleotide sequence of *S.cerevisiae* and/or its expression in a biological sample.

SEQUENCE LISTING

<110>  INSTITUT PASTEUR

<120>  A NOVEL DESIGN OF PROBES FOR SACCHAROMYCES CEREVISIAEWHICH
       MINIMISES CROSS-HYBRIDISATION? TH EOBTAINED PROBES AND DIAGNOSTIC
        USE THEREOF

<130>  P.PAST.32/EP

<140>  EP 03447119.3
<141>  2003-05-22

<160>  1553

<170>  PatentIn version 3.2

<210>  1
<211>  20
<212>  DNA
<213>  ARTIFICIAL SEQUENCE

<220>
<223>  forward primer

<400>  1
cttacctgag caagatccgc                                                  20


<210>  2
<211>  20
<212>  DNA
<213>  ARTIFICIAL SEQUENCE

<220>
<223>  forward primer

<400>  2
cctattggac aagcacggtt                                                  20


<210>  3
<211>  20
<212>  DNA
<213>  ARTIFICIAL SEQUENCE

<220>
<223>  forward primer

<400>  3
tcctcttgaa cctcctggaa                                                  20


<210>  4
<211>  20
<212>  DNA
<213>  ARTIFICIAL SEQUENCE

<220>
<223>  forward primer

<400>  4
attagccaca atggctcacc                                                  20


<210>  5
<211>  20
<212>  DNA
<213>  ARTIFICIAL SEQUENCE

<220>
<223>  forward primer

<400>  5
aaggtgccaa gatccaaatg                                                    20


<210>  6
<211>  20
<212>  DNA
<213>  ARTIFICIAL SEQUENCE

<220>
<223>  forward primer

<400>  6
ttgctttgag aaaggctggt                                                    20


<210>  7
<211>  20
<212>  DNA
<213>  ARTIFICIAL SEQUENCE

<220>
<223>  forward primer

<400>  7
attgctctgc ttcggaacat                                                    20


<210>  8
<211>  20
<212>  DNA
<213>  ARTIFICIAL SEQUENCE

<220>
<223>  forward primer

<400>  8
gtggcatctg gcgttaagtt                                                    20


<210>  9
<211>  20
<212>  DNA
<213>  ARTIFICIAL SEQUENCE

<220>
<223>  forward primer

<400>  9
acaggttttt gcctgctgtc                                                    20


<210>  10
<211>  20
<212>  DNA
<213>  ARTIFICIAL SEQUENCE

<220>
<223>  forward primer

<400>  10
tggtattgag gcgacataag                                                    20


<210>  11

<211>  20
<212>  DNA
<213>  ARTIFICIAL SEQUENCE

<220>
<223>  forward primer

<400>  11
tccaaggccg gtattgttac                                                    20


<210>  12
<211>  20
<212>  DNA
<213>  ARTIFICIAL SEQUENCE

<220>
<223>  forward primer

<400>  12
   ccattcttag gggtttgcct                                                     20


<210>  13
<211>  20
<212>  DNA
<213>  ARTIFICIAL SEQUENCE

<220>
<223>  forward primer

<400>  13
taggaaaaac tcgcattccg                                                    20


<210>  14
<211>  20
<212>  DNA
<213>  ARTIFICIAL SEQUENCE

<220>
<223>  forward primer

<400>  14
tgttcaccca caactgaagg                                                    20


<210>  15
<211>  20
<212>  DNA
<213>  ARTIFICIAL SEQUENCE

<220>
<223>  forward primer

<400>  15
attgctggta ccaatggaca                                                    20


<210>  16
<211>  20
<212>  DNA
<213>  ARTIFICIAL SEQUENCE

<220>
<223>  forward primer

<400>  16
tctatgcaaa ctgcggactg                                                    20

<210>  17
<211>  20
<212>  DNA
<213>  ARTIFICIAL SEQUENCE

<220>
<223>  forward primer

<400>  17
accgattctg aacactggga                                                    20


<210>  18
<211>  20
<212>  DNA
<213>  ARTIFICIAL SEQUENCE

<220>
<223>  forward primer

<400>  18
cacaacattt ccacaccctg                                                    20


<210>  19
<211>  20
<212>  DNA
<213>  ARTIFICIAL SEQUENCE

<220>
<223>  forward primer

<400>  19
ttttgtccag tattgccggt                                                    20


<210>  20
<211>  20
<212>  DNA
<213>  ARTIFICIAL SEQUENCE

<220>
<223>  forward primer

<400>  20
cagacaagcg aacgtctcaa                                                    20


<210>  21
<211>  20
<212>  DNA
<213>  ARTIFICIAL SEQUENCE

<220>
<223>  forward primer

<400>  21
ccgcaagatt aagtggcaat                                                    20


<210>  22
<211>  20
<212>  DNA
<213>  ARTIFICIAL SEQUENCE

<220>
<223>  forward primer

<400>  22
gtcgttagct tctggaacgg                                                    20


<210>  23
<211>  20
<212>  DNA
<213>  ARTIFICIAL SEQUENCE

<220>
<223>  forward primer

<400>  23
ccataaggcc attatcgacg                                                    20


<210>  24
<211>  20
<212>  DNA
<213>  ARTIFICIAL SEQUENCE

<220>
<223>  forward primer

<400>  24
tttgttgatc ctatctatct                                                    20


<210>  25
<211>  20
<212>  DNA
<213>  ARTIFICIAL SEQUENCE

<220>
<223>  forward primer

<400>  25
ctcatggagg tgctccaaat                                                    20


<210>  26
<211>  20
<212>  DNA
<213>  ARTIFICIAL SEQUENCE

<220>
<223>  forward primer

<400>  26
tgtgaatgag gcgatgaaaa                                                    20


<210>  27
<211>  20
<212>  DNA
<213>  ARTIFICIAL SEQUENCE

<220>
<223>  forward primer

<400>  27
ccaggtgcac cactaggatt                                                    20


<210>  28
<211>  20
<212>  DNA
<213>  ARTIFICIAL SEQUENCE

<220>
<223>  forward primer

<400>  28
attgaaggca aatggcaagt                                                    20


<210>  29
<211>  20
<212>  DNA
<213>  ARTIFICIAL SEQUENCE

<220>
<223>  forward primer

<400>  29
cgtaaaagga gccgcattag                                                    20


<210>  30
<211>  20
<212>  DNA
<213>  ARTIFICIAL SEQUENCE

<220>
<223>  forward primer

<400>  30
atgaagaagg agttgaaaat                                                    20


<210>  31
<211>  20
<212>  DNA
<213>  ARTIFICIAL SEQUENCE

<220>
<223>  forward primer

<400>  31
tcgagaacgc ttacacaacg                                                    20


<210>  32
<211>  20
<212>  DNA
<213>  ARTIFICIAL SEQUENCE

<220>
<223>  forward primer

<400>  32
aacagcatgt tgcaaacgag                                                    20


<210>  33
<211>  20
<212>  DNA
<213>  ARTIFICIAL SEQUENCE

<220>
<223>  forward primer

<400>  33
tacggtttgg tcggagtagg                                                    20


<210>  34

<211>  20
<212>  DNA
<213>  ARTIFICIAL SEQUENCE

<220>
<223>  forward primer

<400>  34
tgcactgtac ccacaccaat                                                    20


<210>  35
<211>  20
<212>  DNA
<213>  ARTIFICIAL SEQUENCE

<220>
<223>  forward primer

<400>  35
tgcacatcgg tgttctcaat                                                    20


<210>  36
<211>  20
<212>  DNA
<213>  ARTIFICIAL SEQUENCE

<220>
<223>  forward primer

<400>  36
tcctagcacc agcagaacct                                                    20


<210>  37
<211>  20
<212>  DNA
<213>  ARTIFICIAL SEQUENCE

<220>
<223>  forward primer

<400>  37
ctgaatggaa gcctgaaagc                                                    20


<210>  38
<211>  20
<212>  DNA
<213>  ARTIFICIAL SEQUENCE

<220>
<223>  forward primer

<400>  38
aatgttgctg tggatgcttg                                                    20


<210>  39
<211>  20
<212>  DNA
<213>  ARTIFICIAL SEQUENCE

<220>
<223>  forward primer

<400>  39
accaaaaagg gcaaggaaat                                                    20

<210> 40
<211> 20
<212> DNA
<213> ARTIFICIAL SEQUENCE

<220>
<223> forward primer

<400> 40
tgttgaggtt ttaccagccc 20

<210> 41
<211> 20
<212> DNA
<213> ARTIFICIAL SEQUENCE

<220>
<223> forward primer

<400> 41
ggtacagtgg gcgaagttgt 20

<210> 42
<211> 20
<212> DNA
<213> ARTIFICIAL SEQUENCE

<220>
<223> forward primer

<400> 42
acgaagttgc cacaggagtt 20

<210> 43
<211> 20
<212> DNA
<213> ARTIFICIAL SEQUENCE

<220>
<223> forward primer

<400> 43
atgtctgaca aggaacaaac 20

<210> 44
<211> 20
<212> DNA
<213> ARTIFICIAL SEQUENCE

<220>
<223> forward primer

<400> 44
caactttgga gcgaccattt 20

<210> 45
<211> 20
<212> DNA
<213> ARTIFICIAL SEQUENCE

<220>
<223> forward primer

<400>  45
cctctcttcg caaaataacg                                                    20


<210>  46
<211>  20
<212>  DNA
<213>  ARTIFICIAL SEQUENCE

<220>
<223>  forward primer

<400>  46
ccgtgtttac gctttccaac                                                    20


<210>  47
<211>  20
<212>  DNA
<213>  ARTIFICIAL SEQUENCE

<220>
<223>  forward primer

<400>  47
gtggaattgc gtttcgtttg                                                    20


<210>  48
<211>  20
<212>  DNA
<213>  ARTIFICIAL SEQUENCE

<220>
<223>  forward primer

<400>  48
tgcaagagat ggaacagtcg                                                    20


<210>  49
<211>  20
<212>  DNA
<213>  ARTIFICIAL SEQUENCE

<220>
<223>  forward primer

<400>  49
caagatattc gcgtttcacg                                                    20


<210>  50
<211>  20
<212>  DNA
<213>  ARTIFICIAL SEQUENCE

<220>
<223>  forward primer

<400>  50
caaatcagga gctgcagaca                                                    20


<210>  51
<211>  20
<212>  DNA
<213>  ARTIFICIAL SEQUENCE

<220>
<223>  forward primer

<400>  51
cgctccatta ttgtgtggtg                                                    20


<210>  52
<211>  20
<212>  DNA
<213>  ARTIFICIAL SEQUENCE

<220>
<223>  forward primer

<400>  52
gttgcgtcca aatgacaatg                                                    20


<210>  53
<211>  20
<212>  DNA
<213>  ARTIFICIAL SEQUENCE

<220>
<223>  forward primer

<400>  53
cttaacttcc ggccacttga                                                    20


<210>  54
<211>  20
<212>  DNA
<213>  ARTIFICIAL SEQUENCE

<220>
<223>  forward primer

<400>  54
ccaaggcttg ctcttaatgc                                                    20


<210>  55
<211>  20
<212>  DNA
<213>  ARTIFICIAL SEQUENCE

<220>
<223>  forward primer

<400>  55
gggaaacaat gatggcactt                                                    20


<210>  56
<211>  20
<212>  DNA
<213>  ARTIFICIAL SEQUENCE

<220>
<223>  forward primer

<400>  56
cgatcagaaa acaacccgat                                                    20


<210>  57

<211>  20
<212>  DNA
<213>  ARTIFICIAL SEQUENCE

<220>
<223>  forward primer

<400>  57
atccatctca aattgccacc                                                    20


<210>  58
<211>  20
<212>  DNA
<213>  ARTIFICIAL SEQUENCE

<220>
<223>  forward primer

<400>  58
cagagggctg ctaaatacgc                                                    20


<210>  59
<211>  20
<212>  DNA
<213>  ARTIFICIAL SEQUENCE

<220>
<223>  forward primer

<400>  59
aggtgcttag cctttcccat                                                    20


<210>  60
<211>  20
<212>  DNA
<213>  ARTIFICIAL SEQUENCE

<220>
<223>  forward primer

<400>  60
gttgatcatc cgttgtcgtg                                                    20


<210>  61
<211>  20
<212>  DNA
<213>  ARTIFICIAL SEQUENCE

<220>
<223>  forward primer

<400>  61
tatcgcaaag gcattgacag                                                    20


<210>  62
<211>  20
<212>  DNA
<213>  ARTIFICIAL SEQUENCE

<220>
<223>  forward primer

<400>  62
ttggtgatga ttgcaaggaa                                                    20

<210> 63
<211> 20
<212> DNA
<213> ARTIFICIAL SEQUENCE

<220>
<223> forward primer

<400> 63
ccttctctgc aagacgttcc 20

<210> 64
<211> 20
<212> DNA
<213> ARTIFICIAL SEQUENCE

<220>
<223> forward primer

<400> 64
ttcaatgacc cattcggatt 20

<210> 65
<211> 20
<212> DNA
<213> ARTIFICIAL SEQUENCE

<220>
<223> forward primer

<400> 65
gtggaaagaa atggccaaaa 20

<210> 66
<211> 20
<212> DNA
<213> ARTIFICIAL SEQUENCE

<220>
<223> forward primer

<400> 66
ggccttacca tctcacaagg 20

<210> 67
<211> 20
<212> DNA
<213> ARTIFICIAL SEQUENCE

<220>
<223> forward primer

<400> 67
acgatggcat aacgacaaca 20

<210> 68
<211> 20
<212> DNA
<213> ARTIFICIAL SEQUENCE

<220>
<223> forward primer

<400>  68
ttctgtcaca aagccaggtg                                                    20


<210>  69
<211>  20
<212>  DNA
<213>  ARTIFICIAL SEQUENCE

<220>
<223>  forward primer

<400>  69
cggatatgac cacggttgat                                                    20


<210>  70
<211>  20
<212>  DNA
<213>  ARTIFICIAL SEQUENCE

<220>
<223>  forward primer

<400>  70
agatgggact ggttctgtgg                                                    20


<210>  71
<211>  20
<212>  DNA
<213>  ARTIFICIAL SEQUENCE

<220>
<223>  forward primer

<400>  71
accaagattg acgttaccgc                                                    20


<210>  72
<211>  20
<212>  DNA
<213>  ARTIFICIAL SEQUENCE

<220>
<223>  forward primer

<400>  72
atttccatac cagttgggca                                                    20


<210>  73
<211>  20
<212>  DNA
<213>  ARTIFICIAL SEQUENCE

<220>
<223>  forward primer

<400>  73
gaggatccct gccaactgta                                                    20


<210>  74
<211>  20
<212>  DNA
<213>  ARTIFICIAL SEQUENCE

<220>
<223>  forward primer

<400>  74
ttcaaggagg ttaagggtgg                                                    20


<210>  75
<211>  20
<212>  DNA
<213>  ARTIFICIAL SEQUENCE

<220>
<223>  forward primer

<400>  75
atacgtttca tctcaccgcc                                                    20


<210>  76
<211>  20
<212>  DNA
<213>  ARTIFICIAL SEQUENCE

<220>
<223>  forward primer

<400>  76
tggtggtacc agaaggaaaa                                                    20


<210>  77
<211>  20
<212>  DNA
<213>  ARTIFICIAL SEQUENCE

<220>
<223>  forward primer

<400>  77
aggatcagga tgctgcattt                                                    20


<210>  78
<211>  20
<212>  DNA
<213>  ARTIFICIAL SEQUENCE

<220>
<223>  forward primer

<400>  78
cttgtattag gacgccctgc                                                    20


<210>  79
<211>  20
<212>  DNA
<213>  ARTIFICIAL SEQUENCE

<220>
<223>  forward primer

<400>  79
ggtttgcaag ctccctacag                                                    20


<210>  80

<211>  20
<212>  DNA
<213>  ARTIFICIAL SEQUENCE

<220>
<223>  forward primer

<400>  80
cgaggagaag aggaaagacg                                                    20


<210>  81
<211>  20
<212>  DNA
<213>  ARTIFICIAL SEQUENCE

<220>
<223>  forward primer

<400>  81
gccatggaca aacagtgtca                                                    20


<210>  82
<211>  20
<212>  DNA
<213>  ARTIFICIAL SEQUENCE

<220>
<223>  forward primer

<400>  82
aaatgcttct ggtatcaccc                                                    20


<210>  83
<211>  20
<212>  DNA
<213>  ARTIFICIAL SEQUENCE

<220>
<223>  forward primer

<400>  83
aaaagcaaac gtgggtaacg                                                    20


<210>  84
<211>  20
<212>  DNA
<213>  ARTIFICIAL SEQUENCE

<220>
<223>  forward primer

<400>  84
agttcctcaa ttttcgccct                                                    20


<210>  85
<211>  20
<212>  DNA
<213>  ARTIFICIAL SEQUENCE

<220>
<223>  forward primer

<400>  85
ttgggatatt gcaaacagca                                                    20

<210>  86
<211>  20
<212>  DNA
<213>  ARTIFICIAL SEQUENCE

<220>
<223>  forward primer

<400>  86
tctcacgtca aagacatcgc                                                    20


<210>  87
<211>  20
<212>  DNA
<213>  ARTIFICIAL SEQUENCE

<220>
<223>  forward primer

<400>  87
acagtttcgc cggaatacac                                                    20


<210>  88
<211>  20
<212>  DNA
<213>  ARTIFICIAL SEQUENCE

<220>
<223>  forward primer

<400>  88
gtccgttaat caagagggca                                                    20


<210>  89
<211>  20
<212>  DNA
<213>  ARTIFICIAL SEQUENCE

<220>
<223>  forward primer

<400>  89
atcacgttgt ttgccacaag                                                    20


<210>  90
<211>  20
<212>  DNA
<213>  ARTIFICIAL SEQUENCE

<220>
<223>  forward primer

<400>  90
gcatgaagga aaaacccaaa                                                    20


<210>  91
<211>  20
<212>  DNA
<213>  ARTIFICIAL SEQUENCE

<220>
<223>  forward primer

<400>  91
ttgtggaggc ttcttctgct                                                    20

<210>  92
<211>  20
<212>  DNA
<213>  ARTIFICIAL SEQUENCE

<220>
<223>  forward primer

<400>  92
tcagacaacc agcaagcaac                                                    20

<210>  93
<211>  20
<212>  DNA
<213>  ARTIFICIAL SEQUENCE

<220>
<223>  forward primer

<400>  93
ggttcaggga gatggattga                                                    20

<210>  94
<211>  20
<212>  DNA
<213>  ARTIFICIAL SEQUENCE

<220>
<223>  forward primer

<400>  94
aactggtgat ggtcaaaggc                                                    20

<210>  95
<211>  20
<212>  DNA
<213>  ARTIFICIAL SEQUENCE

<220>
<223>  forward primer

<400>  95
ttgccacata tcttcaacgc                                                    20

<210>  96
<211>  20
<212>  DNA
<213>  ARTIFICIAL SEQUENCE

<220>
<223>  forward primer

<400>  96
caatgaacct cagcagcatc                                                    20

<210>  97
<211>  20
<212>  DNA
<213>  ARTIFICIAL SEQUENCE

<220>
<223>  forward primer

<400>  97
cagatgacaa cgtcgcagtg                                                    20


<210>  98
<211>  20
<212>  DNA
<213>  ARTIFICIAL SEQUENCE

<220>
<223>  forward primer

<400>  98
tggcaaaagc tcttttcgtt                                                    20


<210>  99
<211>  20
<212>  DNA
<213>  ARTIFICIAL SEQUENCE

<220>
<223>  forward primer

<400>  99
acagagtcgc actccacctt                                                    20


<210>  100
<211>  20
<212>  DNA
<213>  ARTIFICIAL SEQUENCE

<220>
<223>  forward primer

<400>  100
cagaagattg cggatcaagc                                                    20


<210>  101
<211>  20
<212>  DNA
<213>  ARTIFICIAL SEQUENCE

<220>
<223>  forward primer

<400>  101
catacccgct ggtgtagtca                                                    20


<210>  102
<211>  20
<212>  DNA
<213>  ARTIFICIAL SEQUENCE

<220>
<223>  forward primer

<400>  102
gattctggca aatggacgat                                                    20


<210>  103

<211>  20
<212>  DNA
<213>  ARTIFICIAL SEQUENCE

<220>
<223>  forward primer

<400>  103
ttcatcggtg tcatcgtcat                                                    20


<210>  104
<211>  20
<212>  DNA
<213>  ARTIFICIAL SEQUENCE

<220>
<223>  forward primer

<400>  104
cggatcatcg atgtctgttg                                                    20


<210>  105
<211>  20
<212>  DNA
<213>  ARTIFICIAL SEQUENCE

<220>
<223>  forward primer

<400>  105
caccaccttc agcagagtga                                                    20


<210>  106
<211>  20
<212>  DNA
<213>  ARTIFICIAL SEQUENCE

<220>
<223>  forward primer

<400>  106
catttacaca acctcgcacg                                                    20


<210>  107
<211>  20
<212>  DNA
<213>  ARTIFICIAL SEQUENCE

<220>
<223>  forward primer

<400>  107
cgttaatgga tgacgctttg                                                    20


<210>  108
<211>  20
<212>  DNA
<213>  ARTIFICIAL SEQUENCE

<220>
<223>  forward primer

<400>  108
catggtggtc tctcaccaga                                                    20

<210>  109
<211>  20
<212>  DNA
<213>  ARTIFICIAL SEQUENCE

<220>
<223>  forward primer

<400>  109
cttactgccc cgtttcacat                                                    20


<210>  110
<211>  20
<212>  DNA
<213>  ARTIFICIAL SEQUENCE

<220>
<223>  forward primer

<400>  110
ctctcaaatg cccaaccatt                                                    20


<210>  111
<211>  20
<212>  DNA
<213>  ARTIFICIAL SEQUENCE

<220>
<223>  forward primer

<400>  111
ccaggaccaa tttacgcaac                                                    20


<210>  112
<211>  20
<212>  DNA
<213>  ARTIFICIAL SEQUENCE

<220>
<223>  forward primer

<400>  112
cctgagagga ggcgagtatg                                                    20


<210>  113
<211>  20
<212>  DNA
<213>  ARTIFICIAL SEQUENCE

<220>
<223>  forward primer

<400>  113
tcgtggtgct atgggtatca                                                    20


<210>  114
<211>  20
<212>  DNA
<213>  ARTIFICIAL SEQUENCE

<220>
<223>  forward primer

<400>  114
tcctcctcaa gtgccttctg                                                    20


<210>  115
<211>  20
<212>  DNA
<213>  ARTIFICIAL SEQUENCE

<220>
<223>  forward primer

<400>  115
atcaatcaac gtgaccgaca                                                    20


<210>  116
<211>  20
<212>  DNA
<213>  ARTIFICIAL SEQUENCE

<220>
<223>  forward primer

<400>  116
gaccaagaat ccttcaacgg                                                    20


<210>  117
<211>  20
<212>  DNA
<213>  ARTIFICIAL SEQUENCE

<220>
<223>  forward primer

<400>  117
ccaaagaatg cggctttaga                                                    20


<210>  118
<211>  20
<212>  DNA
<213>  ARTIFICIAL SEQUENCE

<220>
<223>  forward primer

<400>  118
gatctggcct ttgctgtgtt                                                    20


<210>  119
<211>  20
<212>  DNA
<213>  ARTIFICIAL SEQUENCE

<220>
<223>  forward primer

<400>  119
cggtggttgg agaatgagat                                                    20


<210>  120
<211>  20
<212>  DNA
<213>  ARTIFICIAL SEQUENCE

<220>
<223>  forward primer

<400>  120
cttcactgtg gaggcactga                                                   20


<210>  121
<211>  20
<212>  DNA
<213>  ARTIFICIAL SEQUENCE

<220>
<223>  forward primer

<400>  121
aaggttcgcc ataagctcaa                                                   20


<210>  122
<211>  20
<212>  DNA
<213>  ARTIFICIAL SEQUENCE

<220>
<223>  forward primer

<400>  122
ggtaggataa gaccgcctcc                                                   20


<210>  123
<211>  20
<212>  DNA
<213>  ARTIFICIAL SEQUENCE

<220>
<223>  forward primer

<400>  123
atgaatacat cggccacgtt                                                   20


<210>  124
<211>  20
<212>  DNA
<213>  ARTIFICIAL SEQUENCE

<220>
<223>  forward primer

<400>  124
tggcactttt ggattcagag                                                   20


<210>  125
<211>  20
<212>  DNA
<213>  ARTIFICIAL SEQUENCE

<220>
<223>  forward primer

<400>  125
attgccattc agagtttccg                                                   20


<210>  126

<211>  20
<212>  DNA
<213>  ARTIFICIAL SEQUENCE

<220>
<223>  forward primer

<400>  126
atgactgata catcatcctc                                                    20


<210>  127
<211>  20
<212>  DNA
<213>  ARTIFICIAL SEQUENCE

<220>
<223>  forward primer

<400>  127
cctcaggcaa ctcttgaagg                                                    20


<210>  128
<211>  20
<212>  DNA
<213>  ARTIFICIAL SEQUENCE

<220>
<223>  forward primer

<400>  128
acgaacgtgg aaatgacaca                                                    20


<210>  129
<211>  20
<212>  DNA
<213>  ARTIFICIAL SEQUENCE

<220>
<223>  forward primer

<400>  129
cagagaaact gatgcgtcca                                                    20


<210>  130
<211>  20
<212>  DNA
<213>  ARTIFICIAL SEQUENCE

<220>
<223>  forward primer

<400>  130
gcagtctttt ccttcgtagc                                                    20


<210>  131
<211>  20
<212>  DNA
<213>  ARTIFICIAL SEQUENCE

<220>
<223>  forward primer

<400>  131
ctttcgaaac ctcacccaaa                                                    20

<210>  132
<211>  20
<212>  DNA
<213>  ARTIFICIAL SEQUENCE

<220>
<223>  forward primer

<400>  132
tccaaattgt ccaaagcctc                                                    20


<210>  133
<211>  20
<212>  DNA
<213>  ARTIFICIAL SEQUENCE

<220>
<223>  forward primer

<400>  133
ttatgaacgc tgcattttcg                                                    20


<210>  134
<211>  20
<212>  DNA
<213>  ARTIFICIAL SEQUENCE

<220>
<223>  forward primer

<400>  134
ggatcgcaaa aagattccaa                                                    20


<210>  135
<211>  20
<212>  DNA
<213>  ARTIFICIAL SEQUENCE

<220>
<223>  forward primer

<400>  135
tccaggaggc attttacacc                                                    20


<210>  136
<211>  20
<212>  DNA
<213>  ARTIFICIAL SEQUENCE

<220>
<223>  forward primer

<400>  136
atacaacaga cattcccgcc                                                    20


<210>  137
<211>  20
<212>  DNA
<213>  ARTIFICIAL SEQUENCE

<220>
<223>  forward primer

<400>  137
agagaacgca gatgacaacg                                                    20


<210>  138
<211>  20
<212>  DNA
<213>  ARTIFICIAL SEQUENCE

<220>
<223>  forward primer

<400>  138
acaggtcatg caggccataa                                                    20


<210>  139
<211>  20
<212>  DNA
<213>  ARTIFICIAL SEQUENCE

<220>
<223>  forward primer

<400>  139
gtacggcagc aaccaaaagt                                                    20


<210>  140
<211>  20
<212>  DNA
<213>  ARTIFICIAL SEQUENCE

<220>
<223>  forward primer

<400>  140
acacattggg tatgctgggt                                                    20


<210>  141
<211>  20
<212>  DNA
<213>  ARTIFICIAL SEQUENCE

<220>
<223>  forward primer

<400>  141
ggcgcttgga ttattcatgt                                                    20


<210>  142
<211>  20
<212>  DNA
<213>  ARTIFICIAL SEQUENCE

<220>
<223>  forward primer

<400>  142
ttttacaagg ccctttcagg                                                    20


<210>  143
<211>  20
<212>  DNA
<213>  ARTIFICIAL SEQUENCE

<220>
<223>  forward primer

<400>  143
cgtaatcgac gatgcagaaa                                                    20


<210>  144
<211>  20
<212>  DNA
<213>  ARTIFICIAL SEQUENCE

<220>
<223>  forward primer

<400>  144
atgcacagaa cttactcttt                                                    20


<210>  145
<211>  20
<212>  DNA
<213>  ARTIFICIAL SEQUENCE

<220>
<223>  forward primer

<400>  145
atgctatcaa aatcagaaaa                                                    20


<210>  146
<211>  20
<212>  DNA
<213>  ARTIFICIAL SEQUENCE

<220>
<223>  forward primer

<400>  146
aacaactaat ttgcgccacc                                                    20


<210>  147
<211>  20
<212>  DNA
<213>  ARTIFICIAL SEQUENCE

<220>
<223>  forward primer

<400>  147
ccaaggacca ttctcggtaa                                                    20


<210>  148
<211>  20
<212>  DNA
<213>  ARTIFICIAL SEQUENCE

<220>
<223>  forward primer

<400>  148
atccttccat ttgggacttg                                                    20


<210>  149

<211>  20
<212>  DNA
<213>  ARTIFICIAL SEQUENCE

<220>
<223>  forward primer

<400>  149
ggtctgtgct ccggtatcat                                                    20


<210>  150
<211>  20
<212>  DNA
<213>  ARTIFICIAL SEQUENCE

<220>
<223>  forward primer

<400>  150
ggtcaggaaa tgccgttatg                                                    20


<210>  151
<211>  20
<212>  DNA
<213>  ARTIFICIAL SEQUENCE

<220>
<223>  forward primer

<400>  151
tcaatgactg gaccacgaac                                                    20


<210>  152
<211>  20
<212>  DNA
<213>  ARTIFICIAL SEQUENCE

<220>
<223>  forward primer

<400>  152
ggccaacgaa ttcggtatta                                                    20


<210>  153
<211>  20
<212>  DNA
<213>  ARTIFICIAL SEQUENCE

<220>
<223>  forward primer

<400>  153
ccctcaaaaa ccaactctcg                                                    20


<210>  154
<211>  20
<212>  DNA
<213>  ARTIFICIAL SEQUENCE

<220>
<223>  forward primer

<400>  154
gcacattatg catcagtcgg                                                    20

<210>  155
<211>  20
<212>  DNA
<213>  ARTIFICIAL SEQUENCE

<220>
<223>  forward primer

<400>  155
ctcacccgag agaaatggtc                                                    20


<210>  156
<211>  20
<212>  DNA
<213>  ARTIFICIAL SEQUENCE

<220>
<223>  forward primer

<400>  156
gtcaagcaac attggagggt                                                    20


<210>  157
<211>  20
<212>  DNA
<213>  ARTIFICIAL SEQUENCE

<220>
<223>  forward primer

<400>  157
ctacttggca agggtcaagg                                                    20


<210>  158
<211>  20
<212>  DNA
<213>  ARTIFICIAL SEQUENCE

<220>
<223>  forward primer

<400>  158
ggtaaaggtc gtggtaggca                                                    20


<210>  159
<211>  20
<212>  DNA
<213>  ARTIFICIAL SEQUENCE

<220>
<223>  forward primer

<400>  159
cgggttccag attatgatgg                                                    20


<210>  160
<211>  20
<212>  DNA
<213>  ARTIFICIAL SEQUENCE

<220>
<223>  forward primer

<400>  160
cctggagtcg tagggacaaa                                                    20


<210>  161
<211>  20
<212>  DNA
<213>  ARTIFICIAL SEQUENCE

<220>
<223>  forward primer

<400>  161
ttcttcaaca tcaagtgccg                                                    20


<210>  162
<211>  20
<212>  DNA
<213>  ARTIFICIAL SEQUENCE

<220>
<223>  forward primer

<400>  162
gattcgcgag aatttggttc                                                    20


<210>  163
<211>  20
<212>  DNA
<213>  ARTIFICIAL SEQUENCE

<220>
<223>  forward primer

<400>  163
atttgaacga atcgtcctgc                                                    20


<210>  164
<211>  20
<212>  DNA
<213>  ARTIFICIAL SEQUENCE

<220>
<223>  forward primer

<400>  164
atgtcaaagg cagtaggtga                                                    20


<210>  165
<211>  20
<212>  DNA
<213>  ARTIFICIAL SEQUENCE

<220>
<223>  forward primer

<400>  165
cattgaacgc taacaatggg                                                    20


<210>  166
<211>  20
<212>  DNA
<213>  ARTIFICIAL SEQUENCE

<220>
<223>  forward primer

<400>  166
tgacatcagc tacggacaaa                                                    20


<210>  167
<211>  20
<212>  DNA
<213>  ARTIFICIAL SEQUENCE

<220>
<223>  forward primer

<400>  167
aagccagctc aagcaagaag                                                    20


<210>  168
<211>  20
<212>  DNA
<213>  ARTIFICIAL SEQUENCE

<220>
<223>  forward primer

<400>  168
gtgccaatga ggtttctcgt                                                    20


<210>  169
<211>  20
<212>  DNA
<213>  ARTIFICIAL SEQUENCE

<220>
<223>  forward primer

<400>  169
gcacgtgtcg aattgtcaac                                                    20


<210>  170
<211>  20
<212>  DNA
<213>  ARTIFICIAL SEQUENCE

<220>
<223>  forward primer

<400>  170
tgaaggccgc attctacttc                                                    20


<210>  171
<211>  20
<212>  DNA
<213>  ARTIFICIAL SEQUENCE

<220>
<223>  forward primer

<400>  171
tttacgcaag gggaattctg                                                    20


<210>  172

<211>  20
<212>  DNA
<213>  ARTIFICIAL SEQUENCE

<220>
<223>  forward primer

<400>  172
cgctagcggt gatgattttt                                                    20


<210>  173
<211>  20
<212>  DNA
<213>  ARTIFICIAL SEQUENCE

<220>
<223>  forward primer

<400>  173
aacaaacagg ccatacctgc                                                    20


<210>  174
<211>  20
<212>  DNA
<213>  ARTIFICIAL SEQUENCE

<220>
<223>  forward primer

<400>  174
acagcgctca caataacaga                                                    20


<210>  175
<211>  20
<212>  DNA
<213>  ARTIFICIAL SEQUENCE

<220>
<223>  forward primer

<400>  175
tttagaaagg acctccagcg                                                    20


<210>  176
<211>  20
<212>  DNA
<213>  ARTIFICIAL SEQUENCE

<220>
<223>  forward primer

<400>  176
ggccgcacaa gtaagaagtc                                                    20


<210>  177
<211>  20
<212>  DNA
<213>  ARTIFICIAL SEQUENCE

<220>
<223>  forward primer

<400>  177
atttttggca ttgaccttgg                                                    20

<210> 178
<211> 20
<212> DNA
<213> ARTIFICIAL SEQUENCE

<220>
<223> forward primer

<400> 178
aaatggcgag aaaactgtcg 20


<210> 179
<211> 20
<212> DNA
<213> ARTIFICIAL SEQUENCE

<220>
<223> forward primer

<400> 179
aacgcgcagg tctttatcat 20


<210> 180
<211> 20
<212> DNA
<213> ARTIFICIAL SEQUENCE

<220>
<223> forward primer

<400> 180
gctttgggtg acaccaactt 20


<210> 181
<211> 20
<212> DNA
<213> ARTIFICIAL SEQUENCE

<220>
<223> forward primer

<400> 181
atgtctgctg atttcggttt 20


<210> 182
<211> 20
<212> DNA
<213> ARTIFICIAL SEQUENCE

<220>
<223> forward primer

<400> 182
cattccttga agttggggaa 20


<210> 183
<211> 20
<212> DNA
<213> ARTIFICIAL SEQUENCE

<220>
<223> forward primer

<400>  183
tacgttattg gatgaccgca                                                    20


<210>  184
<211>  20
<212>  DNA
<213>  ARTIFICIAL SEQUENCE

<220>
<223>  forward primer

<400>  184
ctgctcgaca agtgaaatgg                                                    20


<210>  185
<211>  20
<212>  DNA
<213>  ARTIFICIAL SEQUENCE

<220>
<223>  forward primer

<400>  185
gggtcctaag atgtccacca                                                    20


<210>  186
<211>  20
<212>  DNA
<213>  ARTIFICIAL SEQUENCE

<220>
<223>  forward primer

<400>  186
gcttccgtct cagaattgga                                                    20


<210>  187
<211>  20
<212>  DNA
<213>  ARTIFICIAL SEQUENCE

<220>
<223>  forward primer

<400>  187
aagaaggtgt tctgcacgct                                                    20


<210>  188
<211>  20
<212>  DNA
<213>  ARTIFICIAL SEQUENCE

<220>
<223>  forward primer

<400>  188
tgcaaagcca aagttgacag                                                    20


<210>  189
<211>  20
<212>  DNA
<213>  ARTIFICIAL SEQUENCE

<220>
<223>  forward primer

<400>  189
agcaggtctg cttcctttga                                                    20


<210>  190
<211>  20
<212>  DNA
<213>  ARTIFICIAL SEQUENCE

<220>
<223>  forward primer

<400>  190
acagcttcca ggtcaaaacg                                                    20


<210>  191
<211>  20
<212>  DNA
<213>  ARTIFICIAL SEQUENCE

<220>
<223>  forward primer

<400>  191
caacagctgt cacaaccacc                                                    20


<210>  192
<211>  20
<212>  DNA
<213>  ARTIFICIAL SEQUENCE

<220>
<223>  forward primer

<400>  192
gctttgggtg cttatgcaat                                                    20


<210>  193
<211>  20
<212>  DNA
<213>  ARTIFICIAL SEQUENCE

<220>
<223>  forward primer

<400>  193
atggtgaaga taagcttgga                                                    20


<210>  194
<211>  20
<212>  DNA
<213>  ARTIFICIAL SEQUENCE

<220>
<223>  forward primer

<400>  194
atttcgaaga agacggggat                                                    20


<210>  195

<211>  20
<212>  DNA
<213>  ARTIFICIAL SEQUENCE

<220>
<223>  forward primer

<400>  195
cgaatgggct ttaagatgga                                                    20


<210>  196
<211>  20
<212>  DNA
<213>  ARTIFICIAL SEQUENCE

<220>
<223>  forward primer

<400>  196
aggcaaagct gagcaaatgt                                                    20


<210>  197
<211>  20
<212>  DNA
<213>  ARTIFICIAL SEQUENCE

<220>
<223>  forward primer

<400>  197
ctcgacatcc actgggaatc                                                    20


<210>  198
<211>  20
<212>  DNA
<213>  ARTIFICIAL SEQUENCE

<220>
<223>  forward primer

<400>  198
aacgtgacca agagcagctt                                                    20


<210>  199
<211>  20
<212>  DNA
<213>  ARTIFICIAL SEQUENCE

<220>
<223>  forward primer

<400>  199
tccctttctt ctgtcgcatt                                                    20


<210>  200
<211>  20
<212>  DNA
<213>  ARTIFICIAL SEQUENCE

<220>
<223>  forward primer

<400>  200
gaccgttgtc aagtggttca                                                    20

<210> 201
<211> 20
<212> DNA
<213> ARTIFICIAL SEQUENCE

<220>
<223> forward primer

<400> 201
attcgacggc aaggtaacag 20


<210> 202
<211> 20
<212> DNA
<213> ARTIFICIAL SEQUENCE

<220>
<223> forward primer

<400> 202
ctgctatgga acgtttgggt 20


<210> 203
<211> 20
<212> DNA
<213> ARTIFICIAL SEQUENCE

<220>
<223> forward primer

<400> 203
aattcaaaaa cgtcgcccta 20


<210> 204
<211> 20
<212> DNA
<213> ARTIFICIAL SEQUENCE

<220>
<223> forward primer

<400> 204
gtttttcaga ctgcctggga 20


<210> 205
<211> 20
<212> DNA
<213> ARTIFICIAL SEQUENCE

<220>
<223> forward primer

<400> 205
gatggattca agcccaacag 20


<210> 206
<211> 20
<212> DNA
<213> ARTIFICIAL SEQUENCE

<220>
<223> forward primer

<400>  206
tgtagcgttg gctgcaaata                                                    20


<210>  207
<211>  20
<212>  DNA
<213>  ARTIFICIAL SEQUENCE

<220>
<223>  forward primer

<400>  207
gtccacggcc tattctttga                                                    20


<210>  208
<211>  20
<212>  DNA
<213>  ARTIFICIAL SEQUENCE

<220>
<223>  forward primer

<400>  208
tatggacgag aacggaaacc                                                    20


<210>  209
<211>  20
<212>  DNA
<213>  ARTIFICIAL SEQUENCE

<220>
<223>  forward primer

<400>  209
tcaggaatat agcgggttgc                                                    20


<210>  210
<211>  20
<212>  DNA
<213>  ARTIFICIAL SEQUENCE

<220>
<223>  forward primer

<400>  210
cggtgtgtca gcatttgttc                                                    20


<210>  211
<211>  20
<212>  DNA
<213>  ARTIFICIAL SEQUENCE

<220>
<223>  forward primer

<400>  211
catcagccag atcctacggt                                                    20


<210>  212
<211>  20
<212>  DNA
<213>  ARTIFICIAL SEQUENCE

<220>
<223>  forward primer

<400>  212
ccgtcgtcag ctagaaatcc                                                    20


<210>  213
<211>  20
<212>  DNA
<213>  ARTIFICIAL SEQUENCE

<220>
<223>  forward primer

<400>  213
ttgctgtcaa ggcatttttg                                                    20


<210>  214
<211>  20
<212>  DNA
<213>  ARTIFICIAL SEQUENCE

<220>
<223>  forward primer

<400>  214
gtgttattgc ctcaggtggg                                                    20


<210>  215
<211>  20
<212>  DNA
<213>  ARTIFICIAL SEQUENCE

<220>
<223>  forward primer

<400>  215
gcttaacgca aaaggcattc                                                    20


<210>  216
<211>  20
<212>  DNA
<213>  ARTIFICIAL SEQUENCE

<220>
<223>  forward primer

<400>  216
gtcgacaata aagccaagcc                                                    20


<210>  217
<211>  20
<212>  DNA
<213>  ARTIFICIAL SEQUENCE

<220>
<223>  forward primer

<400>  217
ccccgaacaa tccaactgta                                                    20


<210>  218

<211>  20
<212>  DNA
<213>  ARTIFICIAL SEQUENCE

<220>
<223>  forward primer

<400>  218
tacttgcgaa tttcttcccg                                                    20


<210>  219
<211>  20
<212>  DNA
<213>  ARTIFICIAL SEQUENCE

<220>
<223>  forward primer

<400>  219
ccacatgatc gagactggtg                                                    20


<210>  220
<211>  20
<212>  DNA
<213>  ARTIFICIAL SEQUENCE

<220>
<223>  forward primer

<400>  220
ccacaccaac atgcaacttc                                                    20


<210>  221
<211>  20
<212>  DNA
<213>  ARTIFICIAL SEQUENCE

<220>
<223>  forward primer

<400>  221
tccgatgtca tgagcaacat                                                    20


<210>  222
<211>  20
<212>  DNA
<213>  ARTIFICIAL SEQUENCE

<220>
<223>  forward primer

<400>  222
aacctatcac tgcaaaggcg                                                    20


<210>  223
<211>  20
<212>  DNA
<213>  ARTIFICIAL SEQUENCE

<220>
<223>  forward primer

<400>  223
atcctcaaat atgctccccc                                                    20

<210>  224
<211>  20
<212>  DNA
<213>  ARTIFICIAL SEQUENCE

<220>
<223>  forward primer

<400>  224
gtaggcgcat caatgacaag                                                    20


<210>  225
<211>  20
<212>  DNA
<213>  ARTIFICIAL SEQUENCE

<220>
<223>  forward primer

<400>  225
tgctcacagg tcttcctcct                                                    20


<210>  226
<211>  20
<212>  DNA
<213>  ARTIFICIAL SEQUENCE

<220>
<223>  forward primer

<400>  226
ccataggcgt aaccctgaaa                                                    20


<210>  227
<211>  20
<212>  DNA
<213>  ARTIFICIAL SEQUENCE

<220>
<223>  forward primer

<400>  227
tcgaaggaaa aagtcgcagt                                                    20


<210>  228
<211>  20
<212>  DNA
<213>  ARTIFICIAL SEQUENCE

<220>
<223>  forward primer

<400>  228
gcggtatgac acaccacttg                                                    20


<210>  229
<211>  20
<212>  DNA
<213>  ARTIFICIAL SEQUENCE

<220>
<223>  forward primer

<400>  229
ggtaactcct taccggcaca                                                    20


<210>  230
<211>  20
<212>  DNA
<213>  ARTIFICIAL SEQUENCE

<220>
<223>  forward primer

<400>  230
aatccatacg gcgtctcaac                                                    20


<210>  231
<211>  20
<212>  DNA
<213>  ARTIFICIAL SEQUENCE

<220>
<223>  forward primer

<400>  231
acactggttt ccgcgattac                                                    20


<210>  232
<211>  20
<212>  DNA
<213>  ARTIFICIAL SEQUENCE

<220>
<223>  forward primer

<400>  232
ggtattccag aagcaaccca                                                    20


<210>  233
<211>  20
<212>  DNA
<213>  ARTIFICIAL SEQUENCE

<220>
<223>  forward primer

<400>  233
gatgcaatcg aagggactgt                                                    20


<210>  234
<211>  20
<212>  DNA
<213>  ARTIFICIAL SEQUENCE

<220>
<223>  forward primer

<400>  234
aaaaggcaag gaactgggtt                                                    20


<210>  235
<211>  20
<212>  DNA
<213>  ARTIFICIAL SEQUENCE

<220>
<223>  forward primer

<400>  235
gttttggtgc gaaggaagag                                                    20


<210>  236
<211>  20
<212>  DNA
<213>  ARTIFICIAL SEQUENCE

<220>
<223>  forward primer

<400>  236
agagagacat tggccagcat                                                    20


<210>  237
<211>  20
<212>  DNA
<213>  ARTIFICIAL SEQUENCE

<220>
<223>  forward primer

<400>  237
cgtcgagact aacgacgatg                                                    20


<210>  238
<211>  20
<212>  DNA
<213>  ARTIFICIAL SEQUENCE

<220>
<223>  forward primer

<400>  238
ggtagacgtt ttcgcctttg                                                    20


<210>  239
<211>  20
<212>  DNA
<213>  ARTIFICIAL SEQUENCE

<220>
<223>  forward primer

<400>  239
tcatcaacca atgaagaccg                                                    20


<210>  240
<211>  20
<212>  DNA
<213>  ARTIFICIAL SEQUENCE

<220>
<223>  forward primer

<400>  240
attgaaggcc aaatacggtg                                                    20


<210>  241

<211>  20
<212>  DNA
<213>  ARTIFICIAL SEQUENCE

<220>
<223>  forward primer

<400>  241
tccatgcttt ctgttccaca                                                    20


<210>  242
<211>  20
<212>  DNA
<213>  ARTIFICIAL SEQUENCE

<220>
<223>  forward primer

<400>  242
cttccgaatc aaacggctac                                                    20


<210>  243
<211>  20
<212>  DNA
<213>  ARTIFICIAL SEQUENCE

<220>
<223>  forward primer

<400>  243
catcgaccca aatcgaagtt                                                    20


<210>  244
<211>  20
<212>  DNA
<213>  ARTIFICIAL SEQUENCE

<220>
<223>  forward primer

<400>  244
tttgaccaaa tcacacggaa                                                    20


<210>  245
<211>  20
<212>  DNA
<213>  ARTIFICIAL SEQUENCE

<220>
<223>  forward primer

<400>  245
gcgaaaggta cgttctggag                                                    20


<210>  246
<211>  20
<212>  DNA
<213>  ARTIFICIAL SEQUENCE

<220>
<223>  forward primer

<400>  246
ggctgcttac aagcgttttc                                                    20

<210>  247
<211>  20
<212>  DNA
<213>  ARTIFICIAL SEQUENCE

<220>
<223>  forward primer

<400>  247
gcagaaggct actccaaacg                                                    20


<210>  248
<211>  20
<212>  DNA
<213>  ARTIFICIAL SEQUENCE

<220>
<223>  forward primer

<400>  248
tgccaagtgg aacatgaaaa                                                    20


<210>  249
<211>  20
<212>  DNA
<213>  ARTIFICIAL SEQUENCE

<220>
<223>  forward primer

<400>  249
aaagcactta ccgagcaagc                                                    20


<210>  250
<211>  20
<212>  DNA
<213>  ARTIFICIAL SEQUENCE

<220>
<223>  forward primer

<400>  250
cacaaccata ccattgctgc                                                    20


<210>  251
<211>  20
<212>  DNA
<213>  ARTIFICIAL SEQUENCE

<220>
<223>  forward primer

<400>  251
atgtttttcg ttcgccaagt                                                    20


<210>  252
<211>  20
<212>  DNA
<213>  ARTIFICIAL SEQUENCE

<220>
<223>  forward primer

<400>  252
cgattagcca gccaaggata                                                    20


<210>  253
<211>  20
<212>  DNA
<213>  ARTIFICIAL SEQUENCE

<220>
<223>  forward primer

<400>  253
aaggcgggtt catatgtgtc                                                    20


<210>  254
<211>  20
<212>  DNA
<213>  ARTIFICIAL SEQUENCE

<220>
<223>  forward primer

<400>  254
catcatcatt gggagacacg                                                    20


<210>  255
<211>  20
<212>  DNA
<213>  ARTIFICIAL SEQUENCE

<220>
<223>  forward primer

<400>  255
cataaccgaa gcaggcattt                                                    20


<210>  256
<211>  20
<212>  DNA
<213>  ARTIFICIAL SEQUENCE

<220>
<223>  forward primer

<400>  256
acgatcgatt gagacccttg                                                    20


<210>  257
<211>  20
<212>  DNA
<213>  ARTIFICIAL SEQUENCE

<220>
<223>  forward primer

<400>  257
cactgttgaa gcattgaagg                                                    20


<210>  258
<211>  20
<212>  DNA
<213>  ARTIFICIAL SEQUENCE

<220>
<223>  forward primer

<400>  258
tatggagtcg aaccctaccg                                                    20


<210>  259
<211>  20
<212>  DNA
<213>  ARTIFICIAL SEQUENCE

<220>
<223>  forward primer

<400>  259
ctctgcggag gaatacttgg                                                    20


<210>  260
<211>  20
<212>  DNA
<213>  ARTIFICIAL SEQUENCE

<220>
<223>  forward primer

<400>  260
ctccaccagt tccaccagtt                                                    20


<210>  261
<211>  20
<212>  DNA
<213>  ARTIFICIAL SEQUENCE

<220>
<223>  forward primer

<400>  261
tctactcctt gttcgggtcg                                                    20


<210>  262
<211>  20
<212>  DNA
<213>  ARTIFICIAL SEQUENCE

<220>
<223>  forward primer

<400>  262
gattacaagg gtgaagccga                                                    20


<210>  263
<211>  20
<212>  DNA
<213>  ARTIFICIAL SEQUENCE

<220>
<223>  forward primer

<400>  263
ttcttcccaa gtccatgagg                                                    20


<210>  264

<211>  20
<212>  DNA
<213>  ARTIFICIAL SEQUENCE

<220>
<223>  forward primer

<400>  264
atggtatggg gaaatcacga                                                    20


<210>  265
<211>  20
<212>  DNA
<213>  ARTIFICIAL SEQUENCE

<220>
<223>  forward primer

<400>  265
tactggtgtt gatgggcaag                                                    20


<210>  266
<211>  20
<212>  DNA
<213>  ARTIFICIAL SEQUENCE

<220>
<223>  forward primer

<400>  266
cgaagcagaa ggtaatggga                                                    20


<210>  267
<211>  20
<212>  DNA
<213>  ARTIFICIAL SEQUENCE

<220>
<223>  forward primer

<400>  267
ttccttccat tgagaaccca                                                    20


<210>  268
<211>  20
<212>  DNA
<213>  ARTIFICIAL SEQUENCE

<220>
<223>  forward primer

<400>  268
aagctgcgga ggagactgta                                                    20


<210>  269
<211>  20
<212>  DNA
<213>  ARTIFICIAL SEQUENCE

<220>
<223>  forward primer

<400>  269
ggatttccac aaggaccagt                                                    20

<210>  270
<211>  20
<212>  DNA
<213>  ARTIFICIAL SEQUENCE

<220>
<223>  forward primer

<400>  270
aaaggtaagg cagtcgagca                                                    20


<210>  271
<211>  20
<212>  DNA
<213>  ARTIFICIAL SEQUENCE

<220>
<223>  forward primer

<400>  271
tggcaatctc agcaatcaag                                                    20


<210>  272
<211>  20
<212>  DNA
<213>  ARTIFICIAL SEQUENCE

<220>
<223>  forward primer

<400>  272
ctagcacgcc gaaagagaag                                                    20


<210>  273
<211>  20
<212>  DNA
<213>  ARTIFICIAL SEQUENCE

<220>
<223>  forward primer

<400>  273
ttgaggctat tgttcggtga                                                    20


<210>  274
<211>  20
<212>  DNA
<213>  ARTIFICIAL SEQUENCE

<220>
<223>  forward primer

<400>  274
atgagctgga aaacgacacc                                                    20


<210>  275
<211>  20
<212>  DNA
<213>  ARTIFICIAL SEQUENCE

<220>
<223>  forward primer

<400>  275
cctaccaacg cattgaggat                                                   20


<210>  276
<211>  20
<212>  DNA
<213>  ARTIFICIAL SEQUENCE

<220>
<223>  forward primer

<400>  276
acagtcccgc ttattctcca                                                   20


<210>  277
<211>  20
<212>  DNA
<213>  ARTIFICIAL SEQUENCE

<220>
<223>  forward primer

<400>  277
caggatcaac tacagcaccg                                                   20


<210>  278
<211>  20
<212>  DNA
<213>  ARTIFICIAL SEQUENCE

<220>
<223>  forward primer

<400>  278
tagtgttatc ggtgatgcca                                                   20


<210>  279
<211>  20
<212>  DNA
<213>  ARTIFICIAL SEQUENCE

<220>
<223>  forward primer

<400>  279
agtgaaaacc ccactgacca                                                   20


<210>  280
<211>  20
<212>  DNA
<213>  ARTIFICIAL SEQUENCE

<220>
<223>  forward primer

<400>  280
tattcaaatc aggctggcaa                                                   20


<210>  281
<211>  20
<212>  DNA
<213>  ARTIFICIAL SEQUENCE

<220>
<223>  forward primer

<400>  281
aagagaaggc attggcagaa                                                    20


<210>  282
<211>  20
<212>  DNA
<213>  ARTIFICIAL SEQUENCE

<220>
<223>  forward primer

<400>  282
gatggggagc ttcatatcca                                                    20


<210>  283
<211>  20
<212>  DNA
<213>  ARTIFICIAL SEQUENCE

<220>
<223>  forward primer

<400>  283
ttggggcagg gctatactaa                                                    20


<210>  284
<211>  20
<212>  DNA
<213>  ARTIFICIAL SEQUENCE

<220>
<223>  forward primer

<400>  284
cggccgtact ttcttcactc                                                    20


<210>  285
<211>  20
<212>  DNA
<213>  ARTIFICIAL SEQUENCE

<220>
<223>  forward primer

<400>  285
aattgattta acggttgcca                                                    20


<210>  286
<211>  20
<212>  DNA
<213>  ARTIFICIAL SEQUENCE

<220>
<223>  forward primer

<400>  286
agttcccttt gtgcactcgt                                                    20


<210>  287

<211>  20
<212>  DNA
<213>  ARTIFICIAL SEQUENCE

<220>
<223>  forward primer

<400>  287
aacaatccgt acgctgaaaa                                                    20


<210>  288
<211>  20
<212>  DNA
<213>  ARTIFICIAL SEQUENCE

<220>
<223>  forward primer

<400>  288
gcatagtcaa ttctccccca                                                    20


<210>  289
<211>  20
<212>  DNA
<213>  ARTIFICIAL SEQUENCE

<220>
<223>  forward primer

<400>  289
agcagaagtt cgcggtaatg                                                    20


<210>  290
<211>  20
<212>  DNA
<213>  ARTIFICIAL SEQUENCE

<220>
<223>  forward primer

<400>  290
cgagagggag gtaacaagcc                                                    20


<210>  291
<211>  20
<212>  DNA
<213>  ARTIFICIAL SEQUENCE

<220>
<223>  forward primer

<400>  291
ttattgggga agccattacg                                                    20


<210>  292
<211>  20
<212>  DNA
<213>  ARTIFICIAL SEQUENCE

<220>
<223>  forward primer

<400>  292
gaatagggga gaccgtaggc                                                    20

<210>  293
<211>  20
<212>  DNA
<213>  ARTIFICIAL SEQUENCE

<220>
<223>  forward primer

<400>  293
tcgatcaaga tttacgggct                                                    20


<210>  294
<211>  20
<212>  DNA
<213>  ARTIFICIAL SEQUENCE

<220>
<223>  forward primer

<400>  294
tgcccatagc cgagataaac                                                    20


<210>  295
<211>  20
<212>  DNA
<213>  ARTIFICIAL SEQUENCE

<220>
<223>  forward primer

<400>  295
gtggtgccta tcccagagaa                                                    20


<210>  296
<211>  20
<212>  DNA
<213>  ARTIFICIAL SEQUENCE

<220>
<223>  forward primer

<400>  296
ggccttcttc atggatcgta                                                    20


<210>  297
<211>  20
<212>  DNA
<213>  ARTIFICIAL SEQUENCE

<220>
<223>  forward primer

<400>  297
gacagcatgc aaggtcaaga                                                    20


<210>  298
<211>  20
<212>  DNA
<213>  ARTIFICIAL SEQUENCE

<220>
<223>  forward primer

<400>  298
gcattggatc ctccacttgt                                                    20


<210>  299
<211>  20
<212>  DNA
<213>  ARTIFICIAL SEQUENCE

<220>
<223>  forward primer

<400>  299
cgaaaagata gccatcgagc                                                    20


<210>  300
<211>  20
<212>  DNA
<213>  ARTIFICIAL SEQUENCE

<220>
<223>  forward primer

<400>  300
aactcagagc agggcaagaa                                                    20


<210>  301
<211>  20
<212>  DNA
<213>  ARTIFICIAL SEQUENCE

<220>
<223>  forward primer

<400>  301
aacacgcacg aacttccatt                                                    20


<210>  302
<211>  20
<212>  DNA
<213>  ARTIFICIAL SEQUENCE

<220>
<223>  forward primer

<400>  302
cggcttcatt gatcggttat                                                    20


<210>  303
<211>  20
<212>  DNA
<213>  ARTIFICIAL SEQUENCE

<220>
<223>  forward primer

<400>  303
taaggctcaa aactgggtcg                                                    20


<210>  304
<211>  20
<212>  DNA
<213>  ARTIFICIAL SEQUENCE

<220>
<223>  forward primer

<400>  304
aacatccggc gaatcattac                                                    20


<210>  305
<211>  20
<212>  DNA
<213>  ARTIFICIAL SEQUENCE

<220>
<223>  forward primer

<400>  305
aacggcgaag gattcctatt                                                    20


<210>  306
<211>  20
<212>  DNA
<213>  ARTIFICIAL SEQUENCE

<220>
<223>  forward primer

<400>  306
aattttctag cgccgttcaa                                                    20


<210>  307
<211>  20
<212>  DNA
<213>  ARTIFICIAL SEQUENCE

<220>
<223>  forward primer

<400>  307
tcgaggcaca ataaggaacc                                                    20


<210>  308
<211>  20
<212>  DNA
<213>  ARTIFICIAL SEQUENCE

<220>
<223>  forward primer

<400>  308
tatatgctgc aaaccagcca                                                    20


<210>  309
<211>  20
<212>  DNA
<213>  ARTIFICIAL SEQUENCE

<220>
<223>  forward primer

<400>  309
aaagcgctga aactggactc                                                    20


<210>  310

<211>  20
<212>  DNA
<213>  ARTIFICIAL SEQUENCE

<220>
<223>  forward primer

<400>  310
gctatgagaa tgcggacgtt                                                    20


<210>  311
<211>  20
<212>  DNA
<213>  ARTIFICIAL SEQUENCE

<220>
<223>  forward primer

<400>  311
cgaacatgct agttttgcca                                                    20


<210>  312
<211>  20
<212>  DNA
<213>  ARTIFICIAL SEQUENCE

<220>
<223>  forward primer

<400>  312
taacttgcgt catttttgcg                                                    20


<210>  313
<211>  20
<212>  DNA
<213>  ARTIFICIAL SEQUENCE

<220>
<223>  forward primer

<400>  313
acgcaaaaac attcaccaca                                                    20


<210>  314
<211>  20
<212>  DNA
<213>  ARTIFICIAL SEQUENCE

<220>
<223>  forward primer

<400>  314
ttttcattgg ccttttggtc                                                    20


<210>  315
<211>  20
<212>  DNA
<213>  ARTIFICIAL SEQUENCE

<220>
<223>  forward primer

<400>  315
tggcacaggt gaactgagag                                                    20

<210>  316
<211>  20
<212>  DNA
<213>  ARTIFICIAL SEQUENCE

<220>
<223>  forward primer

<400>  316
ccagtatccc cacattacgg                                                    20


<210>  317
<211>  20
<212>  DNA
<213>  ARTIFICIAL SEQUENCE

<220>
<223>  forward primer

<400>  317
tggtagcttc aaggattcgg                                                    20


<210>  318
<211>  20
<212>  DNA
<213>  ARTIFICIAL SEQUENCE

<220>
<223>  forward primer

<400>  318
ttgcatattt gcatgaatcc                                                    20


<210>  319
<211>  20
<212>  DNA
<213>  ARTIFICIAL SEQUENCE

<220>
<223>  forward primer

<400>  319
tttgcaggag tagctgaggc                                                    20


<210>  320
<211>  20
<212>  DNA
<213>  ARTIFICIAL SEQUENCE

<220>
<223>  forward primer

<400>  320
aagactgggt tgcaaaatgg                                                    20


<210>  321
<211>  20
<212>  DNA
<213>  ARTIFICIAL SEQUENCE

<220>
<223>  forward primer

<400>  321
atcagacaat gagggccaac                                                    20


<210>  322
<211>  20
<212>  DNA
<213>  ARTIFICIAL SEQUENCE

<220>
<223>  forward primer

<400>  322
gcccatcgct ttgataatgt                                                    20


<210>  323
<211>  20
<212>  DNA
<213>  ARTIFICIAL SEQUENCE

<220>
<223>  forward primer

<400>  323
ccctgttgag aatccttcca                                                    20


<210>  324
<211>  20
<212>  DNA
<213>  ARTIFICIAL SEQUENCE

<220>
<223>  forward primer

<400>  324
attactttgc ctccggaatg                                                    20


<210>  325
<211>  20
<212>  DNA
<213>  ARTIFICIAL SEQUENCE

<220>
<223>  forward primer

<400>  325
acatacccgg ttagtgcagc                                                    20


<210>  326
<211>  20
<212>  DNA
<213>  ARTIFICIAL SEQUENCE

<220>
<223>  forward primer

<400>  326
tcagaagatc ggactctgcc                                                    20


<210>  327
<211>  20
<212>  DNA
<213>  ARTIFICIAL SEQUENCE

<220>
<223>  forward primer

<400>  327
tttggtggtt agtcaagggc                                                    20


<210>  328
<211>  20
<212>  DNA
<213>  ARTIFICIAL SEQUENCE

<220>
<223>  forward primer

<400>  328
aggggagaaa gtttgctcat                                                    20


<210>  329
<211>  20
<212>  DNA
<213>  ARTIFICIAL SEQUENCE

<220>
<223>  forward primer

<400>  329
aaaccgaaaa agtctcccgt                                                    20


<210>  330
<211>  20
<212>  DNA
<213>  ARTIFICIAL SEQUENCE

<220>
<223>  forward primer

<400>  330
tcgactagca ccatctgcac                                                    20


<210>  331
<211>  20
<212>  DNA
<213>  ARTIFICIAL SEQUENCE

<220>
<223>  forward primer

<400>  331
atccttgaac gacgaaaacg                                                    20


<210>  332
<211>  20
<212>  DNA
<213>  ARTIFICIAL SEQUENCE

<220>
<223>  forward primer

<400>  332
gtaaacgctc ccgaatacga                                                    20


<210>  333

<211>  20
<212>  DNA
<213>  ARTIFICIAL SEQUENCE

<220>
<223>  forward primer

<400>  333
actgcttgcc gatggttatc                                                    20


<210>  334
<211>  20
<212>  DNA
<213>  ARTIFICIAL SEQUENCE

<220>
<223>  forward primer

<400>  334
ggcttatccc gaggaagaac                                                    20


<210>  335
<211>  20
<212>  DNA
<213>  ARTIFICIAL SEQUENCE

<220>
<223>  forward primer

<400>  335
tctaccgcaa attcagctcc                                                    20


<210>  336
<211>  20
<212>  DNA
<213>  ARTIFICIAL SEQUENCE

<220>
<223>  forward primer

<400>  336
ttgcagcagt atcacttggg                                                    20


<210>  337
<211>  20
<212>  DNA
<213>  ARTIFICIAL SEQUENCE

<220>
<223>  forward primer

<400>  337
ctttctgatg ctgagagggc                                                    20


<210>  338
<211>  20
<212>  DNA
<213>  ARTIFICIAL SEQUENCE

<220>
<223>  forward primer

<400>  338
agaaacgcac aagctgctct                                                    20

<210>  339
<211>  20
<212>  DNA
<213>  ARTIFICIAL SEQUENCE

<220>
<223>  forward primer

<400>  339
tatcagctcc caaggagcac                                                    20


<210>  340
<211>  20
<212>  DNA
<213>  ARTIFICIAL SEQUENCE

<220>
<223>  forward primer

<400>  340
tacatgagga ctggggaagg                                                    20


<210>  341
<211>  20
<212>  DNA
<213>  ARTIFICIAL SEQUENCE

<220>
<223>  forward primer

<400>  341
agagctttag tgggccaggt                                                    20


<210>  342
<211>  20
<212>  DNA
<213>  ARTIFICIAL SEQUENCE

<220>
<223>  forward primer

<400>  342
aacaagagca tcccgaagaa                                                    20


<210>  343
<211>  20
<212>  DNA
<213>  ARTIFICIAL SEQUENCE

<220>
<223>  forward primer

<400>  343
tcaacgtatt cccagaaggg                                                    20


<210>  344
<211>  20
<212>  DNA
<213>  ARTIFICIAL SEQUENCE

<220>
<223>  forward primer

<400>  344
tgaaaaatcc ggtgataggg                                                    20


<210>  345
<211>  20
<212>  DNA
<213>  ARTIFICIAL SEQUENCE

<220>
<223>  forward primer

<400>  345
tcgcggcact atctggtaat                                                    20


<210>  346
<211>  20
<212>  DNA
<213>  ARTIFICIAL SEQUENCE

<220>
<223>  forward primer

<400>  346
actgggggtt gtctgttttg                                                    20


<210>  347
<211>  20
<212>  DNA
<213>  ARTIFICIAL SEQUENCE

<220>
<223>  forward primer

<400>  347
ggacaccgtt attggaatcg                                                    20


<210>  348
<211>  20
<212>  DNA
<213>  ARTIFICIAL SEQUENCE

<220>
<223>  forward primer

<400>  348
agactcaaac gcaaagccat                                                    20


<210>  349
<211>  20
<212>  DNA
<213>  ARTIFICIAL SEQUENCE

<220>
<223>  forward primer

<400>  349
cacttaatgg ccaccgactc                                                    20


<210>  350
<211>  20
<212>  DNA
<213>  ARTIFICIAL SEQUENCE

<220>
<223>  forward primer

<400>  350
gctaagcgaa ccgaaaaatg                                                    20


<210>  351
<211>  20
<212>  DNA
<213>  ARTIFICIAL SEQUENCE

<220>
<223>  forward primer

<400>  351
ggtgttggga gaaccaaaga                                                    20


<210>  352
<211>  20
<212>  DNA
<213>  ARTIFICIAL SEQUENCE

<220>
<223>  forward primer

<400>  352
tcccaaactc gacttcatcc                                                    20


<210>  353
<211>  20
<212>  DNA
<213>  ARTIFICIAL SEQUENCE

<220>
<223>  forward primer

<400>  353
acagaagccc acaagagcat                                                    20


<210>  354
<211>  20
<212>  DNA
<213>  ARTIFICIAL SEQUENCE

<220>
<223>  forward primer

<400>  354
cgattggagc caacttcatt                                                    20


<210>  355
<211>  20
<212>  DNA
<213>  ARTIFICIAL SEQUENCE

<220>
<223>  forward primer

<400>  355
caagtctggt tggaaatggc                                                    20


<210>  356

<211>  20
<212>  DNA
<213>  ARTIFICIAL SEQUENCE

<220>
<223>  forward primer

<400>  356
ggcgataact ctcccagtga                                                    20


<210>  357
<211>  20
<212>  DNA
<213>  ARTIFICIAL SEQUENCE

<220>
<223>  forward primer

<400>  357
gaacagcctc tgagggctaa                                                    20


<210>  358
<211>  20
<212>  DNA
<213>  ARTIFICIAL SEQUENCE

<220>
<223>  forward primer

<400>  358
acatgcaacg tctccacatc                                                    20


<210>  359
<211>  20
<212>  DNA
<213>  ARTIFICIAL SEQUENCE

<220>
<223>  forward primer

<400>  359
gtcggggtaa gattatgcga                                                    20


<210>  360
<211>  20
<212>  DNA
<213>  ARTIFICIAL SEQUENCE

<220>
<223>  forward primer

<400>  360
ccatcaatgc tgtggtcatc                                                    20


<210>  361
<211>  20
<212>  DNA
<213>  ARTIFICIAL SEQUENCE

<220>
<223>  forward primer

<400>  361
gattggctgt ggtcaggaat                                                    20

<210>  362
<211>  20
<212>  DNA
<213>  ARTIFICIAL SEQUENCE

<220>
<223>  forward primer

<400>  362
ttaatattat ggcggcgacc                                                    20


<210>  363
<211>  20
<212>  DNA
<213>  ARTIFICIAL SEQUENCE

<220>
<223>  forward primer

<400>  363
cgagaggctt ttgttggaag                                                    20


<210>  364
<211>  20
<212>  DNA
<213>  ARTIFICIAL SEQUENCE

<220>
<223>  forward primer

<400>  364
gtttattgcc gaaggttcca                                                    20


<210>  365
<211>  20
<212>  DNA
<213>  ARTIFICIAL SEQUENCE

<220>
<223>  forward primer

<400>  365
cggcaagtta ccactggttt                                                    20


<210>  366
<211>  20
<212>  DNA
<213>  ARTIFICIAL SEQUENCE

<220>
<223>  forward primer

<400>  366
agttatgact gccgtctcgg                                                    20


<210>  367
<211>  20
<212>  DNA
<213>  ARTIFICIAL SEQUENCE

<220>
<223>  forward primer

<400>  367
attggcaccg tgacgatatt                                                    20


<210>  368
<211>  20
<212>  DNA
<213>  ARTIFICIAL SEQUENCE

<220>
<223>  forward primer

<400>  368
tcttccactg aagcaaagca                                                    20


<210>  369
<211>  20
<212>  DNA
<213>  ARTIFICIAL SEQUENCE

<220>
<223>  forward primer

<400>  369
agctaagttg cacgctgtca                                                    20


<210>  370
<211>  20
<212>  DNA
<213>  ARTIFICIAL SEQUENCE

<220>
<223>  forward primer

<400>  370
aaggttgtta cgccagatgc                                                    20


<210>  371
<211>  20
<212>  DNA
<213>  ARTIFICIAL SEQUENCE

<220>
<223>  forward primer

<400>  371
tttcgatttg ctaacgttgt                                                    20


<210>  372
<211>  20
<212>  DNA
<213>  ARTIFICIAL SEQUENCE

<220>
<223>  forward primer

<400>  372
ccttagagcc aatcggtgtc                                                    20


<210>  373
<211>  20
<212>  DNA
<213>  ARTIFICIAL SEQUENCE

<220>
<223>  forward primer

<400>  373
tcggtgaatc tgagaagctg                                                    20


<210>  374
<211>  20
<212>  DNA
<213>  ARTIFICIAL SEQUENCE

<220>
<223>  forward primer

<400>  374
ataggaatgg ggttctcgct                                                    20


<210>  375
<211>  20
<212>  DNA
<213>  ARTIFICIAL SEQUENCE

<220>
<223>  forward primer

<400>  375
taaagttgag atgcgacccc                                                    20


<210>  376
<211>  20
<212>  DNA
<213>  ARTIFICIAL SEQUENCE

<220>
<223>  forward primer

<400>  376
ctgaacgtga acgtgcctta                                                    20


<210>  377
<211>  20
<212>  DNA
<213>  ARTIFICIAL SEQUENCE

<220>
<223>  forward primer

<400>  377
tatccattgg acgtggtgaa                                                    20


<210>  378
<211>  20
<212>  DNA
<213>  ARTIFICIAL SEQUENCE

<220>
<223>  forward primer

<400>  378
catttaatgg ccaccgattc                                                    20


<210>  379

<211>  20
<212>  DNA
<213>  ARTIFICIAL SEQUENCE

<220>
<223>  forward primer

<400>  379
actcttctgg aggttgctgg                                                    20


<210>  380
<211>  20
<212>  DNA
<213>  ARTIFICIAL SEQUENCE

<220>
<223>  forward primer

<400>  380
aacactcagc ttgcacatcg                                                    20


<210>  381
<211>  20
<212>  DNA
<213>  ARTIFICIAL SEQUENCE

<220>
<223>  forward primer

<400>  381
tccattccat tcttgggaaa                                                    20


<210>  382
<211>  20
<212>  DNA
<213>  ARTIFICIAL SEQUENCE

<220>
<223>  forward primer

<400>  382
tgccgatgat gagtatccaa                                                    20


<210>  383
<211>  20
<212>  DNA
<213>  ARTIFICIAL SEQUENCE

<220>
<223>  forward primer

<400>  383
ggcgggtcca ataacaaaat                                                    20


<210>  384
<211>  20
<212>  DNA
<213>  ARTIFICIAL SEQUENCE

<220>
<223>  forward primer

<400>  384
aatgctgcgt tttctttgct                                                    20

<210>  385
<211>  20
<212>  DNA
<213>  ARTIFICIAL SEQUENCE

<220>
<223>  forward primer

<400>  385
gtgaaacgca ttcccattct                                                    20


<210>  386
<211>  20
<212>  DNA
<213>  ARTIFICIAL SEQUENCE

<220>
<223>  forward primer

<400>  386
aaattcgacg atcacgaagc                                                    20


<210>  387
<211>  20
<212>  DNA
<213>  ARTIFICIAL SEQUENCE

<220>
<223>  forward primer

<400>  387
cagttggaaa ccccttttga                                                    20


<210>  388
<211>  20
<212>  DNA
<213>  ARTIFICIAL SEQUENCE

<220>
<223>  forward primer

<400>  388
cggtcaatgg tgttctaccc                                                    20


<210>  389
<211>  20
<212>  DNA
<213>  ARTIFICIAL SEQUENCE

<220>
<223>  forward primer

<400>  389
gctatatcac gtgctgccaa                                                    20


<210>  390
<211>  20
<212>  DNA
<213>  ARTIFICIAL SEQUENCE

<220>
<223>  forward primer

<400>  390
gcactggtaa ggggactcaa                                                    20


<210>  391
<211>  20
<212>  DNA
<213>  ARTIFICIAL SEQUENCE

<220>
<223>  forward primer

<400>  391
acacgaccaa aagggaagtg                                                    20


<210>  392
<211>  20
<212>  DNA
<213>  ARTIFICIAL SEQUENCE

<220>
<223>  forward primer

<400>  392
ttgcaaacac agactggctc                                                    20


<210>  393
<211>  20
<212>  DNA
<213>  ARTIFICIAL SEQUENCE

<220>
<223>  forward primer

<400>  393
agtttctgcc gcctacaaga                                                    20


<210>  394
<211>  20
<212>  DNA
<213>  ARTIFICIAL SEQUENCE

<220>
<223>  forward primer

<400>  394
acgaaccttc cagtttgacg                                                    20


<210>  395
<211>  20
<212>  DNA
<213>  ARTIFICIAL SEQUENCE

<220>
<223>  forward primer

<400>  395
ggcggctttt gatttaactg                                                    20


<210>  396
<211>  20
<212>  DNA
<213>  ARTIFICIAL SEQUENCE

<220>
<223>  forward primer

<400>  396
ttagaacccc ttgcaaatcg                                                    20


<210>  397
<211>  20
<212>  DNA
<213>  ARTIFICIAL SEQUENCE

<220>
<223>  forward primer

<400>  397
gatgaaatca aagaagggga                                                    20


<210>  398
<211>  20
<212>  DNA
<213>  ARTIFICIAL SEQUENCE

<220>
<223>  forward primer

<400>  398
ctccggcatt atctgtggtt                                                    20


<210>  399
<211>  20
<212>  DNA
<213>  ARTIFICIAL SEQUENCE

<220>
<223>  forward primer

<400>  399
aagactagcc attgtcggtg                                                    20


<210>  400
<211>  20
<212>  DNA
<213>  ARTIFICIAL SEQUENCE

<220>
<223>  forward primer

<400>  400
aaaaagctga agccacagga                                                    20


<210>  401
<211>  20
<212>  DNA
<213>  ARTIFICIAL SEQUENCE

<220>
<223>  forward primer

<400>  401
cagccgcaac aatatatccc                                                    20


<210>  402

<211>  20
<212>  DNA
<213>  ARTIFICIAL SEQUENCE

<220>
<223>  forward primer

<400>  402
agatagtgac gatggtgggg                                                    20


<210>  403
<211>  20
<212>  DNA
<213>  ARTIFICIAL SEQUENCE

<220>
<223>  forward primer

<400>  403
ccttttatgg ggagtgacga                                                    20


<210>  404
<211>  20
<212>  DNA
<213>  ARTIFICIAL SEQUENCE

<220>
<223>  forward primer

<400>  404
gactatgtgg ctggggtgat                                                    20


<210>  405
<211>  20
<212>  DNA
<213>  ARTIFICIAL SEQUENCE

<220>
<223>  forward primer

<400>  405
caccaaaacc atttaggcgt                                                    20


<210>  406
<211>  20
<212>  DNA
<213>  ARTIFICIAL SEQUENCE

<220>
<223>  forward primer

<400>  406
gacgaaaaat tagccgagca                                                    20


<210>  407
<211>  20
<212>  DNA
<213>  ARTIFICIAL SEQUENCE

<220>
<223>  forward primer

<400>  407
gggaagcaag tacaacccaa                                                    20

<210>  408
<211>  20
<212>  DNA
<213>  ARTIFICIAL SEQUENCE

<220>
<223>  forward primer

<400>  408
ccctggcaaa acactagagg                                                    20


<210>  409
<211>  20
<212>  DNA
<213>  ARTIFICIAL SEQUENCE

<220>
<223>  forward primer

<400>  409
gcagcctcat ttagacaaca                                                    20


<210>  410
<211>  20
<212>  DNA
<213>  ARTIFICIAL SEQUENCE

<220>
<223>  forward primer

<400>  410
acagagggga tgtgaaggtg                                                    20


<210>  411
<211>  20
<212>  DNA
<213>  ARTIFICIAL SEQUENCE

<220>
<223>  forward primer

<400>  411
ccgcggcaga gttaatacat                                                    20


<210>  412
<211>  20
<212>  DNA
<213>  ARTIFICIAL SEQUENCE

<220>
<223>  forward primer

<400>  412
cacgttggga aaaaccaatc                                                    20


<210>  413
<211>  20
<212>  DNA
<213>  ARTIFICIAL SEQUENCE

<220>
<223>  forward primer

<400>  413
aaagaggaca cggcagaaga                                                    20


<210>  414
<211>  20
<212>  DNA
<213>  ARTIFICIAL SEQUENCE

<220>
<223>  forward primer

<400>  414
atccattgga cagcatccat                                                    20


<210>  415
<211>  20
<212>  DNA
<213>  ARTIFICIAL SEQUENCE

<220>
<223>  forward primer

<400>  415
caggaagtcg tcatttggct                                                    20


<210>  416
<211>  20
<212>  DNA
<213>  ARTIFICIAL SEQUENCE

<220>
<223>  forward primer

<400>  416
agcaaatatt ggcgtgatcc                                                    20


<210>  417
<211>  20
<212>  DNA
<213>  ARTIFICIAL SEQUENCE

<220>
<223>  forward primer

<400>  417
tcagtgctcg gatgaaattg                                                    20


<210>  418
<211>  20
<212>  DNA
<213>  ARTIFICIAL SEQUENCE

<220>
<223>  forward primer

<400>  418
ccctcaaaga aaggaaaggg                                                    20


<210>  419
<211>  20
<212>  DNA
<213>  ARTIFICIAL SEQUENCE

<220>
<223>  forward primer

<400>  419
aagaaacccc tccccatcta                                                    20


<210>  420
<211>  20
<212>  DNA
<213>  ARTIFICIAL SEQUENCE

<220>
<223>  forward primer

<400>  420
cccgattttc tcccgttatt                                                    20


<210>  421
<211>  20
<212>  DNA
<213>  ARTIFICIAL SEQUENCE

<220>
<223>  forward primer

<400>  421
ctgtattgcc aacaagaagc                                                    20


<210>  422
<211>  20
<212>  DNA
<213>  ARTIFICIAL SEQUENCE

<220>
<223>  forward primer

<400>  422
tgaacgttga aaaacatgcc                                                    20


<210>  423
<211>  20
<212>  DNA
<213>  ARTIFICIAL SEQUENCE

<220>
<223>  forward primer

<400>  423
cacttctggt gccaacttga                                                    20


<210>  424
<211>  20
<212>  DNA
<213>  ARTIFICIAL SEQUENCE

<220>
<223>  forward primer

<400>  424
ataaggggaa tgctcggttc                                                    20


<210>  425

<211>  20
<212>  DNA
<213>  ARTIFICIAL SEQUENCE

<220>
<223>  forward primer

<400>  425
atcaaggcct cgaggatagt                                                    20


<210>  426
<211>  20
<212>  DNA
<213>  ARTIFICIAL SEQUENCE

<220>
<223>  forward primer

<400>  426
cggcacccgt gttattattc                                                    20


<210>  427
<211>  20
<212>  DNA
<213>  ARTIFICIAL SEQUENCE

<220>
<223>  forward primer

<400>  427
ggaggctgct agacgatgac                                                    20


<210>  428
<211>  20
<212>  DNA
<213>  ARTIFICIAL SEQUENCE

<220>
<223>  forward primer

<400>  428
attagcaggg caaaaagcaa                                                    20


<210>  429
<211>  20
<212>  DNA
<213>  ARTIFICIAL SEQUENCE

<220>
<223>  forward primer

<400>  429
aaagcgaaac agcaacaggt                                                    20


<210>  430
<211>  20
<212>  DNA
<213>  ARTIFICIAL SEQUENCE

<220>
<223>  forward primer

<400>  430
gaaagggaaa caacagccac                                                    20

<210> 431
<211> 20
<212> DNA
<213> ARTIFICIAL SEQUENCE

<220>
<223> forward primer

<400> 431
tctgtcgctt tattcgcctt 20


<210> 432
<211> 20
<212> DNA
<213> ARTIFICIAL SEQUENCE

<220>
<223> forward primer

<400> 432
gaaaatgaat tcgcgagctg 20


<210> 433
<211> 20
<212> DNA
<213> ARTIFICIAL SEQUENCE

<220>
<223> forward primer

<400> 433
cgtctcccag atcaaagctc 20


<210> 434
<211> 20
<212> DNA
<213> ARTIFICIAL SEQUENCE

<220>
<223> forward primer

<400> 434
acaaagaaat accgccacca 20


<210> 435
<211> 20
<212> DNA
<213> ARTIFICIAL SEQUENCE

<220>
<223> forward primer

<400> 435
atcaccgaag acacggagac 20


<210> 436
<211> 20
<212> DNA
<213> ARTIFICIAL SEQUENCE

<220>
<223> forward primer

<400>  436
tggtggtcac atgcaagaat                                                    20


<210>  437
<211>  20
<212>  DNA
<213>  ARTIFICIAL SEQUENCE

<220>
<223>  forward primer

<400>  437
aaatgagtcc ggatttgacg                                                    20


<210>  438
<211>  20
<212>  DNA
<213>  ARTIFICIAL SEQUENCE

<220>
<223>  forward primer

<400>  438
caaccaagat tgccagaggt                                                    20


<210>  439
<211>  20
<212>  DNA
<213>  ARTIFICIAL SEQUENCE

<220>
<223>  forward primer

<400>  439
acggactata cccagggacc                                                    20


<210>  440
<211>  20
<212>  DNA
<213>  ARTIFICIAL SEQUENCE

<220>
<223>  forward primer

<400>  440
gagaacgcct taaccgatga                                                    20


<210>  441
<211>  20
<212>  DNA
<213>  ARTIFICIAL SEQUENCE

<220>
<223>  forward primer

<400>  441
tgttttgcgg attactgctg                                                    20


<210>  442
<211>  20
<212>  DNA
<213>  ARTIFICIAL SEQUENCE

<220>
<223>  forward primer

<400>  442
ttgccgggtt tgataacact                                                    20


<210>  443
<211>  20
<212>  DNA
<213>  ARTIFICIAL SEQUENCE

<220>
<223>  forward primer

<400>  443
ggtatgccaa gtggtttgct                                                    20


<210>  444
<211>  20
<212>  DNA
<213>  ARTIFICIAL SEQUENCE

<220>
<223>  forward primer

<400>  444
ggttacagag ttctaggtat                                                    20


<210>  445
<211>  20
<212>  DNA
<213>  ARTIFICIAL SEQUENCE

<220>
<223>  forward primer

<400>  445
ccgatgttga atctagggga                                                    20


<210>  446
<211>  20
<212>  DNA
<213>  ARTIFICIAL SEQUENCE

<220>
<223>  forward primer

<400>  446
atcagaaaca tcctaggtgg                                                    20


<210>  447
<211>  20
<212>  DNA
<213>  ARTIFICIAL SEQUENCE

<220>
<223>  forward primer

<400>  447
gtgccggaaa atccactcta                                                    20


<210>  448

<211>  20
<212>  DNA
<213>  ARTIFICIAL SEQUENCE

<220>
<223>  forward primer

<400>  448
tgtacaaatg tgccgctgat                                                    20


<210>  449
<211>  20
<212>  DNA
<213>  ARTIFICIAL SEQUENCE

<220>
<223>  forward primer

<400>  449
ctcaagcaca agccaaagtg                                                    20


<210>  450
<211>  20
<212>  DNA
<213>  ARTIFICIAL SEQUENCE

<220>
<223>  forward primer

<400>  450
atttccattc aaaacgctgg                                                    20


<210>  451
<211>  20
<212>  DNA
<213>  ARTIFICIAL SEQUENCE

<220>
<223>  forward primer

<400>  451
aaagagaccg gatgcagatg                                                    20


<210>  452
<211>  20
<212>  DNA
<213>  ARTIFICIAL SEQUENCE

<220>
<223>  forward primer

<400>  452
tccaacaaga gtttggctcc                                                    20


<210>  453
<211>  20
<212>  DNA
<213>  ARTIFICIAL SEQUENCE

<220>
<223>  forward primer

<400>  453
tttggtccct tacaccgaag                                                    20

<210>  454
<211>  20
<212>  DNA
<213>  ARTIFICIAL SEQUENCE

<220>
<223>  forward primer

<400>  454
ttttcgctgg taacgaggtc                                                    20


<210>  455
<211>  20
<212>  DNA
<213>  ARTIFICIAL SEQUENCE

<220>
<223>  forward primer

<400>  455
gtgaatactt gcttggccgt                                                    20


<210>  456
<211>  20
<212>  DNA
<213>  ARTIFICIAL SEQUENCE

<220>
<223>  forward primer

<400>  456
taccacatct gcgcattcat                                                    20


<210>  457
<211>  20
<212>  DNA
<213>  ARTIFICIAL SEQUENCE

<220>
<223>  forward primer

<400>  457
gacatgtcgg agatggttcc                                                    20


<210>  458
<211>  20
<212>  DNA
<213>  ARTIFICIAL SEQUENCE

<220>
<223>  forward primer

<400>  458
tcagttgcct gaatctcgtg                                                    20


<210>  459
<211>  20
<212>  DNA
<213>  ARTIFICIAL SEQUENCE

<220>
<223>  forward primer

<400>  459
ttgccactgc ctttttcaac                                                    20


<210>  460
<211>  20
<212>  DNA
<213>  ARTIFICIAL SEQUENCE

<220>
<223>  forward primer

<400>  460
caccggattt tacccaagaa                                                    20


<210>  461
<211>  20
<212>  DNA
<213>  ARTIFICIAL SEQUENCE

<220>
<223>  forward primer

<400>  461
tcgccactga catacctcat                                                    20


<210>  462
<211>  20
<212>  DNA
<213>  ARTIFICIAL SEQUENCE

<220>
<223>  forward primer

<400>  462
aggccattac aaaccatcca                                                    20


<210>  463
<211>  20
<212>  DNA
<213>  ARTIFICIAL SEQUENCE

<220>
<223>  forward primer

<400>  463
ctcagccagt tggtcaaaca                                                    20


<210>  464
<211>  20
<212>  DNA
<213>  ARTIFICIAL SEQUENCE

<220>
<223>  forward primer

<400>  464
gcaaatcccg tgtggtctac                                                    20


<210>  465
<211>  20
<212>  DNA
<213>  ARTIFICIAL SEQUENCE

<220>
<223>  forward primer

<400>  465
gtcgcaggat tctccagttc                                                    20


<210>  466
<211>  20
<212>  DNA
<213>  ARTIFICIAL SEQUENCE

<220>
<223>  forward primer

<400>  466
gcgctggtaa tgatggtttt                                                    20


<210>  467
<211>  20
<212>  DNA
<213>  ARTIFICIAL SEQUENCE

<220>
<223>  forward primer

<400>  467
gctgagcgac acatcaaaaa                                                    20


<210>  468
<211>  20
<212>  DNA
<213>  ARTIFICIAL SEQUENCE

<220>
<223>  forward primer

<400>  468
taaagaggaa gaccctgcga                                                    20


<210>  469
<211>  20
<212>  DNA
<213>  ARTIFICIAL SEQUENCE

<220>
<223>  forward primer

<400>  469
ccagccacaa agactcttcc                                                    20


<210>  470
<211>  20
<212>  DNA
<213>  ARTIFICIAL SEQUENCE

<220>
<223>  forward primer

<400>  470
aatcaagcag aaaaggcgaa                                                    20


<210>  471

<211>  20
<212>  DNA
<213>  ARTIFICIAL SEQUENCE

<220>
<223>  forward primer

<400>  471
ccccaaacaa tgacgaaaat                                                    20


<210>  472
<211>  20
<212>  DNA
<213>  ARTIFICIAL SEQUENCE

<220>
<223>  forward primer

<400>  472
attcaatggg tgcaaagagg                                                    20


<210>  473
<211>  20
<212>  DNA
<213>  ARTIFICIAL SEQUENCE

<220>
<223>  forward primer

<400>  473
gtaacggtga tgggactggt                                                    20


<210>  474
<211>  20
<212>  DNA
<213>  ARTIFICIAL SEQUENCE

<220>
<223>  forward primer

<400>  474
attgttaatg gtgccgcttc                                                    20


<210>  475
<211>  20
<212>  DNA
<213>  ARTIFICIAL SEQUENCE

<220>
<223>  forward primer

<400>  475
gtcttaaaca acccggcaaa                                                    20


<210>  476
<211>  20
<212>  DNA
<213>  ARTIFICIAL SEQUENCE

<220>
<223>  forward primer

<400>  476
cacgcttgga tatcctccat                                                    20

<210>  477
<211>  20
<212>  DNA
<213>  ARTIFICIAL SEQUENCE

<220>
<223>  forward primer

<400>  477
cctggtgtaa accgggaaat                                                    20


<210>  478
<211>  20
<212>  DNA
<213>  ARTIFICIAL SEQUENCE

<220>
<223>  forward primer

<400>  478
cagcaacagc aacaaagcat                                                    20


<210>  479
<211>  20
<212>  DNA
<213>  ARTIFICIAL SEQUENCE

<220>
<223>  forward primer

<400>  479
agtggtatgg ctggcatttc                                                    20


<210>  480
<211>  20
<212>  DNA
<213>  ARTIFICIAL SEQUENCE

<220>
<223>  forward primer

<400>  480
tacggccggt atgatgattt                                                    20


<210>  481
<211>  20
<212>  DNA
<213>  ARTIFICIAL SEQUENCE

<220>
<223>  forward primer

<400>  481
gaaaatggct ccagttccaa                                                    20


<210>  482
<211>  20
<212>  DNA
<213>  ARTIFICIAL SEQUENCE

<220>
<223>  forward primer

<400>  482
ccccaattac ccacagagaa                                                   20


<210>  483
<211>  20
<212>  DNA
<213>  ARTIFICIAL SEQUENCE

<220>
<223>  forward primer

<400>  483
cactctcact gcatcccaaa                                                   20


<210>  484
<211>  20
<212>  DNA
<213>  ARTIFICIAL SEQUENCE

<220>
<223>  forward primer

<400>  484
gagcagctgg cagactcttt                                                   20


<210>  485
<211>  20
<212>  DNA
<213>  ARTIFICIAL SEQUENCE

<220>
<223>  forward primer

<400>  485
catgcgcagg ttactgttgt                                                   20


<210>  486
<211>  20
<212>  DNA
<213>  ARTIFICIAL SEQUENCE

<220>
<223>  forward primer

<400>  486
gcctgtttca tggctatcgt                                                   20


<210>  487
<211>  20
<212>  DNA
<213>  ARTIFICIAL SEQUENCE

<220>
<223>  forward primer

<400>  487
tgtttcctct tcgaccgagt                                                   20


<210>  488
<211>  20
<212>  DNA
<213>  ARTIFICIAL SEQUENCE

<220>
<223>  forward primer

<400>  488
agggaccttt tcacctcgat                                                    20


<210>  489
<211>  20
<212>  DNA
<213>  ARTIFICIAL SEQUENCE

<220>
<223>  forward primer

<400>  489
accagatcac ccacttttgc                                                    20


<210>  490
<211>  20
<212>  DNA
<213>  ARTIFICIAL SEQUENCE

<220>
<223>  forward primer

<400>  490
atgtttcact ggtcgctggt                                                    20


<210>  491
<211>  20
<212>  DNA
<213>  ARTIFICIAL SEQUENCE

<220>
<223>  forward primer

<400>  491
cattccgtat tttcggcact                                                    20


<210>  492
<211>  20
<212>  DNA
<213>  ARTIFICIAL SEQUENCE

<220>
<223>  forward primer

<400>  492
ttaacgaaag aagcccgttc                                                    20


<210>  493
<211>  20
<212>  DNA
<213>  ARTIFICIAL SEQUENCE

<220>
<223>  forward primer

<400>  493
ctgtaaacgc acattcggtg                                                    20


<210>  494

<211>  20
<212>  DNA
<213>  ARTIFICIAL SEQUENCE

<220>
<223>  forward primer

<400>  494
tctgatgcag atgtcgttcc                                                    20


<210>  495
<211>  20
<212>  DNA
<213>  ARTIFICIAL SEQUENCE

<220>
<223>  forward primer

<400>  495
catggtatcc aaacatcccc                                                    20


<210>  496
<211>  20
<212>  DNA
<213>  ARTIFICIAL SEQUENCE

<220>
<223>  forward primer

<400>  496
tgttcttacg aaccagcgtt                                                    20


<210>  497
<211>  20
<212>  DNA
<213>  ARTIFICIAL SEQUENCE

<220>
<223>  forward primer

<400>  497
atttggcctc cagaggtctt                                                    20


<210>  498
<211>  20
<212>  DNA
<213>  ARTIFICIAL SEQUENCE

<220>
<223>  forward primer

<400>  498
aagactttga accccgtcct                                                    20


<210>  499
<211>  20
<212>  DNA
<213>  ARTIFICIAL SEQUENCE

<220>
<223>  forward primer

<400>  499
tcaaggacaa gaagaccgct                                                    20

<210> 500
<211> 20
<212> DNA
<213> ARTIFICIAL SEQUENCE

<220>
<223> forward primer

<400> 500
ggctgagcaa ggtaaaacca 20


<210> 501
<211> 20
<212> DNA
<213> ARTIFICIAL SEQUENCE

<220>
<223> forward primer

<400> 501
tcctgcgggg aatgtattta 20


<210> 502
<211> 20
<212> DNA
<213> ARTIFICIAL SEQUENCE

<220>
<223> forward primer

<400> 502
ccacaacaac ctcgcaacta 20


<210> 503
<211> 20
<212> DNA
<213> ARTIFICIAL SEQUENCE

<220>
<223> forward primer

<400> 503
ttcaatatcc ccttcgatgc 20


<210> 504
<211> 20
<212> DNA
<213> ARTIFICIAL SEQUENCE

<220>
<223> forward primer

<400> 504
ctccatggct ttaatggtgg 20


<210> 505
<211> 20
<212> DNA
<213> ARTIFICIAL SEQUENCE

<220>
<223> forward primer

<400>  505
cacgttgcac agacaaggtt                                                    20

<210>  506
<211>  20
<212>  DNA
<213>  ARTIFICIAL SEQUENCE

<220>
<223>  forward primer

<400>  506
gaggattcag aacgctccag                                                    20

<210>  507
<211>  20
<212>  DNA
<213>  ARTIFICIAL SEQUENCE

<220>
<223>  forward primer

<400>  507
caaagtggca gaaatgtgga                                                    20

<210>  508
<211>  20
<212>  DNA
<213>  ARTIFICIAL SEQUENCE

<220>
<223>  forward primer

<400>  508
agctcttctg ctgctccaac                                                    20

<210>  509
<211>  20
<212>  DNA
<213>  ARTIFICIAL SEQUENCE

<220>
<223>  forward primer

<400>  509
gcctgatttg ggcactttac                                                    20

<210>  510
<211>  20
<212>  DNA
<213>  ARTIFICIAL SEQUENCE

<220>
<223>  forward primer

<400>  510
ccaaaatccg tgtttatggc                                                    20

<210>  511
<211>  20
<212>  DNA
<213>  ARTIFICIAL SEQUENCE

<220>
<223>  forward primer

<400>  511
aacctttgac ggatgctcac                                                    20


<210>  512
<211>  20
<212>  DNA
<213>  ARTIFICIAL SEQUENCE

<220>
<223>  forward primer

<400>  512
ggctgaaaaa ttcgcttctg                                                    20


<210>  513
<211>  20
<212>  DNA
<213>  ARTIFICIAL SEQUENCE

<220>
<223>  forward primer

<400>  513
attgcctgat catgtccctc                                                    20


<210>  514
<211>  20
<212>  DNA
<213>  ARTIFICIAL SEQUENCE

<220>
<223>  forward primer

<400>  514
gcgctgaccg atccttatac                                                    20


<210>  515
<211>  20
<212>  DNA
<213>  ARTIFICIAL SEQUENCE

<220>
<223>  forward primer

<400>  515
ctcctccgcg tcttcttcta                                                    20


<210>  516
<211>  20
<212>  DNA
<213>  ARTIFICIAL SEQUENCE

<220>
<223>  forward primer

<400>  516
taaagaatgg tacgggtggc                                                    20


<210>  517

<211>  20
<212>  DNA
<213>  ARTIFICIAL SEQUENCE

<220>
<223>  forward primer

<400>  517
tccctgtcgt tgacaccata                                                    20


<210>  518
<211>  20
<212>  DNA
<213>  ARTIFICIAL SEQUENCE

<220>
<223>  forward primer

<400>  518
caagagagaa aatgcctgcc                                                    20


<210>  519
<211>  20
<212>  DNA
<213>  ARTIFICIAL SEQUENCE

<220>
<223>  forward primer

<400>  519
tccaaagccc gctagtctaa                                                    20


<210>  520
<211>  20
<212>  DNA
<213>  ARTIFICIAL SEQUENCE

<220>
<223>  forward primer

<400>  520
tcagaagtcc ccagaccaat                                                    20


<210>  521
<211>  20
<212>  DNA
<213>  ARTIFICIAL SEQUENCE

<220>
<223>  forward primer

<400>  521
tcaacttaac ggacgatccc                                                    20


<210>  522
<211>  20
<212>  DNA
<213>  ARTIFICIAL SEQUENCE

<220>
<223>  forward primer

<400>  522
tgccatcgtg tctgctttac                                                    20

<210>  523
<211>  20
<212>  DNA
<213>  ARTIFICIAL SEQUENCE

<220>
<223>  forward primer

<400>  523
acttccacgg tcgaaacaac                                                    20


<210>  524
<211>  20
<212>  DNA
<213>  ARTIFICIAL SEQUENCE

<220>
<223>  forward primer

<400>  524
agacggggga tggttctaat                                                    20


<210>  525
<211>  20
<212>  DNA
<213>  ARTIFICIAL SEQUENCE

<220>
<223>  forward primer

<400>  525
ccgttcatca cccacaagat                                                    20


<210>  526
<211>  20
<212>  DNA
<213>  ARTIFICIAL SEQUENCE

<220>
<223>  forward primer

<400>  526
ccatcactat cacgccttcc                                                    20


<210>  527
<211>  20
<212>  DNA
<213>  ARTIFICIAL SEQUENCE

<220>
<223>  forward primer

<400>  527
atgtggacgc acaagacaaa                                                    20


<210>  528
<211>  20
<212>  DNA
<213>  ARTIFICIAL SEQUENCE

<220>
<223>  forward primer

<400>  528
ggacgactcg ttcatgataa                                                    20


<210>  529
<211>  20
<212>  DNA
<213>  ARTIFICIAL SEQUENCE

<220>
<223>  forward primer

<400>  529
aagcgaccga ctacaggcta                                                    20


<210>  530
<211>  20
<212>  DNA
<213>  ARTIFICIAL SEQUENCE

<220>
<223>  forward primer

<400>  530
aaatggcgtg agatttcgtc                                                    20


<210>  531
<211>  20
<212>  DNA
<213>  ARTIFICIAL SEQUENCE

<220>
<223>  forward primer

<400>  531
ggttcttctg gtttgtccga                                                    20


<210>  532
<211>  20
<212>  DNA
<213>  ARTIFICIAL SEQUENCE

<220>
<223>  forward primer

<400>  532
ccgttggtgg aagatgattc                                                    20


<210>  533
<211>  20
<212>  DNA
<213>  ARTIFICIAL SEQUENCE

<220>
<223>  forward primer

<400>  533
tgtgccggaa ctaagtgtca                                                    20


<210>  534
<211>  20
<212>  DNA
<213>  ARTIFICIAL SEQUENCE

<220>
<223>  forward primer

<400>  534
actgcaacct acaataatat                                                    20


<210>  535
<211>  20
<212>  DNA
<213>  ARTIFICIAL SEQUENCE

<220>
<223>  forward primer

<400>  535
ccgctgcctc ttctactgaa                                                    20


<210>  536
<211>  20
<212>  DNA
<213>  ARTIFICIAL SEQUENCE

<220>
<223>  forward primer

<400>  536
gccgtgcaac atttaggatt                                                    20


<210>  537
<211>  20
<212>  DNA
<213>  ARTIFICIAL SEQUENCE

<220>
<223>  forward primer

<400>  537
ggtgaccgta ccgttcgtat                                                    20


<210>  538
<211>  20
<212>  DNA
<213>  ARTIFICIAL SEQUENCE

<220>
<223>  forward primer

<400>  538
ccaacatgcc atttagagca                                                    20


<210>  539
<211>  20
<212>  DNA
<213>  ARTIFICIAL SEQUENCE

<220>
<223>  forward primer

<400>  539
accctcatgg tactagggca                                                    20


<210>  540

<211>  20
<212>  DNA
<213>  ARTIFICIAL SEQUENCE

<220>
<223>  forward primer

<400>  540
ctttgccagc tttggatacc                                                    20


<210>  541
<211>  20
<212>  DNA
<213>  ARTIFICIAL SEQUENCE

<220>
<223>  forward primer

<400>  541
tctcaagctc tgtcagccaa                                                    20


<210>  542
<211>  20
<212>  DNA
<213>  ARTIFICIAL SEQUENCE

<220>
<223>  forward primer

<400>  542
atgcaggctc taccagcagt                                                    20


<210>  543
<211>  20
<212>  DNA
<213>  ARTIFICIAL SEQUENCE

<220>
<223>  forward primer

<400>  543
tgcactcggc aaaacagtaa                                                    20


<210>  544
<211>  20
<212>  DNA
<213>  ARTIFICIAL SEQUENCE

<220>
<223>  forward primer

<400>  544
gcaagcttga aaactgcaac                                                    20


<210>  545
<211>  20
<212>  DNA
<213>  ARTIFICIAL SEQUENCE

<220>
<223>  forward primer

<400>  545
cttgattggg atgggatttg                                                    20

<210> 546
<211> 20
<212> DNA
<213> ARTIFICIAL SEQUENCE

<220>
<223> forward primer

<400> 546
tgcactcgga aaagaaaagg 20


<210> 547
<211> 20
<212> DNA
<213> ARTIFICIAL SEQUENCE

<220>
<223> forward primer

<400> 547
gggaagcaaa gactcacagc 20


<210> 548
<211> 20
<212> DNA
<213> ARTIFICIAL SEQUENCE

<220>
<223> forward primer

<400> 548
tctttgaaac atggggaagc 20


<210> 549
<211> 20
<212> DNA
<213> ARTIFICIAL SEQUENCE

<220>
<223> forward primer

<400> 549
tcacaagcat tctcagtggc 20


<210> 550
<211> 20
<212> DNA
<213> ARTIFICIAL SEQUENCE

<220>
<223> forward primer

<400> 550
cacagaggct caacgaattg 20


<210> 551
<211> 20
<212> DNA
<213> ARTIFICIAL SEQUENCE

<220>
<223> forward primer

<400>  551
tcctggcaat catgcagtta                                                    20

<210>  552
<211>  20
<212>  DNA
<213>  ARTIFICIAL SEQUENCE

<220>
<223>  forward primer

<400>  552
tgaagaaggg ttaacctcgg                                                    20

<210>  553
<211>  20
<212>  DNA
<213>  ARTIFICIAL SEQUENCE

<220>
<223>  forward primer

<400>  553
gagcatttgg actggcattt                                                    20

<210>  554
<211>  20
<212>  DNA
<213>  ARTIFICIAL SEQUENCE

<220>
<223>  forward primer

<400>  554
tctgggcttt caaggaagtg                                                    20

<210>  555
<211>  20
<212>  DNA
<213>  ARTIFICIAL SEQUENCE

<220>
<223>  forward primer

<400>  555
gccattcaag aaatgcaagg                                                    20

<210>  556
<211>  20
<212>  DNA
<213>  ARTIFICIAL SEQUENCE

<220>
<223>  forward primer

<400>  556
ctgttggttc atccacatcg                                                    20

<210>  557
<211>  20
<212>  DNA
<213>  ARTIFICIAL SEQUENCE

<220>
<223>  forward primer

<400>  557
tctattactc ccgcttccga                                                    20


<210>  558
<211>  20
<212>  DNA
<213>  ARTIFICIAL SEQUENCE

<220>
<223>  forward primer

<400>  558
tgaattcaaa ggcacgaaga                                                    20


<210>  559
<211>  20
<212>  DNA
<213>  ARTIFICIAL SEQUENCE

<220>
<223>  forward primer

<400>  559
agtcaagctc tagcgggtca                                                    20


<210>  560
<211>  20
<212>  DNA
<213>  ARTIFICIAL SEQUENCE

<220>
<223>  forward primer

<400>  560
gggagcgcta tgacaatgat                                                    20


<210>  561
<211>  20
<212>  DNA
<213>  ARTIFICIAL SEQUENCE

<220>
<223>  forward primer

<400>  561
gcccaaagag accttcttcc                                                    20


<210>  562
<211>  20
<212>  DNA
<213>  ARTIFICIAL SEQUENCE

<220>
<223>  forward primer

<400>  562
gttgcaaagg ggtttttgaa                                                    20


<210>  563

<211>  20
<212>  DNA
<213>  ARTIFICIAL SEQUENCE

<220>
<223>  forward primer

<400>  563
ggccatcgaa acaattccta                                                    20


<210>  564
<211>  20
<212>  DNA
<213>  ARTIFICIAL SEQUENCE

<220>
<223>  forward primer

<400>  564
accttatacc ccggttccag                                                    20


<210>  565
<211>  20
<212>  DNA
<213>  ARTIFICIAL SEQUENCE

<220>
<223>  forward primer

<400>  565
gtaaacactc cggcccatta                                                    20


<210>  566
<211>  20
<212>  DNA
<213>  ARTIFICIAL SEQUENCE

<220>
<223>  forward primer

<400>  566
acatggcaca agcaatgttc                                                    20


<210>  567
<211>  20
<212>  DNA
<213>  ARTIFICIAL SEQUENCE

<220>
<223>  forward primer

<400>  567
caatgaaagg gatgaggcat                                                    20


<210>  568
<211>  20
<212>  DNA
<213>  ARTIFICIAL SEQUENCE

<220>
<223>  forward primer

<400>  568
caacccgttt ggaacagact                                                    20

<210>  569
<211>  20
<212>  DNA
<213>  ARTIFICIAL SEQUENCE

<220>
<223>  forward primer

<400>  569
tgtacaatcg caaatcccaa                                                    20


<210>  570
<211>  20
<212>  DNA
<213>  ARTIFICIAL SEQUENCE

<220>
<223>  forward primer

<400>  570
actcttctca ctcagcccca                                                    20


<210>  571
<211>  20
<212>  DNA
<213>  ARTIFICIAL SEQUENCE

<220>
<223>  forward primer

<400>  571
atccatccta accgcatctg                                                    20


<210>  572
<211>  20
<212>  DNA
<213>  ARTIFICIAL SEQUENCE

<220>
<223>  forward primer

<400>  572
ctagctcgaa tgctcgtcct                                                    20


<210>  573
<211>  20
<212>  DNA
<213>  ARTIFICIAL SEQUENCE

<220>
<223>  forward primer

<400>  573
gaacattctg ggaaaaacgc                                                    20


<210>  574
<211>  20
<212>  DNA
<213>  ARTIFICIAL SEQUENCE

<220>
<223>  forward primer

<400>  574
gaacattccc ggaaaaacag                                                    20


<210>  575
<211>  20
<212>  DNA
<213>  ARTIFICIAL SEQUENCE

<220>
<223>  forward primer

<400>  575
gagaacggaa cgttgagagc                                                    20


<210>  576
<211>  20
<212>  DNA
<213>  ARTIFICIAL SEQUENCE

<220>
<223>  forward primer

<400>  576
tccgctattc caagtgaacc                                                    20


<210>  577
<211>  20
<212>  DNA
<213>  ARTIFICIAL SEQUENCE

<220>
<223>  forward primer

<400>  577
gtggtgatct tgacgcagac                                                    20


<210>  578
<211>  20
<212>  DNA
<213>  ARTIFICIAL SEQUENCE

<220>
<223>  forward primer

<400>  578
ttgcaacaag atgaggcaac                                                    20


<210>  579
<211>  20
<212>  DNA
<213>  ARTIFICIAL SEQUENCE

<220>
<223>  forward primer

<400>  579
tcaaaagaaa tacgccgacc                                                    20


<210>  580
<211>  20
<212>  DNA
<213>  ARTIFICIAL SEQUENCE

<220>
<223>  forward primer

<400>  580
cgtcaagaaa ccaaccaacc                                                    20


<210>  581
<211>  20
<212>  DNA
<213>  ARTIFICIAL SEQUENCE

<220>
<223>  forward primer

<400>  581
caatgcctaa taagcccgaa                                                    20


<210>  582
<211>  20
<212>  DNA
<213>  ARTIFICIAL SEQUENCE

<220>
<223>  forward primer

<400>  582
ccaagattgc ccttggttta                                                    20


<210>  583
<211>  20
<212>  DNA
<213>  ARTIFICIAL SEQUENCE

<220>
<223>  forward primer

<400>  583
ttatgatcct cagtgcgtcg                                                    20


<210>  584
<211>  20
<212>  DNA
<213>  ARTIFICIAL SEQUENCE

<220>
<223>  forward primer

<400>  584
aactgccacc aataatccca                                                    20


<210>  585
<211>  20
<212>  DNA
<213>  ARTIFICIAL SEQUENCE

<220>
<223>  forward primer

<400>  585
gcacttgcga atggaggtat                                                    20


<210>  586

<211>  20
<212>  DNA
<213>  ARTIFICIAL SEQUENCE

<220>
<223>  forward primer

<400>  586
tgatccaagt ttgctgatcg                                                    20


<210>  587
<211>  20
<212>  DNA
<213>  ARTIFICIAL SEQUENCE

<220>
<223>  forward primer

<400>  587
actcctggta cagccgagaa                                                    20


<210>  588
<211>  20
<212>  DNA
<213>  ARTIFICIAL SEQUENCE

<220>
<223>  forward primer

<400>  588
ccactcctgg gtttactgga                                                    20


<210>  589
<211>  20
<212>  DNA
<213>  ARTIFICIAL SEQUENCE

<220>
<223>  forward primer

<400>  589
atgggtgcac aaccatcata                                                    20


<210>  590
<211>  20
<212>  DNA
<213>  ARTIFICIAL SEQUENCE

<220>
<223>  forward primer

<400>  590
atccgccgat ttaatcaatg                                                    20


<210>  591
<211>  20
<212>  DNA
<213>  ARTIFICIAL SEQUENCE

<220>
<223>  forward primer

<400>  591
acatgccatg gagactggtt                                                    20

<210>  592
<211>  20
<212>  DNA
<213>  ARTIFICIAL SEQUENCE

<220>
<223>  forward primer

<400>  592
cgcaattgcc tgataaggat                                                    20


<210>  593
<211>  20
<212>  DNA
<213>  ARTIFICIAL SEQUENCE

<220>
<223>  forward primer

<400>  593
aattggggga aaagaggttg                                                    20


<210>  594
<211>  20
<212>  DNA
<213>  ARTIFICIAL SEQUENCE

<220>
<223>  forward primer

<400>  594
gaggtggtga agaagaggca                                                    20


<210>  595
<211>  20
<212>  DNA
<213>  ARTIFICIAL SEQUENCE

<220>
<223>  forward primer

<400>  595
taacgaacgc tgaaccacag                                                    20


<210>  596
<211>  20
<212>  DNA
<213>  ARTIFICIAL SEQUENCE

<220>
<223>  forward primer

<400>  596
atttgactag tgctgccgct                                                    20


<210>  597
<211>  20
<212>  DNA
<213>  ARTIFICIAL SEQUENCE

<220>
<223>  forward primer

<400>  597
agcaggaaat gcttcatcgt                                                    20


<210>  598
<211>  20
<212>  DNA
<213>  ARTIFICIAL SEQUENCE

<220>
<223>  forward primer

<400>  598
cttatgcctt cagtgcctcc                                                    20


<210>  599
<211>  71
<212>  DNA
<213>  Artificial sequence

<220>
<223>  oligonucleotide

<400>  599
ccgtcaattc caccgaatca aggttgactt ggttatctat attgattatc attatcattg        60

ccgttataag t                                                             71


<210>  600
<211>  71
<212>  DNA
<213>  Artificial sequence

<220>
<223>  oligonucleotide

<400>  600
tactcccttt gacccttatg ctctatccga gcacgatgaa gaacgacccc agaatgtaca        60

gtctaagtca a                                                             71


<210>  601
<211>  71
<212>  DNA
<213>  Artificial sequence

<220>
<223>  oligonucleotide

<400>  601
ttgctctata ctcaaaccaa caacaactta ctctacaact aatgtcctct gccgccgaaa        60

agaaaccagc t                                                             71


<210>  602
<211>  71
<212>  DNA
<213>  Artificial sequence

<220>
<223>  oligonucleotide

<400>  602
agagacgctt tgctaaagga agacgcttaa ttgattacaa ataattagct aagaaattac        60

gtaccttctc a                                                          71


<210>  603
<211>  71
<212>  DNA
<213>  Artificial sequence

<220>
<223>  oligonucleotide

<400>  603
tacaagcaat acaaaaccaa cacacctata tataactaat aatgtccggt aacggtgctc     60

aaggtactaa g                                                          71


<210>  604
<211>  71
<212>  DNA
<213>  Artificial sequence

<220>
<223>  oligonucleotide

<400>  604
ggtagaacct tatacggttt cggtggttaa tttagctaat tctaagaaaa cggtaacaac     60

aagcaaatat t                                                          71


<210>  605
<211>  71
<212>  DNA
<213>  Artificial sequence

<220>
<223>  oligonucleotide

<400>  605
taataagaaa aacatctaac ataaatatat aaacgcaaac aatggccaga acaaagcaaa     60

cagcaagaaa g                                                          71


<210>  606
<211>  71
<212>  DNA
<213>  Artificial sequence

<220>
<223>  oligonucleotide

<400>  606
   gttttcaccg aaactttgaa acacgattaa acttattagt acattaaaaa tattctttta     60
   ttttttatat a                                                           71


<210>  607
<211>  71
<212>  DNA
<213>  Artificial sequence

<220>
<223>  oligonucleotide

<400>  607

tgctgaagac ttagaggaaa cgtatcaagc cagggaaaaa gatgtccact gaattaactg 60

ttcaatctga a 71

<210> 608
<211> 71
<212> DNA
<213> Artificial sequence

<220>
<223> oligonucleotide

<400> 608
ctcatatcta caagactgac agacccaagt acgaagctac agccagagaa tggacaaaga 60

aatacgctgt a 71

<210> 609
<211> 71
<212> DNA
<213> Artificial sequence

<220>
<223> oligonucleotide

<400> 609
acgcttcaca tagaaagttg attaagaact ccaaagcaaa aatgtatgtg atgcagtgaa 60

taatgaatct a 71

<210> 610
<211> 71
<212> DNA
<213> Artificial sequence

<220>
<223> oligonucleotide

<400> 610
ccgcaactaa agaagcaaag caaccaaagg aaccaaagaa gaggaccacc aggagaaaga 60

aggatcctaa c 71

<210> 611
<211> 71
<212> DNA
<213> Artificial sequence

<220>
<223> oligonucleotide

<400> 611
gtttttagaa tatacggtca acgaactata attaactaaa catgggtaaa gagaagtctc 60

acattaacgt t 71

<210> 612
<211> 71
<212> DNA
<213> Artificial sequence

<220>
<223> oligonucleotide

<400>  612
aagagaagag cttcttcttt gaaggcttaa gtatgtcttg gacctgcata atttttatac      60

tttttccttt t                                                           71


<210>  613
<211>  71
<212>  DNA
<213>  Artificial sequence

<220>
<223>  oligonucleotide

<400>  613
gctgctgaag aagccgaaga cgaagaataa ataattcgct gatttcataa ctgtataata      60

aattttataa c                                                           71


<210>  614
<211>  71
<212>  DNA
<213>  Artificial sequence

<220>
<223>  oligonucleotide

<400>  614
gccccagttc catacgaaac tttcatctaa tttacccgat gctggttttg tatcgaaaaa      60

gattttcagg t                                                           71


<210>  615
<211>  71
<212>  DNA
<213>  Artificial sequence

<220>
<223>  oligonucleotide

<400>  615
cactccgtcc ctcgtctaat acaagtttga atgacgattt ttctgaaatc tttgtttaca      60

tttcattcta t                                                           71


<210>  616
<211>  71
<212>  DNA
<213>  Artificial sequence

<220>
<223>  oligonucleotide

<400>  616
gctcaagaac tgcgtctcca cttgaattga gaattgctga aattcaaggt aaaacattct      60

acgaaaccgc c                                                           71


<210>  617
<211>  71
<212>  DNA
<213>  Artificial sequence

<220>
<223>  oligonucleotide

<400>  617
tataccgttt tatttggcgt ttcaagggca tttggtattc ttgctcaatt gatcactgat      60

agggccatcg g                                                           71


<210>  618
<211>  71
<212>  DNA
<213>  Artificial sequence

<220>
<223>  oligonucleotide

<400>  618
atgtcacaga acaacacaat cagctcaatg aatcctgaaa gagcttacaa caatgtaacg      60

ctgaaaaatt t                                                           71


<210>  619
<211>  71
<212>  DNA
<213>  Artificial sequence

<220>
<223>  oligonucleotide

<400>  619
   gctagacaaa gaggattgca aagattctaa aaagggcgga gcgagccatt ttatttcctg      60
   tccccttcat c                                                            71


<210>  620
<211>  71
<212>  DNA
<213>  Artificial sequence

<220>
<223>  oligonucleotide

<400>  620
attttaaacc caaggagagt aagaaaccac aagaacccgc catgtctagt atgtttaatc      60

acatagtgaa c                                                           71


<210>  621
<211>  71
<212>  DNA
<213>  Artificial sequence

<220>
<223>  oligonucleotide

<400>  621
agatggcgac tgttttataa ctactcatcc tagaaacaaa gaacttctta ttgcgaattg      60

ctatagctat a                                                           71


<210>  622
<211>  71
<212>  DNA
<213>  Artificial sequence

<220>

<223>  oligonucleotide

<400>  622
acggggagct ttcgaaattt tttatattca aggacattta tgttgtgtgg gttattaaac      60

gtgggacttt g                                                           71


<210>  623
<211>  71
<212>  DNA
<213>  Artificial sequence

<220>
<223>  oligonucleotide

<400>  623
aacgacattg gtttccacaa gtacagataa gtcaagagcg attgtaacat cgtaatcatt      60

aacctttttt g                                                           71


<210>  624
<211>  71
<212>  DNA
<213>  Artificial sequence

<220>
<223>  oligonucleotide

<400>  624
agaaagaatt aataagcaaa cagaactcaa tcaaaggaaa aatggccggt ttgaaagacg      60

ttgtcactcg t                                                           71


<210>  625
<211>  71
<212>  DNA
<213>  Artificial sequence

<220>
<223>  oligonucleotide

<400>  625
gctgaagctg aagctgaaaa gaagaaatag atctaaaaac ttcatcaaag aatttttcat      60

ccacaatttt t                                                           71


<210>  626
<211>  71
<212>  DNA
<213>  Artificial sequence

<220>
<223>  oligonucleotide

<400>  626
agcagtgttt cgaaaaatac agcaagagaa taagcaacaa gatgtctgcc gtcccaagtg      60

tccaagtatg t                                                           71


<210>  627
<211>  71
<212>  DNA
<213>  Artificial sequence

<220>
<223>  oligonucleotide

<400>  627
gagcacccca cttttaaaac cagtaaataa gggtacagat gacgacaata tcgatatttc      60

caatgggcat g                                                           71


<210>  628
<211>  71
<212>  DNA
<213>  Artificial sequence

<220>
<223>  oligonucleotide

<400>  628
tttacttcaa tcctaagata gtcatatcga cttaattcca aatgagagct aagtggagaa      60

agaagagaac t                                                           71


<210>  629
<211>  71
<212>  DNA
<213>  Artificial sequence

<220>
<223>  oligonucleotide

<400>  629
ccacaaagaa agtacgctat caaggcttaa tttcgttaga ttatcatttt aatatttgat      60

ttttggatat a                                                           71


<210>  630
<211>  71
<212>  DNA
<213>  Artificial sequence

<220>
<223>  oligonucleotide

<400>  630
ttaagcaaca acttgaagaa tcaatcctaa tctaaatctg tatagaacgt ttagtcatgc      60

ggaccttgtg t                                                           71


<210>  631
<211>  71
<212>  DNA
<213>  Artificial sequence

<220>
<223>  oligonucleotide

<400>  631
gtcagcactc ctcaagtact atctgcaaaa aagaacaaga agaatgacag cgatgtaccg      60

gaactggcca c                                                           71


<210>  632
<211>  71
<212>  DNA
<213>  Artificial sequence

<220>
<223>  oligonucleotide

<400>  632
attttgtact aaaagaacca gaccacatcg attcaatcga aatgagagcc aagtggagaa      60

agaagagaac t                                                           71


<210>  633
<211>  71
<212>  DNA
<213>  Artificial sequence

<220>
<223>  oligonucleotide

<400>  633
gaccctatgc atggttcaga tgaagatcaa ttatcaccga ctctcgacga agacatgaat      60

agtgatgacg g                                                           71


<210>  634
<211>  71
<212>  DNA
<213>  Artificial sequence

<220>
<223>  oligonucleotide

<400>  634
ccacaaagaa agtacgctat taaggcttaa ttggaagttc aattatatac tatcattatc      60

tatagttcca a                                                           71


<210>  635
<211>  71
<212>  DNA
<213>  Artificial sequence

<220>
<223>  oligonucleotide

<400>  635
aggtataaga aagaactcaa acaggtttaa tagaattaaa aatgggtttg tttgcctcta      60

agttgttcag t                                                           71


<210>  636
<211>  71
<212>  DNA
<213>  Artificial sequence

<220>
<223>  oligonucleotide

<400>  636
gttttcgccg aaaccttgaa gcatgactag aatttatagt atagatcttt aactttaatt      60

taaatatctt t                                                           71


<210>  637
<211>  71
<212>  DNA

<213>  Artificial sequence

<220>
<223>  oligonucleotide

<400>  637
gctttttgga acgtcgaaaa ggaaattaag caaccagcaa gatgtccact gaattaaccg      60

ttcaatccga g                                                          71


<210>  638
<211>  71
<212>  DNA
<213>  Artificial sequence

<220>
<223>  oligonucleotide

<400>  638
cagtgacggt tcaagatctc cttccgcctt ggaattacaa gtacacgaaa ttcaaggcaa      60

gactttctac g                                                          71


<210>  639
<211>  71
<212>  DNA
<213>  Artificial sequence

<220>
<223>  oligonucleotide

<400>  639
ttcattccaa ctttattggt tccaattgct aaccacttcg gtgctgacaa attgggctac      60

catttcggac c                                                          71


<210>  640
<211>  71
<212>  DNA
<213>  Artificial sequence

<220>
<223>  oligonucleotide

<400>  640
tgctcaaatc tacaagacag ataaggctaa gtatgaagcc accgctaagg agtggactaa      60

aaaatatgct g                                                          71


<210>  641
<211>  71
<212>  DNA
<213>  Artificial sequence

<220>
<223>  oligonucleotide

<400>  641
acatcttctt tcgttcgctt caaaataact acaaattaaa aatgcaattc tctaccgtcg      60

cttctatcgc t                                                          71


<210>  642
<211>  71

<212>  DNA
<213>  Artificial sequence

<220>
<223>  oligonucleotide

<400>  642
ttttcaagtt gtacaacgac atagtcccaa agactgcaga aaacttcaga gctctatgta      60

ccggtgaaaa g                                                          71


<210>  643
<211>  71
<212>  DNA
<213>  Artificial sequence

<220>
<223>  oligonucleotide

<400>  643
ggggtccttg ttggtagtaa ccttgttgtt ggctcattag ttttcgctct tttgacttgt      60

ttcgaagacg g                                                          71


<210>  644
<211>  71
<212>  DNA
<213>  Artificial sequence

<220>
<223>  oligonucleotide

<400>  644
caacaaggtt actaccaaca aggaccccca cagcaaggtt attatcaaca aggtccacca      60

caacaaggtt a                                                          71


<210>  645
<211>  71
<212>  DNA
<213>  Artificial sequence

<220>
<223>  oligonucleotide

<400>  645
tatagacaag tcaaaccaca aataaaccat acacacatac aatgtctgct aaagccgaaa      60

agaaaccagc c                                                          71


<210>  646
<211>  71
<212>  DNA
<213>  Artificial sequence

<220>
<223>  oligonucleotide

<400>  646
aaggctacca aggcttctca agaattataa gatcggttct ggtattttaa agaaggcgga      60

aggaactaaa c                                                          71


<210>  647

<211>  71
<212>  DNA
<213>  Artificial sequence

<220>
<223>  oligonucleotide

<400>  647
agctcagcat gaagctataa ggaatgcaat tactatgtta agaagtgagt atgatgtctt      60

atgtaacgaa a                                                           71


<210>  648
<211>  71
<212>  DNA
<213>  Artificial sequence

<2[?]0>
<223>  oligonucleotide

<400>  648
tggctaaaag aaggcagaaa aagagaacgc atgcgcagat cacacccgag caggaacgag      60

atattcctaa a                                                           71


<210>  649
<211>  71
<212>  DNA
<213>  Artificial sequence

<220>
<223>  oligonucleotide

<400>  649
aagctattac ttctgctggc tctggttgta tggccgcttt ggatgctgag aaatacttaa      60

cttccctaga a                                                           71


<210>  650
<211>  71
<212>  DNA
<213>  Artificial sequence

<220>
<223>  oligonucleotide

<400>  650
gactcaaaga ccacaaacac aaactataac ataattgcaa gatggttgct ttcactgttg      60

accaaatgcg t                                                           71


<210>  651
<211>  71
<212>  DNA
<213>  Artificial sequence

<220>
<223>  oligonucleotide

<400>  651
caaccccaag tgcccaatag aaggataaat caatagtcaa tatgcctcca aagtttgatc      60

caaatgaagt t                                                           71

<210>  652
<211>  71
<212>  DNA
<213>  Artificial sequence

<220>
<223>  oligonucleotide

<400>  652
cctagagaag aataaataga taaagaaaaa agcagataaa aatggtacgt accacgagat      60

gttgatgaag c                                                           71


<210>  653
<211>  71
<212>  DNA
<213>  Artificial sequence

<220>
<223>  oligonucleotide

<400>  653
gttgagatac tagtgaacgt atacacaaga gcggataaaa gatgtcttta gttgtccaag      60

aacaaggttc c                                                           71


<210>  654
<211>  71
<212>  DNA
<213>  Artificial sequence

<220>
<223>  oligonucleotide

<400>  654
ggaccccagg agccgccacc atcaaacctg acgttaaaga ttccaaggag tatttcaaaa      60

atgccaataa t                                                           71


<210>  655
<211>  71
<212>  DNA
<213>  Artificial sequence

<220>
<223>  oligonucleotide

<400>  655
atgcaaccat ctaccgctac cgccgctcca aaagaaaaga ccagcagtga aaagaaggac      60

aactatatta t                                                           71


<210>  656
<211>  71
<212>  DNA
<213>  Artificial sequence

<220>
<223>  oligonucleotide

<400>  656
caatggttct tctccaagtt gagattctaa gaagcttttt tccgaaacct aacttttttc      60

tgtgtactat t                                                           71

<210> 657
<211> 71
<212> DNA
<213> Artificial sequence

<220>
<223> oligonucleotide

<400> 657
accggttctg ctaaggctac ttctgcttaa atttgatatt atctataagt tttgttcttt 60

cctaaaacga a 71


<210> 658
<211> 71
<212> DNA
<213> Artificial sequence

<220>
<223> oligonucleotide

<400> 658
aaatttccac cgctgacttg agaactcaac ttcaaaaaga tattgttgaa aaggtcatac 60

caaaggatat g 71


<210> 659
<211> 71
<212> DNA
<213> Artificial sequence

<220>
<223> oligonucleotide

<400> 659
aaaatataga aaacaacgtt atgcctggct tcggttcctt ctgttttcca aaattgaagg 60

ttctctgcca g 71


<210> 660
<211> 71
<212> DNA
<213> Artificial sequence

<220>
<223> oligonucleotide

<400> 660
ggggaaatat tcatcatgta gaatttcaaa tattttcatt gtaatttcta aaattaatga 60

ggaaaattcg a 71


<210> 661
<211> 71
<212> DNA
<213> Artificial sequence

<220>
<223> oligonucleotide

<400> 661
gctagacaaa gaggtttaca aagattctaa gcgatcacta gcaagccaaa cgagcttgat 60

gttgttttct c 71

<210>  662
<211>  71
<212>  DNA
<213>  Artificial sequence

<220>
<223>  oligonucleotide

<400>  662
tccttcaagg aagctgctag aaccgattaa accggttaac atcatggcat gggatataaa      60

tgaaaaaaga a                                                           71

<210>  663
<211>  71
<212>  DNA
<213>  Artificial sequence

<220>
<223>  oligonucleotide

<400>  663
aaagtacggg attcaaacca ggctctgcaa aaaagggtgc tacattgttt aaaacgaggt      60

gtcagcagtg t                                                           71

<210>  664
<211>  71
<212>  DNA
<213>  Artificial sequence

<220>
<223>  oligonucleotide

<400>  664
gggtctagaa agtcgacaac aagaacaaag gatatacaaa aatgcctcca aagtttgatc      60

ctaatgaagt t                                                           71

<210>  665
<211>  71
<212>  DNA
<213>  Artificial sequence

<220>
<223>  oligonucleotide

<400>  665
aagttatcta ttaacgaaat aacagatcat ctactaacac gatggtaaac ttagctaacg      60

ttttaaccaa c                                                           71

<210>  666
<211>  71
<212>  DNA
<213>  Artificial sequence

<220>
<223>  oligonucleotide

<400>  666
gtccttcttt tctatctttc taggatttct gtctggcctg ctgtacgctg tggctaaaca      60

gaaaaagata c                                                          71

<210>  667
<211>  71
<212>  DNA
<213>  Artificial sequence

<220>
<223>  oligonucleotide

<400>  667
aacggtatta acgtgtagac tgcataacag aacaaccaaa gatggcccaa cgtgttactt      60

tcagaagaag a                                                          71

<210>  668
<211>  71
<212>  DNA
<213>  Artificial sequence

<220>
<223>  oligonucleotide

<400>  668
gatcagtgga aaaacataaa acaataaaaa caatattcgg aatgggattg actactaaac      60

ctctatcttt g                                                          71

<210>  669
<211>  71
<212>  DNA
<213>  Artificial sequence

<220>
<223>  oligonucleotide

<400>  669
gaatacaatc accgcattgc acgtttctga tgaggtatta gtgagtggat ctgaaggttt      60

gctcaatatt t                                                          71

<210>  670
<211>  71
<212>  DNA
<213>  Artificial sequence

<220>
<223>  oligonucleotide

<400>  670
aagaaggttg ctcgtcgtaa cgccgattaa gcaaactaac ttcaaaacgt taaatcattt      60

ttacggattg t                                                          71

<210>  671
<211>  71
<212>  DNA
<213>  Artificial sequence

<220>
<223>  oligonucleotide

<400>  671
ctcccttgga ttgattgctt gcttttgtat ctttcttata atagttcttt cagaagagcc      60

caatatgcgg t                                                          71

<210>  672
<211>  71
<212>  DNA
<213>  Artificial sequence

<220>
<223>  oligonucleotide

<400>  672
ccaaagcgat attaaagata tttatgaaca actaaatcaa acctctgcta agatctcaat     60

tagattgcta t                                                          71

<210>  673
<211>  71
<212>  DNA
<213>  Artificial sequence

<220>
<223>  oligonucleotide

<400>  673
cctgcctttc cctttccaga tgctggcatc gaaagtaagg catgtatttg tatgtaagtt     60

ttttaattaa t                                                          71

<210>  674
<211>  71
<212>  DNA
<213>  Artificial sequence

<220>
<223>  oligonucleotide

<400>  674
tcttggtagt caggaaaagg ttttcatatt catcagacat aatgaatttc aaggagccgc     60

tagtgaattt t                                                          71

<210>  675
<211>  71
<212>  DNA
<213>  Artificial sequence

<220>
<223>  oligonucleotide

<400>  675
cgatcaggtg gatatcagcc ctacgattgc agaagctatc agaagattgc ataatggaga     60

atcagtctca t                                                          71

<210>  676
<211>  71
<212>  DNA
<213>  Artificial sequence

<220>
<223>  oligonucleotide

<400>  676

cagtggataa atcaacctac ggcggaaaat tcggatttga aggaaaaaat gaactgcaag     60

aatacgctca a                                                          71


<210>  677
<211>  71
<212>  DNA
<213>  Artificial sequence

<220>
<223>  oligonucleotide

<400>  677
tttcgaaata atcgagaaat cacgataata atacagcaaa aatgtcaggt aacggtgctc     60

aaggtacaaa a                                                          71


<210>  678
<211>  71
<212>  DNA
<213>  Artificial sequence

<220>
<223>  oligonucleotide

<400>  678
ccagcagctg ccgcaaagaa agctttgtaa atatgatgag agaataatat aaatcaaacg     60

ctcgctgatt a                                                          71


<210>  679
<211>  71
<212>  DNA
<213>  Artificial sequence

<220>
<223>  oligonucleotide

<400>  679
gctgccgccg aaggtgaagc accaaagtaa gtattctgat aaatctaaag agaaattact     60

aaaaaaaaga a                                                          71


<210>  680
<211>  71
<212>  DNA
<213>  Artificial sequence

<220>
<223>  oligonucleotide

<400>  680
acgcataaag aatcaaagaa aggcaaaaat tacgctgtac gatggggtcg tttcccttag     60

ctgagtttcc a                                                          71


<210>  681
<211>  71
<212>  DNA
<213>  Artificial sequence

<220>
<223>  oligonucleotide

<400> 681
ggaccgatct ttgcaagtat atatctgtat gtatccatat ttagatggca gcaagcaata 60

tagatttgat g 71


<210> 682
<211> 71
<212> DNA
<213> Artificial sequence

<220>
<223> oligonucleotide

<400> 682
tcttaaatgt atgtatgtgt gtgtgtgtgt gtgtgcgtat tgttctattt cactccagct 60

taaacatggc g 71


<210> 683
<211> 71
<212> DNA
<213> Artificial sequence

<220>
<223> oligonucleotide

<400> 683
aacagaatgt gcatttggat gagcgggtaa cagatggaag tacgctccaa aagaagggta 60

atatgttctg c 71


<210> 684
<211> 71
<212> DNA
<213> Artificial sequence

<220>
<223> oligonucleotide

<400> 684
taaaaaaaag cagaacatat tacccttctt ttggagcgta cttccatctg ttacccgctc 60

atccaaatgc a 71


<210> 685
<211> 71
<212> DNA
<213> Artificial sequence

<220>
<223> oligonucleotide

<400> 685
ggcaaaacca atacaaattg gttttctatc aagtcacccc tgaagatgct gacgaagaag 60

aggatgacga a 71


<210> 686
<211> 71
<212> DNA
<213> Artificial sequence

<220>
<223> oligonucleotide

<400> 686
ccatttctga gcaaactaag aaaccattag atcaataagc aatgggtatg tcagcaccgt 60

gatttaacaa c 71


<210> 687
<211> 71
<212> DNA
<213> Artificial sequence

<220>
<223> oligonucleotide

<400> 687
aagccgccgc taagggtttg agagatatct atggatggac tggtgacgca agcaaagatc 60

caattacgat t 71


<210> 688
<211> 71
<212> DNA
<213> Artificial sequence

<220>
<223> oligonucleotide

<400> 688
ttccaaaagg ttgctgctac ttctcgttaa gatttatgct cgaacttatt atgtacaatg 60

aatatttttc t 71


<210> 689
<211> 71
<212> DNA
<213> Artificial sequence

<220>
<223> oligonucleotide

<400> 689
aagcacggca aagagatcaa ggttgtcttg cacatagctt ctccggttca cttcaacacc 60

actgatttcg a 71


<210> 690
<211> 71
<212> DNA
<213> Artificial sequence

<220>
<223> oligonucleotide

<400> 690
catataaatc caaataacca accaagcaaa ttaagatcac aatggccgct gagtatgtat 60

acgaaatcgt c 71


<210> 691
<211> 71
<212> DNA
<213> Artificial sequence

<220>

<223>  oligonucleotide

<400>  691
caactcaagc caggccaacc aatgtactga aaaatgaccc taaactactt ctaaaccctc      60

tcgatttctt t                                                           71


<210>  692
<211>  71
<212>  DNA
<213>  Artificial sequence

<220>
<223>  oligonucleotide

<400>  692
tccatgggtc cagctttcag attgtactaa tcacttccga gcgattaata catatctcca      60

tctttttaaa t                                                           71


<210>  693
<211>  71
<212>  DNA
<213>  Artificial sequence

<220>
<223>  oligonucleotide

<400>  693
aaagcttctg aaatcaagaa gcccgtacca gaagttcaat catgaaatac atccaaactg      60

aacaacaaat c                                                           71


<210>  694
<211>  71
<212>  DNA
<213>  Artificial sequence

<220>
<223>  oligonucleotide

<400>  694
gcgaccggtc caagcttttt agaaaaaaac tcttgcaagt ttgacaattc taagacaaaa      60

aaactactgg g                                                           71


<210>  695
<211>  71
<212>  DNA
<213>  Artificial sequence

<220>
<223>  oligonucleotide

<400>  695
cctgctgatg ccgccaagaa ggctttataa cttgattaat ataggtgaaa cataagtaaa      60

tttttctact a                                                           71


<210>  696
<211>  71
<212>  DNA
<213>  Artificial sequence

<220>
<223>  oligonucleotide

<400>  696
ggtaagtccg ttggtatcat cggtgcttaa acttaatttg taaattaagt ttgaacaaca      60

agaaggtgcc c                                                          71


<210>  697
<211>  71
<212>  DNA
<213>  Artificial sequence

<220>
<223>  oligonucleotide

<400>  697
gtttgtccac cagaagctgg ttttgattaa tcttttctca catcctcact tatagatatg      60

ttttgattat t                                                          71


<210>  698
<211>  71
<212>  DNA
<213>  Artificial sequence

<220>
<223>  oligonucleotide

<400>  698
gaagagctct gcacacatcc tatgcgtcaa gcttataata cccttgattc aaactcaaga      60

ataattatca c                                                          71


<210>  699
<211>  71
<212>  DNA
<213>  Artificial sequence

<220>
<223>  oligonucleotide

<400>  699
cgactttctt caatgacata tacgtattag aaggaaccta tggtacggac gttgtctctt      60

taaagaattt t                                                          71


<210>  700
<211>  71
<212>  DNA
<213>  Artificial sequence

<220>
<223>  oligonucleotide

<400>  700
atctacacca gagctactgc ttctgaataa acagggaacg ggttatataa ttttaaataa      60

tctgtatttt c                                                          71


<210>  701
<211>  71
<212>  DNA
<213>  Artificial sequence

<220>
<223>  oligonucleotide

<400>  701
atccaaagaa agggtggtaa attggaataa actagctcga ctttaacata gatcatacaa      60

aaaatacaat a                                                           71


<210>  702
<211>  71
<212>  DNA
<213>  Artificial sequence

<220>
<223>  oligonucleotide

<400>  702
tacccttgaa agcccaacat atacaaaaat acgcgtccaa gatgtctact aaagcccaaa      60

accctatgcg t                                                           71


<210>  703
<211>  71
<212>  DNA
<213>  Artificial sequence

<220>
<223>  oligonucleotide

<400>  703
aaagaaaaga aggaaaagcc aagatcataa gcacgaaagt tatcctctcg aggactacat      60

tgtttttgta c                                                           71


<210>  704
<211>  71
<212>  DNA
<213>  Artificial sequence

<220>
<223>  oligonucleotide

<400>  704
tcaccttgtg gtttttcaca gctttttaat ttagaagaac aatgaaggtt gaagttgatt      60

ccttttccgg c                                                           71


<210>  705
<211>  71
<212>  DNA
<213>  Artificial sequence

<220>
<223>  oligonucleotide

<400>  705
gaggtatagc ttagaaaata gcaaactaaa tagagagcga aatgtcctta ccagctactt      60

ttgacttgac t                                                           71


<210>  706
<211>  71
<212>  DNA

<213>  Artificial sequence

<220>
<223>  oligonucleotide

<400>  706
gaaaacttcc accacggtga caaattataa agcttttgat taagccttct agtccaaaaa      60

acacgttttt t                                                          71


<210>  707
<211>  71
<212>  DNA
<213>  Artificial sequence

<220>
<223>  oligonucleotide

<400>  707
ttaaagtaac ttcaggaaat aaacgcgcaa ataaaccaaa aatgtccact gattctattg      60

ttaaagcctc c                                                          71


<210>  708
<211>  71
<212>  DNA
<213>  Artificial sequence

<220>
<223>  oligonucleotide

<400>  708
ttgcttaccc tctattatca catcaaaaca actaattcga aatggcccca ggtaagaaag      60

tcgctccagc t                                                          71


<210>  709
<211>  71
<212>  DNA
<213>  Artificial sequence

<220>
<223>  oligonucleotide

<400>  709
agtctcaacg tgttaaaaca aaagcataac caaagaaaaa aatggctaag ttcttgaaag      60

ctggtaaagt t                                                          71


<210>  710
<211>  71
<212>  DNA
<213>  Artificial sequence

<220>
<223>  oligonucleotide

<400>  710
tctgaaggta cttctttcag aagaaagtaa gttaatttat ttttttgtgt agttaaaatc      60

atcttctttt a                                                          71


<210>  711
<211>  71

<212>  DNA
<213>  Artificial sequence

<220>
<223>  oligonucleotide

<400>  711
tttcagtaga tctttgtctt tttgacctag atgggactat tgtcagcaca acaactgcag      60

cggaaagtgc c                                                          71


<210>  712
<211>  71
<212>  DNA
<213>  Artificial sequence

<220>
<223>  oligonucleotide

<400>  712
tcagctgatc tatgtctttt tgacctagat ggtaccatag tgagtacaac agtggccgca      60

gagaaagcat g                                                          71


<210>  713
<211>  71
<212>  DNA
<213>  Artificial sequence

<220>
<223>  oligonucleotide

<400>  713
gatagtgact tatttagtga ggtcgaataa attgttctcc ggtaccaaga aaattttagt      60

taagcctggc t                                                          71


<210>  714
<211>  71
<212>  DNA
<213>  Artificial sequence

<220>
<223>  oligonucleotide

<400>  714
gtaatgtgga gaacgattta gaagtaaatg gactatttta cgcaataggt cacagccctg      60

ccacagatat a                                                          71


<210>  715
<211>  71
<212>  DNA
<213>  Artificial sequence

<220>
<223>  oligonucleotide

<400>  715
cgatcctctg taatatttct atgaataatt atcacagcaa cgttcaatta tcttcaattc      60

ggcttcagta c                                                          71


<210>  716

<211>  71
<212>  DNA
<213>  Artificial sequence

<220>
<223>  oligonucleotide

<400>  716
acgtaaattg aaggagatta agcaaaaaaa caatcagtaa taatgggtat gtggacgatt      60

aggaatagac a                                                           71


<210>  717
<211>  71
<212>  DNA
<213>  Artificial sequence

<220>
<223>  oligonucleotide

<400>  717
gtcgactaag tataacaata gatacaccac tattgaggaa agatggctag aggaccgtat      60

gttgatttcc a                                                           71


<210>  718
<211>  71
<212>  DNA
<213>  Artificial sequence

<220>
<223>  oligonucleotide

<400>  718
ctacgtggtt ctcaaaagac ccaagattaa tctttttaat tttggtttct tccttctgtc      60

atattatttt a                                                           71


<210>  719
<211>  71
<212>  DNA
<213>  Artificial sequence

<220>
<223>  oligonucleotide

<400>  719
tcggttatca tactaatggg gaacgggtga tagaactttt accaatgaaa agaaatcagc      60

aggccataat t                                                           71


<210>  720
<211>  71
<212>  DNA
<213>  Artificial sequence

<220>
<223>  oligonucleotide

<400>  720
gattgaaact gagaatagat tagcaacgaa caaccctaaa aatggctcaa cgtgttactt      60

tcagaagaag a                                                           71

<210>  721
<211>  71
<212>  DNA
<213>  Artificial sequence

<220>
<223>  oligonucleotide

<400>  721
tgaaacgttt caatgtttta aaatatatca gaacaacaaa agcaaatata caaaccatcg      60

caatgccttt g                                                           71


<210>  722
<211>  71
<212>  DNA
<213>  Artificial sequence

<220>
<223>  oligonucleotide

<400>  722
gaatgagtgg ttttctcaga ccctgaacgt gaccgtccat catgaatttc tccacagttt      60

ttcaagcaat a                                                           71


<210>  723
<211>  71
<212>  DNA
<213>  Artificial sequence

<220>
<223>  oligonucleotide

<400>  723
ctcactgcaa acgctaaggt acctacagaa gatcaataaa aatggtatgt cgaacttgta      60

ctgaaccagc a                                                           71


<210>  724
<211>  71
<212>  DNA
<213>  Artificial sequence

<220>
<223>  oligonucleotide

<400>  724
aaaaagtaac aaacactgat ttcgggtatt tcctccctaa catgtcttat tcagctgccg      60

ataatttaca a                                                           71


<210>  725
<211>  71
<212>  DNA
<213>  Artificial sequence

<220>
<223>  oligonucleotide

<400>  725
gctgggcctc gaaccgcatc cattgaacaa cagcttggcc ttccctgtat tctaccacat      60

ccattgctat t                                                           71

<210> 726
<211> 71
<212> DNA
<213> Artificial sequence

<220>
<223> oligonucleotide

<400> 726
atatatatgt tgaagtgtgt acacacgtac acgcacaagc tacagacgcc acgcggcccg 60

gcattcatat a 71


<210> 727
<211> 71
<212> DNA
<213> Artificial sequence

<220>
<223> oligonucleotide

<400> 727
atttctcttt gaaaacggaa ggtgaagaac aaatcgagaa aatgtctgtt gaaccagttg 60

ttgtcattga t 71


<210> 728
<211> 71
<212> DNA
<213> Artificial sequence

<220>
<223> oligonucleotide

<400> 728
gaatacgcat tggcattgaa cagaacacct ttcatatatg ataactttcc aatggaatta 60

aaaaatcagg g 71


<210> 729
<211> 71
<212> DNA
<213> Artificial sequence

<220>
<223> oligonucleotide

<400> 729
gttggggaag aaaaacaaac tttacatcaa atatttgcgg attccatggt tattaaagga 60

tatagcaccg g 71


<210> 730
<211> 71
<212> DNA
<213> Artificial sequence

<220>
<223> oligonucleotide

<400> 730
cctgtaaaaa tcaagtacag gaatgtaatc gcacatactt tagatgagaa tgaatgcgcc 60

ccagtccctc c 71

<210> 731
<211> 71
<212> DNA
<213> Artificial sequence

<220>
<223> oligonucleotide

<400> 731
ctagtctcgt tgttcattgt gttagcctga tcacatcgag cgagctatcc tgacgctatt 60

ctctcttgtt t 71


<210> 732
<211> 71
<212> DNA
<213> Artificial sequence

<220>
<223> oligonucleotide

<400> 732
gacgacgaag acgttactga tcaagaataa ctacacaagc acattgcata gaaggatttt 60

tcttctctct c 71


<210> 733
<211> 71
<212> DNA
<213> Artificial sequence

<220>
<223> oligonucleotide

<400> 733
aaacgcgatc tcagaaatgt cagaatatga tgcctggcat tatttgcaga agttctcttc 60

ctgtgttaat a 71


<210> 734
<211> 71
<212> DNA
<213> Artificial sequence

<220>
<223> oligonucleotide

<400> 734
ccatcactgt ttttttcgtg gtctttctat atataaacaa agcctctgta aagttttttt 60

ttttcgcata a 71


<210> 735
<211> 71
<212> DNA
<213> Artificial sequence

<220>
<223> oligonucleotide

<400> 735
ttgaagaacg tttggtctta ctcccgttaa aaagataaat attgaataaa aagaagaggg 60

gaagtcgttt a                                                          71

<210>  736
<211>  71
<212>  DNA
<213>  Artificial sequence

<220>
<223>  oligonucleotide

<400>  736
tcttggtgaa tcaaaaaatt aacgaaacga acaaatttaa aatggcaaga tacggtgcta      60

cttcaaccaa c                                                          71

<210>  737
<211>  71
<212>  DNA
<213>  Artificial sequence

<220>
<223>  oligonucleotide

<400>  737
tctggtaaga ttttgggttt cgtttactaa gctacttgtt acaacaacct tttcggtagc      60

tttatatact a                                                          71

<210>  738
<211>  71
<212>  DNA
<213>  Artificial sequence

<220>
<223>  oligonucleotide

<400>  738
aagggtggta gaagaggtag aagattatga tttctttttt ttttactcta ttaacgcatg      60

gaaatacggc t                                                          71

<210>  739
<211>  71
<212>  DNA
<213>  Artificial sequence

<220>
<223>  oligonucleotide

<400>  739
tccttcaagg aagctccaag atctgattag atattttaga tttctatagc ttacaaaata      60

cgcgatgttt t                                                          71

<210>  740
<211>  71
<212>  DNA
<213>  Artificial sequence

<220>
<223>  oligonucleotide

<400>  740
gactgaattc aaggccggtt ctgctaagaa aggtgctaca cttttcaaga ctagatgtct      60

acaatgccac a                                                          71

<210>  741
<211>  71
<212>  DNA
<213>  Artificial sequence

<220>
<223>  oligonucleotide

<400>  741
agaagattaa gagaaatggt tgaagcttaa acttctttca ttcattttct cttggctttc      60

actaggtata t                                                          71

<210>  742
<211>  71
<212>  DNA
<213>  Artificial sequence

<220>
<223>  oligonucleotide

<400>  742
atttttctgc ccaaaacaaa ccaaagaatc aatacgcaaa gatggctaga ggaccgtatg      60

tttgactata g                                                          71

<210>  743
<211>  71
<212>  DNA
<213>  Artificial sequence

<220>
<223>  oligonucleotide

<400>  743
gaaatttaag ggaaagcatc tccacgagtt ttaagaacga tatgaccttg gcacccctag      60

acgcctccaa a                                                          71

<210>  744
<211>  71
<212>  DNA
<213>  Artificial sequence

<220>
<223>  oligonucleotide

<400>  744
gtcttagtga acaaagagag tcgtgaaaaa taaaataaac aatgtccacc gattctattg      60

tcaaggcttc t                                                          71

<210>  745
<211>  71
<212>  DNA
<213>  Artificial sequence

<220>
<223>  oligonucleotide

<400>  745

cttctcatca acacagagga tatttatact gaaactttaa atgaaactct cgcagaattt 60

ggtaagggac c 71

<210> 746
<211> 71
<212> DNA
<213> Artificial sequence

<220>
<223> oligonucleotide

<400> 746
atttctcaaa tcactgacgg tcaaatccaa gctactacca ctgctaccac cgaagctacc 60

accactgctg c 71

<210> 747
<211> 71
<212> DNA
<213> Artificial sequence

<220>
<223> oligonucleotide

<400> 747
ctgccccagt gactttgaaa tacagaaggg ttatcgatca cactttggac gaaaaggaat 60

gtccttccgt a 71

<210> 748
<211> 71
<212> DNA
<213> Artificial sequence

<220>
<223> oligonucleotide

<400> 748
tctgagggta catctttcag aagaaagtaa tttcattact agacagatga aacgaaattt 60

acttcaaaag a 71

<210> 749
<211> 71
<212> DNA
<213> Artificial sequence

<220>
<223> oligonucleotide

<400> 749
agagcaaaca aagagtcctc aagaactact aatagattaa aatggctaga tacggtgcta 60

cttccactaa c 71

<210> 750
<211> 71
<212> DNA
<213> Artificial sequence

<220>
<223> oligonucleotide

<400>  750
ttaaagaacg tctggtctta ctcacgttaa gagaaaagaa aaagaggatt cgatttaagc      60

ttcatatcgt t                                                           71


<210>  751
<211>  71
<212>  DNA
<213>  Artificial sequence

<220>
<223>  oligonucleotide

<400>  751
aaaagacctg gtgtggaaga atttttggaa agagtcggaa aattattcga ggttgtagtc      60

ttcacggcta g                                                           71


<210>  752
<211>  71
<212>  DNA
<213>  Artificial sequence

<220>
<223>  oligonucleotide

<400>  752
tctgtttttc aatacagtga acacgataac tgaaagaaaa gatggctcca ggtaagaaag      60

ttgctccagc t                                                           71


<210>  753
<211>  71
<212>  DNA
<213>  Artificial sequence

<220>
<223>  oligonucleotide

<400>  753
ttacaatcta agaaaaccac gatcaaacaa ataaatcagc aatgggtgcc tacaaatatt      60

tggaagaatt g                                                           71


<210>  754
<211>  71
<212>  DNA
<213>  Artificial sequence

<220>
<223>  oligonucleotide

<400>  754
gaagaaaatg cagacaacgt tgaatggtaa tcatttgaaa accttgcaat gtgtgtgaat      60

attgtaagga a                                                           71


<210>  755
<211>  71
<212>  DNA
<213>  Artificial sequence

<220>
<223>  oligonucleotide

<400>  755
caagaccaac gaatacagat tggccttcta ccaagtcact ccagaagaag acgaagaaga      60

agacgaagaa t                                                           71


<210>  756
<211>  71
<212>  DNA
<213>  Artificial sequence

<220>
<223>  oligonucleotide

<400>  756
attgaccact gaagccccaa agaacggtac ttctactgct gctccagtta cctctactga      60

agctccaaag a                                                           71


<210>  757
<211>  71
<212>  DNA
<213>  Artificial sequence

<220>
<223>  oligonucleotide

<400>  757
   aattgtaaca taccggcttg ctgtgtgtga acccctttcc ccaattgtat atcagaaacg      60
   tctgagtgga g                                                           71


<210>  758
<211>  71
<212>  DNA
<213>  Artificial sequence

<220>
<223>  oligonucleotide

<400>  758
taatgcaaaa gcggctccta aacagaaatt cttcagtagt caaatctcga aattgccttg      60

ctcgtcatca a                                                           71


<210>  759
<211>  71
<212>  DNA
<213>  Artificial sequence

<220>
<223>  oligonucleotide

<400>  759
acgaaatcac gaccgaggac ttaagagcgc aactaaagtc cgagatcatt gaaaaagtca      60

tcccaagaga c                                                           71


<210>  760
<211>  71
<212>  DNA
<213>  Artificial sequence

<220>

<223>  oligonucleotide

<400>  760
accggctctg cttctaaggc ttctgcttaa tttttatact aagatctatt tctttaaatg     60

aaaactcaat a                                                          71


<210>  761
<211>  71
<212>  DNA
<213>  Artificial sequence

<220>
<223>  oligonucleotide

<400>  761
tctgaacgtg aagctcgtcg tttgcgttaa aggtatatta aaaaatttac tattggtgct     60

cttcgagcat t                                                          71


<210>  762
<211>  71
<212>  DNA
<213>  Artificial sequence

<220>
<223>  oligonucleotide

<400>  762
agaatgaacc caggtccatc cgtccaataa gaaaattaac agttgtggaa aaggagctta     60

aaagtaacaa g                                                          71


<210>  763
<211>  71
<212>  DNA
<213>  Artificial sequence

<220>
<223>  oligonucleotide

<400>  763
tctgccccag ctgctaacgg tgaagttcca acattcaaac tagttcttgt cggtgatggt     60

ggtactggta a                                                          71


<210>  764
<211>  71
<212>  DNA
<213>  Artificial sequence

<220>
<223>  oligonucleotide

<400>  764
atctatacca gagctgctgc ttccgaataa atataaaatc aagaaataag gtaataagtt     60

gtttgtcttc t                                                          71


<210>  765
<211>  71
<212>  DNA
<213>  Artificial sequence

<220>
<223>  oligonucleotide

<400>  765
ggtaggcttc ttcaacaaac ggttgtgcgc tttttagtcg tatgctttgt ctcatatcaa      60

aaactttctt c                                                           71


<210>  766
<211>  71
<212>  DNA
<213>  Artificial sequence

<220>
<223>  oligonucleotide

<400>  766
atccaaagaa agggcggtaa gttggaataa attatgtaat tccctgaaaa cttcctcgaa      60

atttattgca c                                                           71


<210>  767
<211>  71
<212>  DNA
<213>  Artificial sequence

<220>
<223>  oligonucleotide

<400>  767
gtataatact aacgtttcaa aacatagtaa ttaggtaaaa aatgactcgc tcttccgttt      60

tagctgatgc t                                                           71


<210>  768
<211>  71
<212>  DNA
<213>  Artificial sequence

<220>
<223>  oligonucleotide

<400>  768
attcatttgt actaacaacg tttcctatta ttgcagataa aatggctcac gaaaacgtct      60

ggttctctca c                                                           71


<210>  769
<211>  71
<212>  DNA
<213>  Artificial sequence

<220>
<223>  oligonucleotide

<400>  769
atatttcaat tgcacgagca gataatccaa agtacttgaa aatggccggt ttaaaagacg      60

ttgtcactcg t                                                           71


<210>  770
<211>  71
<212>  DNA
<213>  Artificial sequence

<220>
<223>  oligonucleotide

<400>  770
ggcctggcca ggcaaaaaag gacgcagcac gcctcgagcg ttatttccaa atcgggcgta      60

ctatcagcca a                                                           71


<210>  771
<211>  71
<212>  DNA
<213>  Artificial sequence

<220>
<223>  oligonucleotide

<400>  771
aacccttttt cctcttcgtt gatcctaaaa aaagcgcgtc tggaaaacaa ggcgaagctt      60

cctcacatgg a                                                           71


<210>  772
<211>  71
<212>  DNA
<213>  Artificial sequence

<220>
<223>  oligonucleotide

<400>  772
aaggaaacaa aacaagcaca cgaataacca atcgtttaat catggctgtc ggaaagaata      60

agagactatc c                                                           71


<210>  773
<211>  71
<212>  DNA
<213>  Artificial sequence

<220>
<223>  oligonucleotide

<400>  773
ggtgacaagc cacacctatt gaaattctaa attgaactga aaaaattata aatgatttaa      60

aatagaattt c                                                           71


<210>  774
<211>  71
<212>  DNA
<213>  Artificial sequence

<220>
<223>  oligonucleotide

<400>  774
agagacagac gttacagaaa gagagtttaa aattaaatta gaaagctatt taaaataatt      60

tactattcaa a                                                           71


<210>  775
<211>  71
<212>  DNA

<213>  Artificial sequence

<220>
<223>  oligonucleotide

<400>  775
accgatctta cgaaaagcca atagagcaga aagtggtaaa gatgtcttta gttgtacaag      60

aacaaggttc c                                                           71


<210>  776
<211>  71
<212>  DNA
<213>  Artificial sequence

<220>
<223>  oligonucleotide

<400>  776
ctccaggtgc tgctaccatc aagccaaccg ttgaagactc caaggaatac ttcgaagctg      60

ccaacaaata a                                                           71


<210>  777
<211>  71
<212>  DNA
<213>  Artificial sequence

<220>
<223>  oligonucleotide

<400>  777
gcctttgagg aaaccaagca aaaaaccgat ctagactaat catggctgtt ggtaagaata      60

agagactatc c                                                           71


<210>  778
<211>  71
<212>  DNA
<213>  Artificial sequence

<220>
<223>  oligonucleotide

<400>  778
ttacattagg cgaaacaaca aagtaatagc catccaacga aatgagtgcc caaaaagtat      60

gttcctacat g                                                           71


<210>  779
<211>  71
<212>  DNA
<213>  Artificial sequence

<220>
<223>  oligonucleotide

<400>  779
gactcatacg ctgaggaaga ggaattttaa ggttcactct cgcccccaac ctccttatcc      60

tttctatatt t                                                           71


<210>  780
<211>  71

<212>  DNA
<213>  Artificial sequence

<220>
<223>  oligonucleotide

<400>  780
gactctttta gtagatttgt agagcttttc ctggcataca ataagcaaag actttatttc      60

cgctcgagtc c                                                          71


<210>  781
<211>  71
<212>  DNA
<213>  Artificial sequence

<220>
<223>  oligonucleotide

<400>  781
aaactgacaa aaaattgtgt cagagttgaa gctatagctc cttttatgat gtgtcttggt      60

tctttgggtg c                                                          71


<210>  782
<211>  71
<212>  DNA
<213>  Artificial sequence

<220>
<223>  oligonucleotide

<400>  782
gggtcaggta gttggctcca taattgctct tgatagacag gaagttgtga gtactgacga      60

taaagaagga c                                                          71


<210>  783
<211>  71
<212>  DNA
<213>  Artificial sequence

<220>
<223>  oligonucleotide

<400>  783
ggtgccgatt cttacgctga ggagttctaa gaaagtggct ctgatcccga tacccactta      60

tagatttata t                                                          71


<210>  784
<211>  71
<212>  DNA
<213>  Artificial sequence

<220>
<223>  oligonucleotide

<400>  784
gctaccgtaa agacagtcac aaattaccta ccacacaagc aatgggagcc tacaaatatt      60

tagaagaatt a                                                          71


<210>  785

<211>  71
<212>  DNA
<213>  Artificial sequence

<220>
<223>  oligonucleotide

<400>  785
aggaaataat ttcggaaaag taaagcagga atcaagtaac aatgggtatg ttgaactgaa      60

gcaataagag c                                                          71


<210>  786
<211>  71
<212>  DNA
<213>  Artificial sequence

<220>
<223>  oligonucleotide

<400>  786
cgttccagat tccaaaaatc ttaccgttaa gaaattgtgg ggattatttt tcgctctttt      60

catttcttta t                                                          71


<210>  787
<211>  71
<212>  DNA
<213>  Artificial sequence

<220>
<223>  oligonucleotide

<400>  787
tgctaaacaa gacttagccc agattataga acttacaaga tactatgcgg gggcggtcga      60

caagttcaat a                                                          71


<210>  788
<211>  71
<212>  DNA
<213>  Artificial sequence

<220>
<223>  oligonucleotide

<400>  788
ccaaaggtga tttggcacaa attttacagc ttaccagata ttttgctggg tccgctgata      60

agtttgacaa a                                                          71


<210>  789
<211>  71
<212>  DNA
<213>  Artificial sequence

<220>
<223>  oligonucleotide

<400>  789
aacgctacag aaccaataga aaaatagaat cattctgaaa tatggctggt gaaacttttg      60

aatttcaagc t                                                          71

<210> 790
<211> 71
<212> DNA
<213> Artificial sequence

<220>
<223> oligonucleotide

<400> 790
aacatcattg ctgcttctcg tcgtcactaa gtttctaaaa actgattttt gtaatatata 60

tatagatatc a 71


<210> 791
<211> 71
<212> DNA
<213> Artificial sequence

<220>
<223> oligonucleotide

<400> 791
actgaaacca agaaaacaca gatcataact aaccgttcaa gatgttgatg ccaaagcaag 60

aaagaaacaa a 71


<210> 792
<211> 71
<212> DNA
<213> Artificial sequence

<220>
<223> oligonucleotide

<400> 792
tcctacaaga gaccttccac tttctactga actgtgcata tttgaatttt aagatataaa 60

atatttttct g 71


<210> 793
<211> 71
<212> DNA
<213> Artificial sequence

<220>
<223> oligonucleotide

<400> 793
gggtagtggg ttgtattgtt gctttagata ggcaagaagt gattcatgaa tctgatccgg 60

aaagaacaag t 71


<210> 794
<211> 71
<212> DNA
<213> Artificial sequence

<220>
<223> oligonucleotide

<400> 794
aagtttcttc ttttttttat attctggcat tgtcccgctt tttcaacgca ggaagggaac 60

gagcttgtga t 71

<210> 795
<211> 71
<212> DNA
<213> Artificial sequence

<220>
<223> oligonucleotide

<400> 795
gtaaaagaaa acggggaaat ttatcgtttc gtagtaaata gatgatggac ttgggcgaca 60

aaataaatcc t 71

<210> 796
<211> 71
<212> DNA
<213> Artificial sequence

<220>
<223> oligonucleotide

<400> 796
ggtagaacct tatatggttt cggtggttaa acaatcggtg gttaaacaat cggtgtttga 60

aattattttc a 71

<210> 797
<211> 71
<212> DNA
<213> Artificial sequence

<220>
<223> oligonucleotide

<400> 797
ttcttcccct tttactaaag gatccaagca aacactccac aatggccaga actaaacaaa 60

cagctagaaa a 71

<210> 798
<211> 71
<212> DNA
<213> Artificial sequence

<220>
<223> oligonucleotide

<400> 798
cacaagggtt tcattgtcga agacatgtaa atggaacaaa aaagtgtctt tgcgcttaga 60

ggcgcttaaa t 71

<210> 799
<211> 71
<212> DNA
<213> Artificial sequence

<220>
<223> oligonucleotide

<400> 799
gcattttgtt aaggtattaa acaaccaacc aaccataaaa gatgtctcaa ccagtcgttg 60

ttattgatgg t 71

<210>  800
<211>  71
<212>  DNA
<213>  Artificial sequence

<220>
<223>  oligonucleotide

<400>  800
caagatctta tcccaagctc caagtgagtt ggaattacaa gtcgccaaga ccttcatcga      60

tctagaaagc t                                                           71


<210>  801
<211>  71
<212>  DNA
<213>  Artificial sequence

<220>
<223>  oligonucleotide

<400>  801
ctaaaggaca cgggagaaaa ggtcacccca gaattaaccg aaaagattct aaaagaacaa      60

gtggaaagac t                                                           71


<210>  802
<211>  71
<212>  DNA
<213>  Artificial sequence

<220>
<223>  oligonucleotide

<400>  802
accgatcacc actgcttcca cacaagccac tcagaaggat aaatcctctg aaaagaaaga      60

caactatata a                                                           71


<210>  803
<211>  71
<212>  DNA
<213>  Artificial sequence

<220>
<223>  oligonucleotide

<400>  803
aagcaaaagg gtcaagcttt gcaattctga atctctttat gggtttcgct tttgtatttt      60

ctttgcttta a                                                           71


<210>  804
<211>  71
<212>  DNA
<213>  Artificial sequence

<220>
<223>  oligonucleotide

<400>  804
agaaatcacc actggaaact aagacagtag atgctcaaac gagaagggac tactggctct      60

ctaaaggcca g                                                          71


<210>  805
<211>  71
<212>  DNA
<213>  Artificial sequence

<220>
<223>  oligonucleotide

<400>  805
agaagaagat ctaagggttt caaggtgtaa tctagtatgg tttgaaacct tacaattttt     60

cttctttgtt c                                                          71


<210>  806
<211>  71
<212>  DNA
<213>  Artificial sequence

<220>
<223>  oligonucleotide

<400>  806
tgaatattga caacaaaggt atagaaccaa agataataaa gatggcaggt gtttccgtta     60

ggtacgtata a                                                          71


<210>  807
<211>  71
<212>  DNA
<213>  Artificial sequence

<220>
<223>  oligonucleotide

<400>  807
tacactgtat tgtttggtgt tgccagagct attggtgtgt taccccaatt aatcatcgat     60

agggctgttg g                                                          71


<210>  808
<211>  71
<212>  DNA
<213>  Artificial sequence

<220>
<223>  oligonucleotide

<400>  808
gaattctaga tgcctataag gacaaggaat ccgaccttgg aaatgcgcta gggttttacc     60

tcctaggatg g                                                          71


<210>  809
<211>  71
<212>  DNA
<213>  Artificial sequence

<220>
<223>  oligonucleotide

<400>  809
ggatcttaaa gtcaagagga ttaccccaag acatctgcaa ttggccatta gaggtgacga     60

tgagttagat t                                                          71


<210>  810
<211>  71
<212>  DNA
<213>  Artificial sequence

<220>
<223>  oligonucleotide

<400>  810
gatccatcac aagaagagat catcaagctt acggattcac aatgggtatc gatcacactt     60

ccaagcaaca c                                                          71


<210>  811
<211>  71
<212>  DNA
<213>  Artificial sequence

<220>
<223>  oligonucleotide

<400>  811
agcttgaaag cgaagtgata cgatcgacaa atagagtaaa aatgccaggt gtttccgtta     60

ggtacgtaaa a                                                          71


<210>  812
<211>  71
<212>  DNA
<213>  Artificial sequence

<220>
<223>  oligonucleotide

<400>  812
actgagtcaa gattaacatg gctctcatta ctaattatgg gggtaatggt tggtatcagt     60

atagtgcagg c                                                          71


<210>  813
<211>  71
<212>  DNA
<213>  Artificial sequence

<220>
<223>  oligonucleotide

<400>  813
gtctgctcca caagccaaga ttttgtctca agctccaact gaattggaat tacaagttgc     60

tcaagctttc g                                                          71


<210>  814
<211>  71
<212>  DNA
<213>  Artificial sequence

<220>
<223>  oligonucleotide

<400>  814

gccagaccta cgcacccaag aatttttaat aacagataaa aatggttgct ttcactgttg     60

accaaatgcg t                                                          71


<210>  815
<211>  71
<212>  DNA
<213>  Artificial sequence

<220>
<223>  oligonucleotide

<400>  815
tctgaacgtg aagctcgtcg tttgcgttag gaagcttatt tgcagattga aggttgcagc     60

tatacaaaat c                                                          71


<210>  816
<211>  71
<212>  DNA
<213>  Artificial sequence

<220>
<223>  oligonucleotide

<400>  816
agaatgaacc caggtccatc tgtccaatag atactatcta aaattctata cttttattgt     60

tctacatgtg t                                                          71


<210>  817
<211>  71
<212>  DNA
<213>  Artificial sequence

<220>
<223>  oligonucleotide

<400>  817
tgtcagcacc tgctcaaaac aatgccgagg ttcccacttt caagttagtt cttgttggtg     60

atggaggtac t                                                          71


<210>  818
<211>  71
<212>  DNA
<213>  Artificial sequence

<220>
<223>  oligonucleotide

<400>  818
acaacctttg caaaagtaga accagataaa tcatagataa aatggctgaa tcccatagac     60

gtatgttatg a                                                          71


<210>  819
<211>  71
<212>  DNA
<213>  Artificial sequence

<220>
<223>  oligonucleotide

<400> 819
ccaactcaaa gaccacaaag aagatattaa ggaattcgta gctccaattg aattttctct 60

tttgtaacat t 71


<210> 820
<211> 71
<212> DNA
<213> Artificial sequence

<220>
<223> oligonucleotide

<400> 820
ccgtaacaaa ggaatcaagc aaagaaaaag tatgtaatag aatgtattgt atgcttgtgg 60

aatgaatgtc a 71


<210> 821
<211> 71
<212> DNA
<213> Artificial sequence

<220>
<223> oligonucleotide

<400> 821
gaatgtgttt attcttagtt gttattattt tactagtggt aattatcgtt cctatcgtcg 60

tccatttcag c 71


<210> 822
<211> 71
<212> DNA
<213> Artificial sequence

<220>
<223> oligonucleotide

<400> 822
cactgcaacc accactgctg aaacttcttc ctctgctaca tcctctagca ccgctgaatt 60

atctagttac a 71


<210> 823
<211> 71
<212> DNA
<213> Artificial sequence

<220>
<223> oligonucleotide

<400> 823
gtttgtccac cagacgctgg ttttgcttga ttttttccag ctgcataatc tgtagacata 60

ttttaagtat a 71


<210> 824
<211> 71
<212> DNA
<213> Artificial sequence

<220>
<223> oligonucleotide

<400> 824
cgcaagacat gaatgtagat gaaatatttc tcaaacaagc ggcagaagct atagcggtaa 60

tctcatcaag t 71


<210> 825
<211> 71
<212> DNA
<213> Artificial sequence

<220>
<223> oligonucleotide

<400> 825
gctccagttc catacgaaac cttcatttaa tctacctcgt ttggtatatt tatcgaagtt 60

atcacattcc t 71


<210> 826
<211> 71
<212> DNA
<213> Artificial sequence

<220>
<223> oligonucleotide

<400> 826
gatgaagctg atgaggccga tgaagaataa ttgtgctgat ttttatttct taattacgtg 60

ttgaaatcac g 71


<210> 827
<211> 71
<212> DNA
<213> Artificial sequence

<220>
<223> oligonucleotide

<400> 827
cttaagagaa gtgacaaaag agtgaagaca gactatacat catgaaggta tgatttatga 60

tactattatt g 71


<210> 828
<211> 71
<212> DNA
<213> Artificial sequence

<220>
<223> oligonucleotide

<400> 828
agaatcttct tgtacccatc caacatctaa atttgttgat tgtataagta ctctattaaa 60

catcgttatt t 71


<210> 829
<211> 71
<212> DNA
<213> Artificial sequence

<220>

<223>  oligonucleotide

<400>  829
tttacaaaga ctctaaagat agcaaggaca aggaaaaggc attatacaca atatcaggcc      60

aatggtctgg c                                                          71


<210>  830
<211>  71
<212>  DNA
<213>  Artificial sequence

<220>
<223>  oligonucleotide

<400>  830
atgtatgtac gtacgtatgc aaagaagtgg atatagagaa ggaaagtagg ggagagaggg      60

aaagaacaaa a                                                          71


<210>  831
<211>  71
<212>  DNA
<213>  Artificial sequence

<220>
<223>  oligonucleotide

<400>  831
tttttactgc agttttattc gcagcatcct ccgcattagc tgctccagtc aacactacaa      60

cagaagatga a                                                          71


<210>  832
<211>  71
<212>  DNA
<213>  Artificial sequence

<220>
<223>  oligonucleotide

<400>  832
atcaataaat tggttaaggc tatgaactaa cattattccg tgtggcaata atctcaatgt      60

ataataaata a                                                          71


<210>  833
<211>  71
<212>  DNA
<213>  Artificial sequence

<220>
<223>  oligonucleotide

<400>  833
acgttactat ttcaatttaa cagtcaacca gtcgtccaaa aatgtccaag atcacttctt      60

ctcaagtcag a                                                          71


<210>  834
<211>  71
<212>  DNA
<213>  Artificial sequence

<220>
<223>  oligonucleotide

<400>  834
gctgacaccg aaatggaaga ggtagattag gtagaacatc atggccttga ataggttata      60

aacaaaacat a                                                           71


<210>  835
<211>  71
<212>  DNA
<213>  Artificial sequence

<220>
<223>  oligonucleotide

<400>  835
aacatcattg ctgcctctcg tcgtcattaa acagcgtgag ggggccacag tcaagcaaat      60

ccaactccgc c                                                           71


<210>  836
<211>  71
<212>  DNA
<213>  Artificial sequence

<220>
<223>  oligonucleotide

<400>  836
gatcgcaaac agatgctttt tatacccaat ataaaaatgg cactgcagct tctgaaccca      60

ctccaataac a                                                           71


<210>  837
<211>  71
<212>  DNA
<213>  Artificial sequence

<220>
<223>  oligonucleotide

<400>  837
ttagctctcg aagaatccat tgaccactgt ttagatagag aatgcgattc catcgtcaag      60

gtatctcagc c                                                           71


<210>  838
<211>  71
<212>  DNA
<213>  Artificial sequence

<220>
<223>  oligonucleotide

<400>  838
agaagattga gagaaatggt tgaagcttga ttttatttta attatatgta taaaaatcat      60

aaaacaacta t                                                           71


<210>  839
<211>  71
<212>  DNA
<213>  Artificial sequence

<220>
<223>  oligonucleotide

<400>  839
gttttttaat actagcaaac tgctcttcgt tacgtatgtc caatggcttg tacactattt      60

ccatgtcctg a                                                          71


<210>  840
<211>  71
<212>  DNA
<213>  Artificial sequence

<220>
<223>  oligonucleotide

<400>  840
gatgttccgc tactacttga atgaagggga cgcctttaaa ctgatattgc cgttgactga      60

aaagagctgc a                                                          71


<210>  841
<211>  71
<212>  DNA
<213>  Artificial sequence

<220>
<223>  oligonucleotide

<400>  841
aaggagttat ataacgccac tttggcttag ggatacttcc tccccgcgtt tttgtctgtg      60

tgatttaatt t                                                          71


<210>  842
<211>  71
<212>  DNA
<213>  Artificial sequence

<220>
<223>  oligonucleotide

<400>  842
attggtgcat ttatccatta catgacggct aatttattca aagagtatta tttacataac      60

ggcccactag a                                                          71


<210>  843
<211>  71
<212>  DNA
<213>  Artificial sequence

<220>
<223>  oligonucleotide

<400>  843
tggtacaagc ataaccgtgt aaaaggttaa tgagcaaacg ttccttgctt gtgtcaagac      60

atgttcgggc a                                                          71


<210>  844
<211>  71
<212>  DNA

<213>  Artificial sequence

<220>
<223>  oligonucleotide

<400>  844
gaaagaaagc atagcaatct aatctaagtt ttaattacaa aatgggtaaa gagaagtctc      60

acattaacgt t                                                           71


<210>  845
<211>  71
<212>  DNA
<213>  Artificial sequence

<220>
<223>  oligonucleotide

<400>  845
ggaagggaag gtgaaccaag aacatacaaa catagccaaa gatgtctgcc aaagctcaaa      60

accctatgcg t                                                           71


<210>  846
<211>  71
<212>  DNA
<213>  Artificial sequence

<220>
<223>  oligonucleotide

<400>  846
gcttgttgga tttactttta ggcatagaga tggaaaacaa ggttcaagcg ggatacttta      60

caatggttca g                                                           71


<210>  847
<211>  71
<212>  DNA
<213>  Artificial sequence

<220>
<223>  oligonucleotide

<400>  847
aaggaaaaga aggaaaagcc aagatcttaa gcagagttat attacgattt cataattcga      60

aaaattctac t                                                           71


<210>  848
<211>  71
<212>  DNA
<213>  Artificial sequence

<220>
<223>  oligonucleotide

<400>  848
aagactgcca aagcttctca agaactgtaa gaactgagtt gaaaagaaac aaagcaaatc      60

aaacattctt g                                                           71


<210>  849
<211>  71

<212>  DNA
<213>  Artificial sequence

<220>
<223>  oligonucleotide

<400>  849
   cttccaacgc ccaagtcaaa accccattac ctccagcccc agctccaaag aaggaatcta      60
   actttttgat t                                                          71


<210>  850
<211>  71
<212>  DNA
<213>  Artificial sequence

<220>
<223>  oligonucleotide

<400>  850
atcgatatta gtcccacgtt cgctgaagct ataagaagac tacacaatgg tgaatccgtg      60

tcatatttgt t                                                           71


<210>  851
<211>  71
<212>  DNA
<213>  Artificial sequence

<220>
<223>  oligonucleotide

<400>  851
aatacttctg gaccaatcgg tgataccatc ttccttattt atggtattat tatcattatt      60

ggaccccgcc g                                                           71


<210>  852
<211>  71
<212>  DNA
<213>  Artificial sequence

<220>
<223>  oligonucleotide

<400>  852
agacggttta cctgaagtga tatactgtgc cttgagaaac aatgagtagc gacgctaagc      60

aacaagaaac a                                                           71


<210>  853
<211>  71
<212>  DNA
<213>  Artificial sequence

<220>
<223>  oligonucleotide

<400>  853
tgccaaagca gcagctgcag aaattaatgt taaagatccg aaggaagatt tggaaacttc      60

tgtggtagat g                                                           71


<210>  854

<211>  71
<212>  DNA
<213>  Artificial sequence

<220>
<223>  oligonucleotide

<400>  854
tttgtcttac gccgccttga ttttggctga ctctgaaatc gaaatctctt ctgaaaagtt      60

gttgactttg a                                                           71


<210>  855
<211>  71
<212>  DNA
<213>  Artificial sequence

<220>
<223>  oligonucleotide

<400>  855
gtgcctatgc aagatgctgt taccgaacag ctgactccca cagtatctga agatatggat      60

ctcaacaaca a                                                           71


<210>  856
<211>  71
<212>  DNA
<213>  Artificial sequence

<220>
<223>  oligonucleotide

<400>  856
aacacgatcc atttgaagcg tatagcacat ccacaaccct tcaaagcgaa attaatatca      60

gtgtcaagca t                                                           71


<210>  857
<211>  71
<212>  DNA
<213>  Artificial sequence

<220>
<223>  oligonucleotide

<400>  857
aattaaagaa aaaacttcgt acacagaatt gaaaattttc gatgggcgaa ggaactacta      60

aggaaaacaa t                                                           71


<210>  858
<211>  71
<212>  DNA
<213>  Artificial sequence

<220>
<223>  oligonucleotide

<400>  858
ggaagaggga aataatgttt acgaaatcca agacttggag aagagatctc ctgtaatagg      60

ctcaagcttg g                                                           71

<210>  859
<211>  71
<212>  DNA
<213>  Artificial sequence

<220>
<223>  oligonucleotide

<400>  859
cgagttagga ctaagttggg catttatagt ttacaacatt gctagaccct ttgagctcaa      60

gtactttggt c                                                           71


<210>  860
<211>  71
<212>  DNA
<213>  Artificial sequence

<220>
<223>  oligonucleotide

<400>  860
tgtcaatggt cgatataaaa gatgcgggga gtgaactaag ggacctcgaa tctggagctc      60

tggacacaaa a                                                           71


<210>  861
<211>  71
<212>  DNA
<213>  Artificial sequence

<220>
<223>  oligonucleotide

<400>  861
atgctaatac tgttacgttt atctgaggtt tgtgtgaatt ttgtgataat tatcgggatt      60

ccattgttga t                                                           71


<210>  862
<211>  71
<212>  DNA
<213>  Artificial sequence

<220>
<223>  oligonucleotide

<400>  862
gacttggttg aacacgttgc caaggcttaa gtgaatttac tttaaatctt gcatttaaat      60

aaattttctt t                                                           71


<210>  863
<211>  71
<212>  DNA
<213>  Artificial sequence

<220>
<223>  oligonucleotide

<400>  863
actcctatgt gaaaaatatc tgtgtttatg ccgatgaaag tagggttaaa ccggtgggta      60

ttgtggtacc c                                                           71

<210>  864
<211>  71
<212>  DNA
<213>  Artificial sequence

<220>
<223>  oligonucleotide

<400>  864
cacaaaaaac agtacttcac taaatttaca cacaaaacaa aatgatcaga attgctatta      60

acggtttcgg t                                                          71


<210>  865
<211>  71
<212>  DNA
<213>  Artificial sequence

<220>
<223>  oligonucleotide

<400>  865
ccatccgaca ttgctacctt gttgaactaa tctctaagga gaagaaatcc ataactatat      60

tacctttatc t                                                          71


<210>  866
<211>  71
<212>  DNA
<213>  Artificial sequence

<220>
<223>  oligonucleotide

<400>  866
gacttggttg aacacgttgc caaggcttaa atttaactcc ttaagttact ttaatgattt      60

agtttttatt a                                                          71


<210>  867
<211>  71
<212>  DNA
<213>  Artificial sequence

<220>
<223>  oligonucleotide

<400>  867
atcacaccag aaggggatct gccccttagt ggccaagata tcttttatca atactcgttc      60

agcagttttt a                                                          71


<210>  868
<211>  71
<212>  DNA
<213>  Artificial sequence

<220>
<223>  oligonucleotide

<400>  868
tcggcgcttt tactgcatgc tgtcttttga agagagtttc attgaaaagt agtgaagaaa      60

aaaaaaaaaa a                                                          71

<210> 869
<211> 71
<212> DNA
<213> Artificial sequence

<220>
<223> oligonucleotide

<400> 869
ccatccgaca tcgctacttt gttgaactaa gggagacaaa aacttcttaa aaaagtatct 60

tgtgaggcta t 71

<210> 870
<211> 71
<212> DNA
<213> Artificial sequence

<220>
<223> oligonucleotide

<400> 870
acgaccttga aaaaaacatt tcgcacttgg acccgaccgg tgttgacaat gcttatattc 60

cacctgaaca g 71

<210> 871
<211> 71
<212> DNA
<213> Artificial sequence

<220>
<223> oligonucleotide

<400> 871
tatgactcca ggcaaggttc tctttactaa tgctttgggt ttaggctgtt ccagatctag 60

ggggttgttt t 71

<210> 872
<211> 71
<212> DNA
<213> Artificial sequence

<220>
<223> oligonucleotide

<400> 872
catcatttaa aggactgtag agccaattgc attatatcaa tatgagtttc ggtagtaaag 60

tctccagggc t 71

<210> 873
<211> 71
<212> DNA
<213> Artificial sequence

<220>
<223> oligonucleotide

<400> 873
ccaatacgaa cgattatcaa attattgcaa catttcccag tgaaatcttc accaaatgct 60

catacagcgt g                                                          71

<210>  874
<211>  71
<212>  DNA
<213>  Artificial sequence

<220>
<223>  oligonucleotide

<400>  874
ttaaatcatc taatcctgta aggttggcca ctgcagtagt agaggctacc actcactttg     60

acaatcccag c                                                          71

<210>  875
<211>  71
<212>  DNA
<213>  Artificial sequence

<220>
<223>  oligonucleotide

<400>  875
aactttacaa aataaacata ttgacttgtg ggcactatcc cctcaactct attccattta     60

cagttacata a                                                          71

<210>  876
<211>  71
<212>  DNA
<213>  Artificial sequence

<220>
<223>  oligonucleotide

<400>  876
gatattactc caagtcatat gcacgatttg gacatgtctc tttattccgt tactcaatgc     60

agaggaattc g                                                          71

<210>  877
<211>  71
<212>  DNA
<213>  Artificial sequence

<220>
<223>  oligonucleotide

<400>  877
ttttggaccg ctgcgctaga atacgagtag ataagatggc ttatgcacaa tagataaaga     60

tatacctaca t                                                          71

<210>  878
<211>  71
<212>  DNA
<213>  Artificial sequence

<220>
<223>  oligonucleotide

<400>  878
attataacta tcaaaaaaag gacttcagca cctgtatcat catgagcttc agtagtatag     60

tctctaagtt t                                                          71

<210>  879
<211>  71
<212>  DNA
<213>  Artificial sequence

<220>
<223>  oligonucleotide

<400>  879
gatatactat caagcaaaaa aaactctagc attacaccat tatgagtttc ggtacgagaa      60

tctccagggc t                                                          71

<210>  880
<211>  71
<212>  DNA
<213>  Artificial sequence

<220>
<223>  oligonucleotide

<400>  880
aaggtttgag cctgcaagca acaacagtga tgaattcggt agcgggaaag actttgctgt      60

ctaccaatat g                                                          71

<210>  881
<211>  71
<212>  DNA
<213>  Artificial sequence

<220>
<223>  oligonucleotide

<400>  881
cgttaggaaa tatacataag gaaagcaact gtcttgccac aatgacctat tctacagtta      60

atattaatac a                                                          71

<210>  882
<211>  71
<212>  DNA
<213>  Artificial sequence

<220>
<223>  oligonucleotide

<400>  882
agtggataaa tatacaattt ccaaagcagt atgcctttcc tatggttatc atcctcctag      60

gtctttgcac a                                                          71

<210>  883
<211>  71
<212>  DNA
<213>  Artificial sequence

<220>
<223>  oligonucleotide

<400>  883

ggctttgaat cctgctatgt cgctttcgct gtgcttggcg agagccgtct ctcctacaag 60

atgtgtcgtt a 71


<210> 884
<211> 71
<212> DNA
<213> Artificial sequence

<220>
<223> oligonucleotide

<400> 884
aagcaacgct gaggaaaatg tacaggagac tgaagctggc gaatctttca cattaaatcc 60

cggagaagat t 71


<210> 885
<211> 71
<212> DNA
<213> Artificial sequence

<220>
<223> oligonucleotide

<400> 885
tgaaaaacaa aaggctattg tcttttatga gacagatgga aaattggaat ataaagacgt 60

cacagttccg g 71


<210> 886
<211> 71
<212> DNA
<213> Artificial sequence

<220>
<223> oligonucleotide

<400> 886
ggtaccaagt ttttctcaaa cgccaataat gctcacatat tggtcgaaat tgcaaaggat 60

atgctggacg a 71


<210> 887
<211> 71
<212> DNA
<213> Artificial sequence

<220>
<223> oligonucleotide

<400> 887
gtgtttatac aattgctatt gtttatataa tgtagtgaca ttttctctta atcttatact 60

aatttctatg a 71


<210> 888
<211> 71
<212> DNA
<213> Artificial sequence

<220>
<223> oligonucleotide

<400>  888
ggccatgaac attccatgtg aaaaaatcct gagggagagc tcgaatggta agaaaatagc      60

aaccattgaa a                                                           71


<210>  889
<211>  71
<212>  DNA
<213>  Artificial sequence

<220>
<223>  oligonucleotide

<400>  889
aatattacca acaacaccca cagcagccca tttatgtcca acaacaaccg gccagttctg      60

gaaatgaaga c                                                           71


<210>  890
<211>  71
<212>  DNA
<213>  Artificial sequence

<220>
<223>  oligonucleotide

<400>  890
ttatttcctt tgctgctttc atcctagctg atgctggttt ggaaatcacc tctgacaact      60

tattgactat c                                                           71


<210>  891
<211>  71
<212>  DNA
<213>  Artificial sequence

<220>
<223>  oligonucleotide

<400>  891
cgtagttttt gtacagatca cttatacagc tttacacaga ttttcccgct tgttgtgcac      60

tttttttcg a                                                            71


<210>  892
<211>  71
<212>  DNA
<213>  Artificial sequence

<220>
<223>  oligonucleotide

<400>  892
gacctgttcg cttgggttat tcttcaaagc acaaatcaat atctttcttc ctccttttgc      60

tttaaccgtt g                                                           71


<210>  893
<211>  71
<212>  DNA
<213>  Artificial sequence

<220>
<223>  oligonucleotide

<400>  893
caattgcgtc caaagaagaa gttaaaatga tctgtttcgc ccaatcaatc tatacattta      60

tttactgtat a                                                           71


<210>  894
<211>  71
<212>  DNA
<213>  Artificial sequence

<220>
<223>  oligonucleotide

<400>  894
ctccagtcca cttggaagct atcgatttga tagactgtta agcagaaaac tattagttct      60

tttatcctga t                                                           71


<210>  895
<211>  71
<212>  DNA
<213>  Artificial sequence

<220>
<223>  oligonucleotide

<400>  895
ttttcccgtt cagcaagagg taaagccacc aaaggttcaa aatgcaaatg tatgttacgg      60

cgaatacaga a                                                           71


<210>  896
<211>  71
<212>  DNA
<213>  Artificial sequence

<220>
<223>  oligonucleotide

<400>  896
ctctcgaact ctccctccca ctttacttta actaatagat tatgcagatt ttcgtcaaga      60

ctttgaccgg t                                                           71


<210>  897
<211>  71
<212>  DNA
<213>  Artificial sequence

<220>
<223>  oligonucleotide

<400>  897
aagagaaaga agaaggtcta caccacccca aagaagatca agcacaagca caagaaggtc      60

aagttggctg t                                                           71


<210>  898
<211>  71
<212>  DNA
<213>  Artificial sequence

<220>

<223>  oligonucleotide

<400>  898
cattcttacg aaaagcgttt acataactaa ttggtaacta gggttcatta ataataaaat      60

aaaactaaaa a                                                          71


<210>  899
<211>  71
<212>  DNA
<213>  Artificial sequence

<220>
<223>  oligonucleotide

<400>  899
atacaatcaa ctatcaacta ttaactatat cgtaatacac aatgtctatt ccagaaactc      60

aaaaagccat t                                                          71


<210>  900
<211>  71
<212>  DNA
<213>  Artificial sequence

<220>
<223>  oligonucleotide

<400>  900
ttcaagctat accaagcata caatcaacta tctcatatac aatgtctatc ccagaaactc      60

aaaaaggtgt t                                                          71


<210>  901
<211>  71
<212>  DNA
<213>  Artificial sequence

<220>
<223>  oligonucleotide

<400>  901
ccttagctac caacagtgtt aatggttcct atactgaaga ctctctaaaa aggatatgga      60

atgaacttca c                                                          71


<210>  902
<211>  71
<212>  DNA
<213>  Artificial sequence

<220>
<223>  oligonucleotide

<400>  902
tccaccgtat tgctggcaat cgtatggtaa acgcacggca tcatcttata ttaataattc      60

tatcatcacg c                                                          71


<210>  903
<211>  71
<212>  DNA
<213>  Artificial sequence

<220>
<223>  oligonucleotide

<400>  903
aaaaaatact acagtaaaga aagggccatt ccaaattacc tatgtttaag tctgttgttt      60

attcggttct a                                                           71


<210>  904
<211>  71
<212>  DNA
<213>  Artificial sequence

<220>
<223>  oligonucleotide

<400>  904
cattacaacg ccagtctatt gagacaatag ttttgtataa ctaaataata ttggaaacta      60

aatacgaata c                                                           71


<210>  905
<211>  71
<212>  DNA
<213>  Artificial sequence

<220>
<223>  oligonucleotide

<400>  905
atgaaatact tagctgctta cttattattg gttcaaggtg gtaacgctgc cccatccgcc      60

gctgacatca a                                                           71


<210>  906
<211>  71
<212>  DNA
<213>  Artificial sequence

<220>
<223>  oligonucleotide

<400>  906
atgtctagtg cgcaatcctc tattgatagc gatggagatg ttcgagatgc tgatattcat      60

gtcgcaccac c                                                           71


<210>  907
<211>  71
<212>  DNA
<213>  Artificial sequence

<220>
<223>  oligonucleotide

<400>  907
tgaatatctc ttctatatca ttttcaatag cgatttttaa tatggctgat ttatttgctc      60

ctgctcctga a                                                           71


<210>  908
<211>  71
<212>  DNA
<213>  Artificial sequence

<220>
<223>  oligonucleotide

<400>  908
gtggagtttc attccgatta cgtaaaggtg aagaacgcta cgttcctcgg tgtacaaatg      60

aaatttgcac t                                                           71


<210>  909
<211>  71
<212>  DNA
<213>  Artificial sequence

<220>
<223>  oligonucleotide

<400>  909
aaaacttcca ccacggtgac aagttgtaaa gtgcttttaa ctaagaatta ttagtctttt      60

ctgcttattt t                                                           71


<210>  910
<211>  71
<212>  DNA
<213>  Artificial sequence

<220>
<223>  oligonucleotide

<400>  910
ctattccaga agtcgtcaag gactacaaac ctgttgctgt cccagctaga gttccaatta      60

ccaagtctac t                                                           71


<210>  911
<211>  71
<212>  DNA
<213>  Artificial sequence

<220>
<223>  oligonucleotide

<400>  911
gtccaaaaca tctgtgtcta cgctgatgaa aacaaagtta agcctgtcgg tattgtggtc      60

cctaacttag g                                                           71


<210>  912
<211>  71
<212>  DNA
<213>  Artificial sequence

<220>
<223>  oligonucleotide

<400>  912
tattattgaa cgctgctggt aacaccccag atgccaccaa gattaaggct attttggaat      60

ccgtcggtat c                                                           71


<210>  913
<211>  71
<212>  DNA

<213>  Artificial sequence

<220>
<223>  oligonucleotide

<400>  913
tatgcgatcg atttttctgc tgcattttga ctataaaacg tgtgaagagg aagatttcga      60

agactggaat t                                                           71


<210>  914
<211>  71
<212>  DNA
<213>  Artificial sequence

<220>
<223>  oligonucleotide

<400>  914
aactagaagg tgttgcaaac cccattatga gtaaatttta cggagctgca ggtggtgccc      60

caggagcagg c                                                           71


<210>  915
<211>  71
<212>  DNA
<213>  Artificial sequence

<220>
<223>  oligonucleotide

<400>  915
cttgtatgat aagcacattg gttctcagtg ccatgaagtt tatttgcaag ctatagattt      60

aattgactgt t                                                           71


<210>  916
<211>  71
<212>  DNA
<213>  Artificial sequence

<220>
<223>  oligonucleotide

<400>  916
ctacaagcta tcgatttgct caactgttaa tcataagttt acctctcata gtcgcatgaa      60

aacataaagt c                                                           71


<210>  917
<211>  71
<212>  DNA
<213>  Artificial sequence

<220>
<223>  oligonucleotide

<400>  917
accactattg ctgacgccgc taagggttac aagccagttg ctgtcccagc tagaactcca      60

gctaacgctg c                                                           71


<210>  918
<211>  71

<212>  DNA
<213>  Artificial sequence

<220>
<223>  oligonucleotide

<400>  918
actcatctag caagttagga cgcctccgat ttctttctga aactgccgct attaaagtat      60

ccccgttaat c                                                           71


<210>  919
<211>  71
<212>  DNA
<213>  Artificial sequence

<220>
<223>  oligonucleotide

<400>  919
gtgtccaggt aagttagttg ggttagtcac ctctcgtgat atacaattct tagaagatga      60

ttctttggtt g                                                           71


<210>  920
<211>  71
<212>  DNA
<213>  Artificial sequence

<220>
<223>  oligonucleotide

<400>  920
aaaaaaaaaa aaaaaaagaa aagaaaagaa aaattaataa aatgcaatca tccactgaaa      60

gtgatagaga t                                                           71


<210>  921
<211>  71
<212>  DNA
<213>  Artificial sequence

<220>
<223>  oligonucleotide

<400>  921
tgccataaat ttttcaaaag ccttatcaaa cagacaaggt cgaatgcaca tttgagttca      60

ccaacgaacg g                                                           71


<210>  922
<211>  71
<212>  DNA
<213>  Artificial sequence

<220>
<223>  oligonucleotide

<400>  922
tgtcgcctca ataccatttt tattttgtat cattccggaa cttagtattg aatgaaaaat      60

gcctccgaag t                                                           71


<210>  923

<211>  71
<212>  DNA
<213>  Artificial sequence

<220>
<223>  oligonucleotide

<400>  923
taagccatca tttggtgtgt cctaaacggt ttccatgcaa agcaacatct ttgttactca      60

ttcctgaagg t                                                          71


<210>  924
<211>  71
<212>  DNA
<213>  Artificial sequence

<220>
<223>  oligonucleotide

<400>  924
agtccggagg ttggattccc agttctagaa gaacaccaca accaacaagc agtacaccat      60

tagttattgt t                                                          71


<210>  925
<211>  71
<212>  DNA
<213>  Artificial sequence

<220>
<223>  oligonucleotide

<400>  925
aaacgtgtat tgggagtcgc ttatttgtag agtttgtagt ttttaagcgt tctcaggaaa      60

ttcattgacg c                                                          71


<210>  926
<211>  71
<212>  DNA
<213>  Artificial sequence

<220>
<223>  oligonucleotide

<400>  926
gaaggtccaa ccgttgaaga agttgattaa gccaattggt gcggcaattg ataataacga      60

aaatgtcttt t                                                          71


<210>  927
<211>  71
<212>  DNA
<213>  Artificial sequence

<220>
<223>  oligonucleotide

<400>  927
agttggaagg cattgccaat ccaataatga cgaaatttta cggtgctggt gccggcgcag      60

gtcctggagc g                                                          71

<210>  928
<211>  71
<212>  DNA
<213>  Artificial sequence

<220>
<223>  oligonucleotide

<400>  928
cctgggtacc agcttctaga agggatggtg attatgataa cgaaaaatta cagcatgacg      60

agaaaccctt c                                                           71


<210>  929
<211>  71
<212>  DNA
<213>  Artificial sequence

<220>
<223>  oligonucleotide

<400>  929
agatgaccca gcggtaacca agaagctttc acccctcaca tcgatgtctg ccaggatagc      60

ttttgacaat t                                                           71


<210>  930
<211>  71
<212>  DNA
<213>  Artificial sequence

<220>
<223>  oligonucleotide

<400>  930
ttacggaggt gttgttgctt ccagcgctac accctcatcc acatctacca catcaggggc      60

taccaactct a                                                           71


<210>  931
<211>  71
<212>  DNA
<213>  Artificial sequence

<220>
<223>  oligonucleotide

<400>  931
ttgtttacta ctagccatcg tcacattgct gggattaact aatgttgtct ctgcgactac      60

agcggcatgc c                                                           71


<210>  932
<211>  71
<212>  DNA
<213>  Artificial sequence

<220>
<223>  oligonucleotide

<400>  932
ttatttttaa ttcttgtcac cagtaaacag aacatccaaa aatgacaatg cctcatcgct      60

atatgttttt g                                                           71

<210> 933
<211> 71
<212> DNA
<213> Artificial sequence

<220>
<223> oligonucleotide

<400> 933
caaatgattt tctttaaatt gattagcacc actaaaaaaa atgacaattg cacaccactg 60

catatttttg g 71


<210> 934
<211> 71
<212> DNA
<213> Artificial sequence

<220>
<223> oligonucleotide

<400> 934
cagacgcatt gccagcacgt agtgctattc acaagcctga ttcaactgct gtgcgcaacg 60

aggacgagta a 71


<210> 935
<211> 71
<212> DNA
<213> Artificial sequence

<220>
<223> oligonucleotide

<400> 935
tgggaaggaa gaatatatat cagcgaatga gtattctcaa agaataataa tgataatgtg 60

cgaagaaacc c 71


<210> 936
<211> 71
<212> DNA
<213> Artificial sequence

<220>
<223> oligonucleotide

<400> 936
atctcagcct caagcgcaaa ctcaatctca gcagtttggc gctcgttatc aaccacaaca 60

acaaccttct g 71


<210> 937
<211> 71
<212> DNA
<213> Artificial sequence

<220>
<223> oligonucleotide

<400> 937
atgtctgcca agattagctt cgaagattag tcaaatcaat agttttagta tgttcataat 60

gtgaaaaatg t 71

<210>  938
<211>  71
<212>  DNA
<213>  Artificial sequence

<220>
<223>  oligonucleotide

<400>  938
gaaggtccaa ctgtcgaaga agttgattaa gcttagcgat cgccctgtaa aatatccgaa      60

aagttttact t                                                           71


<210>  939
<211>  71
<212>  DNA
<213>  Artificial sequence

<220>
<223>  oligonucleotide

<400>  939
aacagcaaca gcaacagcaa caacagcaac aacaagctcc agctgaacaa actcaaggtg      60

aaccaaccaa a                                                           71


<210>  940
<211>  71
<212>  DNA
<213>  Artificial sequence

<220>
<223>  oligonucleotide

<400>  940
gtcaagatac agcaacaaca acccggctac gcggatcttg aacacaagga ggactgtgtg      60

gaggagtttt t                                                           71


<210>  941
<211>  71
<212>  DNA
<213>  Artificial sequence

<220>
<223>  oligonucleotide

<400>  941
tgtatgttga aggtgtcaaa ccatggaaat ctggtagctg gatctcaaaa gaaaaaagag      60

tttccgagga a                                                           71


<210>  942
<211>  71
<212>  DNA
<213>  Artificial sequence

<220>
<223>  oligonucleotide

<400>  942
ttctacaaga aaatgttcgg caagaaataa tttcacgcta aaccgtagtg ctgaataata      60

gtgtatttta a                                                          71

<210>  943
<211>  71
<212>  DNA
<213>  Artificial sequence

<220>
<223>  oligonucleotide

<400>  943
ccaatctaca agaggttctt ttccagctaa tcttccggtt tttagtaact ggaaaaaaat      60

acatgaactt a                                                          71

<210>  944
<211>  71
<212>  DNA
<213>  Artificial sequence

<220>
<223>  oligonucleotide

<400>  944
gaagcacaat ccgtggtaac aagtctttag tgaaaaagtg aacatgtttt tttcaaccta      60

aaaggtgggt t                                                          71

<210>  945
<211>  71
<212>  DNA
<213>  Artificial sequence

<220>
<223>  oligonucleotide

<400>  945
ggctttttcc agcatttatt ctgtttctgg cttgtatcta tgtgtcatgg gataagggcc      60

atctatttcg t                                                          71

<210>  946
<211>  71
<212>  DNA
<213>  Artificial sequence

<220>
<223>  oligonucleotide

<400>  946
tcggcccgca atgagaagcc cctaaagtag gaacaaatgt tccttttcca aaatatacta      60

tgtgtagttg a                                                          71

<210>  947
<211>  71
<212>  DNA
<213>  Artificial sequence

<220>
<223>  oligonucleotide

<400>  947
cctatcaaga ggatacagca gtccaaaata ctccagctga tgctttgtcg ccagttgaat      60

ccgattctaa t                                                          71

<210>  948
<211>  71
<212>  DNA
<213>  Artificial sequence

<220>
<223>  oligonucleotide

<400>  948
ccgatctaat atctcctcag aaatccaatt catccaactc atatgaattg gaatctggtc     60

gttcaaaggc c                                                          71

<210>  949
<211>  71
<212>  DNA
<213>  Artificial sequence

<220>
<223>  oligonucleotide

<400>  949
gtcttcattt cacgaaatga tggcactata gcacgtccct tgtttcttgt ggtgctgttt     60

tttatcatga c                                                          71

<210>  950
<211>  71
<212>  DNA
<213>  Artificial sequence

<220>
<223>  oligonucleotide

<400>  950
ggagaacaac agattatgac ctagacgcta ctagaaatga tccgagacca ttttataaaa     60

ggatgttcac t                                                          71

<210>  951
<211>  71
<212>  DNA
<213>  Artificial sequence

<220>
<223>  oligonucleotide

<400>  951
gcagacggta tctacactat tgcaaattag agatagattc ccttagggat aaaatgtgat     60

acgaattaga g                                                          71

<210>  952
<211>  71
<212>  DNA
<213>  Artificial sequence

<220>
<223>  oligonucleotide

<400>  952

cagaagaggt aataattacg atttagagga tttacaacat gacgacaaac cgtggtacaa     60

ggccatgcta g                                                          71

<210>  953
<211>  71
<212>  DNA
<213>  Artificial sequence

<220>
<223>  oligonucleotide

<400>  953
gacggtatct acactgctgt accaaactag gatttgacgc ccttacgaat agaactttac     60

gagtattaga a                                                          71

<210>  954
<211>  71
<212>  DNA
<213>  Artificial sequence

<220>
<223>  oligonucleotide

<400>  954
gacggtatct acactgctgt tccaaactaa gccgatggcc tcacaattgg tatttagcac     60

tggattgctt t                                                          71

<210>  955
<211>  71
<212>  DNA
<213>  Artificial sequence

<220>
<223>  oligonucleotide

<400>  955
cgaagaaggt acgcctacta ataatgggca acagaaggag agaagaaaga tagaaattaa     60

gttcatcgag a                                                          71

<210>  956
<211>  20
<212>  DNA
<213>  Artificial sequence

<220>
<223>  reverse primer

<400>  956
caatatgcgg ccatagtcct                                                 20

<210>  957
<211>  20
<212>  DNA
<213>  Artificial sequence

<220>
<223>  reverse primer

<400>  957
attcgtatac ggcggagttg                                                 20

<210>  958
<211>  20
<212>  DNA
<213>  Artificial sequence

<220>
<223>  reverse primer

<400>  958
gcctcgttgc ggtatttaga                                                    20


<210>  959
<211>  20
<212>  DNA
<213>  Artificial sequence

<220>
<223>  reverse primer

<400>  959
agaccccttc cactcgatct                                                    20


<210>  960
<211>  20
<212>  DNA
<213>  Artificial sequence

<220>
<223>  reverse primer

<400>  960
atcccacatg acttgcaaca                                                    20


<210>  961
<211>  20
<212>  DNA
<213>  Artificial sequence

<220>
<223>  reverse primer

<400>  961
cgttcaaagc cttgaccttc                                                    20


<210>  962
<211>  20
<212>  DNA
<213>  Artificial sequence

<220>
<223>  reverse primer

<400>  962
cattgcccaa accaaaaagt                                                    20


<210>  963
<211>  20
<212>  DNA
<213>  Artificial sequence

<220>
<223>  reverse primer

<400>  963
caatgatggc aaggtgtttg                                                    20


<210>  964
<211>  20
<212>  DNA
<213>  Artificial sequence

<220>
<223>  reverse primer

<400>  964
ctaatttcgc cgatcttgga                                                    20


<210>  965
<211>  20
<212>  DNA
<213>  Artificial sequence

<220>
<223>  reverse primer

<400>  965
gctttccaag acagcggata                                                    20


<210>  966
<211>  20
<212>  DNA
<213>  Artificial sequence

<220>
<223>  reverse primer

<400>  966
tgactttttg ggcatcaaca                                                    20


<210>  967
<211>  20
<212>  DNA
<213>  Artificial sequence

<220>
<223>  reverse primer

<400>  967
agttgaattt gttttcgccg                                                    20


<210>  968
<211>  20
<212>  DNA
<213>  Artificial sequence

<220>
<223>  reverse primer

<400>  968
catttgcgct ggtcatagaa                                                    20


<210>  969
<211>  20
<212>  DNA
<213>  Artificial sequence

<220>
<223>  reverse primer

<400>  969
gcattttcat tcttgccgtt                                                    20


<210>  970
<211>  20
<212>  DNA
<213>  Artificial sequence

<220>
<223>  reverse primer

<400>  970
aatattgagc actggcccac                                                    20


<210>  971
<211>  20
<212>  DNA
<213>  Artificial sequence

<220>
<223>  reverse primer

<400>  971
tttccaggta atcgctggtc                                                    20


<210>  972
<211>  20
<212>  DNA
<213>  Artificial sequence

<220>
<223>  reverse primer

<400>  972
ccaggctaat atttggtgcg                                                    20


<210>  973
<211>  20
<212>  DNA
<213>  Artificial sequence

<220>
<223>  reverse primer

<400>  973
atagacagct gcccaatgct                                                    20


<210>  974
<211>  20
<212>  DNA
<213>  Artificial sequence

<220>
<223>  reverse primer

<400>  974
tcttttcgtg ttcgtgttcg                                                    20


<210>  975

<211>  20
<212>  DNA
<213>  Artificial sequence

<220>
<223>  reverse primer

<400>  975
taaattgaaa ggaccgccaa                                                    20


<210>  976
<211>  20
<212>  DNA
<213>  Artificial sequence

<220>
<223>  reverse primer

<400>  976
atcctccaga tggaagggtc                                                    20


<210>  977
<211>  20
<212>  DNA
<213>  Artificial sequence

<220>
<223>  reverse primer

<400>  977
tgagccgcta ccttcaactt                                                    20


<210>  978
<211>  20
<212>  DNA
<213>  Artificial sequence

<220>
<223>  reverse primer

<400>  978
gcctgtcgga actccatatc                                                    20


<210>  979
<211>  20
<212>  DNA
<213>  Artificial sequence

<220>
<223>  reverse primer

<400>  979
ttactcagag tcggtggcta                                                    20


<210>  980
<211>  20
<212>  DNA
<213>  Artificial sequence

<220>
<223>  reverse primer

<400>  980
tacttcgatt tttgccccac                                                    20

<210> 981
<211> 20
<212> DNA
<213> Artificial sequence

<220>
<223> reverse primer

<400> 981
ttcaaacgtc aggaatgtcg 20


<210> 982
<211> 20
<212> DNA
<213> Artificial sequence

<220>
<223> reverse primer

<400> 982
aaccatatgc ctcgtatcgc 20


<210> 983
<211> 20
<212> DNA
<213> Artificial sequence

<220>
<223> reverse primer

<400> 983
tcagcttggg caataaaagg 20


<210> 984
<211> 20
<212> DNA
<213> Artificial sequence

<220>
<223> reverse primer

<400> 984
gccacttgat gttcaccctc 20


<210> 985
<211> 20
<212> DNA
<213> Artificial sequence

<220>
<223> reverse primer

<400> 985
tgaaataccg tattgcatcc 20


<210> 986
<211> 20
<212> DNA
<213> Artificial sequence

<220>
<223> reverse primer

<400>  986
tcgctatcat cacttgcagc                                                    20


<210>  987
<211>  20
<212>  DNA
<213>  Artificial sequence

<220>
<223>  reverse primer

<400>  987
ggcttttcgg aaatttaggc                                                    20


<210>  988
<211>  20
<212>  DNA
<213>  Artificial sequence

<220>
<223>  reverse primer

<400>  988
tgacgttgag cgtaatgacc                                                    20


<210>  989
<211>  20
<212>  DNA
<213>  Artificial sequence

<220>
<223>  reverse primer

<400>  989
atgcttgttc atgatgctcg                                                    20


<210>  990
<211>  20
<212>  DNA
<213>  Artificial sequence

<220>
<223>  reverse primer

<400>  990
ttgctgtcat caacggagag                                                    20


<210>  991
<211>  20
<212>  DNA
<213>  Artificial sequence

<220>
<223>  reverse primer

<400>  991
accatttcca ttttcatggc                                                    20


<210>  992
<211>  20
<212>  DNA
<213>  Artificial sequence

<220>
<223>  reverse primer

<400>  992
ataagccaac caatcgtcca                                                    20


<210>  993
<211>  20
<212>  DNA
<213>  Artificial sequence

<220>
<223>  reverse primer

<400>  993
agcagccaat ttcctcaaga                                                    20


<210>  994
<211>  20
<212>  DNA
<213>  Artificial sequence

<220>
<223>  reverse primer

<400>  994
gaaatctcgc cagacaaagc                                                    20


<210>  995
<211>  20
<212>  DNA
<213>  Artificial sequence

<220>
<223>  reverse primer

<400>  995
gaaaactaga atggccagcg                                                    20


<210>  996
<211>  20
<212>  DNA
<213>  Artificial sequence

<220>
<223>  reverse primer

<400>  996
ggcccaaaca agtcgaataa                                                    20


<210>  997
<211>  20
<212>  DNA
<213>  Artificial sequence

<220>
<223>  reverse primer

<400>  997
ttgaacacgc tctgttggag                                                    20


<210>  998

<211>  20
<212>  DNA
<213>  Artificial sequence

<220>
<223>  reverse primer

<400>  998
atgtcgtcaa cgcgaaggct                                                    20


<210>  999
<211>  20
<212>  DNA
<213>  Artificial sequence

<220>
<223>  reverse primer

<400>  999
gcccatcaat tgggtgtaac                                                    20


<210>  1000
<211>  20
<212>  DNA
<213>  Artificial sequence

<220>
<223>  reverse primer

<400>  1000
tgctcccatt accttaccgt                                                    20


<210>  1001
<211>  20
<212>  DNA
<213>  Artificial sequence

<220>
<223>  reverse primer

<400>  1001
tgcaccttta tcatagcccc                                                    20


<210>  1002
<211>  20
<212>  DNA
<213>  Artificial sequence

<220>
<223>  reverse primer

<400>  1002
gatactgggc cctgaacaga                                                    20


<210>  1003
<211>  20
<212>  DNA
<213>  Artificial sequence

<220>
<223>  reverse primer

<400>  1003
aacatccaag gtgatagcgg                                                    20

<210> 1004
<211> 20
<212> DNA
<213> Artificial sequence

<220>
<223> reverse primer

<400> 1004
ccaccgtaca tcgtctcgtt 20


<210> 1005
<211> 20
<212> DNA
<213> Artificial sequence

<220>
<223> reverse primer

<400> 1005
ctgttatgcc ttggccttgt 20


<210> 1006
<211> 20
<212> DNA
<213> Artificial sequence

<220>
<223> reverse primer

<400> 1006
ttcttcgctg atcggaagtt 20


<210> 1007
<211> 20
<212> DNA
<213> Artificial sequence

<220>
<223> reverse primer

<400> 1007
tttaacccaa tatttgcccg 20


<210> 1008
<211> 20
<212> DNA
<213> Artificial sequence

<220>
<223> reverse primer

<400> 1008
agatacgggg caaaaattga 20


<210> 1009
<211> 20
<212> DNA
<213> Artificial sequence

<220>
<223> reverse primer

<400>  1009
cttctcagag cccattctgg                                                    20


<210>  1010
<211>  20
<212>  DNA
<213>  Artificial sequence

<220>
<223>  reverse primer

<400>  1010
atatcggcta cgcttgatga                                                    20


<210>  1011
<211>  20
<212>  DNA
<213>  Artificial sequence

<220>
<223>  reverse primer

<400>  1011
cacgctcttg cgagttgata                                                    20


<210>  1012
<211>  20
<212>  DNA
<213>  Artificial sequence

<220>
<223>  reverse primer

<400>  1012
catttccgta ccttccctga                                                    20


<210>  1013
<211>  20
<212>  DNA
<213>  Artificial sequence

<220>
<223>  reverse primer

<400>  1013
cttcctccgc attagaacca                                                    20


<210>  1014
<211>  20
<212>  DNA
<213>  Artificial sequence

<220>
<223>  reverse primer

<400>  1014
ggctgtgctt actagcgagg                                                    20


<210>  1015
<211>  20
<212>  DNA
<213>  Artificial sequence

<220>
<223>  reverse primer

<400>  1015
tgacattttc cgaaacacca                                                    20


<210>  1016
<211>  20
<212>  DNA
<213>  Artificial sequence

<220>
<223>  reverse primer

<400>  1016
ttgcaattga aattgtgcgt                                                    20


<210>  1017
<211>  20
<212>  DNA
<213>  Artificial sequence

<220>
<223>  reverse primer

<400>  1017
tcgcccctta tagttggttg                                                    20


<210>  1018
<211>  20
<212>  DNA
<213>  Artificial sequence

<220>
<223>  reverse primer

<400>  1018
tagtggcgtt ttcttcgctt                                                    20


<210>  1019
<211>  20
<212>  DNA
<213>  Artificial sequence

<220>
<223>  reverse primer

<400>  1019
tttccgtttg gagattcagg                                                    20


<210>  1020
<211>  20
<212>  DNA
<213>  Artificial sequence

<220>
<223>  reverse primer

<400>  1020
cccaagcaag aatttgatgg                                                    20


<210>  1021

<211>  20
<212>  DNA
<213>  Artificial sequence

<220>
<223>  reverse primer

<400>  1021
gccactagga acaaggtgga                                                    20


<210>  1022
<211>  20
<212>  DNA
<213>  Artificial sequence

<220>
<223>  reverse primer

<400>  1022
ttgcccaaaa cccatctcta                                                    20


<210>  1023
<211>  20
<212>  DNA
<213>  Artificial sequence

<220>
<223>  reverse primer

<400>  1023
caccttctgc caatagctcc                                                    20


<210>  1024
<211>  20
<212>  DNA
<213>  Artificial sequence

<220>
<223>  reverse primer

<400>  1024
cgacttcatt ttgtgcctga                                                    20


<210>  1025
<211>  20
<212>  DNA
<213>  Artificial sequence

<220>
<223>  reverse primer

<400>  1025
gatccagcga agttctgagc                                                    20


<210>  1026
<211>  20
<212>  DNA
<213>  Artificial sequence

<220>
<223>  reverse primer

<400>  1026
ttttggcgaa ttccagtacc                                                    20

<210>  1027
<211>  20
<212>  DNA
<213>  Artificial sequence

<220>
<223>  reverse primer

<400>  1027
cccctcattt acgtttgcat                                                    20


<210>  1028
<211>  20
<212>  DNA
<213>  Artificial sequence

<220>
<223>  reverse primer

<400>  1028
ggttgcttgt gctgttctca                                                    20


<210>  1029
<211>  20
<212>  DNA
<213>  Artificial sequence

<220>
<223>  reverse primer

<400>  1029
aaaatgcaca ccggctaaac                                                    20


<210>  1030
<211>  20
<212>  DNA
<213>  Artificial sequence

<220>
<223>  reverse primer

<400>  1030
ccttgacctc tgtggcaaat                                                    20


<210>  1031
<211>  20
<212>  DNA
<213>  Artificial sequence

<220>
<223>  reverse primer

<400>  1031
ttgttgtttt gtctcgctgc                                                    20


<210>  1032
<211>  20
<212>  DNA
<213>  Artificial sequence

<220>
<223>  reverse primer

<400>  1032
tgtcttttcg tgccttctca                                                   20


<210>  1033
<211>  20
<212>  DNA
<213>  Artificial sequence

<220>
<223>  reverse primer

<400>  1033
ttattcgtat ctccggcagc                                                   20


<210>  1034
<211>  20
<212>  DNA
<213>  Artificial sequence

<220>
<223>  reverse primer

<400>  1034
agaatgttgg atgggtctgc                                                   20


<210>  1035
<211>  20
<212>  DNA
<213>  Artificial sequence

<220>
<223>  reverse primer

<400>  1035
tatccgtttg ggccaataat                                                   20


<210>  1036
<211>  20
<212>  DNA
<213>  Artificial sequence

<220>
<223>  reverse primer

<400>  1036
aaccgttggc aataaacctg                                                   20


<210>  1037
<211>  20
<212>  DNA
<213>  Artificial sequence

<220>
<223>  reverse primer

<400>  1037
aaacctaaca agcgcatacc                                                   20


<210>  1038
<211>  20
<212>  DNA
<213>  Artificial sequence

<220>
<223>  reverse primer

<400>  1038
ttgtagcgca cacccaaata                                                    20


<210>  1039
<211>  20
<212>  DNA
<213>  Artificial sequence

<220>
<223>  reverse primer

<400>  1039
tgaaattcac tttctccggc                                                    20


<210>  1040
<211>  20
<212>  DNA
<213>  Artificial sequence

<220>
<223>  reverse primer

<400>  1040
atgcattgaa ctttaggcgg                                                    20


<210>  1041
<211>  20
<212>  DNA
<213>  Artificial sequence

<220>
<223>  reverse primer

<400>  1041
ctgtaagatt gcgccaatga                                                    20


<210>  1042
<211>  20
<212>  DNA
<213>  Artificial sequence

<220>
<223>  reverse primer

<400>  1042
tcccattttt caaagcaacc                                                    20


<210>  1043
<211>  20
<212>  DNA
<213>  Artificial sequence

<220>
<223>  reverse primer

<400>  1043
tgccatataa ccccactggt                                                    20


<210>  1044

<211>  20
<212>  DNA
<213>  Artificial sequence

<220>
<223>  reverse primer

<400>  1044
tcaaagtttc caaatcgctg                                                    20


<210>  1045
<211>  20
<212>  DNA
<213>  Artificial sequence

<220>
<223>  reverse primer

<400>  1045
ccttctcgta atggaacgga                                                    20


<210>  1046
<211>  20
<212>  DNA
<213>  Artificial sequence

<220>
<223>  reverse primer

<400>  1046
ggtacgaaca gattgggcat                                                    20


<210>  1047
<211>  20
<212>  DNA
<213>  Artificial sequence

<220>
<223>  reverse primer

<400>  1047
cggagaattt ttcctcctcc                                                    20


<210>  1048
<211>  20
<212>  DNA
<213>  Artificial sequence

<220>
<223>  reverse primer

<400>  1048
ctgacgagtc cgttcaacaa                                                    20


<210>  1049
<211>  20
<212>  DNA
<213>  Artificial sequence

<220>
<223>  reverse primer

<400>  1049
cgttcttgcc aatgacttga                                                    20

<210>  1050
<211>  20
<212>  DNA
<213>  Artificial sequence

<220>
<223>  reverse primer

<400>  1050
tcgacatctc caatcttcca                                                    20


<210>  1051
<211>  20
<212>  DNA
<213>  Artificial sequence

<220>
<223>  reverse primer

<400>  1051
cagtttgcgc gtttgagtaa                                                    20


<210>  1052
<211>  20
<212>  DNA
<213>  Artificial sequence

<220>
<223>  reverse primer

<400>  1052
tgttttcgtt tggtgttggt                                                    20


<210>  1053
<211>  20
<212>  DNA
<213>  Artificial sequence

<220>
<223>  reverse primer

<400>  1053
atgctaaaac tggtggtggc                                                    20


<210>  1054
<211>  20
<212>  DNA
<213>  Artificial sequence

<220>
<223>  reverse primer

<400>  1054
tctggtcctc tggtaatggg                                                    20


<210>  1055
<211>  20
<212>  DNA
<213>  Artificial sequence

<220>
<223>  reverse primer

<400>  1055
tcgaactcgg tttcctcatc                                                    20


<210>  1056
<211>  20
<212>  DNA
<213>  Artificial sequence

<220>
<223>  reverse primer

<400>  1056
gggtcaccga cctttagtga                                                    20


<210>  1057
<211>  20
<212>  DNA
<213>  Artificial sequence

<220>
<223>  reverse primer

<400>  1057
ggcaactact ctcagctcgg                                                    20


<210>  1058
<211>  20
<212>  DNA
<213>  Artificial sequence

<220>
<223>  reverse primer

<400>  1058
cttgcgggtt tttggaatta                                                    20


<210>  1059
<211>  20
<212>  DNA
<213>  Artificial sequence

<220>
<223>  reverse primer

<400>  1059
aggtgacttt gttcccgttg                                                    20


<210>  1060
<211>  20
<212>  DNA
<213>  Artificial sequence

<220>
<223>  reverse primer

<400>  1060
tgtagcttcc tcggttggat                                                    20


<210>  1061
<211>  20
<212>  DNA
<213>  Artificial sequence

<220>
<223>  reverse primer

<400>  1061
ccccgaagta atcctcttcc                                                    20


<210>  1062
<211>  20
<212>  DNA
<213>  Artificial sequence

<220>
<223>  reverse primer

<400>  1062
tgcaaggttt gtgtagtggt                                                    20


<210>  1063
<211>  20
<212>  DNA
<213>  Artificial sequence

<220>
<223>  reverse primer

<400>  1063
ccagcttgcc ttggtagact                                                    20


<210>  1064
<211>  20
<212>  DNA
<213>  Artificial sequence

<220>
<223>  reverse primer

<400>  1064
tgtgttccga ttaaggaggc                                                    20


<210>  1065
<211>  20
<212>  DNA
<213>  Artificial sequence

<220>
<223>  reverse primer

<400>  1065
ttgtccagat cgtcatttcg                                                    20


<210>  1066
<211>  20
<212>  DNA
<213>  Artificial sequence

<220>
<223>  reverse primer

<400>  1066
tcttcatcat cggaagaccc                                                    20


<210>  1067

<211>  20
<212>  DNA
<213>  Artificial sequence

<220>
<223>  reverse primer

<400>  1067
aaagtggcct tgggttttct                                                    20


<210>  1068
<211>  20
<212>  DNA
<213>  Artificial sequence

<220>
<223>  reverse primer

<400>  1068
ctctttaccg ttaccgacgc                                                    20


<210>  1069
<211>  20
<212>  DNA
<213>  Artificial sequence

<220>
<223>  reverse primer

<400>  1069
cgcaccaaca ccaagtaaac                                                    20


<210>  1070
<211>  20
<212>  DNA
<213>  Artificial sequence

<220>
<223>  reverse primer

<400>  1070
agctttcgat tcgacttcca                                                    20


<210>  1071
<211>  20
<212>  DNA
<213>  Artificial sequence

<220>
<223>  reverse primer

<400>  1071
caggttgacg ttccctttgt                                                    20


<210>  1072
<211>  20
<212>  DNA
<213>  Artificial sequence

<220>
<223>  reverse primer

<400>  1072
aggatcctga agtggaagca                                                    20

<210> 1073
<211> 20
<212> DNA
<213> Artificial sequence

<220>
<223> reverse primer

<400> 1073
actttccttc caagctgctg 20

<210> 1074
<211> 20
<212> DNA
<213> Artificial sequence

<220>
<223> reverse primer

<400> 1074
cctacaaggg caattctgga 20

<210> 1075
<211> 20
<212> DNA
<213> Artificial sequence

<220>
<223> reverse primer

<400> 1075
cgatcactcg aatcattgaa 20

<210> 1076
<211> 20
<212> DNA
<213> Artificial sequence

<220>
<223> reverse primer

<400> 1076
gagatagcga tctggaagcg 20

<210> 1077
<211> 20
<212> DNA
<213> Artificial sequence

<220>
<223> reverse primer

<400> 1077
gggatgttgg agcagtgaat 20

<210> 1078
<211> 20
<212> DNA
<213> Artificial sequence

<220>
<223> reverse primer

<400>  1078
gcccttgtgg acaataggaa                                                    20


<210>  1079
<211>  20
<212>  DNA
<213>  Artificial sequence

<220>
<223>  reverse primer

<400>  1079
tccgcagtat tcattcgttg                                                    20


<210>  1080
<211>  20
<212>  DNA
<213>  Artificial sequence

<220>
<223>  reverse primer

<400>  1080
tttgcatgtc caacattgct                                                    20


<210>  1081
<211>  20
<212>  DNA
<213>  Artificial sequence

<220>
<223>  reverse primer

<400>  1081
accgtacttc tttcttcttt                                                    20


<210>  1082
<211>  20
<212>  DNA
<213>  Artificial sequence

<220>
<223>  reverse primer

<400>  1082
gaacctgttt tcgtggaagc                                                    20


<210>  1083
<211>  20
<212>  DNA
<213>  Artificial sequence

<220>
<223>  reverse primer

<400>  1083
cgtgttcgaa tggtatgacg                                                    20


<210>  1084
<211>  20
<212>  DNA
<213>  Artificial sequence

<220>
<223>  reverse primer

<400>  1084
acgttaacca gtccaccagc                                                    20


<210>  1085
<211>  20
<212>  DNA
<213>  Artificial sequence

<220>
<223>  reverse primer

<400>  1085
tccgtgcctc ttttcttctt                                                    20


<210>  1086
<211>  20
<212>  DNA
<213>  Artificial sequence

<220>
<223>  reverse primer

<400>  1086
ttccctagat gcgctttcac                                                    20


<210>  1087
<211>  20
<212>  DNA
<213>  Artificial sequence

<220>
<223>  reverse primer

<400>  1087
cggaagtact ctgcctggag                                                    20


<210>  1088
<211>  20
<212>  DNA
<213>  Artificial sequence

<220>
<223>  reverse primer

<400>  1088
ggtctccttc agtggagtcg                                                    20


<210>  1089
<211>  20
<212>  DNA
<213>  Artificial sequence

<220>
<223>  reverse primer

<400>  1089
tgaaccattg ctgtgctgtt                                                    20


<210>  1090

<211>  20
<212>  DNA
<213>  Artificial sequence

<220>
<223>  reverse primer

<400>  1090
tcctcttctt ctgtcagggc                                                    20


<210>  1091
<211>  20
<212>  DNA
<213>  Artificial sequence

<220>
<223>  reverse primer

<400>  1091
acgaggactc tatgggcgta                                                    20


<210>  1092
<211>  20
<212>  DNA
<213>  Artificial sequence

<220>
<223>  reverse primer

<400>  1092
tcttgtcttc ctttggtgcc                                                    20


<210>  1093
<211>  20
<212>  DNA
<213>  Artificial sequence

<220>
<223>  reverse primer

<400>  1093
tggtgtaaat tggaacagcc                                                    20


<210>  1094
<211>  20
<212>  DNA
<213>  Artificial sequence

<220>
<223>  reverse primer

<400>  1094
tcggcaactt tatcatgcaa                                                    20


<210>  1095
<211>  20
<212>  DNA
<213>  Artificial sequence

<220>
<223>  reverse primer

<400>  1095
tgccaacctg ttggtattca                                                    20

<210>  1096
<211>  20
<212>  DNA
<213>  Artificial sequence

<220>
<223>  reverse primer

<400>  1096
caacagcacc aaaggttgtc                                                    20


<210>  1097
<211>  20
<212>  DNA
<213>  Artificial sequence

<220>
<223>  reverse primer

<400>  1097
acagtgcttc aatgtctgcg                                                    20


<210>  1098
<211>  20
<212>  DNA
<213>  Artificial sequence

<220>
<223>  reverse primer

<400>  1098
ggtaggaaca cgcgaactgt                                                    20


<210>  1099
<211>  20
<212>  DNA
<213>  Artificial sequence

<220>
<223>  reverse primer

<400>  1099
aaccttcgat atctctgatg                                                    20


<210>  1100
<211>  20
<212>  DNA
<213>  Artificial sequence

<220>
<223>  reverse primer

<400>  1100
tctgtgtttt atttgaagtt                                                    20


<210>  1101
<211>  20
<212>  DNA
<213>  Artificial sequence

<220>
<223>  reverse primer

<400>  1101
tgcaccctag aagctgttga                                                    20


<210>  1102
<211>  20
<212>  DNA
<213>  Artificial sequence

<220>
<223>  reverse primer

<400>  1102
attcgcctta tttggcaatg                                                    20


<210>  1103
<211>  20
<212>  DNA
<213>  Artificial sequence

<220>
<223>  reverse primer

<400>  1103
accagggtta tcgtggttca                                                    20


<210>  1104
<211>  20
<212>  DNA
<213>  Artificial sequence

<220>
<223>  reverse primer

<400>  1104
tagccgagca caaagcagat                                                    20


<210>  1105
<211>  20
<212>  DNA
<213>  Artificial sequence

<220>
<223>  reverse primer

<400>  1105
tggagactga gaagaacgcc                                                    20


<210>  1106
<211>  20
<212>  DNA
<213>  Artificial sequence

<220>
<223>  reverse primer

<400>  1106
ttcttgcttc aaggcctcat                                                    20


<210>  1107
<211>  20
<212>  DNA
<213>  Artificial sequence

<220>
<223>  reverse primer

<400>  1107
atcccgaaga ctcgctgtta                                                    20


<210>  1108
<211>  20
<212>  DNA
<213>  Artificial sequence

<220>
<223>  reverse primer

<400>  1108
ttttcaccgc tctggtcttc                                                    20


<210>  1109
<211>  20
<212>  DNA
<213>  Artificial sequence

<220>
<223>  reverse primer

<400>  1109
tgaaacatac gcttcccctc                                                    20


<210>  1110
<211>  20
<212>  DNA
<213>  Artificial sequence

<220>
<223>  reverse primer

<400>  1110
tcgtcaacga acctccttct                                                    20


<210>  1111
<211>  20
<212>  DNA
<213>  Artificial sequence

<220>
<223>  reverse primer

<400>  1111
ggagcccttt ggctatttct                                                    20


<210>  1112
<211>  20
<212>  DNA
<213>  Artificial sequence

<220>
<223>  reverse primer

<400>  1112
ttccaagcat gcaaccataa                                                    20


<210>  1113

<211>  20
<212>  DNA
<213>  Artificial sequence

<220>
<223>  reverse primer

<400>  1113
ctgtggttta agccgcactt                                                    20


<210>  1114
<211>  20
<212>  DNA
<213>  Artificial sequence

<220>
<223>  reverse primer

<400>  1114
tccatgaatt cgaatgggtt                                                    20


<210>  1115
<211>  20
<212>  DNA
<213>  Artificial sequence

<220>
<223>  reverse primer

<400>  1115
attccgcttt atcaccatgc                                                    20


<210>  1116
<211>  20
<212>  DNA
<213>  Artificial sequence

<220>
<223>  reverse primer

<400>  1116
tcaatcttgc gttgtgcttc                                                    20


<210>  1117
<211>  20
<212>  DNA
<213>  Artificial sequence

<220>
<223>  reverse primer

<400>  1117
ttatttgacc ccttccatcg                                                    20


<210>  1118
<211>  20
<212>  DNA
<213>  Artificial sequence

<220>
<223>  reverse primer

<400>  1118
tagcagttcg tccttgaggg                                                    20

<210>  1119
<211>  20
<212>  DNA
<213>  Artificial sequence

<220>
<223>  reverse primer

<400>  1119
tcgataataa tgtcgccttt                                                    20


<210>  1120
<211>  20
<212>  DNA
<213>  Artificial sequence

<220>
<223>  reverse primer

<400>  1120
atccttggga taaccatcca                                                    20


<210>  1121
<211>  20
<212>  DNA
<213>  Artificial sequence

<220>
<223>  reverse primer

<400>  1121
agcgggtttg attctgttct                                                    20


<210>  1122
<211>  20
<212>  DNA
<213>  Artificial sequence

<220>
<223>  reverse primer

<400>  1122
cctagaaacc acttcaggcg                                                    20


<210>  1123
<211>  20
<212>  DNA
<213>  Artificial sequence

<220>
<223>  reverse primer

<400>  1123
tcaccattat gcaaacgacg                                                    20


<210>  1124
<211>  20
<212>  DNA
<213>  Artificial sequence

<220>
<223>  reverse primer

<400>  1124
caaaatagag ccatcgggaa                                                    20


<210>  1125
<211>  20
<212>  DNA
<213>  Artificial sequence

<220>
<223>  reverse primer

<400>  1125
agcaattgcc tttcaccttg                                                    20


<210>  1126
<211>  20
<212>  DNA
<213>  Artificial sequence

<220>
<223>  reverse primer

<400>  1126
tctgtcctgg taaaatcggg                                                    20


<210>  1127
<211>  20
<212>  DNA
<213>  Artificial sequence

<220>
<223>  reverse primer

<400>  1127
ggccatgacc aaggctataa                                                    20


<210>  1128
<211>  20
<212>  DNA
<213>  Artificial sequence

<220>
<223>  reverse primer

<400>  1128
acgatctctg ctcacccatt                                                    20


<210>  1129
<211>  20
<212>  DNA
<213>  Artificial sequence

<220>
<223>  reverse primer

<400>  1129
tttggcttcg aatcaacctc                                                    20


<210>  1130
<211>  20
<212>  DNA
<213>  Artificial sequence

<220>
<223>  reverse primer

<400>  1130
tagtcccaaa agcgacattg                                                    20


<210>  1131
<211>  20
<212>  DNA
<213>  Artificial sequence

<220>
<223>  reverse primer

<400>  1131
atagcttgtt gccggtgttt                                                    20


<210>  1132
<211>  20
<212>  DNA
<213>  Artificial sequence

<220>
<223>  reverse primer

<400>  1132
atggctgcgt tgaatttctc                                                    20


<210>  1133
<211>  20
<212>  DNA
<213>  Artificial sequence

<220>
<223>  reverse primer

<400>  1133
tcgctgtcag catcttcatt                                                    20


<210>  1134
<211>  20
<212>  DNA
<213>  Artificial sequence

<220>
<223>  reverse primer

<400>  1134
gactggtttt ggttgtcggt                                                    20


<210>  1135
<211>  20
<212>  DNA
<213>  Artificial sequence

<220>
<223>  reverse primer

<400>  1135
gagtttttgg cagcttggac                                                    20


<210>  1136

<211>  20
<212>  DNA
<213>  Artificial sequence

<220>
<223>  reverse primer

<400>  1136
ccaccatcga tgataatatc                                                    20


<210>  1137
<211>  20
<212>  DNA
<213>  Artificial sequence

<220>
<223>  reverse primer

<400>  1137
gacttgttca tacgggccat                                                    20


<210>  1138
<211>  20
<212>  DNA
<213>  Artificial sequence

<220>
<223>  reverse primer

<400>  1138
tccaatgaaa gtcgtgatgc                                                    20


<210>  1139
<211>  20
<212>  DNA
<213>  Artificial sequence

<220>
<223>  reverse primer

<400>  1139
acgctttgtt cgtttggatt                                                    20


<210>  1140
<211>  20
<212>  DNA
<213>  Artificial sequence

<220>
<223>  reverse primer

<400>  1140
caccttgaat ttcttggcgt                                                    20


<210>  1141
<211>  20
<212>  DNA
<213>  Artificial sequence

<220>
<223>  reverse primer

<400>  1141
aaccggaaca tgactcacaa                                                    20

<210>  1142
<211>  20
<212>  DNA
<213>  Artificial sequence

<220>
<223>  reverse primer

<400>  1142
tgttagaaga tgatcggccc                                                    20


<210>  1143
<211>  20
<212>  DNA
<213>  Artificial sequence

<220>
<223>  reverse primer

<400>  1143
tgtcctttat ggagggcttg                                                    20


<210>  1144
<211>  20
<212>  DNA
<213>  Artificial sequence

<220>
<223>  reverse primer

<400>  1144
ggaaattctt tcaatcggca                                                    20


<210>  1145
<211>  20
<212>  DNA
<213>  Artificial sequence

<220>
<223>  reverse primer

<400>  1145
agcatcaacg taaacccagg                                                    20


<210>  1146
<211>  20
<212>  DNA
<213>  Artificial sequence

<220>
<223>  reverse primer

<400>  1146
agccacgcta gaagcagaag                                                    20


<210>  1147
<211>  20
<212>  DNA
<213>  Artificial sequence

<220>
<223>  reverse primer

<400>  1147
ctatcttttc ctcggtgcca                                                   20


<210>  1148
<211>  20
<212>  DNA
<213>  Artificial sequence

<220>
<223>  reverse primer

<400>  1148
cagtaatctc gctggatctg                                                   20


<210>  1149
<211>  20
<212>  DNA
<213>  Artificial sequence

<220>
<223>  reverse primer

<400>  1149
tcatcctttc atttcccagc                                                   20


<210>  1150
<211>  20
<212>  DNA
<213>  Artificial sequence

<220>
<223>  reverse primer

<400>  1150
caccggcttc ttgcttagtc                                                   20


<210>  1151
<211>  20
<212>  DNA
<213>  Artificial sequence

<220>
<223>  reverse primer

<400>  1151
aattcgtcga tggtagcctg                                                   20


<210>  1152
<211>  20
<212>  DNA
<213>  Artificial sequence

<220>
<223>  reverse primer

<400>  1152
cctgagccct gtgtgaaaat                                                   20


<210>  1153
<211>  20
<212>  DNA
<213>  Artificial sequence

<220>
<223>  reverse primer

<400>  1153
ttaatcgcgc ctgttcttct                                                   20


<210>  1154
<211>  20
<212>  DNA
<213>  Artificial sequence

<220>
<223>  reverse primer

<400>  1154
gacgaagtgt tggcatccat                                                   20


<210>  1155
<211>  20
<212>  DNA
<213>  Artificial sequence

<220>
<223>  reverse primer

<400>  1155
gttggactgg gaagagcaag                                                   20


<210>  1156
<211>  20
<212>  DNA
<213>  Artificial sequence

<220>
<223>  reverse primer

<400>  1156
tcgatccatt cctttaccca                                                   20


<210>  1157
<211>  20
<212>  DNA
<213>  Artificial sequence

<220>
<223>  reverse primer

<400>  1157
acgtgcatat tcttcagggc                                                   20


<210>  1158
<211>  20
<212>  DNA
<213>  Artificial sequence

<220>
<223>  reverse primer

<400>  1158
tcaaagatgg agtggcacaa                                                   20


<210>  1159

<211>  20
<212>  DNA
<213>  Artificial sequence

<220>
<223>  reverse primer

<400>  1159
tgttttcctc atccaggagc                                                    20


<210>  1160
<211>  20
<212>  DNA
<213>  Artificial sequence

<220>
<223>  reverse primer

<400>  1160
tcagcagcac atgtcaacaa                                                    20


<210>  1161
<211>  20
<212>  DNA
<213>  Artificial sequence

<220>
<223>  reverse primer

<400>  1161
gatagcaaac ccgaggatga                                                    20


<210>  1162
<211>  20
<212>  DNA
<213>  Artificial sequence

<220>
<223>  reverse primer

<400>  1162
cgctcgaagg ctttaatttg                                                    20


<210>  1163
<211>  20
<212>  DNA
<213>  Artificial sequence

<220>
<223>  reverse primer

<400>  1163
ttgtcggatc agatcgttga                                                    20


<210>  1164
<211>  20
<212>  DNA
<213>  Artificial sequence

<220>
<223>  reverse primer

<400>  1164
tctaatggcc cgtgaaactc                                                    20

<210>  1165
<211>  20
<212>  DNA
<213>  Artificial sequence

<220>
<223>  reverse primer

<400>  1165
agtcttactg ttgccgccat                                                   20


<210>  1166
<211>  20
<212>  DNA
<213>  Artificial sequence

<220>
<223>  reverse primer

<400>  1166
atatgtccca ggaacagcca                                                   20


<210>  1167
<211>  20
<212>  DNA
<213>  Artificial sequence

<220>
<223>  reverse primer

<400>  1167
tggtattgag tgcccacgta                                                   20


<210>  1168
<211>  20
<212>  DNA
<213>  Artificial sequence

<220>
<223>  reverse primer

<400>  1168
caaagccgtt tgaggttcat                                                   20


<210>  1169
<211>  20
<212>  DNA
<213>  Artificial sequence

<220>
<223>  reverse primer

<400>  1169
cacgctgaaa aacattgcat                                                   20


<210>  1170
<211>  20
<212>  DNA
<213>  Artificial sequence

<220>
<223>  reverse primer

<400>  1170
ttcgcgtaga gccattttct                                                   20


<210>  1171
<211>  20
<212>  DNA
<213>  Artificial sequence

<220>
<223>  reverse primer

<400>  1171
ttggtatgac acagcgacaa                                                   20


<210>  1172
<211>  20
<212>  DNA
<213>  Artificial sequence

<220>
<223>  reverse primer

<400>  1172
gttttcatcc tttcgttgcc                                                   20


<210>  1173
<211>  20
<212>  DNA
<213>  Artificial sequence

<220>
<223>  reverse primer

<400>  1173
acagcatgtg cttctggatg                                                   20


<210>  1174
<211>  20
<212>  DNA
<213>  Artificial sequence

<220>
<223>  reverse primer

<400>  1174
   acgattttgg ggatgtgagg                                                    20


<210>  1175
<211>  20
<212>  DNA
<213>  Artificial sequence

<220>
<223>  reverse primer

<400>  1175
tgatgcggaa tcagacagag                                                   20


<210>  1176
<211>  20
<212>  DNA
<213>  Artificial sequence

<220>
<223>  reverse primer

<400>  1176
cagcatcatc tgctggctta                                                    20


<210>  1177
<211>  20
<212>  DNA
<213>  Artificial sequence

<220>
<223>  reverse primer

<400>  1177
gggtcagtgg tgaccaatct                                                    20


<210>  1178
<211>  20
<212>  DNA
<213>  Artificial sequence

<220>
<223>  reverse primer

<400>  1178
cagcacagtg cacgaagttt                                                    20


<210>  1179
<211>  20
<212>  DNA
<213>  Artificial sequence

<220>
<223>  reverse primer

<400>  1179
acaccaattt caggaccagc                                                    20


<210>  1180
<211>  20
<212>  DNA
<213>  Artificial sequence

<220>
<223>  reverse primer

<400>  1180
ccttgcacca ttgagctacc                                                    20


<210>  1181
<211>  20
<212>  DNA
<213>  Artificial sequence

<220>
<223>  reverse primer

<400>  1181
tatgtgcgga ctgttggttc                                                    20


<210>  1182

<211>  20
<212>  DNA
<213>  Artificial sequence

<220>
<223>  reverse primer

<400>  1182
taaaccgtcg ctaaacgtcc                                                    20


<210>  1183
<211>  20
<212>  DNA
<213>  Artificial sequence

<220>
<223>  reverse primer

<400>  1183
acctaatgtg acgacggagc                                                    20


<210>  1184
<211>  20
<212>  DNA
<213>  Artificial sequence

<220>
<223>  reverse primer

<400>  1184
ttccgtagtc tggcacacag                                                    20


<210>  1185
<211>  20
<212>  DNA
<213>  Artificial sequence

<220>
<223>  reverse primer

<400>  1185
caaaacttga caccacacgc                                                    20


<210>  1186
<211>  20
<212>  DNA
<213>  Artificial sequence

<220>
<223>  reverse primer

<400>  1186
gttcttcgct aagcgtgagg                                                    20


<210>  1187
<211>  20
<212>  DNA
<213>  Artificial sequence

<220>
<223>  reverse primer

<400>  1187
atcagccatt cgtaagcgtc                                                    20

<210>  1188
<211>  20
<212>  DNA
<213>  Artificial sequence

<220>
<223>  reverse primer

<400>  1188
ttttgctggg ttctgctttt                                                    20


<210>  1189
<211>  20
<212>  DNA
<213>  Artificial sequence

<220>
<223>  reverse primer

<400>  1189
tattgcctcc accgatttgt                                                    20


<210>  1190
<211>  20
<212>  DNA
<213>  Artificial sequence

<220>
<223>  reverse primer

<400>  1190
tatcaacgga attgaacgca                                                    20


<210>  1191
<211>  20
<212>  DNA
<213>  Artificial sequence

<220>
<223>  reverse primer

<400>  1191
gctgggtcaa catggttctt                                                    20


<210>  1192
<211>  20
<212>  DNA
<213>  Artificial sequence

<220>
<223>  reverse primer

<400>  1192
gagtagccct cctcattgcc                                                    20


<210>  1193
<211>  20
<212>  DNA
<213>  Artificial sequence

<220>
<223>  reverse primer

<400>  1193
ggtcttttct tgctcccaca                                                    20


<210>  1194
<211>  20
<212>  DNA
<213>  Artificial sequence

<220>
<223>  reverse primer

<400>  1194
cagtttcgct gcctctaacc                                                    20


<210>  1195
<211>  20
<212>  DNA
<213>  Artificial sequence

<220>
<223>  reverse primer

<400>  1195
aggacgaata tctggcttgg                                                    20


<210>  1196
<211>  20
<212>  DNA
<213>  Artificial sequence

<220>
<223>  reverse primer

<400>  1196
cggtcatttt gagctcctgt                                                    20


<210>  1197
<211>  20
<212>  DNA
<213>  Artificial sequence

<220>
<223>  reverse primer

<400>  1197
tgccaagcac aaaagttgtc                                                    20


<210>  1198
<211>  20
<212>  DNA
<213>  Artificial sequence

<220>
<223>  reverse primer

<400>  1198
aaaatgggtg gcctcttctt                                                    20


<210>  1199
<211>  20
<212>  DNA
<213>  Artificial sequence

<220>
<223>  reverse primer

<400>  1199
tcatttcttc cgcatccttc                                                    20


<210>  1200
<211>  20
<212>  DNA
<213>  Artificial sequence

<220>
<223>  reverse primer

<400>  1200
atgaaatgga acacaacgca                                                    20


<210>  1201
<211>  20
<212>  DNA
<213>  Artificial sequence

<220>
<223>  reverse primer

<400>  1201
tacagctgat gcaatttcgg                                                    20


<210>  1202
<211>  20
<212>  DNA
<213>  Artificial sequence

<220>
<223>  reverse primer

<400>  1202
aattcttgca acttgggtgg                                                    20


<210>  1203
<211>  20
<212>  DNA
<213>  Artificial sequence

<220>
<223>  reverse primer

<400>  1203
   accttttgcc attagcctca                                                   20


<210>  1204
<211>  20
<212>  DNA
<213>  Artificial sequence

<220>
<223>  reverse primer

<400>  1204
ttggtgaaag gttcagggac                                                    20


<210>  1205

<211>  20
<212>  DNA
<213>  Artificial sequence

<220>
<223>  reverse primer

<400>  1205
aaacatcaga gccttcgtcg                                                    20


<210>  1206
<211>  20
<212>  DNA
<213>  Artificial sequence

<220>
<223>  reverse primer

<400>  1206
gatggagcca aatggaaaga                                                    20


<210>  1207
<211>  20
<212>  DNA
<213>  Artificial sequence

<220>
<223>  reverse primer

<400>  1207
cccatggtga aatgataccc                                                    20


<210>  1208
<211>  20
<212>  DNA
<213>  Artificial sequence

<220>
<223>  reverse primer

<400>  1208
aatgtacacc tgctaggccg                                                    20


<210>  1209
<211>  20
<212>  DNA
<213>  Artificial sequence

<220>
<223>  reverse primer

<400>  1209
taccagatgc caagggattc                                                    20


<210>  1210
<211>  20
<212>  DNA
<213>  Artificial sequence

<220>
<223>  reverse primer

<400>  1210
acttggatgt ctccgacagg                                                    20

<210>  1211
<211>  20
<212>  DNA
<213>  Artificial sequence

<220>
<223>  reverse primer

<400>  1211
cttcaaaccg cgttgtgtta                                                    20


<210>  1212
<211>  20
<212>  DNA
<213>  Artificial sequence

<220>
<223>  reverse primer

<400>  1212
cacgatcact tgggctattg                                                    20


<210>  1213
<211>  20
<212>  DNA
<213>  Artificial sequence

<220>
<223>  reverse primer

<400>  1213
   ttcttccaga ccccttttcc                                                     20


<210>  1214
<211>  20
<212>  DNA
<213>  Artificial sequence

<220>
<223>  reverse primer

<400>  1214
aaaactccgg taccccattc                                                    20


<210>  1215
<211>  20
<212>  DNA
<213>  Artificial sequence

<220>
<223>  reverse primer

<400>  1215
tattttgaaa gggacgtggc                                                    20


<210>  1216
<211>  20
<212>  DNA
<213>  Artificial sequence

<220>
<223>  reverse primer

<400>  1216
tgcgtaattc ccagcttagg                                                    20


<210>  1217
<211>  20
<212>  DNA
<213>  Artificial sequence

<220>
<223>  reverse primer

<400>  1217
ttcagtggcc atagcgtctt                                                    20


<210>  1218
<211>  20
<212>  DNA
<213>  Artificial sequence

<220>
<223>  reverse primer

<400>  1218
ccttgaaaat ttggatggct                                                    20


<210>  1219
<211>  20
<212>  DNA
<213>  Artificial sequence

<220>
<223>  reverse primer

<400>  1219
cgccatagca tagccagatt                                                    20


<210>  1220
<211>  20
<212>  DNA
<213>  Artificial sequence

<220>
<223>  reverse primer

<400>  1220
tagcgcatcc gtagcttttt                                                    20


<210>  1221
<211>  20
<212>  DNA
<213>  Artificial sequence

<220>
<223>  reverse primer

<400>  1221
caaattgcaa atttttgggg                                                    20


<210>  1222
<211>  20
<212>  DNA
<213>  Artificial sequence

<220>
<223>  reverse primer

<400>  1222
caccgtgatg gtaagtgtgc                                                    20


<210>  1223
<211>  20
<212>  DNA
<213>  Artificial sequence

<220>
<223>  reverse primer

<400>  1223
cagttgtggc tttttctccg                                                    20


<210>  1224
<211>  20
<212>  DNA
<213>  Artificial sequence

<220>
<223>  reverse primer

<400>  1224
ttaaagtctt ctcttggatc                                                    20


<210>  1225
<211>  20
<212>  DNA
<213>  Artificial sequence

<220>
<223>  reverse primer

<400>  1225
tggtttgctc tcttccgagt                                                    20


<210>  1226
<211>  20
<212>  DNA
<213>  Artificial sequence

<220>
<223>  reverse primer

<400>  1226
cgcacgagtt gtgtcctcta                                                    20


<210>  1227
<211>  20
<212>  DNA
<213>  Artificial sequence

<220>
<223>  reverse primer

<400>  1227
acgttttcac gggagtcaac                                                    20


<210>  1228

<211>  20
<212>  DNA
<213>  Artificial sequence

<220>
<223>  reverse primer

<400>  1228
   ttctggggga aatatgcctt                                                    20


<210>  1229
<211>  20
<212>  DNA
<213>  Artificial sequence

<220>
<223>  reverse primer

<400>  1229
taaatcgtgc tttcttgccc                                                   20


<210>  1230
<211>  20
<212>  DNA
<213>  Artificial sequence

<220>
<223>  reverse primer

<400>  1230
ctgccaaaat tgaaagagcc                                                   20


<210>  1231
<211>  20
<212>  DNA
<213>  Artificial sequence

<220>
<223>  reverse primer

<400>  1231
aacctgcctt gtcatgcttt                                                   20


<210>  1232
<211>  20
<212>  DNA
<213>  Artificial sequence

<220>
<223>  reverse primer

<400>  1232
gaggcctacg ctgattctga                                                   20


<210>  1233
<211>  20
<212>  DNA
<213>  Artificial sequence

<220>
<223>  reverse primer

<400>  1233
caagcacagg agacgatgaa                                                   20

<210>  1234
<211>  20
<212>  DNA
<213>  Artificial sequence

<220>
<223>  reverse primer

<400>  1234
aacacgtttc aagattgggc                                                    20


<210>  1235
<211>  20
<212>  DNA
<213>  Artificial sequence

<220>
<223>  reverse primer

<400>  1235
ccaatccttc gaccacttgt                                                    20


<210>  1236
<211>  20
<212>  DNA
<213>  Artificial sequence

<220>
<223>  reverse primer

<400>  1236
aatgcggtag agaaaccgaa                                                    20


<210>  1237
<211>  20
<212>  DNA
<213>  Artificial sequence

<220>
<223>  reverse primer

<400>  1237
accaaatctt ccgacatcca                                                    20


<210>  1238
<211>  20
<212>  DNA
<213>  Artificial sequence

<220>
<223>  reverse primer

<400>  1238
ccaactgttc gtcaccaatg                                                    20


<210>  1239
<211>  20
<212>  DNA
<213>  Artificial sequence

<220>
<223>  reverse primer

<400>  1239
acgaaccgtt ggttcttcag                                                    20


<210>  1240
<211>  20
<212>  DNA
<213>  Artificial sequence

<220>
<223>  reverse primer

<400>  1240
gttttggaat ctcctcgaac                                                    20


<210>  1241
<211>  20
<212>  DNA
<213>  Artificial sequence

<220>
<223>  reverse primer

<400>  1241
ttccaaggtt aaatgtccgc                                                    20


<210>  1242
<211>  20
<212>  DNA
<213>  Artificial sequence

<220>
<223>  reverse primer

<400>  1242
agtcgtgcaa tccgtctctc                                                    20


<210>  1243
<211>  20
<212>  DNA
<213>  Artificial sequence

<220>
<223>  reverse primer

<400>  1243
cacgttgtgg tatttcagcg                                                    20


<210>  1244
<211>  20
<212>  DNA
<213>  Artificial sequence

<220>
<223>  reverse primer

<400>  1244
caatcaattg ggctactggg                                                    20


<210>  1245
<211>  20
<212>  DNA
<213>  Artificial sequence

<220>
<223>  reverse primer

<400>  1245
gcgagcgagt atatccgagt                                                    20


<210>  1246
<211>  20
<212>  DNA
<213>  Artificial sequence

<220>
<223>  reverse primer

<400>  1246
tgttccttgg cataagtgga                                                    20


<210>  1247
<211>  20
<212>  DNA
<213>  Artificial sequence

<220>
<223>  reverse primer

<400>  1247
tatcaatctt cgctacgcca                                                    20


<210>  1248
<211>  20
<212>  DNA
<213>  Artificial sequence

<220>
<223>  reverse primer

<400>  1248
aatgatagac cagcccatgc                                                    20


<210>  1249
<211>  20
<212>  DNA
<213>  Artificial sequence

<220>
<223>  reverse primer

<400>  1249
ataatgagct gccacgaacc                                                    20


<210>  1250
<211>  20
<212>  DNA
<213>  Artificial sequence

<220>
<223>  reverse primer

<400>  1250
ctttcgaagg cttcatggac                                                    20


<210>  1251

<211>  20
<212>  DNA
<213>  Artificial sequence

<220>
<223>  reverse primer

<400>  1251
gcccctttgg aatcatacct                                                    20


<210>  1252
<211>  20
<212>  DNA
<213>  Artificial sequence

<220>
<223>  reverse primer

<400>  1252
tgccaaggca acaatacaaa                                                    20


<210>  1253
<211>  20
<212>  DNA
<213>  Artificial sequence

<220>
<223>  reverse primer

<400>  1253
ggccacttga tttcagcatt                                                    20


<210>  1254
<211>  20
<212>  DNA
<213>  Artificial sequence

<220>
<223>  reverse primer

<400>  1254
catttcattg ccgttgagtg                                                    20


<210>  1255
<211>  20
<212>  DNA
<213>  Artificial sequence

<220>
<223>  reverse primer

<400>  1255
tggaatggaa ttcaagggaa                                                    20


<210>  1256
<211>  20
<212>  DNA
<213>  Artificial sequence

<220>
<223>  reverse primer

<400>  1256
atcgacttgg gaaacgctta                                                    20

<210>  1257
<211>  20
<212>  DNA
<213>  Artificial sequence

<220>
<223>  reverse primer

<400>  1257
tggaagctgc tgaagatgtg                                                    20


<210>  1258
<211>  20
<212>  DNA
<213>  Artificial sequence

<220>
<223>  reverse primer

<400>  1258
gttacaaccc ccagtctccc                                                    20


<210>  1259
<211>  20
<212>  DNA
<213>  Artificial sequence

<220>
<223>  reverse primer

<400>  1259
accattagcg ggtattgcag                                                    20


<210>  1260
<211>  20
<212>  DNA
<213>  Artificial sequence

<220>
<223>  reverse primer

<400>  1260
ttgctgcttc ctagcctgtt                                                    20


<210>  1261
<211>  20
<212>  DNA
<213>  Artificial sequence

<220>
<223>  reverse primer

<400>  1261
accgacatct tcggagacac                                                    20


<210>  1262
<211>  20
<212>  DNA
<213>  Artificial sequence

<220>
<223>  reverse primer

<400>  1262
tcttcgcagg aacttttggt                                                    20


<210>  1263
<211>  20
<212>  DNA
<213>  Artificial sequence

<220>
<223>  reverse primer

<400>  1263
tagatttctt ccgtgccacc                                                    20


<210>  1264
<211>  20
<212>  DNA
<213>  Artificial sequence

<220>
<223>  reverse primer

<400>  1264
actttagcgg cttcagcaaa                                                    20


<210>  1265
<211>  20
<212>  DNA
<213>  Artificial sequence

<220>
<223>  reverse primer

<400>  1265
cagttttcaa aaacccgcag                                                    20


<210>  1266
<211>  20
<212>  DNA
<213>  Artificial sequence

<220>
<223>  reverse primer

<400>  1266
ttgctttttg attgctcgtg                                                    20


<210>  1267
<211>  20
<212>  DNA
<213>  Artificial sequence

<220>
<223>  reverse primer

<400>  1267
ttaagggaat ttccgccttc                                                    20


<210>  1268
<211>  20
<212>  DNA
<213>  Artificial sequence

<220>
<223>  reverse primer

<400>  1268
gaaatgtgct gcgattgaga                                                    20


<210>  1269
<211>  20
<212>  DNA
<213>  Artificial sequence

<220>
<223>  reverse primer

<400>  1269
aggctgaaaa gtaggtgcga                                                    20


<210>  1270
<211>  20
<212>  DNA
<213>  Artificial sequence

<220>
<223>  reverse primer

<400>  1270
catattgcga accacccttc                                                    20


<210>  1271
<211>  20
<212>  DNA
<213>  Artificial sequence

<220>
<223>  reverse primer

<400>  1271
ctctgcgcct tttccttatg                                                    20


<210>  1272
<211>  20
<212>  DNA
<213>  Artificial sequence

<220>
<223>  reverse primer

<400>  1272
tggaaacatg gtgaaccaaa                                                    20


<210>  1273
<211>  20
<212>  DNA
<213>  Artificial sequence

<220>
<223>  reverse primer

<400>  1273
ccaagtttgc ctttggtggt                                                    20


<210>  1274

<211>  20
<212>  DNA
<213>  Artificial sequence

<220>
<223>  reverse primer

<400>  1274
cccattgaac gtcgtctctt                                                    20


<210>  1275
<211>  20
<212>  DNA
<213>  Artificial sequence

<220>
<223>  reverse primer

<400>  1275
cgatctgttg gagtttggag                                                    20


<210>  1276
<211>  20
<212>  DNA
<213>  Artificial sequence

<220>
<223>  reverse primer

<400>  1276
tacttggcga aatccgtacc                                                    20


<210>  1277
<211>  20
<212>  DNA
<213>  Artificial sequence

<220>
<223>  reverse primer

<400>  1277
caggcttggt attcctccaa                                                    20


<210>  1278
<211>  20
<212>  DNA
<213>  Artificial sequence

<220>
<223>  reverse primer

<400>  1278
tgatgtgaga ttcgggatga                                                    20


<210>  1279
<211>  20
<212>  DNA
<213>  Artificial sequence

<220>
<223>  reverse primer

<400>  1279
actgctccgg taaacatcaa                                                    20

<210> 1280
<211> 20
<212> DNA
<213> Artificial sequence

<220>
<223> reverse primer

<400> 1280
aggcaacacc attaaggacg 20


<210> 1281
<211> 20
<212> DNA
<213> Artificial sequence

<220>
<223> reverse primer

<400> 1281
ttttagacac gagatggccc 20


<210> 1282
<211> 20
<212> DNA
<213> Artificial sequence

<220>
<223> reverse primer

<400> 1282
ttgggtaatc gaagccttgt 20


<210> 1283
<211> 20
<212> DNA
<213> Artificial sequence

<220>
<223> reverse primer

<400> 1283
cgaaagcaag tggaacacaa 20


<210> 1284
<211> 20
<212> DNA
<213> Artificial sequence

<220>
<223> reverse primer

<400> 1284
ttgtgcgcgg taatttgtaa 20


<210> 1285
<211> 20
<212> DNA
<213> Artificial sequence

<220>
<223> reverse primer

<400>  1285
acttcaaacc ttggcacgtc                                                   20


<210>  1286
<211>  20
<212>  DNA
<213>  Artificial sequence

<220>
<223>  reverse primer

<400>  1286
atgatccaaa cacgtgcaaa                                                   20


<210>  1287
<211>  20
<212>  DNA
<213>  Artificial sequence

<220>
<223>  reverse primer

<400>  1287
cctcatatca cgggtccaat                                                   20


<210>  1288
<211>  20
<212>  DNA
<213>  Artificial sequence

<220>
<223>  reverse primer

<400>  1288
ccttcctttt cccttcatcc                                                   20


<210>  1289
<211>  20
<212>  DNA
<213>  Artificial sequence

<220>
<223>  reverse primer

<400>  1289
gtgaccttcg attgtgggtt                                                   20


<210>  1290
<211>  20
<212>  DNA
<213>  Artificial sequence

<220>
<223>  reverse primer

<400>  1290
ttaccgatca actggccatc                                                   20


<210>  1291
<211>  20
<212>  DNA
<213>  Artificial sequence

<220>
<223>  reverse primer

<400>  1291
taagtcccag cagtatcgcc                                                   20


<210>  1292
<211>  20
<212>  DNA
<213>  Artificial sequence

<220>
<223>  reverse primer

<400>  1292
agccgggttg actgttgtag                                                   20


<210>  1293
<211>  20
<212>  DNA
<213>  Artificial sequence

<220>
<223>  reverse primer

<400>  1293
gcagcgttca aatccactct                                                   20


<210>  1294
<211>  20
<212>  DNA
<213>  Artificial sequence

<220>
<223>  reverse primer

<400>  1294
acaagacacc aaagatcggc                                                   20


<210>  1295
<211>  20
<212>  DNA
<213>  Artificial sequence

<220>
<223>  reverse primer

<400>  1295
tgtggctcag cagactgttt                                                   20


<210>  1296
<211>  20
<212>  DNA
<213>  Artificial sequence

<220>
<223>  reverse primer

<400>  1296
tctgccagta gaacatcccc                                                   20


<210>  1297

<211>  20
<212>  DNA
<213>  Artificial sequence

<220>
<223>  reverse primer

<400>  1297
gcccattgac gtctcttgat                                                    20


<210>  1298
<211>  20
<212>  DNA
<213>  Artificial sequence

<220>
<223>  reverse primer

<400>  1298
taccgatcga catcgtttga                                                    20


<210>  1299
<211>  20
<212>  DNA
<213>  Artificial sequence

<220>
<223>  reverse primer

<400>  1299
tgtttaacgg aagattgccc                                                    20


<210>  1300
<211>  20
<212>  DNA
<213>  Artificial sequence

<220>
<223>  reverse primer

<400>  1300
tggaataata acttcgcggc                                                    20


<210>  1301
<211>  20
<212>  DNA
<213>  Artificial sequence

<220>
<223>  reverse primer

<400>  1301
agcctctctc ctcgcctact                                                    20


<210>  1302
<211>  20
<212>  DNA
<213>  Artificial sequence

<220>
<223>  reverse primer

<400>  1302
tctgttttct ctcttcgggc                                                    20

<210>  1303
<211>  20
<212>  DNA
<213>  Artificial sequence

<220>
<223>  reverse primer

<400>  1303
caccattcca aagagcgttt                                                    20


<210>  1304
<211>  20
<212>  DNA
<213>  Artificial sequence

<220>
<223>  reverse primer

<400>  1304
tttcatcttc aacaaggggc                                                    20


<210>  1305
<211>  20
<212>  DNA
<213>  Artificial sequence

<220>
<223>  reverse primer

<400>  1305
aagtcactgg ccagaaatgg                                                    20


<210>  1306
<211>  20
<212>  DNA
<213>  Artificial sequence

<220>
<223>  reverse primer

<400>  1306
gctttcctcg gtatagggct                                                    20


<210>  1307
<211>  20
<212>  DNA
<213>  Artificial sequence

<220>
<223>  reverse primer

<400>  1307
ccggctcctt ctcctagaat                                                    20


<210>  1308
<211>  20
<212>  DNA
<213>  Artificial sequence

<220>
<223>  reverse primer

<400>  1308
tttcgttctc cctcatccac                                                    20


<210>  1309
<211>  20
<212>  DNA
<213>  Artificial sequence

<220>
<223>  reverse primer

<400>  1309
cagcctcgaa gtcaaattcc                                                    20


<210>  1310
<211>  20
<212>  DNA
<213>  Artificial sequence

<220>
<223>  reverse primer

<400>  1310
ttccgtcctt aacccaatca                                                    20


<210>  1311
<211>  20
<212>  DNA
<213>  Artificial sequence

<220>
<223>  reverse primer

<400>  1311
cagccacgct tgaagtaaca                                                    20


<210>  1312
<211>  20
<212>  DNA
<213>  Artificial sequence

<220>
<223>  reverse primer

<400>  1312
tccagcagca tatcttcacg                                                    20


<210>  1313
<211>  20
<212>  DNA
<213>  Artificial sequence

<220>
<223>  reverse primer

<400>  1313
ttggtcggca taaacacaaa                                                    20


<210>  1314
<211>  20
<212>  DNA
<213>  Artificial sequence

<220>
<223>  reverse primer

<400>  1314
acgtaggtag ccatgccaag                                                    20


<210>  1315
<211>  20
<212>  DNA
<213>  Artificial sequence

<220>
<223>  reverse primer

<400>  1315
ctgctatgct cgtgaccctt                                                    20


<210>  1316
<211>  20
<212>  DNA
<213>  Artificial sequence

<220>
<223>  reverse primer

<400>  1316
attcactacg ccctgtgagg                                                    20


<210>  1317
<211>  20
<212>  DNA
<213>  Artificial sequence

<220>
<223>  reverse primer

<400>  1317
gaaagacctc gccatttcaa                                                    20


<210>  1318
<211>  20
<212>  DNA
<213>  Artificial sequence

<220>
<223>  reverse primer

<400>  1318
attgacggag gcatgttttc                                                    20


<210>  1319
<211>  20
<212>  DNA
<213>  Artificial sequence

<220>
<223>  reverse primer

<400>  1319
tttgaccaaa gatggcaaga                                                    20


<210>  1320

<211>  20
<212>  DNA
<213>  Artificial sequence

<220>
<223>  reverse primer

<400>  1320
gacgcatgtt taccccagat                                                    20


<210>  1321
<211>  20
<212>  DNA
<213>  Artificial sequence

<220>
<223>  reverse primer

<400>  1321
gatgattcga cggcttgagt                                                    20


<210>  1322
<211>  20
<212>  DNA
<213>  Artificial sequence

<220>
<223>  reverse primer

<400>  1322
gcactgtctg tatcgtcgga                                                    20


<210>  1323
<211>  20
<212>  DNA
<213>  Artificial sequence

<220>
<223>  reverse primer

<400>  1323
tttcaggaac aaccctttgg                                                    20


<210>  1324
<211>  20
<212>  DNA
<213>  Artificial sequence

<220>
<223>  reverse primer

<400>  1324
ttcgacgtca gcatcaaaag                                                    20


<210>  1325
<211>  20
<212>  DNA
<213>  Artificial sequence

<220>
<223>  reverse primer

<400>  1325
aagggtgagg ctctaacggt                                                    20

<210>  1326
<211>  20
<212>  DNA
<213>  Artificial sequence

<220>
<223>  reverse primer

<400>  1326
aaagtaacga atggcatcgg                                                    20


<210>  1327
<211>  20
<212>  DNA
<213>  Artificial sequence

<220>
<223>  reverse primer

<400>  1327
agccttgaaa gcagccaata                                                    20


<210>  1328
<211>  20
<212>  DNA
<213>  Artificial sequence

<220>
<223>  reverse primer

<400>  1328
tcaaaatccg caaacatcaa                                                    20


<210>  1329
<211>  20
<212>  DNA
<213>  Artificial sequence

<220>
<223>  reverse primer

<400>  1329
tcatttggat ttccatgggt                                                    20


<210>  1330
<211>  20
<212>  DNA
<213>  Artificial sequence

<220>
<223>  reverse primer

<400>  1330
catttgggat gcttcttgct                                                    20


<210>  1331
<211>  20
<212>  DNA
<213>  Artificial sequence

<220>
<223>  reverse primer

<400>  1331
aaccccaatt tgatgttcca                                                    20


<210>  1332
<211>  20
<212>  DNA
<213>  Artificial sequence

<220>
<223>  reverse primer

<400>  1332
taaccggcat tccaaagaac                                                    20


<210>  1333
<211>  20
<212>  DNA
<213>  Artificial sequence

<220>
<223>  reverse primer

<400>  1333
tattgggcgc agatacatca                                                    20


<210>  1334
<211>  20
<212>  DNA
<213>  Artificial sequence

<220>
<223>  reverse primer

<400>  1334
   tcccatcctt ttccttttcc                                                     20


<210>  1335
<211>  20
<212>  DNA
<213>  Artificial sequence

<220>
<223>  reverse primer

<400>  1335
agaattctta gccgcatgga                                                    20


<210>  1336
<211>  20
<212>  DNA
<213>  Artificial sequence

<220>
<223>  reverse primer

<400>  1336
ggtctgggtc actccacagt                                                    20


<210>  1337
<211>  20
<212>  DNA
<213>  Artificial sequence

<220>
<223>  reverse primer

<400>  1337
cgtcctaacg ctcccagtag                                                    20


<210>  1338
<211>  20
<212>  DNA
<213>  Artificial sequence

<220>
<223>  reverse primer

<400>  1338
ggcttaccat taccgcagtc                                                    20


<210>  1339
<211>  20
<212>  DNA
<213>  Artificial sequence

<220>
<223>  reverse primer

<400>  1339
caggatccag gatgtttgct                                                    20


<210>  1340
<211>  20
<212>  DNA
<213>  Artificial sequence

<220>
<223>  reverse primer

<400>  1340
ggcacttcca aagttgtggt                                                    20


<210>  1341
<211>  20
<212>  DNA
<213>  Artificial sequence

<220>
<223>  reverse primer

<400>  1341
tgtacctttt ggcgatgaca                                                    20


<210>  1342
<211>  20
<212>  DNA
<213>  Artificial sequence

<220>
<223>  reverse primer

<400>  1342
gagcttgctt gttggtgtca                                                    20


<210>  1343

<211>  20
<212>  DNA
<213>  Artificial sequence

<220>
<223>  reverse primer

<400>  1343
ggcactgcta gatttaccgc                                                    20


<210>  1344
<211>  20
<212>  DNA
<213>  Artificial sequence

<220>
<223>  reverse primer

<400>  1344
cgaaaagcca agtgaagacc                                                    20


<210>  1345
<211>  20
<212>  DNA
<213>  Artificial sequence

<220>
<223>  reverse primer

<400>  1345
cttcctgcat ggttggaagt                                                    20


<210>  1346
<211>  20
<212>  DNA
<213>  Artificial sequence

<220>
<223>  reverse primer

<400>  1346
gggagtactg ctcgttgctc                                                    20


<210>  1347
<211>  20
<212>  DNA
<213>  Artificial sequence

<220>
<223>  reverse primer

<400>  1347
caacccaata acctcatcgc                                                    20


<210>  1348
<211>  20
<212>  DNA
<213>  Artificial sequence

<220>
<223>  reverse primer

<400>  1348
atgattggaa cgttgtctgg                                                    20

<210>  1349
<211>  20
<212>  DNA
<213>  Artificial sequence

<220>
<223>  reverse primer

<400>  1349
ccgctaataa tgctttccgt                                                    20


<210>  1350
<211>  20
<212>  DNA
<213>  Artificial sequence

<220>
<223>  reverse primer

<400>  1350
ccaaatggtg ttcccaattc                                                    20


<210>  1351
<211>  20
<212>  DNA
<213>  Artificial sequence

<220>
<223>  reverse primer

<400>  1351
tgcgcgagtg aagagactta                                                    20


<210>  1352
<211>  20
<212>  DNA
<213>  Artificial sequence

<220>
<223>  reverse primer

<400>  1352
attatcatgt gacacacggg                                                    20


<210>  1353
<211>  20
<212>  DNA
<213>  Artificial sequence

<220>
<223>  reverse primer

<400>  1353
tcggcttcct cagtgactct                                                    20


<210>  1354
<211>  20
<212>  DNA
<213>  Artificial sequence

<220>
<223>  reverse primer

<400>  1354
tctttggaga agcggaagag                                                    20


<210>  1355
<211>  20
<212>  DNA
<213>  Artificial sequence

<220>
<223>  reverse primer

<400>  1355
tttgcctcca cttcttggac                                                    20


<210>  1356
<211>  20
<212>  DNA
<213>  Artificial sequence

<220>
<223>  reverse primer

<400>  1356
cttcgtcatt cgcaccacta                                                    20


<210>  1357
<211>  20
<212>  DNA
<213>  Artificial sequence

<220>
<223>  reverse primer

<400>  1357
gtgctctcag agtccaagcc                                                    20


<210>  1358
<211>  20
<212>  DNA
<213>  Artificial sequence

<220>
<223>  reverse primer

<400>  1358
cagtcttgcc tcttcttcgg                                                    20


<210>  1359
<211>  20
<212>  DNA
<213>  Artificial sequence

<220>
<223>  reverse primer

<400>  1359
atgtcccaag tggaaaacca                                                    20


<210>  1360
<211>  20
<212>  DNA
<213>  Artificial sequence

<220>
<223>  reverse primer

<400>  1360
tctgagggaa cagggctaga                                                    20


<210>  1361
<211>  20
<212>  DNA
<213>  Artificial sequence

<220>
<223>  reverse primer

<400>  1361
atcttgggcg ctcacattag                                                    20


<210>  1362
<211>  20
<212>  DNA
<213>  Artificial sequence

<220>
<223>  reverse primer

<400>  1362
tccaaaaacg cccagaatac                                                    20


<210>  1363
<211>  20
<212>  DNA
<213>  Artificial sequence

<220>
<223>  reverse primer

<400>  1363
atgagtggtt tcgttttccg                                                    20


<210>  1364
<211>  20
<212>  DNA
<213>  Artificial sequence

<220>
<223>  reverse primer

<400>  1364
ctggcctgtg ggtgataact                                                    20


<210>  1365
<211>  20
<212>  DNA
<213>  Artificial sequence

<220>
<223>  reverse primer

<400>  1365
gcttgtttaa aatcctccgc                                                    20


<210>  1366

<211>  20
<212>  DNA
<213>  Artificial sequence

<220>
<223>  reverse primer

<400>  1366
ccttgttgtg ttccaatccc                                                    20


<210>  1367
<211>  20
<212>  DNA
<213>  Artificial sequence

<220>
<223>  reverse primer

<400>  1367
aaatgtcttt gaaccgaccg                                                    20


<210>  1368
<211>  20
<212>  DNA
<213>  Artificial sequence

<220>
<223>  reverse primer

<400>  1368
gcctggtttg tcatgtttga                                                    20


<210>  1369
<211>  20
<212>  DNA
<213>  Artificial sequence

<220>
<223>  reverse primer

<400>  1369
ctgagccctt tcgtaagcag                                                    20


<210>  1370
<211>  20
<212>  DNA
<213>  Artificial sequence

<220>
<223>  reverse primer

<400>  1370
gggcaaggaa taattcacga                                                    20


<210>  1371
<211>  20
<212>  DNA
<213>  Artificial sequence

<220>
<223>  reverse primer

<400>  1371
ggcacagaat gcaatgacac                                                    20

<210>  1372
<211>  20
<212>  DNA
<213>  Artificial sequence

<220>
<223>  reverse primer

<400>  1372
gactggcttt cagcatctcc                                                   20


<210>  1373
<211>  20
<212>  DNA
<213>  Artificial sequence

<220>
<223>  reverse primer

<400>  1373
cttgcgatat ttttctgggc                                                   20


<210>  1374
<211>  20
<212>  DNA
<213>  Artificial sequence

<220>
<223>  reverse primer

<400>  1374
gatgggtacg gaactcgaaa                                                   20


<210>  1375
<211>  20
<212>  DNA
<213>  Artificial sequence

<220>
<223>  reverse primer

<400>  1375
ccccattttc cgtgttctta                                                   20


<210>  1376
<211>  20
<212>  DNA
<213>  Artificial sequence

<220>
<223>  reverse primer

<400>  1376
ttttcatggc gcttaatacg                                                   20


<210>  1377
<211>  20
<212>  DNA
<213>  Artificial sequence

<220>
<223>  reverse primer

<400>  1377
gccaatcatg gaccaaatct                                                    20


<210>  1378
<211>  20
<212>  DNA
<213>  Artificial sequence

<220>
<223>  reverse primer

<400>  1378
tcgcttcata tccgcttacc                                                    20


<210>  1379
<211>  20
<212>  DNA
<213>  Artificial sequence

<220>
<223>  reverse primer

<400>  1379
aaactgctgg ctttgcttgt                                                    20


<210>  1380
<211>  20
<212>  DNA
<213>  Artificial sequence

<220>
<223>  reverse primer

<400>  1380
gatttcagtg ttcggaaggc                                                    20


<210>  1381
<211>  20
<212>  DNA
<213>  Artificial sequence

<220>
<223>  reverse primer

<400>  1381
attgtggaag cgacaccttc                                                    20


<210>  1382
<211>  20
<212>  DNA
<213>  Artificial sequence

<220>
<223>  reverse primer

<400>  1382
tggcataatg aaacgaagca                                                    20


<210>  1383
<211>  20
<212>  DNA
<213>  Artificial sequence

<220>
<223>  reverse primer

<400>  1383
cgtccctctc aatgttgtcc                                                    20


<210>  1384
<211>  20
<212>  DNA
<213>  Artificial sequence

<220>
<223>  reverse primer

<400>  1384
cctcagccac atggaaatct                                                    20


<210>  1385
<211>  20
<212>  DNA
<213>  Artificial sequence

<220>
<223>  reverse primer

<400>  1385
gtcgttggta tgctccacct                                                    20


<210>  1386
<211>  20
<212>  DNA
<213>  Artificial sequence

<220>
<223>  reverse primer

<400>  1386
gtaagacgac ccgtccttga                                                    20


<210>  1387
<211>  20
<212>  DNA
<213>  Artificial sequence

<220>
<223>  reverse primer

<400>  1387
ggacgctttt gttttcttgc                                                    20


<210>  1388
<211>  20
<212>  DNA
<213>  Artificial sequence

<220>
<223>  reverse primer

<400>  1388
aagaggagga agaagaggcg                                                    20


<210>  1389

<211>  20
<212>  DNA
<213>  Artificial sequence

<220>
<223>  reverse primer

<400>  1389
gatcttcgca tcggaactgt                                                    20


<210>  1390
<211>  20
<212>  DNA
<213>  Artificial sequence

<220>
<223>  reverse primer

<400>  1390
cttgtgatgt ttgtggtgcc                                                    20


<210>  1391
<211>  20
<212>  DNA
<213>  Artificial sequence

<220>
<223>  reverse primer

<400>  1391
attgttctgg ggtgttttcg                                                    20


<210>  1392
<211>  20
<212>  DNA
<213>  Artificial sequence

<220>
<223>  reverse primer

<400>  1392
tgttgttctt gctgctggtc                                                    20


<210>  1393
<211>  20
<212>  DNA
<213>  Artificial sequence

<220>
<223>  reverse primer

<400>  1393
gtggtaggaa gcagcaatgg                                                    20


<210>  1394
<211>  20
<212>  DNA
<213>  Artificial sequence

<220>
<223>  reverse primer

<400>  1394
cgacgaattt ggttctccat                                                    20

<210>  1395
<211>  20
<212>  DNA
<213>  Artificial sequence

<220>
<223>  reverse primer

<400>  1395
ttttgtttcc ggaagattgc                                                    20


<210>  1396
<211>  20
<212>  DNA
<213>  Artificial sequence

<220>
<223>  reverse primer

<400>  1396
gttcgcatag gaaatagccg                                                    20


<210>  1397
<211>  20
<212>  DNA
<213>  Artificial sequence

<220>
<223>  reverse primer

<400>  1397
ctgccaaatt gacgctgata                                                    20


<210>  1398
<211>  20
<212>  DNA
<213>  Artificial sequence

<220>
<223>  reverse primer

<400>  1398
agtggaagca cccatggtaa                                                    20


<210>  1399
<211>  20
<212>  DNA
<213>  Artificial sequence

<220>
<223>  reverse primer

<400>  1399
atcagctctg ttaccaacat                                                    20


<210>  1400
<211>  20
<212>  DNA
<213>  Artificial sequence

<220>
<223>  reverse primer

<400>  1400
ctcatcgatc aacagcgaga                                                    20


<210>  1401
<211>  20
<212>  DNA
<213>  Artificial sequence

<220>
<223>  reverse primer

<400>  1401
gatgctcgta cgttattttt                                                    20


<210>  1402
<211>  20
<212>  DNA
<213>  Artificial sequence

<220>
<223>  reverse primer

<400>  1402
ccaggagttg ggaattgaaa                                                    20


<210>  1403
<211>  20
<212>  DNA
<213>  Artificial sequence

<220>
<223>  reverse primer

<400>  1403
caagacggca accaactttt                                                    20


<210>  1404
<211>  20
<212>  DNA
<213>  Artificial sequence

<220>
<223>  reverse primer

<400>  1404
atttctatct tgcgctgcgt                                                    20


<210>  1405
<211>  20
<212>  DNA
<213>  Artificial sequence

<220>
<223>  reverse primer

<400>  1405
tggcaaccaa tgaacacaac                                                    20


<210>  1406
<211>  20
<212>  DNA
<213>  Artificial sequence

<220>
<223>  reverse primer

<400>  1406
caagccttgg caagtttctc                                                   20


<210>  1407
<211>  20
<212>  DNA
<213>  Artificial sequence

<220>
<223>  reverse primer

<400>  1407
cattgtcatg gtcgcttctg                                                   20


<210>  1408
<211>  20
<212>  DNA
<213>  Artificial sequence

<220>
<223>  reverse primer

<400>  1408
tgtatcccat ttttcagggc                                                   20


<210>  1409
<211>  20
<212>  DNA
<213>  Artificial sequence

<220>
<223>  reverse primer

<400>  1409
ttgccttctg ggttggatac                                                   20


<210>  1410
<211>  20
<212>  DNA
<213>  Artificial sequence

<220>
<223>  reverse primer

<400>  1410
atgatggtgc actcctcctc                                                   20


<210>  1411
<211>  20
<212>  DNA
<213>  Artificial sequence

<220>
<223>  reverse primer

<400>  1411
cctggtaaga gcaatacgcc                                                   20


<210>  1412

<211>  20
<212>  DNA
<213>  Artificial sequence

<220>
<223>  reverse primer

<400>  1412
caggcttctt ccctctgtgt                                                    20


<210>  1413
<211>  20
<212>  DNA
<213>  Artificial sequence

<220>
<223>  reverse primer

<400>  1413
gctcgcacct ttccaaatta                                                    20


<210>  1414
<211>  20
<212>  DNA
<213>  Artificial sequence

<220>
<223>  reverse primer

<400>  1414
gagttgtttg aaccaccgct                                                    20


<210>  1415
<211>  20
<212>  DNA
<213>  Artificial sequence

<220>
<223>  reverse primer

<400>  1415
ttgcgtcagg ttgctcatac                                                    20


<210>  1416
<211>  20
<212>  DNA
<213>  Artificial sequence

<220>
<223>  reverse primer

<400>  1416
tttgcttttc tttggaaccc                                                    20


<210>  1417
<211>  20
<212>  DNA
<213>  Artificial sequence

<220>
<223>  reverse primer

<400>  1417
tgagaggttt gtgcaccttg                                                    20

<210>  1418
<211>  20
<212>  DNA
<213>  Artificial sequence

<220>
<223>  reverse primer

<400>  1418
gcatacgtcg ttcgtcattg                                                    20


<210>  1419
<211>  20
<212>  DNA
<213>  Artificial sequence

<220>
<223>  reverse primer

<400>  1419
cgcctgtaaa ctcacgacaa                                                    20


<210>  1420
<211>  20
<212>  DNA
<213>  Artificial sequence

<220>
<223>  reverse primer

<400>  1420
acaagtgggt gacgttttcc                                                    20


<210>  1421
<211>  20
<212>  DNA
<213>  Artificial sequence

<220>
<223>  reverse primer

<400>  1421
caatcaatgg tctaccggct                                                    20


<210>  1422
<211>  20
<212>  DNA
<213>  Artificial sequence

<220>
<223>  reverse primer

<400>  1422
gccccagata gaagggtttc                                                    20


<210>  1423
<211>  20
<212>  DNA
<213>  Artificial sequence

<220>
<223>  reverse primer

<400>  1423
atttcgccca tcacatcaat                                                    20


<210>  1424
<211>  20
<212>  DNA
<213>  Artificial sequence

<220>
<223>  reverse primer

<400>  1424
cgatgaagtt cgcaaattgt                                                    20


<210>  1425
<211>  20
<212>  DNA
<213>  Artificial sequence

<220>
<223>  reverse primer

<400>  1425
ttcatcttcg aggggaattg                                                    20


<210>  1426
<211>  20
<212>  DNA
<213>  Artificial sequence

<220>
<223>  reverse primer

<400>  1426
cgtaaatgag ttcccgcaat                                                    20


<210>  1427
<211>  20
<212>  DNA
<213>  Artificial sequence

<220>
<223>  reverse primer

<400>  1427
cggattgagg ttttggtttg                                                    20


<210>  1428
<211>  20
<212>  DNA
<213>  Artificial sequence

<220>
<223>  reverse primer

<400>  1428
ttaggataag tgccggatgg                                                    20


<210>  1429
<211>  20
<212>  DNA
<213>  Artificial sequence

<220>
<223>  reverse primer

<400>  1429
acaccgtctt ggatttggtc                                                    20


<210>  1430
<211>  20
<212>  DNA
<213>  Artificial sequence

<220>
<223>  reverse primer

<400>  1430
ccttggcttt tcagccagta                                                    20


<210>  1431
<211>  20
<212>  DNA
<213>  Artificial sequence

<220>
<223>  reverse primer

<400>  1431
tttattgaac gcgcttgttg                                                    20


<210>  1432
<211>  20
<212>  DNA
<213>  Artificial sequence

<220>
<223>  reverse primer

<400>  1432
cagtgtcagg cattgattcg                                                    20


<210>  1433
<211>  20
<212>  DNA
<213>  Artificial sequence

<220>
<223>  reverse primer

<400>  1433
caaatcgccc cttttcagta                                                    20


<210>  1434
<211>  20
<212>  DNA
<213>  Artificial sequence

<220>
<223>  reverse primer

<400>  1434
tcttggcaga ggccttagaa                                                    20


<210>  1435

<211>  20
<212>  DNA
<213>  Artificial sequence

<220>
<223>  reverse primer

<400>  1435
aattggatcc tgagacgctg                                                    20


<210>  1436
<211>  20
<212>  DNA
<213>  Artificial sequence

<220>
<223>  reverse primer

<400>  1436
ggagtggccc ctgtgatatt                                                    20


<210>  1437
<211>  20
<212>  DNA
<213>  Artificial sequence

<220>
<223>  reverse primer

<400>  1437
tgtgcatttt tggctctcag                                                    20


<210>  1438
<211>  20
<212>  DNA
<213>  Artificial sequence

<220>
<223>  reverse primer

<400>  1438
gcctcctcgt catcactctc                                                    20


<210>  1439
<211>  20
<212>  DNA
<213>  Artificial sequence

<220>
<223>  reverse primer

<400>  1439
gttaaccaag ccacctgcat                                                    20


<210>  1440
<211>  20
<212>  DNA
<213>  Artificial sequence

<220>
<223>  reverse primer

<400>  1440
acggcgtctt gaattttgtc                                                    20

<210>  1441
<211>  20
<212>  DNA
<213>  Artificial sequence

<220>
<223>  reverse primer

<400>  1441
cccagtccca aattcaaaaa                                                    20


<210>  1442
<211>  20
<212>  DNA
<213>  Artificial sequence

<220>
<223>  reverse primer

<400>  1442
cttcttagct tgacaggcgg                                                    20


<210>  1443
<211>  20
<212>  DNA
<213>  Artificial sequence

<220>
<223>  reverse primer

<400>  1443
tcgctcgata ggattggaac                                                    20


<210>  1444
<211>  20
<212>  DNA
<213>  Artificial sequence

<220>
<223>  reverse primer

<400>  1444
tggataaatg ttcttgcgct                                                    20


<210>  1445
<211>  20
<212>  DNA
<213>  Artificial sequence

<220>
<223>  reverse primer

<400>  1445
atacggggct taggaaggaa                                                    20


<210>  1446
<211>  20
<212>  DNA
<213>  Artificial sequence

<220>
<223>  reverse primer

<400>  1446
tcataaagag gtccgcaagg                                                   20


<210>  1447
<211>  20
<212>  DNA
<213>  Artificial sequence

<220>
<223>  reverse primer

<400>  1447
agttcttctg caccctccct                                                   20


<210>  1448
<211>  20
<212>  DNA
<213>  Artificial sequence

<220>
<223>  reverse primer

<400>  1448
aacaattgaa agcattcggc                                                   20


<210>  1449
<211>  20
<212>  DNA
<213>  Artificial sequence

<220>
<223>  reverse primer

<400>  1449
ctccttggcc atacttagcg                                                   20


<210>  1450
<211>  20
<212>  DNA
<213>  Artificial sequence

<220>
<223>  reverse primer

<400>  1450
ccctttctgt taacgtcgga                                                   20


<210>  1451
<211>  20
<212>  DNA
<213>  Artificial sequence

<220>
<223>  reverse primer

<400>  1451
ataccctcta atcttgtagg                                                   20


<210>  1452
<211>  20
<212>  DNA
<213>  Artificial sequence

<220>
<223>  reverse primer

<400>  1452
gatctgcaat acggtcggtt                                                    20


<210>  1453
<211>  20
<212>  DNA
<213>  Artificial sequence

<220>
<223>  reverse primer

<400>  1453
gactcgatgc tttcctggtc                                                    20


<210>  1454
<211>  20
<212>  DNA
<213>  Artificial sequence

<220>
<223>  reverse primer

<400>  1454
tcccacatgg tttcagacaa                                                    20


<210>  1455
<211>  20
<212>  DNA
<213>  Artificial sequence

<220>
<223>  reverse primer

<400>  1455
gcttttcttg ccattccaaa                                                    20


<210>  1456
<211>  20
<212>  DNA
<213>  Artificial sequence

<220>
<223>  reverse primer

<400>  1456
tcctctatac catgccgtcc                                                    20


<210>  1457
<211>  20
<212>  DNA
<213>  Artificial sequence

<220>
<223>  reverse primer

<400>  1457
ccgggacatt cttgttcatt                                                    20


<210>  1458

<211>  20
<212>  DNA
<213>  Artificial sequence

<220>
<223>  reverse primer

<400>  1458
cacaccaact gcatcattcc                                                    20


<210>  1459
<211>  20
<212>  DNA
<213>  Artificial sequence

<220>
<223>  reverse primer

<400>  1459
tgccttcaaa acatatgcca                                                    20


<210>  1460
<211>  20
<212>  DNA
<213>  Artificial sequence

<220>
<223>  reverse primer

<400>  1460
aaatttcaga cacgatgccc                                                    20


<210>  1461
<211>  20
<212>  DNA
<213>  Artificial sequence

<220>
<223>  reverse primer

<400>  1461
gcattcggga aacaggagta                                                    20


<210>  1462
<211>  20
<212>  DNA
<213>  Artificial sequence

<220>
<223>  reverse primer

<400>  1462
tttttcatcg tccagtgtgc                                                    20


<210>  1463
<211>  20
<212>  DNA
<213>  Artificial sequence

<220>
<223>  reverse primer

<400>  1463
agagatggca gaggtcttgg                                                    20

<210>  1464
<211>  20
<212>  DNA
<213>  Artificial sequence

<220>
<223>  reverse primer

<400>  1464
acttccatca ccatccgttc                                                    20


<210>  1465
<211>  20
<212>  DNA
<213>  Artificial sequence

<220>
<223>  reverse primer

<400>  1465
ccagacgatg ggtaacctgt                                                    20


<210>  1466
<211>  20
<212>  DNA
<213>  Artificial sequence

<220>
<223>  reverse primer

<400>  1466
tgtgaattgg catttgggta                                                    20


<210>  1467
<211>  20
<212>  DNA
<213>  Artificial sequence

<220>
<223>  reverse primer

<400>  1467
caattcgtcg tgttcgaaat                                                    20


<210>  1468
<211>  20
<212>  DNA
<213>  Artificial sequence

<220>
<223>  reverse primer

<400>  1468
aatgatagtc accggaacgc                                                    20


<210>  1469
<211>  20
<212>  DNA
<213>  Artificial sequence

<220>
<223>  reverse primer

<400>  1469
tgatgtgtcg gataacccaa                                                    20

<210>  1470
<211>  20
<212>  DNA
<213>  Artificial sequence

<220>
<223>  reverse primer

<400>  1470
cgttgaaact ttctcagccc                                                    20

<210>  1471
<211>  20
<212>  DNA
<213>  Artificial sequence

<220>
<223>  reverse primer

<400>  1471
gcttgaccaa caagagaggc                                                    20

<210>  1472
<211>  20
<212>  DNA
<213>  Artificial sequence

<220>
<223>  reverse primer

<400>  1472
aagaccccat cttgggtagc                                                    20

<210>  1473
<211>  20
<212>  DNA
<213>  Artificial sequence

<220>
<223>  reverse primer

<400>  1473
tcgtcacctg gaactgtttg                                                    20

<210>  1474
<211>  20
<212>  DNA
<213>  Artificial sequence

<220>
<223>  reverse primer

<400>  1474
actccgttgg aacgctctta                                                    20

<210>  1475
<211>  20
<212>  DNA
<213>  Artificial sequence

<220>
<223>  reverse primer

<400>  1475
cgatatttga tgttgtggcg                                                    20


<210>  1476
<211>  20
<212>  DNA
<213>  Artificial sequence

<220>
<223>  reverse primer

<400>  1476
ggttaagtct tgccggttca                                                    20


<210>  1477
<211>  20
<212>  DNA
<213>  Artificial sequence

<220>
<223>  reverse primer

<400>  1477
ctagattggg ttgcggaaga                                                    20


<210>  1478
<211>  20
<212>  DNA
<213>  Artificial sequence

<220>
<223>  reverse primer

<400>  1478
gagcaatgga agtagccgag                                                    20


<210>  1479
<211>  20
<212>  DNA
<213>  Artificial sequence

<220>
<223>  reverse primer

<400>  1479
cttgaagtgg ggtcgaggta                                                    20


<210>  1480
<211>  20
<212>  DNA
<213>  Artificial sequence

<220>
<223>  reverse primer

<400>  1480
   atggttgcgt ttttctctgt                                                     20


<210>  1481

<211>  20
<212>  DNA
<213>  Artificial sequence

<220>
<223>  reverse primer

<400>  1481
gttcagcggc cttttgaata                                                    20


<210>  1482
<211>  20
<212>  DNA
<213>  Artificial sequence

<220>
<223>  reverse primer

<400>  1482
cctcgtcaaa cccttcttga                                                    20


<210>  1483
<211>  20
<212>  DNA
<213>  Artificial sequence

<220>
<223>  reverse primer

<400>  1483
ttttcaggaa ttcattggca                                                    20


<210>  1484
<211>  20
<212>  DNA
<213>  Artificial sequence

<220>
<223>  reverse primer

<400>  1484
cgtttcatca gaatcgctca                                                    20


<210>  1485
<211>  20
<212>  DNA
<213>  Artificial sequence

<220>
<223>  reverse primer

<400>  1485
aagtcttcct tcccatggct                                                    20


<210>  1486
<211>  20
<212>  DNA
<213>  Artificial sequence

<220>
<223>  reverse primer

<400>  1486
gtttcaccag attcggcatt                                                    20

<210>  1487
<211>  20
<212>  DNA
<213>  Artificial sequence

<220>
<223>  reverse primer

<400>  1487
tcttccattt cttgctcggt                                                    20


<210>  1488
<211>  20
<212>  DNA
<213>  Artificial sequence

<220>
<223>  reverse primer

<400>  1488
ttctcttcga tgtgtgtcgc                                                    20


<210>  1489
<211>  20
<212>  DNA
<213>  Artificial sequence

<220>
<223>  reverse primer

<400>  1489
ttaatctata tccatgtaat                                                    20


<210>  1490
<211>  20
<212>  DNA
<213>  Artificial sequence

<220>
<223>  reverse primer

<400>  1490
cggaaacagt gctagtggct                                                    20


<210>  1491
<211>  20
<212>  DNA
<213>  Artificial sequence

<220>
<223>  reverse primer

<400>  1491
ctagttttgg tcctcccgtg                                                    20


<210>  1492
<211>  20
<212>  DNA
<213>  Artificial sequence

<220>
<223>  reverse primer

<400>  1492
ttatattctg gacccagcgg                                                   20


<210>  1493
<211>  20
<212>  DNA
<213>  Artificial sequence

<220>
<223>  reverse primer

<400>  1493
ccgaaagcgc tattatcacc                                                   20


<210>  1494
<211>  20
<212>  DNA
<213>  Artificial sequence

<220>
<223>  reverse primer

<400>  1494
tttccagaat cattggctcc                                                   20


<210>  1495
<211>  20
<212>  DNA
<213>  Artificial sequence

<220>
<223>  reverse primer

<400>  1495
tgcaacggtt tattttgttg                                                   20


<210>  1496
<211>  20
<212>  DNA
<213>  Artificial sequence

<220>
<223>  reverse primer

<400>  1496
accagaagcg gatgagactg                                                   20


<210>  1497
<211>  20
<212>  DNA
<213>  Artificial sequence

<220>
<223>  reverse primer

<400>  1497
taaaccttgg gattgaacgc                                                   20


<210>  1498
<211>  20
<212>  DNA
<213>  Artificial sequence

<220>
<223>  reverse primer

<400>  1498
gatttgttac cccaggctga                                                    20


<210>  1499
<211>  20
<212>  DNA
<213>  Artificial sequence

<220>
<223>  reverse primer

<400>  1499
ggttggcgcc attaatacac                                                    20


<210>  1500
<211>  20
<212>  DNA
<213>  Artificial sequence

<220>
<223>  reverse primer

<400>  1500
tattgaccaa tgagacgcca                                                    20


<210>  1501
<211>  20
<212>  DNA
<213>  Artificial sequence

<220>
<223>  reverse primer

<400>  1501
cttgccgttc ttggtgctat                                                    20


<210>  1502
<211>  20
<212>  DNA
<213>  Artificial sequence

<220>
<223>  reverse primer

<400>  1502
tgcggtatgc aatctgtctc                                                    20


<210>  1503
<211>  20
<212>  DNA
<213>  Artificial sequence

<220>
<223>  reverse primer

<400>  1503
atggactggt tgtattcggg                                                    20


<210>  1504

<211>  20
<212>  DNA
<213>  Artificial sequence

<220>
<223>  reverse primer

<400>  1504
caattgtgca aactccggta                                                    20


<210>  1505
<211>  20
<212>  DNA
<213>  Artificial sequence

<220>
<223>  reverse primer

<400>  1505
ttcccacaga cgcaatgtta                                                    20


<210>  1506
<211>  20
<212>  DNA
<213>  Artificial sequence

<220>
<223>  reverse primer

<400>  1506
gttgcattgt cctggtgttg                                                    20


<210>  1507
<211>  20
<212>  DNA
<213>  Artificial sequence

<220>
<223>  reverse primer

<400>  1507
aaggcatgtc tgttgttctg                                                    20


<210>  1508
<211>  20
<212>  DNA
<213>  Artificial sequence

<220>
<223>  reverse primer

<400>  1508
caaaccgacc ttgccactat                                                    20


<210>  1509
<211>  20
<212>  DNA
<213>  Artificial sequence

<220>
<223>  reverse primer

<400>  1509
acaaagacgc ttgcgaagat                                                    20

<210>  1510
<211>  20
<212>  DNA
<213>  Artificial sequence

<220>
<223>  reverse primer

<400>  1510
tcgttgtttg gtcttggaga                                                    20


<210>  1511
<211>  20
<212>  DNA
<213>  Artificial sequence

<220>
<223>  reverse primer

<400>  1511
gtagcagaaa gagggcacca                                                    20


<210>  1512
<211>  20
<212>  DNA
<213>  Artificial sequence

<220>
<223>  reverse primer

<400>  1512
taggggtgta ggggttgtca                                                    20


<210>  1513
<211>  20
<212>  DNA
<213>  Artificial sequence

<220>
<223>  reverse primer

<400>  1513
ttttgaggat ttacggccag                                                    20


<210>  1514
<211>  20
<212>  DNA
<213>  Artificial sequence

<220>
<223>  reverse primer

<400>  1514
aaaatgcttt ctttggccct                                                    20


<210>  1515
<211>  20
<212>  DNA
<213>  Artificial sequence

<220>
<223>  reverse primer

<400>  1515
ttgtcccgat ttctcctgtc                                                    20


<210>  1516
<211>  20
<212>  DNA
<213>  Artificial sequence

<220>
<223>  reverse primer

<400>  1516
tgcggttatg tgctcgatac                                                    20


<210>  1517
<211>  20
<212>  DNA
<213>  Artificial sequence

<220>
<223>  reverse primer

<400>  1517
atgcgcattc gctatcctta                                                    20


<210>  1518
<211>  20
<212>  DNA
<213>  Artificial sequence

<220>
<223>  reverse primer

<400>  1518
ttttcaacca atgctgcaag                                                    20


<210>  1519
<211>  20
<212>  DNA
<213>  Artificial sequence

<220>
<223>  reverse primer

<400>  1519
tgtcgtcgcc aataatttca                                                    20


<210>  1520
<211>  20
<212>  DNA
<213>  Artificial sequence

<220>
<223>  reverse primer

<400>  1520
gggggacgtt accacatcta                                                    20


<210>  1521
<211>  20
<212>  DNA
<213>  Artificial sequence

<220>
<223>  reverse primer

<400>  1521
ataccacgga tcaggaacca                                                    20


<210>  1522
<211>  20
<212>  DNA
<213>  Artificial sequence

<220>
<223>  reverse primer

<400>  1522
ttctgtaagc tctggcggtt                                                    20


<210>  1523
<211>  20
<212>  DNA
<213>  Artificial sequence

<220>
<223>  reverse primer

<400>  1523
tcgagctcaa aggcattttt                                                    20


<210>  1524
<211>  20
<212>  DNA
<213>  Artificial sequence

<220>
<223>  reverse primer

<400>  1524
atcgtccttg agttccgttg                                                    20


<210>  1525
<211>  20
<212>  DNA
<213>  Artificial sequence

<220>
<223>  reverse primer

<400>  1525
aagcaccaag ggcatagaaa                                                    20


<210>  1526
<211>  20
<212>  DNA
<213>  Artificial sequence

<220>
<223>  reverse primer

<400>  1526
aggagctacc atcagcctca                                                    20


<210>  1527

<211>  20
<212>  DNA
<213>  Artificial sequence

<220>
<223>  reverse primer

<400>  1527
tgatggctca tagcacggta                                                    20


<210>  1528
<211>  20
<212>  DNA
<213>  Artificial sequence

<220>
<223>  reverse primer

<400>  1528
gttgcagtgt tgggaggatt                                                    20


<210>  1529
<211>  20
<212>  DNA
<213>  Artificial sequence

<220>
<223>  reverse primer

<400>  1529
taccgtggac cccacataat                                                    20


<210>  1530
<211>  20
<212>  DNA
<213>  Artificial sequence

<220>
<223>  reverse primer

<400>  1530
ggggcaggag tgtcatctaa                                                    20


<210>  1531
<211>  20
<212>  DNA
<213>  Artificial sequence

<220>
<223>  reverse primer

<400>  1531
aggattgttt gtgtttcccg                                                    20


<210>  1532
<211>  20
<212>  DNA
<213>  Artificial sequence

<220>
<223>  reverse primer

<400>  1532
tcttgtttcg ctcggttttt                                                    20

<210>  1533
<211>  20
<212>  DNA
<213>  Artificial sequence

<220>
<223>  reverse primer

<400>  1533
gcacttccac cttgctttgt                                                    20


<210>  1534
<211>  20
<212>  DNA
<213>  Artificial sequence

<220>
<223>  reverse primer

<400>  1534
ctgcccagcg attgttactt                                                    20


<210>  1535
<211>  20
<212>  DNA
<213>  Artificial sequence

<220>
<223>  reverse primer

<400>  1535
tgacactgac cggaattgaa                                                    20


<210>  1536
<211>  20
<212>  DNA
<213>  Artificial sequence

<220>
<223>  reverse primer

<400>  1536
ataatgggct gtggcatcat                                                    20


<210>  1537
<211>  20
<212>  DNA
<213>  Artificial sequence

<220>
<223>  reverse primer

<400>  1537
ctcttcttct tcctttcccg                                                    20


<210>  1538
<211>  20
<212>  DNA
<213>  Artificial sequence

<220>
<223>  reverse primer

<400>  1538
gagtcaccat tagcatcgca                                                    20


<210>  1539
<211>  20
<212>  DNA
<213>  Artificial sequence

<220>
<223>  reverse primer

<400>  1539
gtctgcggaa taaccaggaa                                                    20


<210>  1540
<211>  20
<212>  DNA
<213>  Artificial sequence

<220>
<223>  reverse primer

<400>  1540
aacacgagat gctgacgatg                                                    20


<210>  1541
<211>  20
<212>  DNA
<213>  Artificial sequence

<220>
<223>  reverse primer

<400>  1541
ctaaatccgt ggggaacaaa                                                    20


<210>  1542
<211>  20
<212>  DNA
<213>  Artificial sequence

<220>
<223>  reverse primer

<400>  1542
cgtgacccat agcgaagttt                                                    20


<210>  1543
<211>  20
<212>  DNA
<213>  Artificial sequence

<220>
<223>  reverse primer

<400>  1543
ttaagtcgtc gctggaaggt                                                    20


<210>  1544
<211>  20
<212>  DNA
<213>  Artificial sequence

<220>
<223>  reverse primer

<400>  1544
atagcaccga ggctatcgaa                                                    20


<210>  1545
<211>  20
<212>  DNA
<213>  Artificial sequence

<220>
<223>  reverse primer

<400>  1545
gggtcttgat gatgctggtt                                                    20


<210>  1546
<211>  20
<212>  DNA
<213>  Artificial sequence

<220>
<223>  reverse primer

<400>  1546
atcagcgcca gtagtctcgt                                                    20


<210>  1547
<211>  20
<212>  DNA
<213>  Artificial sequence

<220>
<223>  reverse primer

<400>  1547
acgtcatctg tggcatcaag                                                    20


<210>  1548
<211>  20
<212>  DNA
<213>  Artificial sequence

<220>
<223>  reverse primer

<400>  1548
atcattatcg ttctgcgcct                                                    20


<210>  1549
<211>  20
<212>  DNA
<213>  Artificial sequence

<220>
<223>  reverse primer

<400>  1549
ctgagcggct tagaggattc                                                    20


<210>  1550

<211>  20
<212>  DNA
<213>  Artificial sequence

<220>
<223>  reverse primer

<400>  1550
cctgtgggga tttttcttga                                                    20


<210>  1551
<211>  20
<212>  DNA
<213>  Artificial sequence

<220>
<223>  reverse primer

<400>  1551
tgcatttgtt gttgtgggtt                                                    20


<210>  1552
<211>  20
<212>  DNA
<213>  Artificial sequence

<220>
<223>  reverse primer

<400>  1552
tcatgtggtg ctactggagg                                                    20


<210>  1553
<211>  20
<212>  DNA
<213>  Artificial sequence

<220>
<223>  reverse primer

<400>  1553
catagggttt tgtccggatg                                                    20

## Description

### Field of the invention

The present invention is related to new nucleotide sequences which are specific of *Saccharomyces cerevisiae* genes and which can be used as probes in hybridisation tests especially by using microarray technology for the identification of nucleic acid sequences comprising said genes and present in a biological sample.

### State of the art

DNA array-based technologies enable the determination of gene expression patterns of thousands of genes in a single experiment. Typically, said DNA array technologies involve (*i*) in situ synthesis of oligonucleotides using photochemical techniques or ink-jet oligonucleotide synthetiser, (*ii*) robotic deposition or "spotting" of presynthesized DNA molecules or oligomers on glass slides or filter membranes, (*iii*) hybridisation of the set of "spotted" DNA molecules with complex cDNA targets derived from reverse transcription of mRNA populations from various sources, (*iv*) collection of data by quantitative analysis of signal intensities of arrayed DNA spots, and processing of said collected data using adapted software tools.

Spotted arrays are commonly referred to as "cDNA microarrays", although recombinant plasmids, PCR products, or oligonucleotides can all be spotted.

In *Saccharomyces cerevisiae,* several genomewide expression patterns have already been established with microarray technology. Such experiments involve, for example, the response of yeast cells to environmental changes, the gene transcription during the cell cycle, the mitochondrial dysfunction, or the transcriptional activation in multidrug resistance.

Two DNA chip formats are currently in wide use in *S. cerevisiae.* In said DNA chip formats, the PCR product microarrays are made from entire coding sequences (CDS) (DeRisi *et al.,* 1997) and the synthetic oligonucleotide microarrays either from short oligonucleotides (20-25 mers) (Lipshutz *et al.,* 1999) or long ones (60-70 mers) (Hughes *et al.,* 2001). The oligonucleotide approach allows the user to design probes for each gene that avoid repetitive regions or regions very similar to other known genes. In all cases, the microarrays contain probes for all computer predicted-CDSs from the original yeast sequence.

A few years after the complete sequencing of the *S.cerevisiae* genome, many studies have been carried out in order to refine the annotation of the yeast genome or to identify new genes. Those studies involve computational methods, comparative methods, transposon tagging methods or overlapping transcriptional clusters and expression profiling combined with mass spectroscopy of proteins.

However, since many *S. cerevisiae* genes belong to gene families or may contain repeated sequences, cross-hybridisation between members of a gene family remains an issue in PCR-product-based microarray experiments.

Several software tools have been written to design and evaluate primer pairs for large-scale DNA microarray purposes (see for example the one known as PrimerArray (Raddatz et al., 2001)). Hence, compared to the numerous improvements in data analysis and clustering methods, the problem of cross-hybridisation between genes of a given organism has been rarely addressed so far.

Recently, an automated approach (ProbeWiz) to design PCR products with minimal homology to other expressed sequences from a given organism has been reported (Nielsen and Knudsen 2002). In this algorithm, the designed PCR primer sets are evaluated according to the sum weight of three penalty parameters (paralogy, primer quality and 3' proximity penalties) out of which the chances of cross-hybridisation must be deduced by the user.

A genome-scale oligonucleotide design (OligoArray) has also been recently proposed (Rouillard et *al.,* 2002), wherein gene-specific and secondary structure-free oligonucleotides are computed for microarray construction.

### Aims of the invention

The present invention aims to provide nucleotide sequences specific for *Saccharomyces cerevisiae.*

A preferred aim of the present invention is to provide said improved nucleotide sequences specific for *Saccharomyces cerevisiae* genes, so as to minimize cross-hybridisation between related sequences, especially between nucleotide sequences and nucleic acid sequences including one or more regions similar (homologous) to one or more regions of said *Saccharomyces cerevisiae* genes, but not strictly identical (such as nucleic acid sequences phylogenetically related to different *Saccharomyces cerevisiae* genes).

In other words, the present invention aims to provide nucleotide sequences which could be used as specific probes for detecting *Saccharomyces cerevisiae* genes, each of said probes being able to hybridise with one and only one gene of the *Saccharomyces cerevisiae* genome and not with the other genes of said genome, thereby ensuring the detection specificity of said hybridised gene.

Another aim of the present invention is to provide a detection tool such as a solid support, preferably under the format of a microarray, comprising said nucleotide sequences fixed on its surface and used as probes for specifically detecting and/ or quantifying corresponding nucleotide acid sequences comprising said genes of *Saccharomyces cerevisiae* and present in a biological sample.

In particular, the present invention aims to provide said microarray allowing an improved discrimination between related sequences and avoiding or reducing artefactual cross-hybridisation between related sequences.

### Summary of the invention

A first aspect of the present invention is related to a nucleotide sequence preferably selected specifically for genes of *Saccharomyces cerevisiae*, which is selected from a group consisting of SEQ.ID. No.1 to SEQ.ID.No. 955, their corresponding strands and their variants. The term variants finds a definition hereafter.

More specifically, the present invention is related to a first group of nucleotide sequences, starting from SEQ. ID. No. 1 to SEQ. ID. No. 598 and to a second group of inventions starting from SEQ. ID. No. 599 to SEQ. ID. No. 955, as well as their corresponding strands.

Another aspect of the present invention is related to a nucleotide primer for obtaining by PCR amplification said nucleotide sequence, preferably said nucleotide primer was selected from the group of primer pairs consisting of SEQ. ID. No. 956 to SEQ. ID. No. 1553.

The present invention is also related to any amplicon nucleotide sequence amplified by said nucleotide primer.

The present invention is also related to the peptides encoded by said nucleotide sequences and amplicon nucleotide sequences.

Another aspect of the present invention is related to a kit of parts comprising said nucleotide sequence or said nucleotide primer, preferably a diagnostic kit for the identification of *Saccharomyces cerevisiae* genes, comprising at least one of said sequences or of said primers. Preferably, in the diagnostic kit, the nucleotide sequences are probes bound to the surface of a solid support preferably according to a microarray.

Another aspect of the present invention is related to microarray comprising preferably at least 500, more preferably at least 700, more specifically at least 900 probes, each probes being specific for a unique target gene of *Saccharomyces cerevisiae.* Preferably, said microarray comprises 955 probes selected from the group consisting of SEQ. ID. No. 1 to SEQ. ID. No. 955.

The present invention also concerns a method for the selection of a DNA coding sequence specific for a *Saccharomyces cerevisiae* CDS comprising the steps of:
a) comparing the CDS, which is the test nucleotide sequence, to all the hit sequences of the *Saccharomyces cerevisiae genome* so as to obtain sequence alignments;
b) classifying the alignments obtained in step a) into two classes, a class(i), also referred as "Type4 alignment", and class (ii), also referred as "Types 1,2,3", class(i) corresponding to the alignments wherein two or more test sequence regions arranged in a given order within the entire test sequence have matches against two or several hit sequence regions in the same order along a unique hit sequence, and class (ii) corresponding to the other test sequence alignments;
c) identifying for each of said alignments the potential cross-hybridising regions by calculating the values of at least three parameters, which are the size AS, the percentage of identity percent and the longest contiguous perfect match segment and considering the existence of cross-hybridizing regions when alignments meet one of the two following conditions (i) the size AS is greater than 50 and the identity is greater than 70 for Type4 alignments or (ii) the longest contiguous perfect match segment LC is greater than 30 for Types 1-3 alignments;
d) comparing any nucleotide sequence longer than 72 nucleotides and devoid of potential cross-hybridising regions, introns and trinucleotide repeats with intergenic sequences of *Saccharomyces cerevisiae* genome;
   wherein the remaining non-cross-hybridising regions are sequences specific for *Saccharomyces cerevisiae* CDS;
e) selecting said remaining sequences
f) possibly fixing said nucleotide sequences at dedicated locations upon the surface of a solid support according to a micro-array.

It is meant by "about 72 nucleotides" preferably between 61 and 80 nucleotides, more preferably between 64 and 76 nucleotides, and more particularly 72 nucleotides for DNA probes.

The present invention also concerns a method for the selection of a RNA coding sequence specific for a *Saccharomyces cerevisiae* CDS comprising the steps of:
a) comparing the CDS, which is the test nucleotide sequence, to all the hit sequences of the *Saccharomyces cerevisiae* genome so as to obtain sequence alignments;
b) classifying the alignments obtained in step a) into two classes, a class(i), also referred as "Type4 alignment", and class(ii), also referred as "Types 1,2,3", class(i) corresponding to the alignments wherein two or more test sequence regions arranged in a given order within the entire test sequence have matches against two or several hit sequence regions in the same order along a unique hit sequence, and class (ii) corresponding to the other test sequence alignments;
c) identifying for each of said alignments the potential cross-hybridising regions by calculating the values of at least three parameters, which are the size AS, the percentage of identity percent and the longest contiguous perfect match segment and considering the existence of cross-hybridizing regions when alignments meet one of the two following conditions (i) the size AS is greater than 50 and the identity is greater than 70 for Type4 alignments or (ii) the longest contiguous perfect match segment LC is greater than 30 for Types 1-3 alignments;
d) comparing any sequence longer than about 25 nucleotides and devoid of potential cross-hybridising regions, introns and trinucleotide repeats with intergenic sequences of *Saccharomyces cerevisiae* genome;
   wherein the remaining non-cross-hybridising regions are sequences specific for *Saccharomyces cerevisiae* CDS;
e) selecting said remaining sequences
f) possibly fixing said sequences at dedicated locations upon the surface of a solid support according to a micro-array.

It is meant by "about 25 nucleotides" preferably between 20 and 35 nucleotides, more preferably between 22 and 30 nucleotides, and more particularly 25 nucleotides for RNA probes.

The present invention is also related to a method for designing an oligonucleotide probe specific for a *Saccharomyces cerevisiae* CDS comprising the steps of:
a) providing a sequence specific for a *Saccharomyces cerevisiae* CDS selected by the method according to claim 14;
b) designing a synthetic oligonucleotide that meets the criteria of having a GC content in percent between 34-46 and of being devoid of monotonous hexanucleotides and of secondary structures.

Another aspect of the present invention is also related to a preparation and selection method for obtaining the specific nucleotide sequence and primer according to the invention, which is described in more detail in the following examples.

In particular, the present invention is also related to a method for designing a primer pair which can be used for the amplification of a sequence specific for a *Saccharomyces cerevisiae* CDS comprising the steps of:
a) providing a sequence specific for a *Saccharomyces cerevisiae* CDS selected by the method of claim 14;
b) designing a primer pair with the following criteria: a primer length about 20 pb, a primer melting temperature range of about 56-63°C and an optimum PCR product size about 300-700 pb.

Moreover, another aspect of the present invention also concerns a method for detecting and/or possibly quantifying a target nucleotide sequence of *Saccharomyces cerevisiae* and/or its expression in a biological sample, which comprises the steps of:
a) possibly purifying said sample in order to obtain a sufficient amount of said target sequence;
b) putting said target sequence in contact with the set of other sequences selected from the group consisting of SEQ.ID.NO.1 to SEQ.ID.NO.955, or with the primer pairs consisting of SEQ.ID.NO.956 to SEQ.ID.NO.1553;
and identifying if said target sequence corresponds to a sequence specific *for Saccharomyces cerevisiae* by testing the possibility of having a hybridisation between said sequence with at least one of the said other sequences or an amplification of the target sequence by complementary base pairing with the primer pairs.

### Definitions

The term "polynucleotide" or "nucleotide sequence" generally refers to any polyribonucleotide or polydeoxyribonucleotide, which may be unmodified RNA or DNA or modified RNA or DNA. "Polynucleotides" or "nucleotide sequences" include, without limitation single- and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. The term "Polynucleotide" or "nucleotide sequence" also includes DNAs or RNAs containing one or more modified bases and DNAs or RNAs with backbones modified for stability or for other reasons. (Preferably said modification does not alter the hybridisation rate of said polynucleotide sequence) "Modified" bases include, for example, tritylated bases and unusual bases such as inosine. A variety of modifications have been made to DNA and RNA; thus, "Polynucleotide" or "nucleotide sequence" embraces chemically, enzymatically or metabolically modified forms of polynucleotides as typically found in nature, as well as the chemical forms of DNA and RNA characteristic of viruses and cells. "Polynucleotide" also embraces relatively short polynucleotides comprising preferably from 10 to 100 bases, and more preferably from 20 to 30 bases, often referred to as oligonucleotides.

The term "variant" as used herein, refers to a polynucleotide (nucleotide sequence) or its corresponding polypeptide (peptide sequence) that differs from a reference polynucleotide (nucleotide sequence) or its corresponding polypeptide (peptide sequence) respectively, but retains essential properties specific of the micro-organism (*Saccharomyces cerevisiae*) to be characterised. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polynucleotide or polypeptide may differ in their nucleotide or amino acid sequence by one, two, three, four, five, ten, twenty or more substitutions (preferably conservative), additions and deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide. For instance, they should have similar antigenic or immunogenic activities as the reference polypeptide. Antigenicity can be tested using standard immunoblot experiments, preferably using polyclonal sera against the reference polypeptide. The immunogenicity can be tested by measuring antibody responses (using polyclonal sera generated against the variant polypeptide) against purified reference polypeptide in a standard ELISA test. Preferably, a variant would retain all of the above biological activities. Preferably, a polypeptide or polynucleotide variant differs from the sequence of reference by a small percentage of difference and shares an "identity of sequence" higher than 75%, 80%, 85%, 90%, 95%, 97%, 98% or 99%.

The term "identity" is a measure of the identity of nucleotide sequences or amino acid sequences. In general, the sequences are aligned so that the highest order match is obtained. "Identify" *per se* has an art-recognised meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds, Humana Press, New Jersey, 1994; SEQUENCE ANALYSIS IN MOLECULAR BIOLOGY, von Heijne, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1998) 48 : 1073). Methods commonly employed to determine identity or similarity between two sequences include, but are not limited to those disclosed in Guide to Huge Computers, Martin J. Bishop, ed., Academic Press, San Diego, 1994, and Carillo, H., and Lipton, D., *SIAM J Applied Math* (1988) 48 : 1073. Methods to determine identity and similarity are codified in computer programs. Preferred computer program methods to determine identity and similarity between two sequences include, but are not limited to, GCG program package (Devereux, J., *et al.*, *J Molec Biol* (1990) 215 : 403). Most preferably, the program used to determine identity levels was the BLAST program.

As an illustration, by a polynucleotide having a nucleotide sequence having at least, for example, 95% "identity" to a reference nucleotide sequence is intended that the nucleotide sequence of the polynucleotide is identical to the reference sequence except that the polynucleotide sequence may include an average up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

### Short description of the drawings

Fig. 1 corresponds to a schematic representation of the design procedure according to the present invention.

Fig. 2 represents the different types of BLAST alignments considered in the method of the invention.

Fig. 3 represents the protocol strategy used in the array tests according to the method of the invention.

Fig. 4 represents the distances between reverse primers and stop codons in the present invention. Negative distances refer to reverse primers localized within the 3'untranslated region (see Table 2).

Fig. 5 gives a comparison of transcript level measurements between duplicated probe spots within a single array from Batch616.

Fig. 6 corresponds to the experimental validation of probes containing with 5' or 3' untranslated regions. A particular microarray glass was printed in duplicates with PCR products (**A**) or oligonucleotides (**B**).

### Detailed description of the invention

### Material and methods

### DNA sequences and databases

DNA sequences of the 16 *S.cerevisiae* chromosomes were taken from MIPS (http://mips.gsf.de) at the date of February 2001. A set of 5803 protein coding genes was used and referred to here as the GSC database. The GSC database is composed of CDSs originally described at MIPS plus novel short CDSs discovered in Genolevures (Blandin et *al.,* 2000) or other comparative sequence data (Cliften *et al.*, 2001; Goffeau et al., 1997).

All remaining sequences from *S. cerevisiae* were considered as "intergenic" sequences and called GSCIG database.

The list of intron-containing CDSs was compiled from MIPS, YIDB (Lopez and Seraphin 2000) and ARES V 2.0 (Grate and Ares 2002) intron databases.

Position and length of trinucleotide repeats relative to *S. cerevisiae* CDSs were taken from Richard and Dujon, 1997.

The final set of *S. cerevisiae* polypeptide coding genes has 241 intron-containing CDSs and 564 CDSs in which at least one array of at least 5 repeats of trinucleotides is found.

### Design of DNA probes

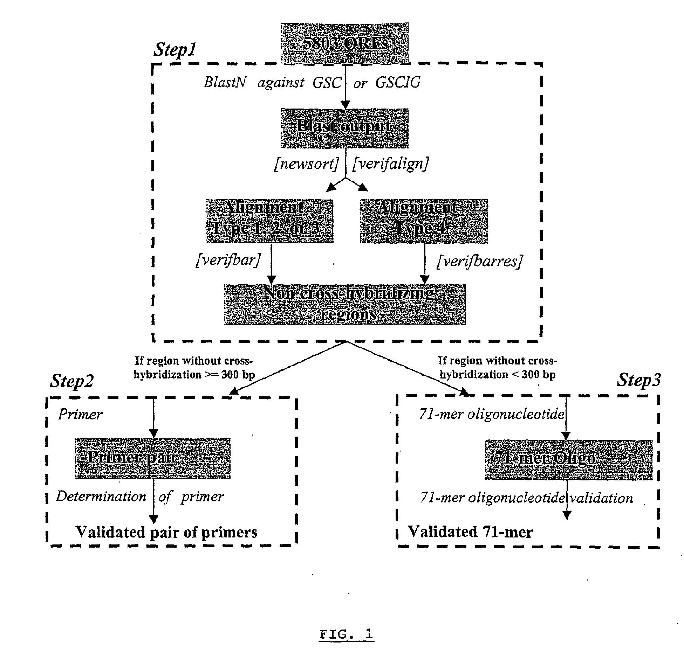

The probe design according to the invention involves 3 steps as illustrated in **Figure 1.** The main scripts (written in *perl* and *sh shell* programming languages) used in this work are in brackets []. [*Newsort*] allows the rearrangement of the BLAST output table in a positional order along the query and subjet sequences. [*Verifalign*] detects the different alignment types. [*Verifbar*] and [*Verifbarres*] allow the detection of potential cross-hybridisation regions in Type 4 and Type 1, 2, or 3 alignements, respectively.

It is meant by "probe" the spots on the glass slide and it is meant by "target" the nucleotide sequence sample hybridised to the glass-bound probe array.

### - Step1 : Detection of non cross-hybridising regions within open reading frames

Each test sequence (here the entire CDS as query) was first compared to the present GSC database using *BLASTN* 2.1.2 algorithm (Altschul *et al*., 1997). Low complexity filter was not applied so that all significant matches were reported from the *BLAST* output. *BLAST* output files were parsed in order to extract the size (AS), the percentage of identity (ID), and longest contiguous perfect match segment (LC) in each alignment.

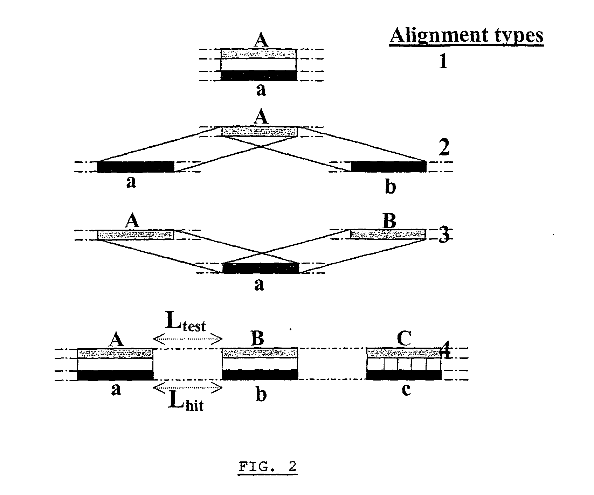

The four possible types of alignments between the query sequence and its homolog are described in **Figure 2.**

Four types of blast alignments can be observed when a test sequence is compared to all other sequences of the *S. cerevisiae* genome: (1) A test sequence region (e.g. A) has a unique match region "a" in a hit sequence; (2) A test sequence region (e.g. A) has matches against several hit regions (e.g. a and b) from a unique hit sequence; (3) Several test sequence regions (e.g. A and B) have matches against a unique hit region (e.g. a); (4) Two or more test sequence regions (e.g. A, B, and C) arranged in a given order within the test sequence have matches against two or several hit sequence regions (e.g. a, b, and c) in the same order along a unique hit sequence. Lₜₑₛₜ and Lₕᵢₜ are the nucleotide distances (in bp) between two successive alignments in test and hit sequences, respectively. Alignment Types 1, 2, or 3 were computed separately from Type 4 (see hereafter).

Type 4 alignments were detected and redirected into a specific table if the following two conditions were simultaneously met: (*i*) *L*_{*test*} and *L*_{*hit*} distances are less than 500 bp and more than -7 bp (cases of partial overlaps); (*ii*) the successive alignments between test and hit sequences have the same strand orientation. The *L*_{*test*} and *L*_{*hit*} threshold value of 500 bp was chosen, because it corresponds to the average size of probes to be designed. Overlapping alignment threshold was arbitrary set at 7 bp. In subsequent analysis, Type 4 alignments were treated separately from other types of alignment.

Potential cross-hybridising regions were identified when alignments meet either of the following two conditions: (i) AS > 50 and ID > 70; (ii) LC >30. For Type 4 alignments, AS and ID were re-calculated globally from all partial alignments. The remaining part of each CDS (without potential cross-hybridising regions) was examined for the presence of introns or trinucleotide repeats.

For each CDS, all segments larger than 72 nucleotides and devoid of potential cross-hybridising regions, introns and trinucleotide repeats, were extracted and compared against the GSCIG database using *BLASTN* as above in order to eliminate potential cross-hybridisation with intergenic regions.

For the final probe design of each CDS, preference was given to subsegments of 800 bp in length selected by their proximity to the 3'-end of the CDS.

In some cases, sequences were extended to the neighbouring intergenic regions.

Based on the size of available non cross-hybridising regions two distinct routes were followed (see **Figure 2):** *Step 2* or *Step 3*.

### - Step 2: Design of primer pairs

The selected non cross-hybridising regions were subjected to *PRIMER3 a*lgorithm (Rozen and Skaletsky 1998) with the following parameters: primer length: 20 bp, primer melting temperature range: 56-63°C, and optimum PCR product size: 300-700 bp. Secondary structure parameters of *PRIMER3* including hairpins, self-dimers, and cross-dimers in primer pairs were set as "default". *PRIMER3* was allowed to return up to 10 pairs of primers as candidates (the *forward primer* or the *reverse primer* designate oligonucleotide localized near the start or the stop codon of the CDS, respectively). Data from *PRIMER3* output file were extracted and entered into an Excel spreadsheet for subsequent analysis.

To select the best primer pair for each CDS, the following was applied. Each candidate primer sequence was compared to the 16 yeast chromosome sequences using *BLASTN* algorithm without low-complexity filter and *W* set to 14. Primers were considered as "unique" if no match of 14 nucleotides was found or if the match equal or greater than 14 nucleotides leaves mismatches at the 3'-end.

For each CDS, preference was given to "unique" primers showing no match, or in their absence, to those exhibiting minimal size match. Therefore, each designed primer was assigned a "unique" or "non-unique" mark.

Finally, the best primer pair for each CDS was selected based on the following preferences: (*i*) an optimal probe length of 500 bp; (*ii*) at least one of the two primers is "unique"; and (*iii*) position of reverse primer is as close as possible to the 3'-end of the open reading frame.

### - Step3: Design of long oligonucleotide probes

For CDSs in which the remaining non cross-hybridising regions are shorter than 300 nt, no PCR probe was designed. Instead, 71-mer synthetic oligonucleotides were synthesized. For this purpose, the CDS subsegments defined at *Step 1* were screened for the presence of 71 nucleotidic sequences that met the following criteria: GC percent between 34-46 (optimum 39%); absence of monotonous hexanucleotides (TTTTTT, CCCCCC, GGGGGG and AAAAAA); and absence of secondary structures. The first two criteria were determined by using EMBOSS applications *geecee* and *compseq,* respectively (http://www.uk.embnet.org/). The last criteria was estimated using nearest-neighbour thermodynamic calculations with the aid of the *MFOLD 2.3* DNA/RNA folding algorithm (Walter *et al.,* 1994) (temperature set to 68°C, with other variables set as "default").

Melting temperatures of designed probes were computed using the *MELTING* algorithm (Le Novere 2001) with sodium concentration and nucleic acid concentration in excess set to 0.825 M and 0.02 M, respectively. All other parameters were used as "defaults".

### Microarray preparation

Primer pairs for each of the 4972 PCR products selected as probes were synthesized at 40 nmoles scale using standard cyanoethyl phosphoramidite chemistry method. The forward and reverse primers are 34-35 nucleotides long and are composed of 20 nucleotides of the designed primer with a common 5'-tag sequence (forward primer-tag: 5'-CGACGCCCGCTGATA; reverse primer-tag: 5'-GTCCGGGAGCCATG).

Synthesis of the 71-mer oligonucleotides was done using the same chemistry but the 5'-end is modified by an the aryl amino-group NH₂-[CH₂]₆- at the 5'-end of the oligonucleotide.

PCR amplification reactions were done in two rounds. In the first round, one hundred-microliter reactions were performed using each primer pair with the following reagents: 20 ng of *S. cerevisiae* DNA template, 50 pmoles of each primer, 0.25 units of Taq polymerase (Eurogentec s.a.), 1x PCR buffer (Eurogentec s.a.), and 1.8 mM MgCl₂. Thermalcycling was carried out in Gene Amp PCR system (Applied Biosystems) with 5 min denaturation step at 95°C, followed by 40 cycles of 1 min at 95°C, 30 sec at 55°C, 1 min at 72°C, and a final cycle at 72°C for 10 min. All PCR reactions were analyzed by agarose gel electrophoresis. Reactions that failed to amplify or show multiple bands were repeated using different conditions (e.g. higher annealing temperatures) to favor amplification of the desired product.

The second round of PCR amplification followed the same protocol as the first round except for the use of 40 ng of first round PCR products as templates, 50 pmoles each of forward (with an aryl amino-group NH₂-[CH₂]₁₂- at the 5'-end of the oligonucleotide) and reverse tag-primers, and the annealing temperature of 45°C.

PCR reactions were purified with 96-well Millipore MultiScreen-FB filters (Millipore), then eluted in a final volume of 40 µl NaPO₄ 20 mM, pH 8.0, and stored at -20°C until use.

A set of PCR products and synthetic oligonucleotides was also selected as controls. This includes the luciferase gene and RNA spike (Promega) for signal normalization; *YFL039c* (*ACT1*) as control of hybridisation homogeneity; *YKL182w* (6153 bp) and *YLR310c* (4767 bp) as controls of reverse transcription efficiency; 10 intergenic regions, 10 intronic regions, and 17 mitochondrial genes of *S. cerevisiae;* 3 *E. coli* genes (*tuf*A*, ace*F, *kdt*A) as negative controls; the LexA binding domain, LacZ, Pho4 binding domain, Gal4 binding domain, GFP, and GST as commonly used tags or reporter genes; *Leu1, His5,* and *Ura4* for *S. pombe, Ura3* for *K. lactis*, human CBF2 and MEF2C genes, *kan*^{R} gene as heterologous genes and markers; *MEL1* and *FLO1* genes of *S*. *cerevisiae* which are absent from the sequenced strain S288c; the 20 non-monotonous trinucleotide repeats (72-mer oligonucleotides); and total genomic DNA from the wild-type strain S288c. Printing buffer was deposited on the empty spots.

Using the robot arrayer SDDC-2 (Engineering Services Inc., ESI), DNAs (PCR products, 71-mer oligonucleotides, and control genes) were deposited in duplicates (1 femtomol DNA of PCR product and 10 femtomoles DNA of long oligonucleotide) onto Superamine glass slides with 200-µm spacing between neighbouring spot centers. The complete array is composed of 32 grids of 400 spots each, with ca.200 empty spots for background controls. Subsequent microarray treatments were performed as recommended by the manufacturer (Telechem International).

The final design of arrays with probes is described in **Annexes 2A and 2B.**

It should be noted that batches 800, 400, and 616 (prepared sucessively) as used in the present hybridisation tests differ from the final ones described above, since the probes corresponding to the genes *YBR145w, YDR225w, YEL033w, YGL147c, YGR148c, YJR148w, YMR011w, YMR169c, YMR170c,* and *YNL143c* are present in the form of PCR products instead of 71-mer oligonucleotides.

### Yeast strains

All strains used herein were provided by the Euroscarf collection of *S*. *cerevisiae* deletions (http://www.uni-frankfurt.de/fb15/mikro/euroscarf). These include the parental strain BY4742 *(MATα his3Δ1 leu2Δ0 lys2Δ0 ura3Δ0*) (Brachmann *et al.,* 1998) as well as the corresponding deletants in which each of the following genes was replaced by the KANMX4 cassette: *YBR025c, YBR145w, YDR225w, YDR497c, YEL033w, YGL147c, YGR086c, YGR148c, YJR148w, YLR058c, YLR109w, YML072c, YML113w, YmR011w, YMR169c, YMR170c, YNL143c,* and *YOL103w.*

The correct replacement of the target CDS by the KANMX4 cassette in each deletion strain was confirmed by PCR amplification as described by Lucau-Danila *et al.*, 2000.

PCR amplification of the target CDS-region was also used in the present invention to verify the complete absence of the CDS in each deletion strain.

All the deletant strains have normal growth phenotype.

### mRNA preparation, cDNA synthesis and hybridisation:

For each test, freshly grown yeast cells were inoculated into 400-ml glucose-rich liquid medium, grown to mid log phase (OD₆₀₀ = 0.5), and then collected by centrifugation at 1,600 x g for 5 min at room temperature. Each pellet was washed once with 20 ml of ice cold water, and stored at -80°C until RNA preparation. Total RNA isolation was essentially performed as described by Ausubel *et al*., 1993.

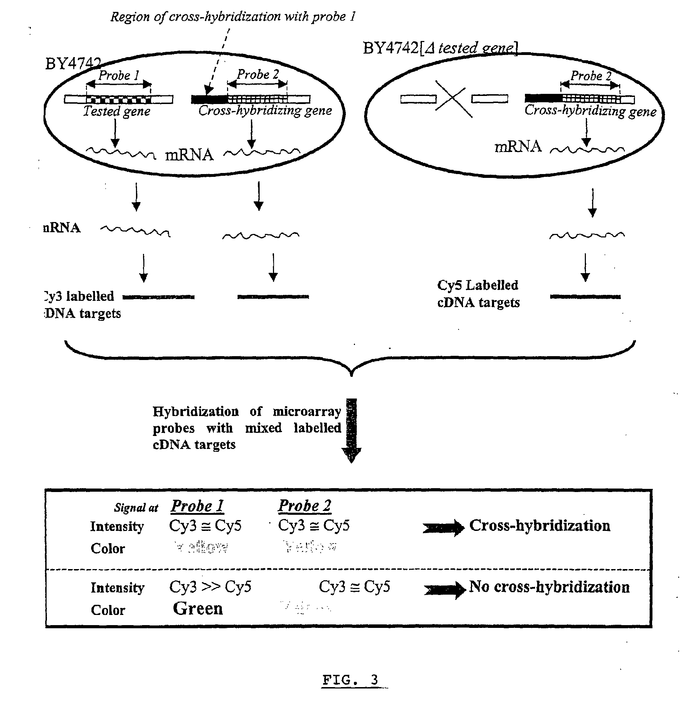

Analysis of deleted mutants were performed as described in **Figure 3**. For each test, about ten micrograms of total RNA were converted to either Cy3-(the BY4742 wild-type strain used as reference) or Cy5-(the deletion mutant studied) labelled cDNA targets using a custom labelling kit (Amersham-Pharmacia-Biotech). All tests were performed using the same RNA preparation from the wild-type strain BY4742 as reference.

The amount of cDNA as well as the incorporation of Cy3 and Cy5 dyes into cDNA targets were quantified by measuring the absorbance of each sample at 260 nm, 550 nm and 650 nm, respectively. For each test, labelled-Cy3 and -Cy5 cDNA targets were then combined in equal amount, dried by Speed-vac, and resuspended in 50 µl hybridisation buffer composed of Dig Easy Hyb solution (Roche diagnostics) with 0.5 mg/ml of salmon sperm DNA.

After covering the array with a 24 x 50 mm coverslip, the slide was placed in a humidified hybridisation chamber (Telechem, CA) and processed to prehybridisation in 50 µl hybridisation buffer for 2 hours at 42°C.

The hybridisation was performed with resuspended CyDye-labelled cDNA at 42°C for approximately 16 h. Following hybridisation, slides were washed twice in 0.1x SSC, 0.1% SDS for 5 min and finally in 0.1x SSC for 30s. Then, slides were immediately dried by centrifugation (2 min at 400 x g).

### Array data processing

Hybridised microarrays were scanned for Cy3-and Cy5-labelled targets with a Genepix 4000A fluorescence reader (Axon) with a resolution of 10 µm. According to the manufacturer specifications, the photomultiplier tube settings were adjusted in order to balance the signal intensities of the two channels, and to get the greatest dynamic range for each scan. Signal quantification for each probe on the microarray was performed with Genepix image acquisition software (Axon).

For each test, intensity signals of the ca.200 empty spots were measured and the 95% upper confidence interval of these figures was used as the lower limit for significant measurements. Intensity signals of duplicated DNA spots were processed independently. Since the dye bias appears to be dependent on grid location in the array, a "within-print-tip-group" normalization method (Yang *et al.,* 2001) was applied to the data. This procedure corrects the differential rates of incorporation of Cy3 and Cy5 so that the ratio is close to 1. Fluorescence ratios were computed based on hybridisation signals normalised with background corrections.

### • Results

### Determination of a list of protein coding sequences (CDS) in Saccharomyces cerevisiae useful for microarray analysis

Most microarrays commonly used until now were designed according to the initial yeast genome annotation with a widely accepted number of protein coding genes of about 6200 (Goffeau et al., 1996; Mewes et al., 1997).

The *S*. *cerevisiae* coding sequences (CDS) were originally predicted using, as primary criteria, their size (>= 100 codons from ATG to stop) and ignoring all reading frames entirely included within longer ones (Dujon et al., 1994).

Additional criteria such as the codon adaptation index, CAI (Sharp and Li 1987) were sometimes used, in particular to identify short CDSs without obvious homology or to estimate the likelyhood of partially overlapping CDSs.

Shorter CDSs were considered only when previously described or when their predicted product show homology to another gene product in public databases.

In addition, original annotations, were not carried out uniformly for all chromosomes (e.g. chromosome I annotations (Bussey et al., 1995) differed from chromosome VIII (Johnston et al., 1994)).

On the contrary to the prior art, the present invention has firstly established a uniform definition of the S. cerevisiae CDS. Indeed, the small CDSs discovered by the Genolevures project (Souciet et al., 2000) were added to the list of all CDS exceeding 99 codons and not entirely included in another CDS, yielding to a total of 6252 CDSs (in which Ty elements are ignored). This list includes a number of partially overlapping CDSs (993 in total). The majority (83%) of them are antiparallel overlappings, excluding frameshift sequencing errors. This high proportion of partially overlapping CDSs in the genome of *S. cerevisiae* (16%) is primarily due to the overprediction of CDS as judged from the fact that 519 of them (58%) do not show any homology with other hemiascomycetes whereas this fraction drops to 8% when the non-overlapping CDSs are considered.

Using as criteria the total absence of homology in other hemiascomycete species, the absence of known function in litterature and the short size, a total of 449 CDSs (see **Annex** 1) were eliminated in the present list (their size range from 100 to 394 codons, average of 130; and their CAI range between 0.028-0.330, average of 0.108), yielding a final list of 5803 CDS that was used to construct the present microarray probes (see **Annexes 2A and 2B)**. More than 85% of eliminated CDSs are questionable ORFs (Class6 at MIPS).

### Design of microarray probes

The microarray of the invention had to respect the three following characteristics: having probes of minimised lengths, preferentially located near the 3' end of the coding sequences and for which cross-hybridisation is minimised as far as possible.

### Prediction of potential cross-hybridising regions within the present selected CDSs.

A particular approach was performed in order to determine region within each coding sequence not susceptible to cross-hybridise with any other sequence in the yeast genome.

In classical gene hybridisation tests, similar sequences that are not completely identical can be distinguished by increasing the stringency of hybridisation conditions.

In the present case, however, it was not possible to perform hybridisation with the most stringent conditions for each of the 5803 genes at the same time. The sequence similarity was therefore examined between each of the 5803 selected CDS and the entire yeast genome as described in "Materials and Methods". The entire yeast genome was used instead of CDS only, because of the presence of 5' and 3' UTR regions (untranslated regions) in transcripts and because some short genes may not have been identified yet.

Cross-hybridisations of a CDS with one or several yeast transcript(s) may theoretically result from the existence of a single region having similarity with one or several other sequence(s) in the yeast genome, or from the existence of several short stretches of sequence similarities as illustrated in **Figure 2**.

Here, BLAST alignments revealed that 17.6% and 5.1% of the test sequences (entire CDS) give rise to Type 4 alignments when compared to other coding sequences and to intergenic sequences, respectively. Type 4 alignments are of critical importance since a previous study suggested that short regions of sequence identity spread over the entire length of a probe may result in cross-hybridisation artifacts using cDNA microarrays (Heller et al., 1997). Other test sequences give rise to Type 1, 2 or 3 alignments.

In order to predict putative cross-hybridising regions in each CDS, three parameters were combined (see "Materials and Methods").

The alignment size (AS) threshold was set at 50 nucleotides, based on the predicted melting temperatures (Bolton and McCarthy 1962) with an average GC percent of 39%, the average value for the *S. cerevisiae* genome.

The percentage of identity (ID) was set at 70% based in a recent study (Girke et al., 2000) and experiments in E. coli showing that cross-hybridisation occurs between paralogous genes sharing more than 70% identity over at least 200 bp (Richmond et al., 1999).

A longest contiguous stretch of sequence identity (LC) of 30 nucleotides was considered sufficient for cross-hybridisation.

Using said criteria, 4523 (78%) of the selected CDS were expected not to show cross-hybridisation with any other transcript in the yeast genome.

Thus, the corresponding microarray probes could be easily designed in the preferred region as described below.

For the remaining 1280 CDS, putative cross-hybridisations are expected with other CDS (841 or 14.5%), with intergenic regions (191 or 3.3%), or with both simultaneously (248 or 4.2%)(**Table 1**). For those cases, probes were designed avoiding the putative cross-hybridising regions as far as possible.

Compared to the microarrays that use entire CDS as probes, the design according to the invention predicts that a total of 5660 CDS (97.5%) should be free of cross-hybridisation (a gain of 1137 CDS compared to full length probes).

For example, HXT2 (*YMR011w*), a high-affinity hexose transporter is known to share high aminoacid sequence identity with other members of the same family (Bisson *et al.*, 1993). At the nucleic acid level, it was found that *YMR011w* has cross-hybridising regions with all the other 15 members of the hexose family: HXT1; HXT3-12; HXT14-16 as well as with YLR081w, the gene encoding the GAL2 protein. Comparatively, in a recent study, the HXT2 signal was considered non-significant because of the high sequence similarity of HXT2 with the numerous hexose transporters gene (DeRisi *et al.*, 2000). Here, the present designed *YMR011w* probe (an oligonucleotide of 71 mers) do not show cross-hybridisation with members of the same family (see **Table 6**).

Among the remaining 143 cross-hybridising probes, 9 cross-hybridise with intergenic sequences **(Table 1**). The other 134 cases cross-hybridise with CDS only or with both CDS and intergenic sequences.

It should be noted that among the 143 cross-hybridising probes, we have also reduced the number of cross-hybridants for 31 probes. The list of the remaining cases of cross-hybridising probes as well as their putative cross-hybridising CDS is given by the **Annexes 2A and 2B.**

### Designed primers and corresponding PCR products

From the non cross-hybridising regions defined in each CDS, 4972 pairs of 20 base primers were designed as described in "Materials and Methods" of which 97.2% are "unique". For the remaining 278 "non-unique" primers, the second member of the pair is "unique". The average GC percent of the designed primers is 47.8 with no significant difference between forward and reverse primers.

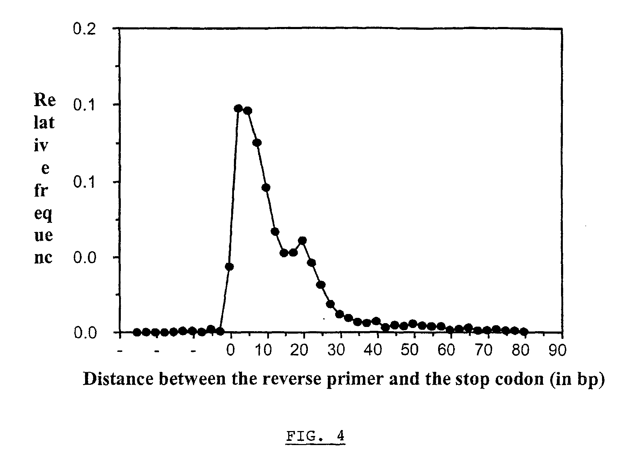

Reverse primers, and therefore the probes according to the invention for each CDS, were selected for their proximity to the 3'-end of each gene. This feature was chosen because, in most tests, the hybridising target will be prepared by reverse transcription with oligo(dT)-primed mRNA. In the present design, distances (in base pairs) between the reverse primer and the stop codon is less than 100 bp for 62% of the CDS and less than 300 bp for 94% of the CDS (**Figure 4**).

The remaining 316 cases are due to the presence of putative cross-hybridising regions near the 3'-end of the CDS. In 37 cases, the forward or reverse primers were selected in the 5'- or 3'-untranslated regions because the non-crosshybridising region of the corresponding CDS was shorter than 300 nucleotides **(Table 2).** Those primers were designed so that they do not overlap the next CDS.

PCR amplification using above primer pairs gave excellent results since up to 98.8% of all PCR products have the correct size and good quality (see "Materials and Methods").

Average size of the present PCR products is 521 bp (70% of all the probes are longer than 500 bp). Shorter probes are due either to short CDS (9.3% of the *S*. *cerevisiae* CDS are less than 500 bp) or to the fact that the non cross-hybridising regions are shorter than 500 bp. Minimal size of the PCR products was limited to 300 bp because it was noticed that small genes (<300 bp) often showed lower intensity signals in hybridisation (Richmond *et al.,* 1999).

The GC percentage of the designed PCR probes is comprised between 20 and 62 % (with an average of 40.6) compared to 20 to 59% (with an average of 40%) for entire yeast CDS.

### Long oligonucleotides as probes

For the 831 remaining CDS in which the non cross-hybridising regions are shorter than 300 bp, PCR probes were replaced by synthetic 71-mer oligonucleotides (see "Materials and Methods"). Using this strategy, the number of cross-hybridising probes could be further reduced by 688, leaving only 143 CDS in which cross-hybridisation is unavoidable (See **Table 1**).

Long oligonucleotide probes were selected within the last 800 nucleotides of each CDS (distance to the stop codon range from 0 to 779 bp with an average of 187 bp). Melting temperature of the oligonucleotide probe is about 70°C. It should be noted that 199 selected oligonucleotides partially overlap the 5'- or the 3'-UTR (mainly for short CDS). For each of these oligonucleotides, the overlapping sequence in 5' or 3'-UTR is 41 bp and do not cross the next CDS.

### Sensitivity and specificity of the present microarrays

The sensitivity and specificity of the microarrays according to the present invention were determined. 82-92% of all probe spots produce detectable transcript signals *i.e.* showing Cy3 or Cy5 intensities higher than the 95% upper limit of the distribution of empty spot signals. This is indicative of the high sensitivity of the system according to the invention.

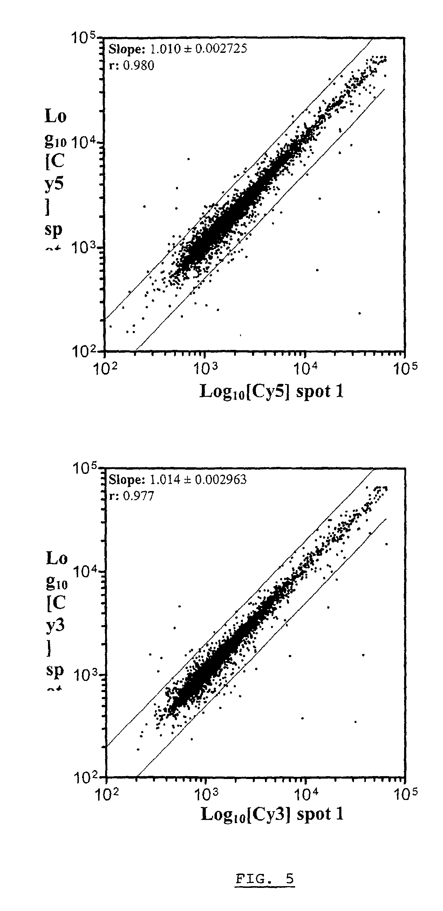

An example of the dynamic range and reproducibility of the duplicate measurements of an array is shown in **Figure 5**.

A scatter plot of Cy5 (A) and Cy3 (B) intensities at duplicated probe spots from a single hybridisation is shown. cDNA targets were prepared from total RNA isolated from wild-type BY4742 (Cy3 labelled) and *Δydr225w* (Cy5 labelled) strain as described in "Materials and Methods". Linear regression over the scattered points gives a slope with a correlation coefficient r. The 2x limits are shown in figure 5.

As can be seen, transcript intensities can be measured over a 3-log range and the reproducibility of measurements of the two duplicates is high (slope close to 1). In addition, only ca.30 pairs of spots differ by a factor greater than 2x (and only ca.10 pairs greater than 3x).

Within one hybridisation test involving BY4742 Cy3- and Cy5-labelled cDNA, signals of oligonucleotides were compared to those of PCR probes. As can be seen in **Table 3,** hybridisation signals of oligonucleotides tend to be lower than those of PCR products (respective median values 277 and 250 for Cy5 channel, and 525 and 500 for Cy3 channel). The same is true for mean values. Nevertheless, signals of oligonucleotide spots remains highly significant in comparison to background signals (28.5 and 11.3 fold).

Here, sensitivity of the oligonucleotide probe spots (74%) was lower than for the PCR probe spots (95%). In separate tests which involved PCR and oligonucleotides probes with extension in the 5'- or 3'-UTR, more than 87 % of the probe spots produce a detectable signal.

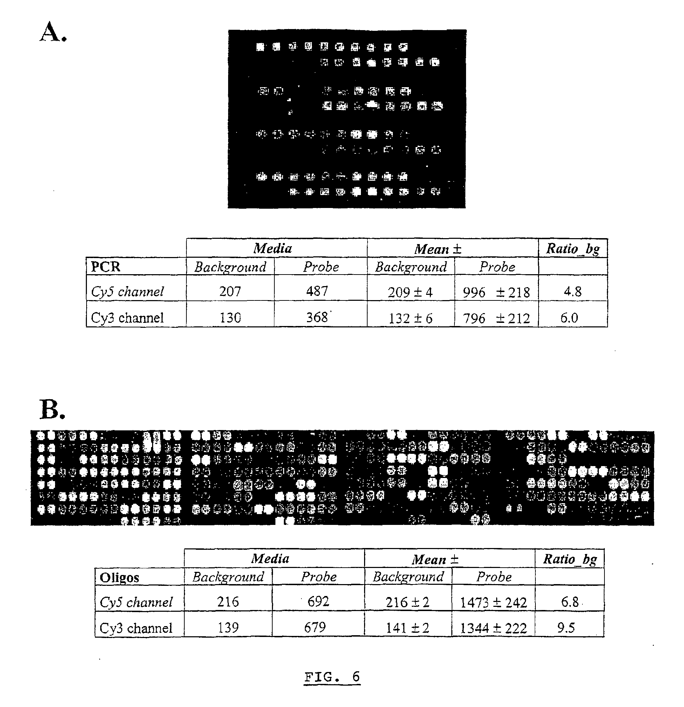

Moreover, the hybridisation signals tend to be significant when compared to background signals in PCR or oligonucleotides (respective mean values 996 ± 218 or 796 ± 212 for Cy5 channel, and 1473 ± 242 and 1344 ± 222 for Cy3 channel)(**Figure 6).**

cDNA targets were prepared from total RNA isolated from wild-type BY4742 (Cy3 labelled) and *Δydr225w* (Cy5 labelled) strain and hybridised to microarrays as described in "Materials and Methods". Microarray images are shown as well as description table of the hybridisation signals. For each channel, the distribution of intensities for spotted probes is computed and is given by the median, the mean (with 2 x standard error). Ratio_bg is the mean intensity of the probe over mean intensity of the background signal for probes within the microarray experiment.

The results of hybridisation tests using arrays from 3 sucessively prepared batches (Batch800, Batch400, and Batch616) were also compared. Median and mean values of probe signals and background measurements were computed as above **(Table 4)**. For both channels, it was found that the median and mean of background signals were higher in Batch400, compared to Batch800 and Batch616. Moreover, the Ratio_bg are 24.0, 5.9, and 52.2 for Cy5 channel, and 15.6, 10.5, and 28.5 for Cy3 channel in arrays from Batch800, Batch400, and Batch616, respectively. Although the median and mean intensities are lower in Batch800 (in comparison to the two other batches), these results indicate a higher specificity for Batch800 and Batch616. This last batch was the most frequently used in the hybridisation tests (See **Tables 5 & 6)**.

### Experimental validation of predicted cross-hybridisation profiles

In order to test experimentally the cross-hybridisation predictions, a collection of yeast deletion mutants was used. First was selected a set of probes representative of the various situations encountered with regard to potential cross-hybridisations (i.e. different alignment size and different percentage of identity between the tested probe and the potential cross-hybridising gene)(Part I in **Tables 5 & 6).**

Then, microarray hybridisations were done as described in "Materials and Methods" and transcript signals were compared (for the tested probes and their putative cross-hybridising genes) from the wild-type strain (labelled with Cy3) and from the strain deleted in the corresponding gene (labelled with Cy5).

Eleven deletants were selected to represent cases in which the probe shows potential cross-hybridisation with only one other coding sequence and the 2 other deletants (*YML113w* and *YMR011w*) to represent cases with potential cross-hybridisation to several other coding sequences **(Table 6).** One deletant (*YEL033w*) was selected for its predicted cross-hybridisation with an intergenic sequence (**Table 5**) and a last one (*YNL143c*) have putative cross-hybridising regions with one other coding sequence and 14 intergenic sequences (**Table 5**). The last 3 deletants (*YBR025c, YML072c,* and *YLR109w*) were selected as negative controls (no putative cross-hybridisation)(**Table 5**).

Putative cross-hybridising regions range from 31 to 494 nucleotides with identities higher than 79%.

For each of the 19 different hybridisation tests (BY4742 x BY4742 experiment is included), intensities were normalised and the Cy5/Cy3 ratios (RatioI) were calculated for all the probes. In theory, a RatioI close to 1 or -1 (yellow color) should be observed at the spot corresponding to the cross-hybridising gene (used as control) whereas the color at the spot corresponding to the tested probe should be either yellow (in case of cross-hybridisation) or green (in case of non cross-hybridisation) (see **Figure 3**)**.**

Results are given by **Tables 5 & 6** (see *Part II*). For each spot, the transcript abundance (given by Rb5 and Rb3) was determined by comparing the hybridisation signal of the spot with the background of the array. In order to allow specific comparisons, the mean of the Cy5/Cy3 ratios (given by mean of ratios) was also calculated for the spot of interest for the 17 tests in which the gene studied is not deleted.

As expected, three control genes (*YBR025c, YML072c, YLR109w*) predicted not to cross-hybridise, show highly negative RatioI (-100 to -33.3) at the tested spots. Correspondingly, the transcript abundance in the mutant (Rb5) is significantly lower than in the wild-type (Rb3).

For the probes predicted to cross-hybridise with only one other *S. cerevisiae* gene, cross-hybridisation was observed for *YMR170c, YMR169c, YDR225w, YGR148c, YGL147c, YBR145w,* and *YNL143c* (RatioI at the tested spots range from -2.1 to -1.2) but not for *YOL103w, YDR497c, YGR086c, YJR148w* and *YLR058c* (RatioI at the tested spots range from -50 to -9). It should be noted that, with the exception of *YGR148c* (missing spot of the putative cross-hybridising gene *YGL031c* on our array) and *YNL143c* (hybridisation signal of the putative cross-hybridising gene *YGR069w* below the background signal), all RatioI at the cross-hybridising spots are close to 1 or -1, as expected. It should also be noted that the mean of ratios (for all 17 other tests) at the tested spots are very close to 1 or -1 (*Part III* in **Table 5**).

The different behaviour between the first 7 genes (that cross-hybridise) and the last 5 genes (that do not cross-hybridise) can result from different sources.

The absolute transcript abundance of the cross-hybridising gene may of course interfere with the signal at the tested spots, in addition to the cross-hybridisation parameters (%ID, ML and LC). However, transcript abundance of the cross-hybridising gene (given by Rb3 and Rb5) shows low and high levels in each category, suggesting that this factor has little importance in the present tests.

Similarly, the alignment size (ML) does not seem to play a major role (67 to 348 for the non cross-hybridising probes; and 31 to 494 for the cross-hybridising probes).

In contrast, the LC value seem to play the most important part in our results. If one excepts *YBR145w,* the LC values for the 6 tested probes which cross-hybridise, range from 31 to 187 nucleotides, and for the 5 tested probes which do not cross-hybridise, from 15 to 24 nucleotides. The cross-hybridisation observed for *YBR145w* (with a LC value of 14 nucleotides) is probably due to the high transcript abundance of the cross-hybridising gene YMR303c (Rb5 of 338).

The cross-hybridisation observed for *YNL143c* (LC of 31 nucleotides) seems in contradiction with the previous statement because the transcript intensity of the cross-hybridising gene (*YGR069w*) is very low. However, *YNL143c* also shares 31-34 nucleotides in common with several intergenic regions (sequence positions 223113-223147 on chromosome 1; 145863-145894 on chromosome 2; 678007-678040, 892693-892725, and 1357469- 1357501 on chromosome 4; 80602-80633 on chromosome 7; 236960-236991 and 549757-549835 on chromosome 8; 387143-387174 on chromosome 9; 22042-22073 on chromosome 11; 122007-122039 and 459804-459835 on chromosome 12; 822590-822623 on chromosome 13; 359342-359376 on chromosome 14). Except position 459804-459835 on chromosome 12 (rDNA loci), none of those intergenic regions contains genetic objects such as Tys, tRNAs, snRNAs, LTRs, rDNAs, etc... but are 6 to 1794 nucleotide distances from the stop or ATG of the next CDS. For *YEL033w,* the observed RatioI was -16.6, indicating that there is no cross-hybridisation of the intergenic region (the sequence position is 92686-92717 on the chromosome 4; and locates 45 or 220 nucleotide distances from the next and previous CDS) with this probe. These results suggest that target transcripts from intergenic regions can lead to cross-hybridisation, thus validating our concern to examine cross-hybridisation of probe with intergenic sequences.

A last explanation for the absence of cross-hybridisation could be that the cross-hybridising region lies upstream of a long CDS such that this region is underrepresented in our labelled reverse transcriptase-generated cDNA. This, however, seems unlikely because in two cases, the position of the corresponding region from the 3'-end of the gene ranges from 18 to 246 nucleotides (for probes showing cross-hybridisation) and from 38 to 240 nucleotides (for probes not showing cross-hybridisation).

Results of the tests for the probes (*YML113w* and *YMR011w*) having putative cross-hybridisation with several other yeast genes are presented in **Table 6.** Despite the presence of various cross-hybridising genes, those results are compatible with the above observations in the sense that *YMR011w* (whose LC value range from 11 to 20) do not show cross-hybridisation (RatioI of -16.6) while *YML113w* does (LC value from 11 to 32; Cy5/Cy3 ratio of -1.5). As before, the RatioI for the cross-hybridising spots are close to 1 or -1.

In summary, of all the parameters that may interfere with cross-hybridisation results, it seems that LC plays the most important part. From the present data, similar sequences with LC < 25 should not significantly cross-hybridise in regular experiments while sequences with LC >24 do.

Effect of the transcript abundance of the cross-hybridising gene may of course play some role, in particular when ML and LC are low (i.e. ML of 53 with LC of 14).

Considering all those results, a total of 957 probes in the microarray design of the invention are each specific for a unique target gene.

**Table 1.**

| **Total number of Saccharomyces cerevisiae protein coding genes and corresponding probes** | | | | | |
|---|---|---|---|---|---|
| | ***No CH*** | ***CH with other CDS only*** | ***CH with IR only*** | ***CH with both CDS* & *IR*** | ***Total*** |
| *Natural CDSs* | 4523 | 841 | 191 | 248 | 5803 |
| *Designed probes* | | | | | |
| PCR products | 4972 | 0 | 0 | 0 | 4972 |
| Oligonucleotides | 688 | 65 | 9 | 69 | 831 |
| **CH:** crosshybridisation; **CDS:** Codingsequences; **IR:** Intergenic regions | | | | | |

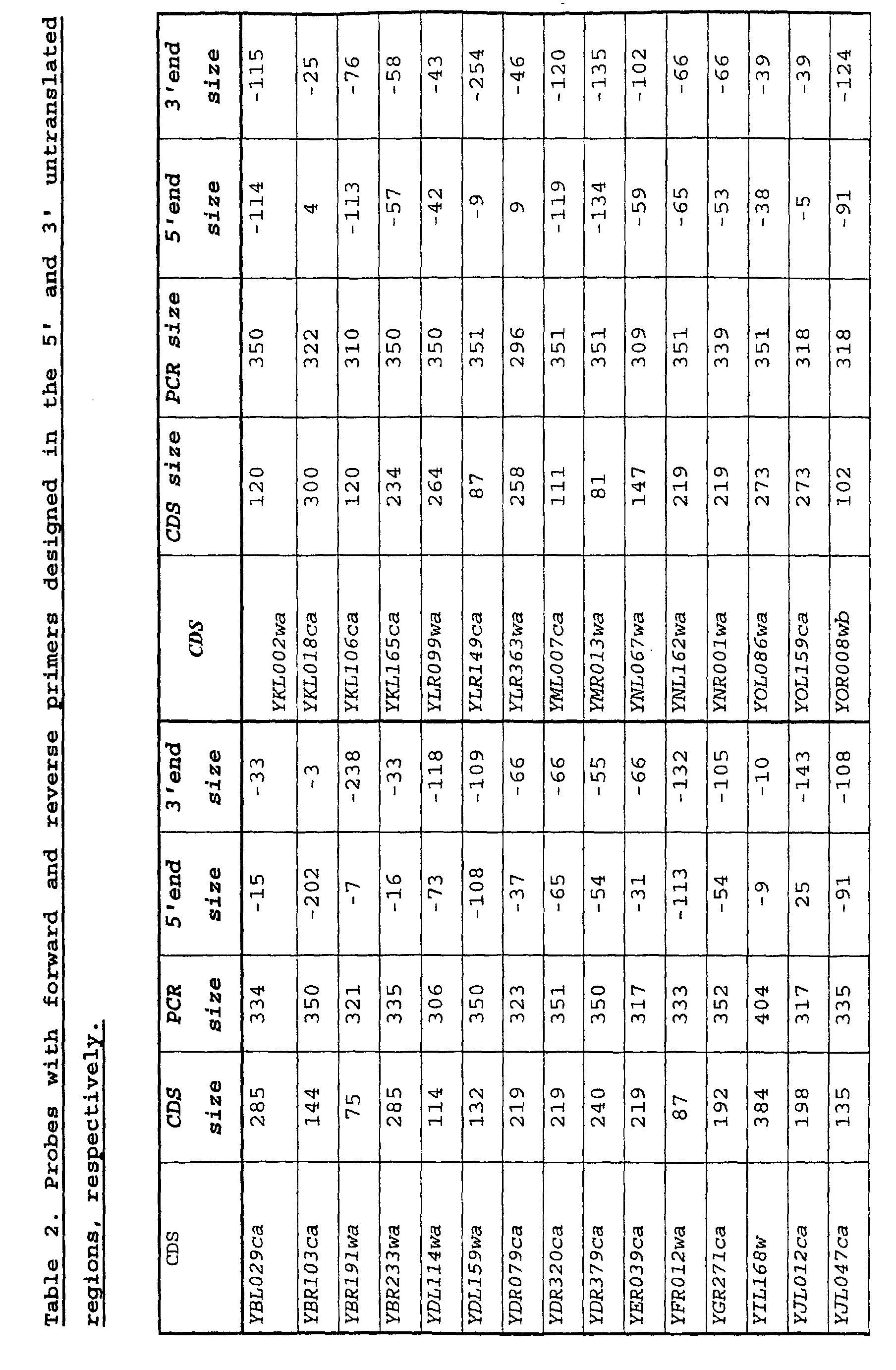

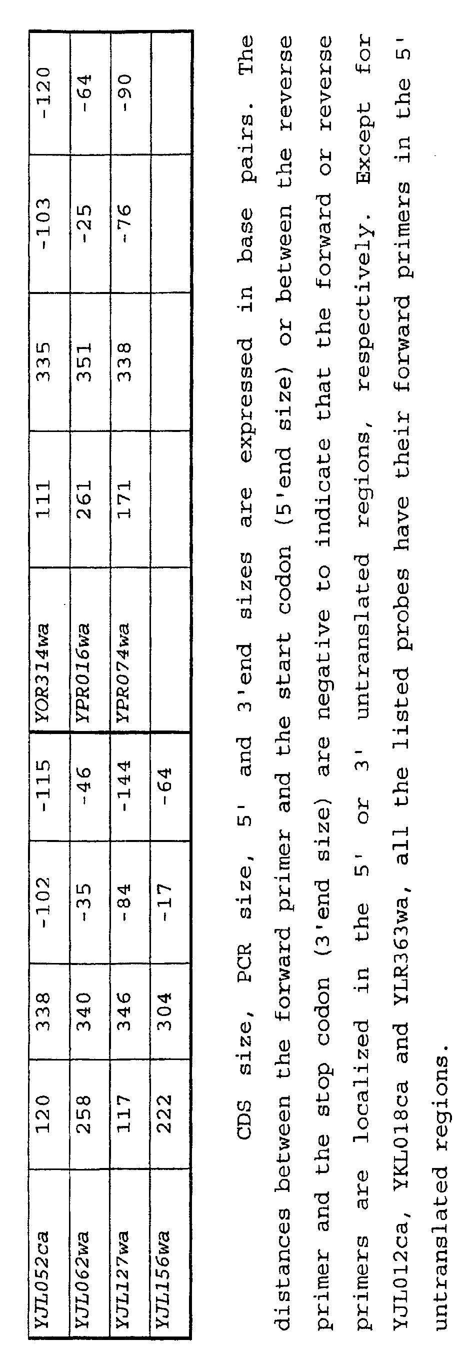

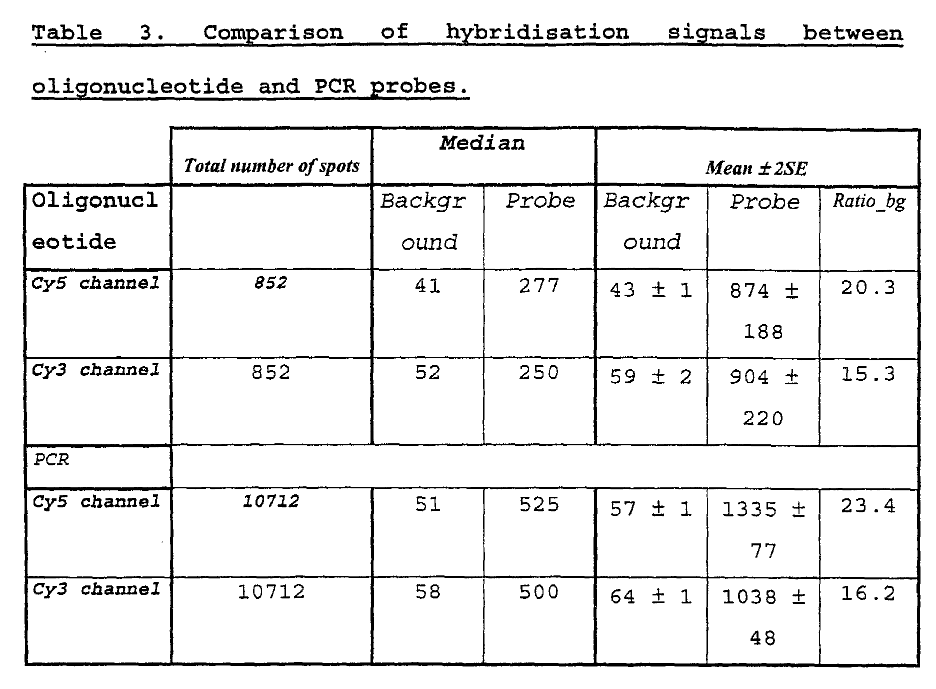

Intensities and corresponding background values were computed separately for oligonucleotides and PCR probes in experiment *BY4742* using an array of Batch800. Cy3- and Cy5-labelled cDNA targets were prepared from the total RNA isolated from the wild-type strain BY4742. For each channel, the distribution of intensities for all genes computed either for oligonucleotides or for PCRs are given by the median, the mean (with 2 x standard error). Ratio_bg is mean intensity of the probe over mean intensity of the background signal for oligonucleotides and PCR probes within the microarray experiment.

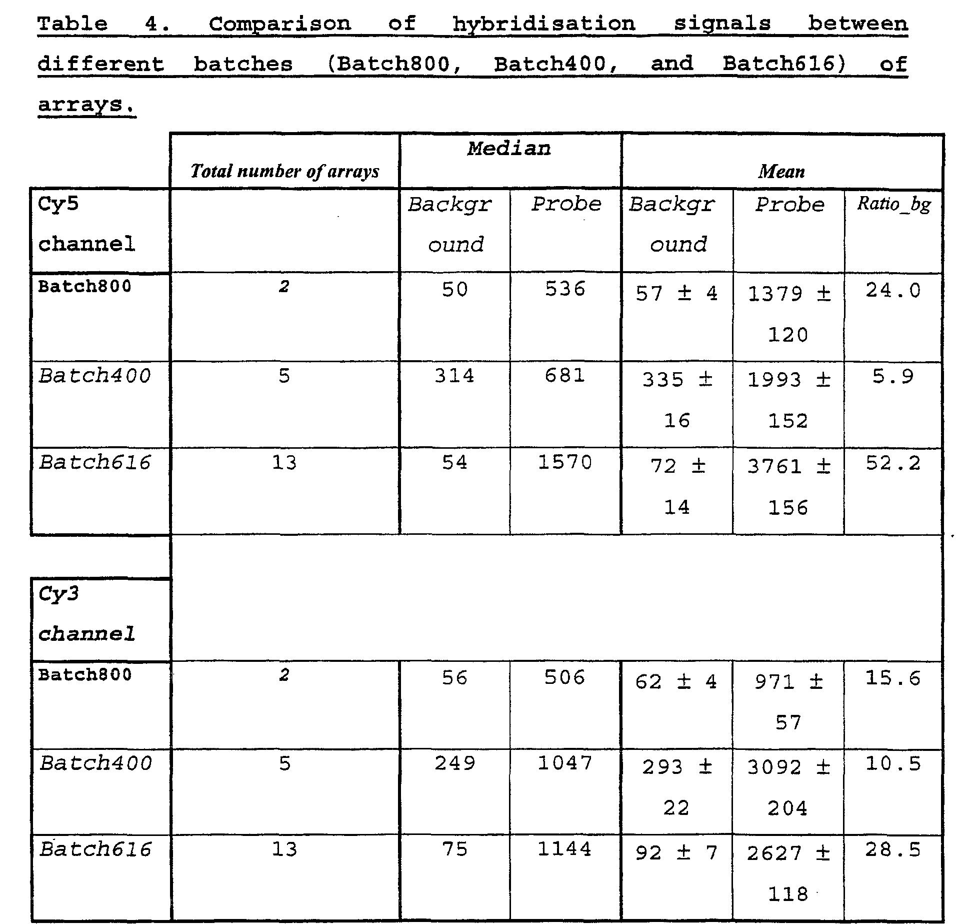

For all arrays of a given batch, the mean intensity value for each gene was first calculated considering all intensity measurements above 95% of the backgroung threshold (see "Materials and Methods"). The figures were then used to compute the distribution of intensities for all genes. These distributions are described in this table by their medians, and means (with 2 x standard error). Ratio_bg is the mean intensity of the probe in the batch over mean intensity of the background signal.

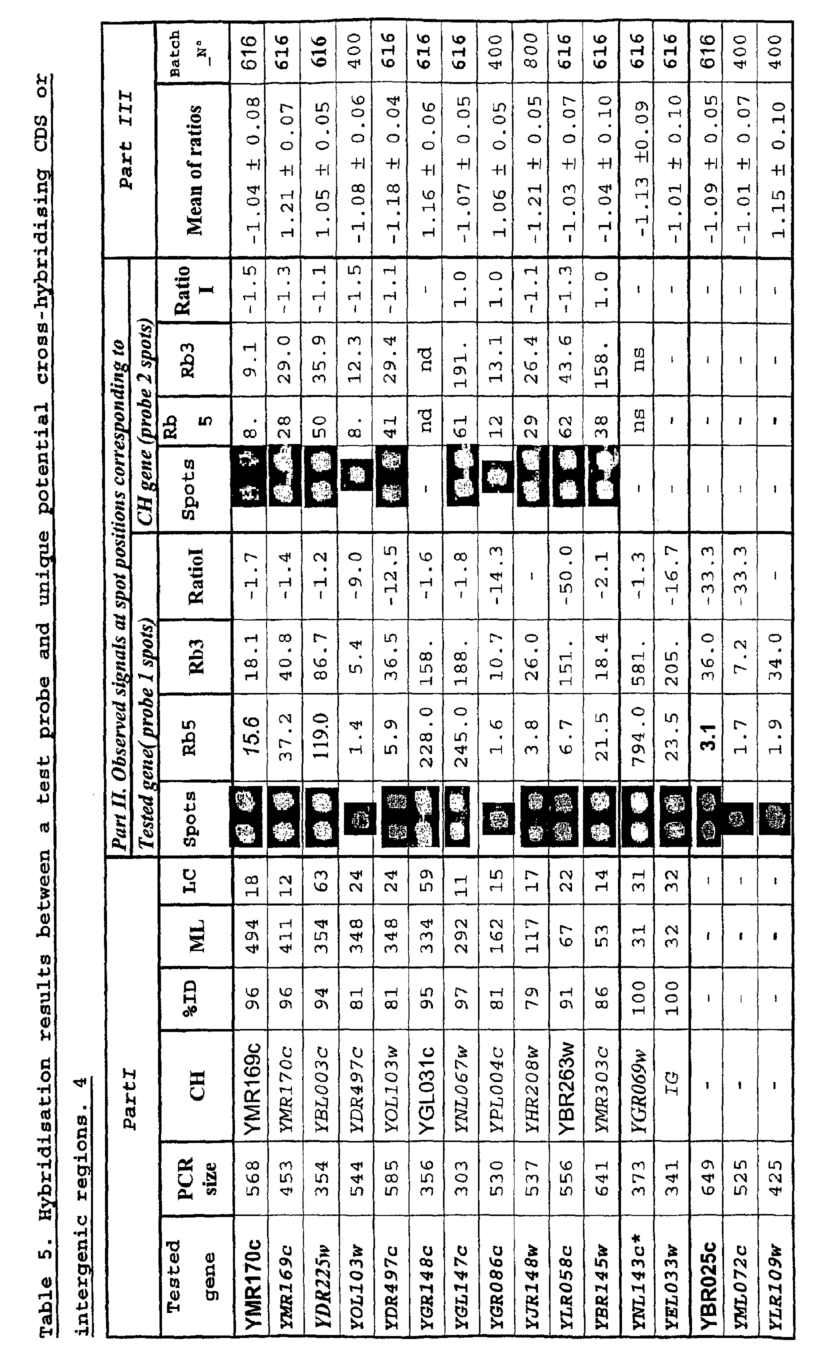

"Part I" describes the tested probes (indicated by the CDS name) with its size (in bp), the name of the potential cross-hybridising CDS (CH) and the alignment parameters of the potential cross-hybridising region (%ID, percentage of identity; ML, alignment size in bp; LC, longest contigous match in bp). IG, intergenic sequences. (*) indicates that the gene also cross-hybridise with intergenic sequences. *"Part II"* describes the results of hybridisation experiments using the deletant of the gene corresponding to the tested probe (Cy5-labelled cDNA) compared with the wild-type strain BY4742 as reference (Cy3-labelled cDNA). Figures (Rb5 or Rb3) represent the transcript abundance (hybridisation signals divided by the mean of background values of the array) for the probe and for the crosshybridising gene. Images of the spots are shown. White spots denotes saturated pixels. RatioI is the normal ratio (normalised Cy5 hybridisation signal over normalised Cy3 hybridisation signal). Ratio below 1.0 were inverted and multiplied by -1 for symmetry. nd, non-determined. ns, non-significant signal. *Part III* indicates for each probe, the average value (± 2*standard error, with 95% confidence) of the normalised ratio for all experiments in which the corresponding CDS is not deleted. Batch_N° referred to the batch number of the array used. Note that the probes from microarrays of the Batch400 are not printed in duplicate.

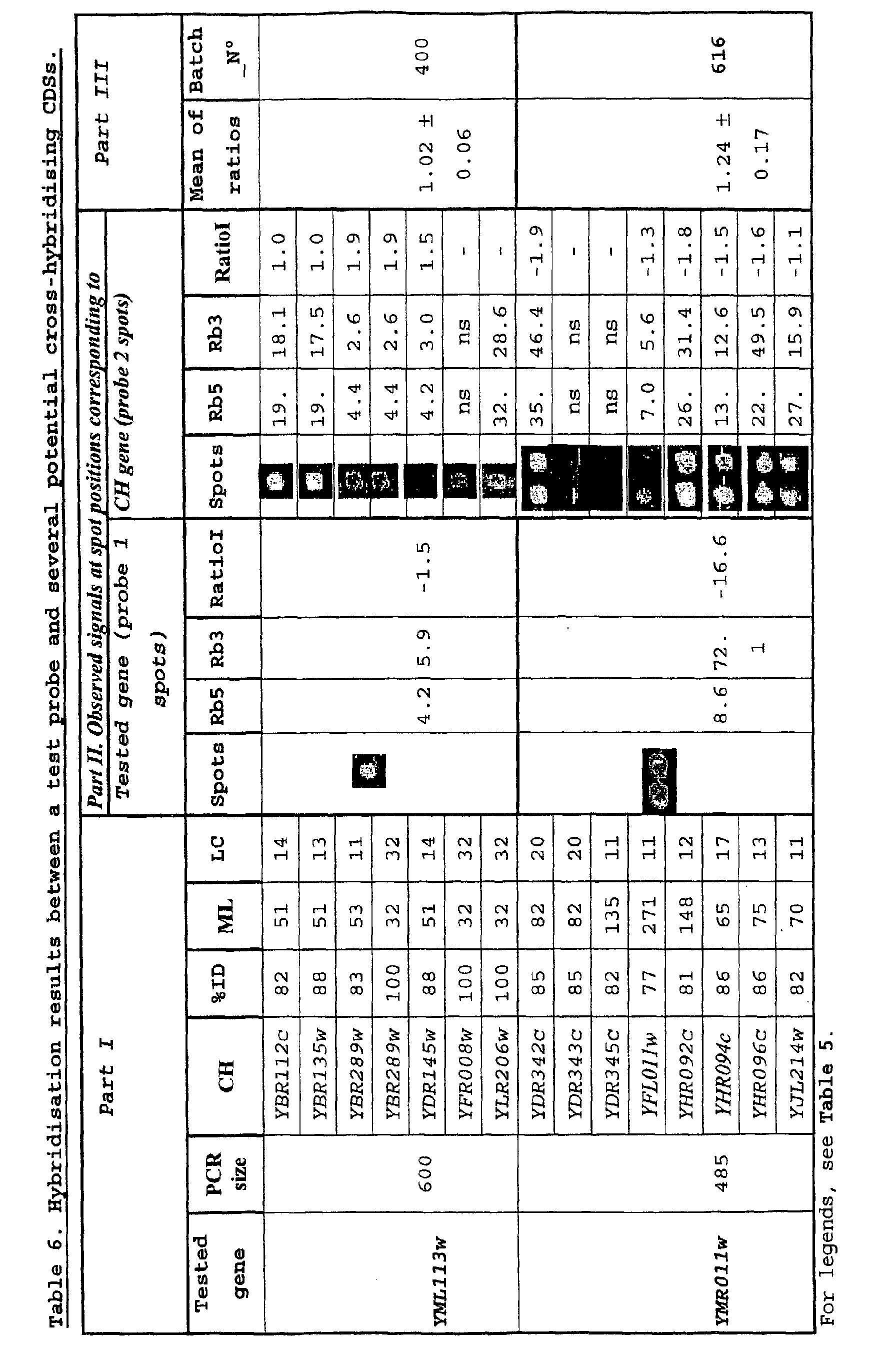

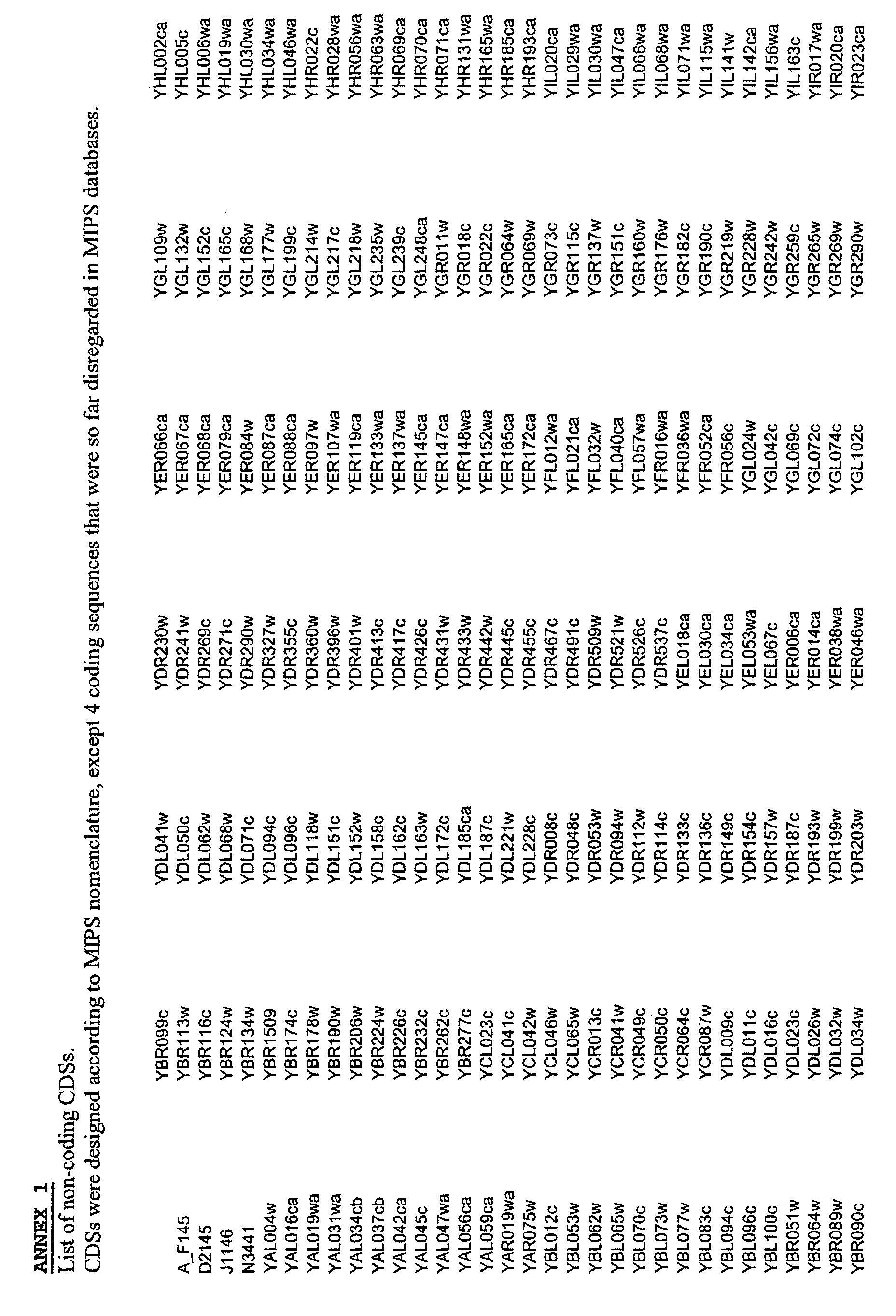

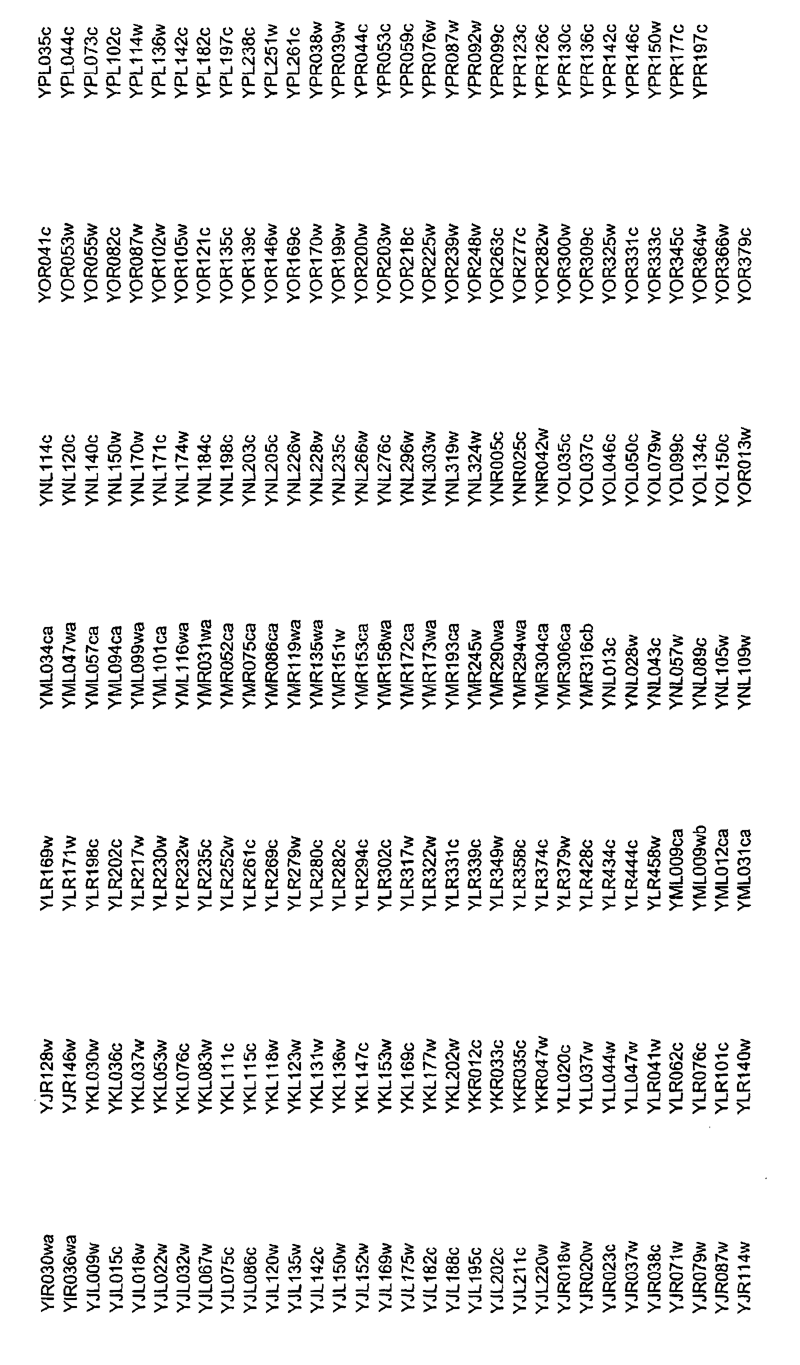

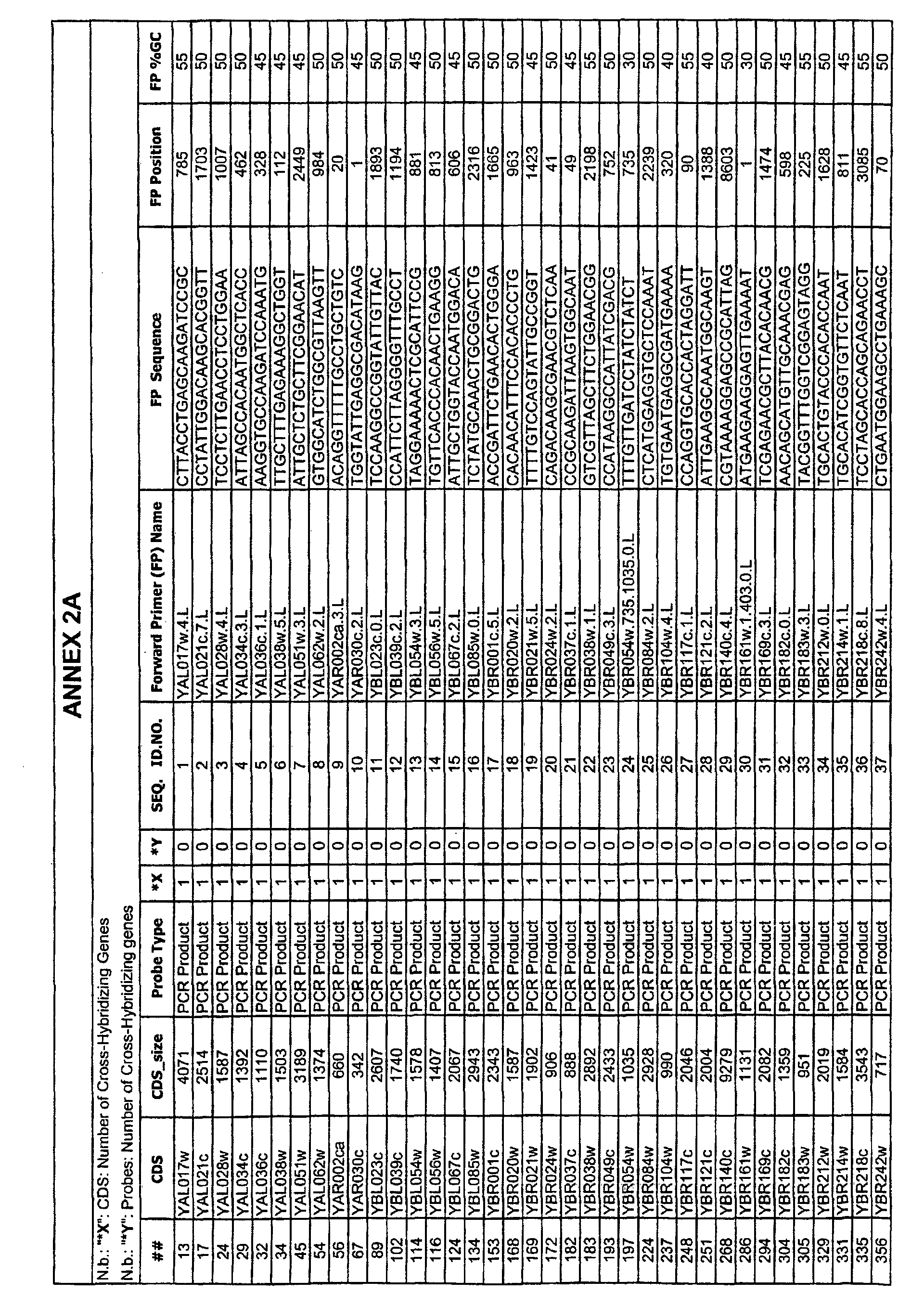

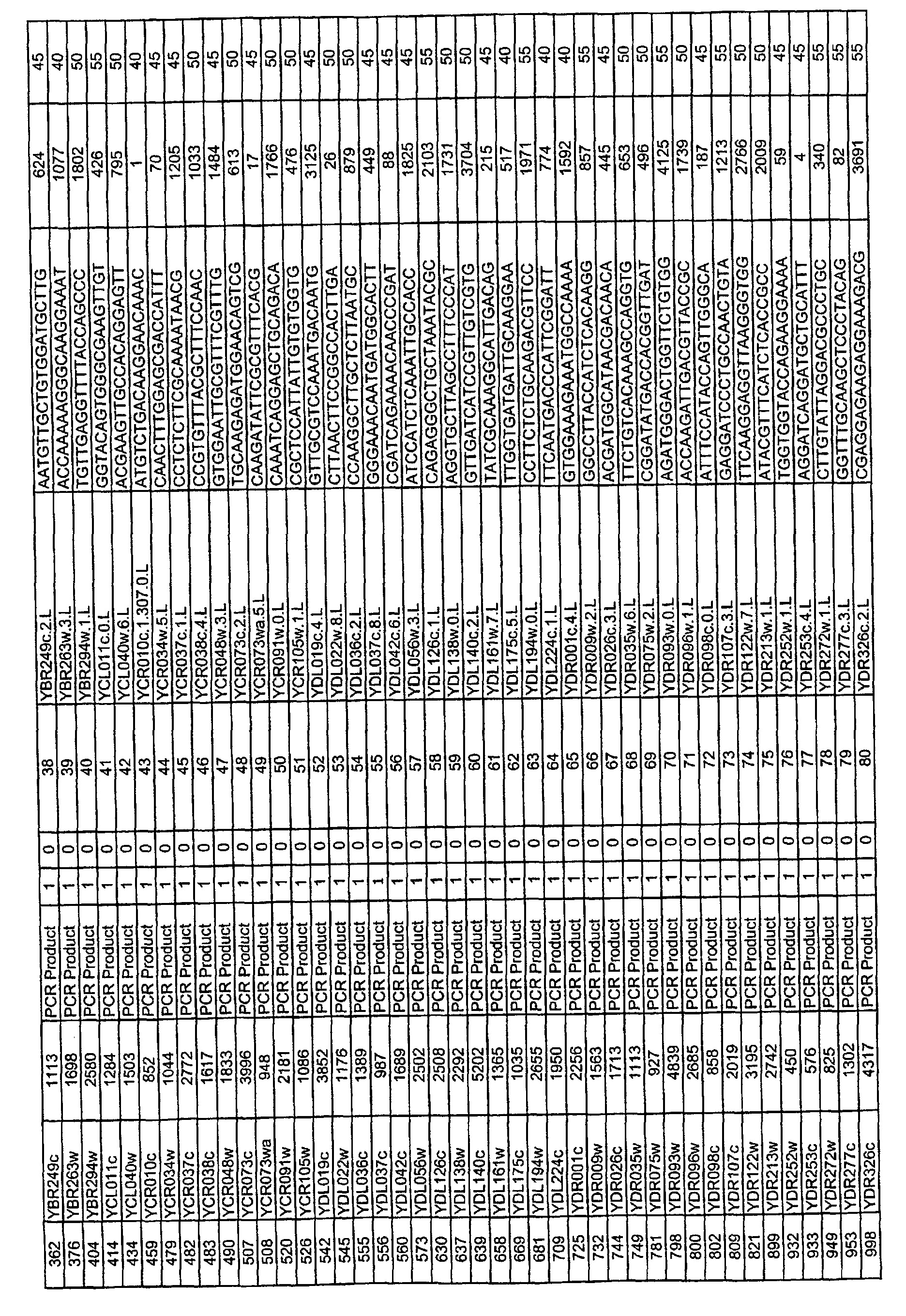

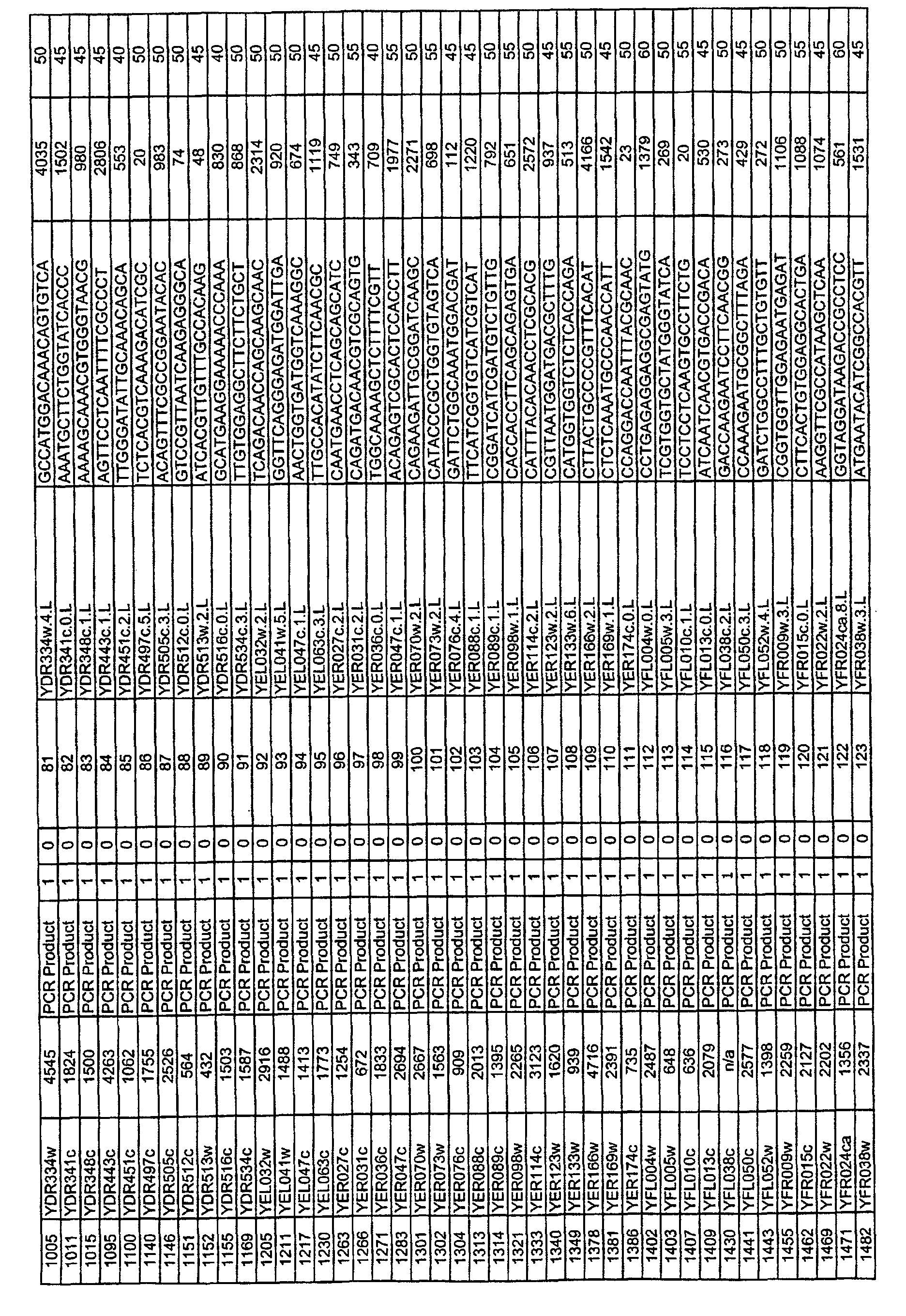

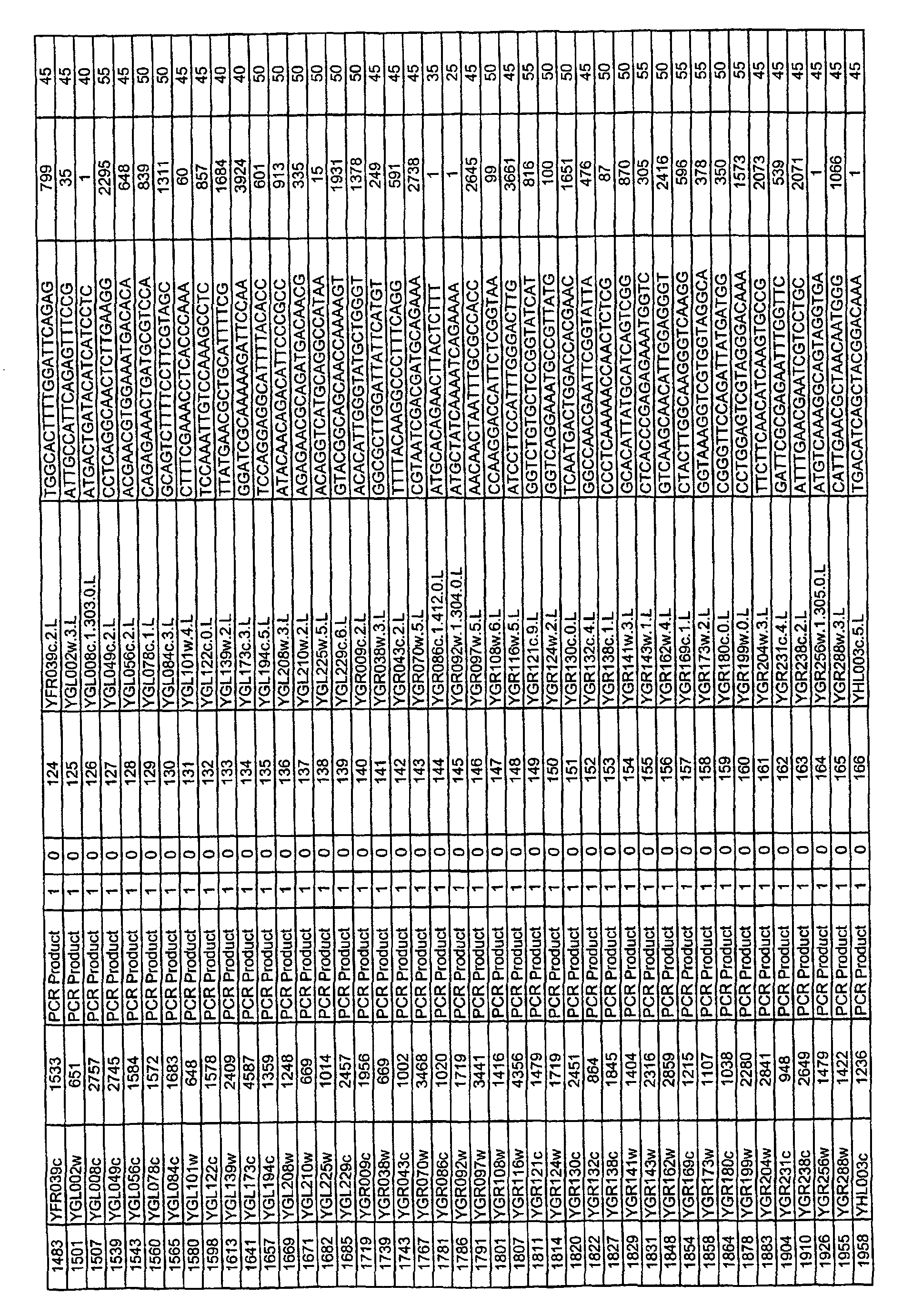

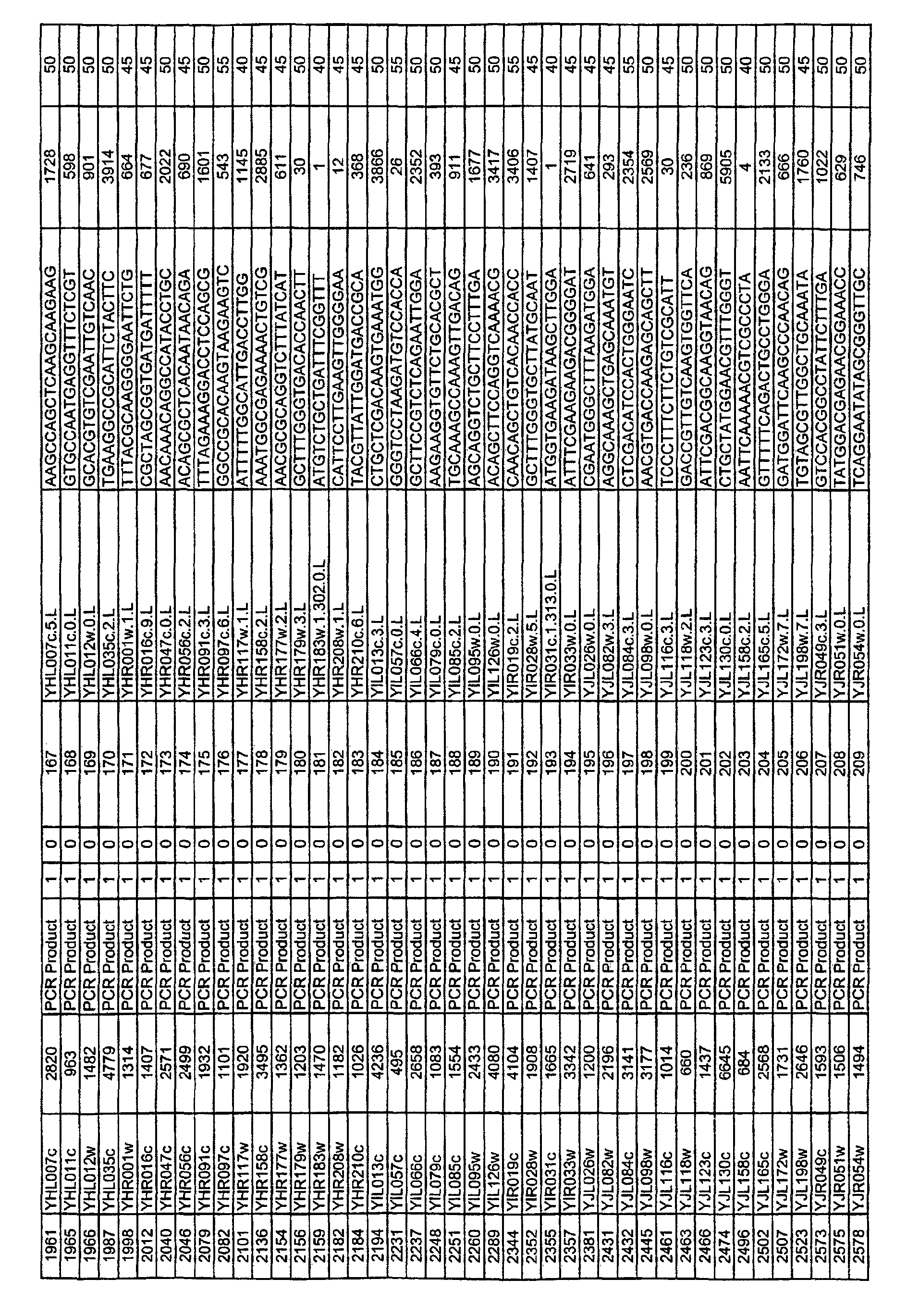

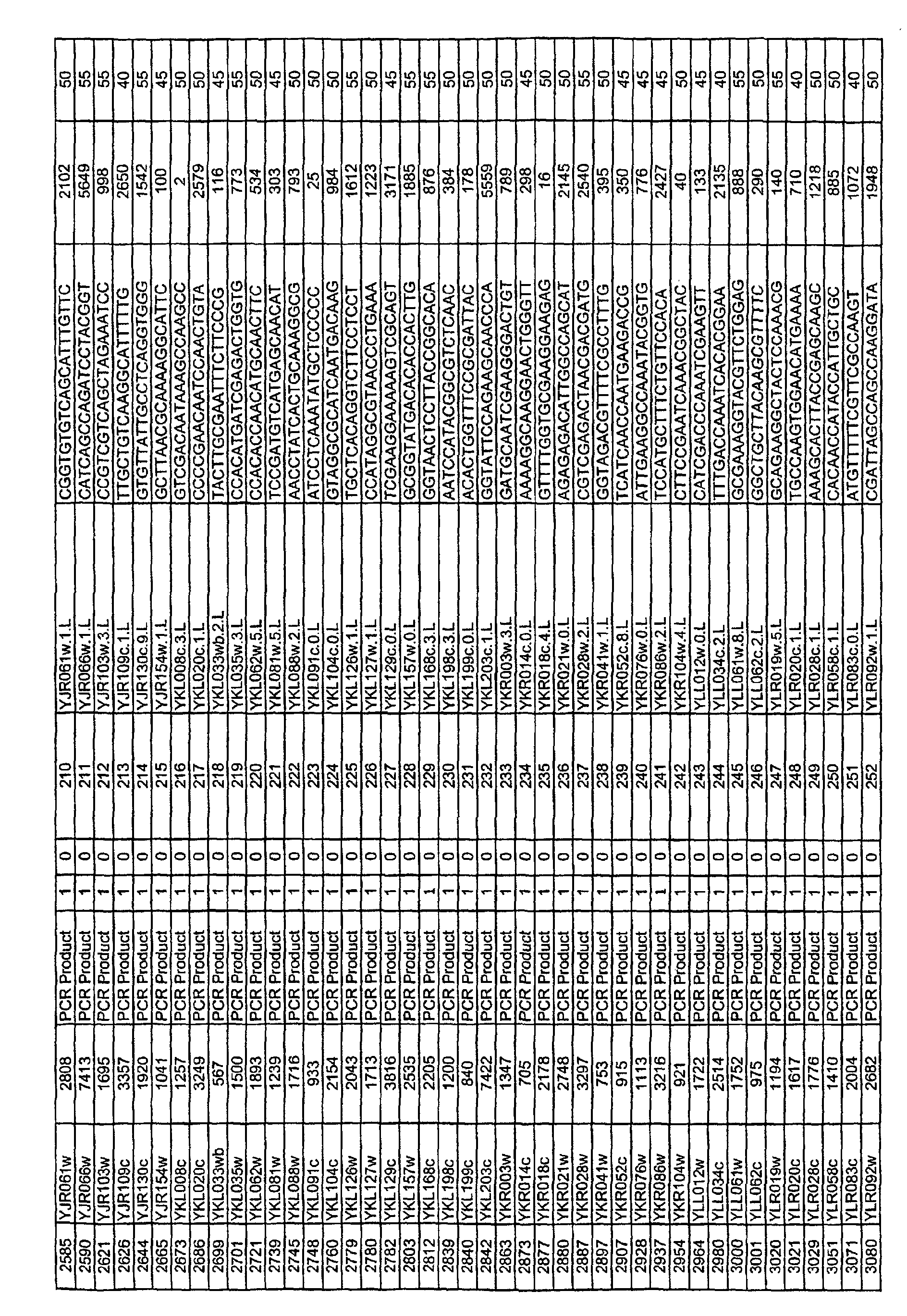

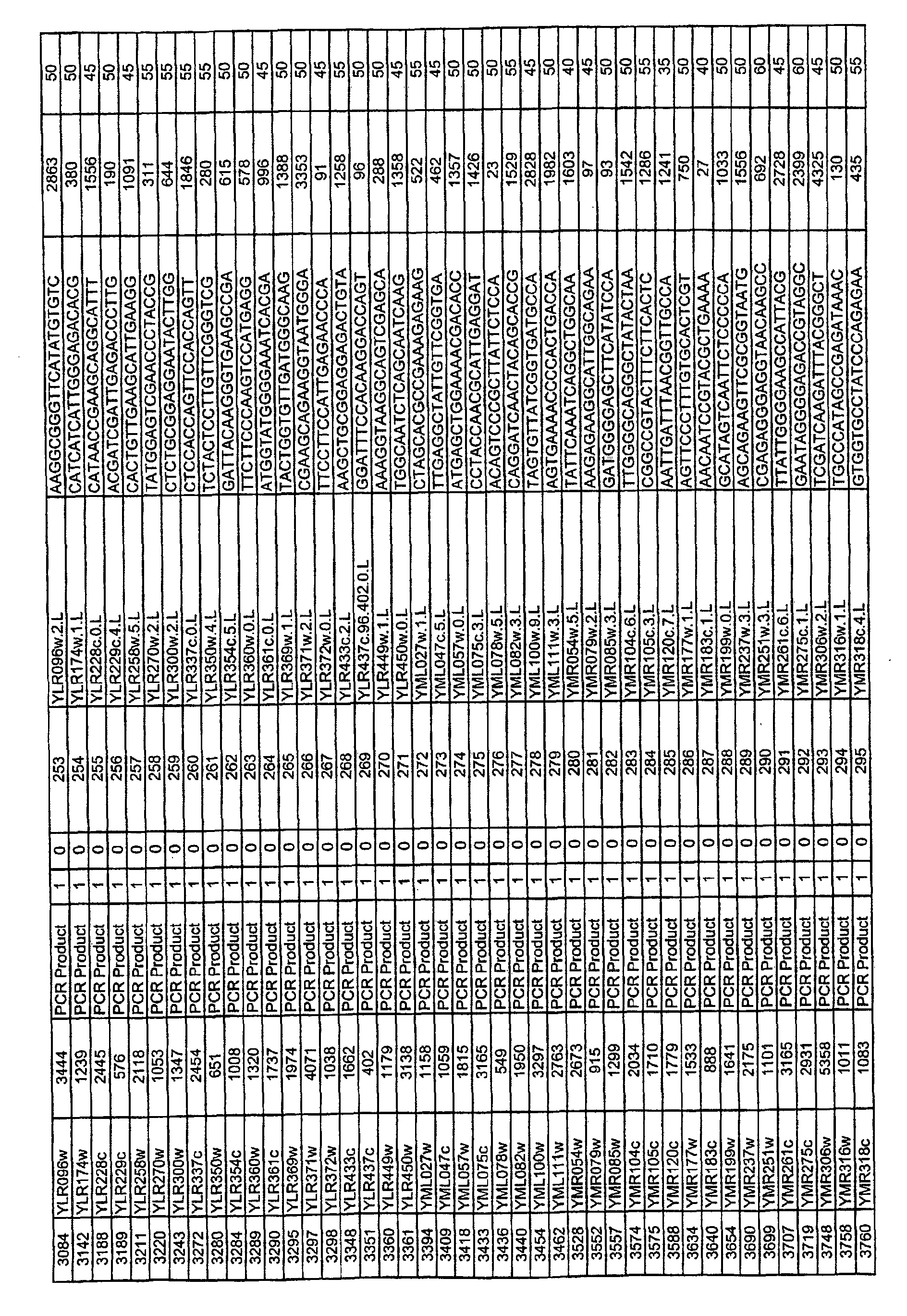

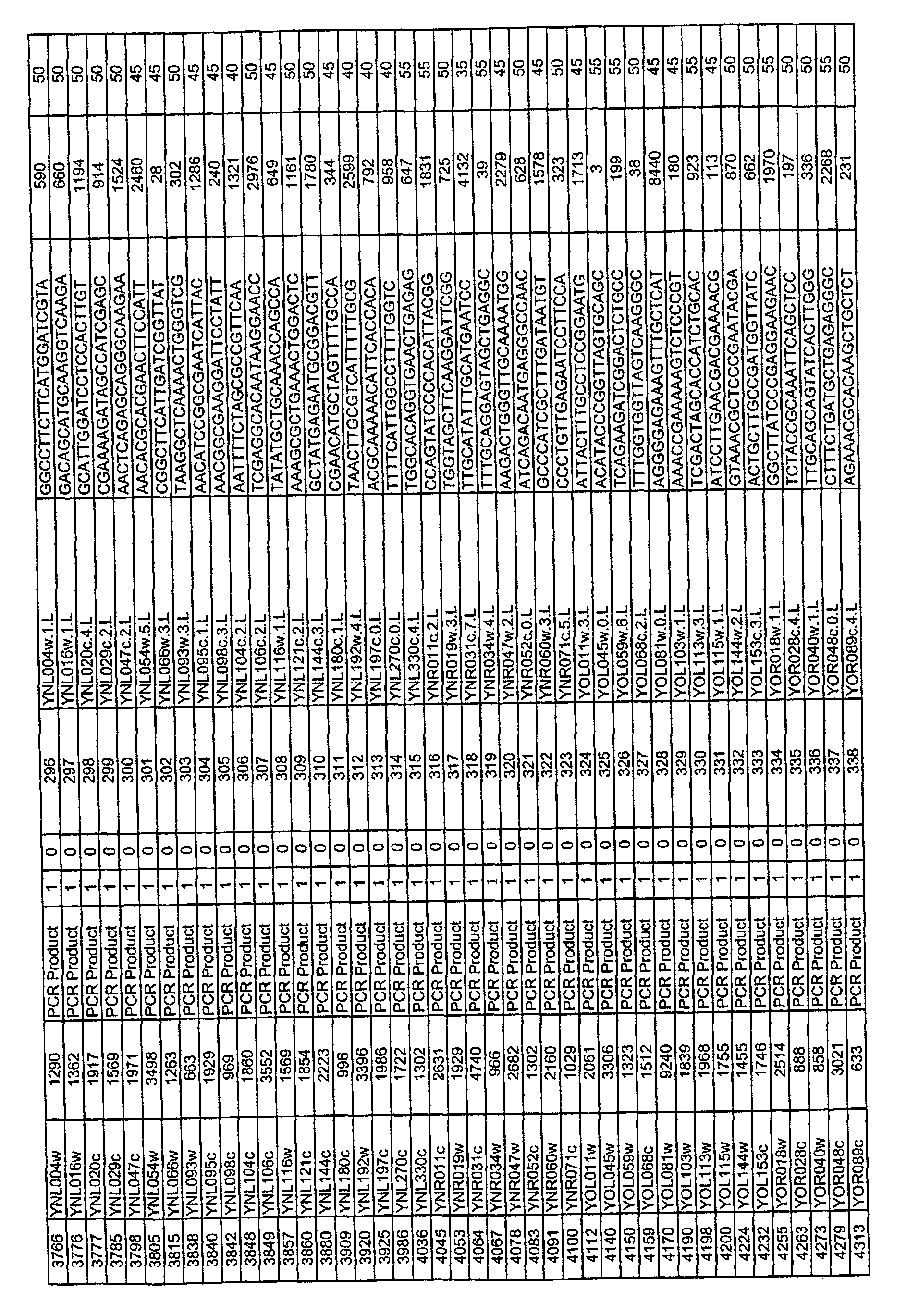

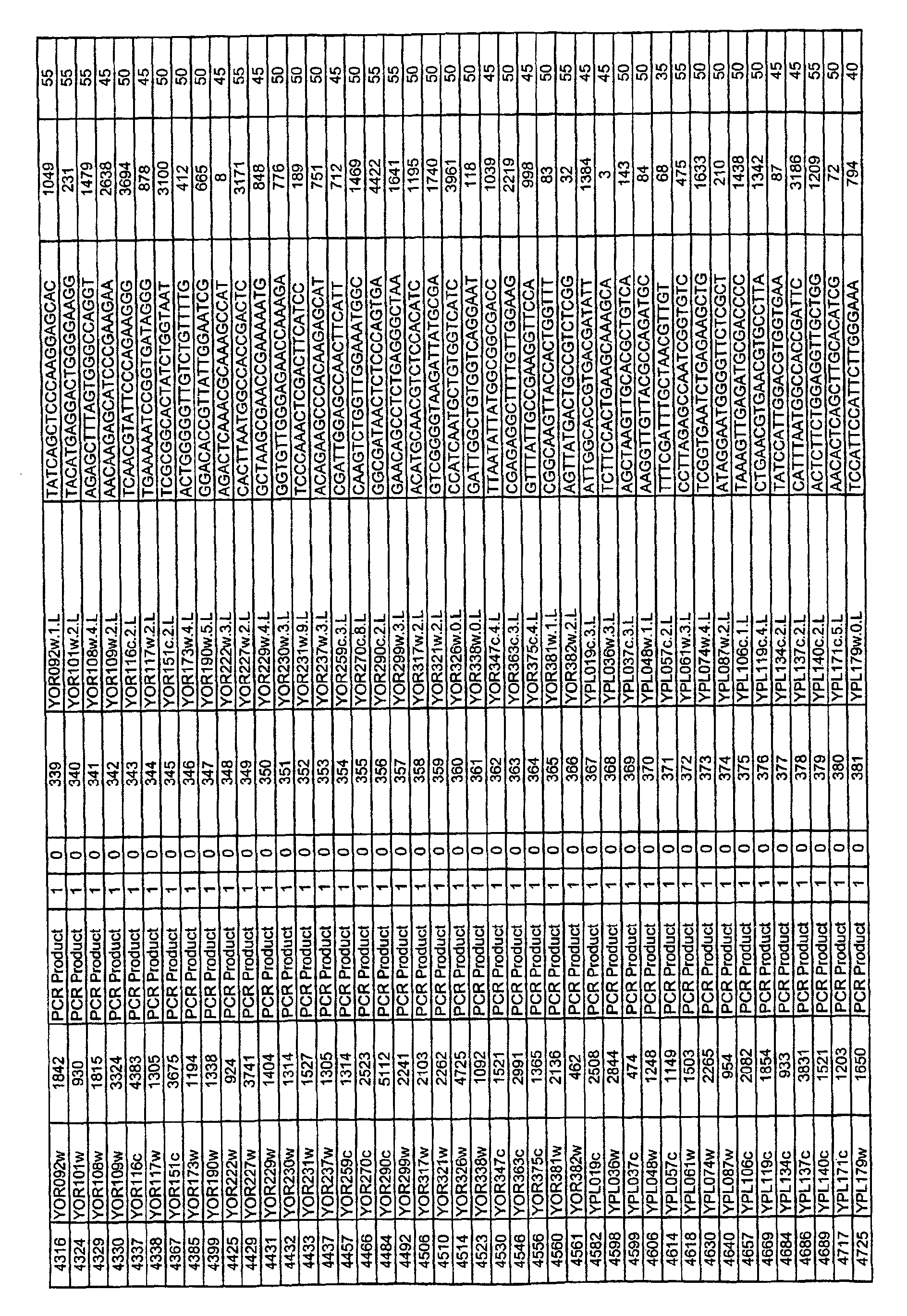

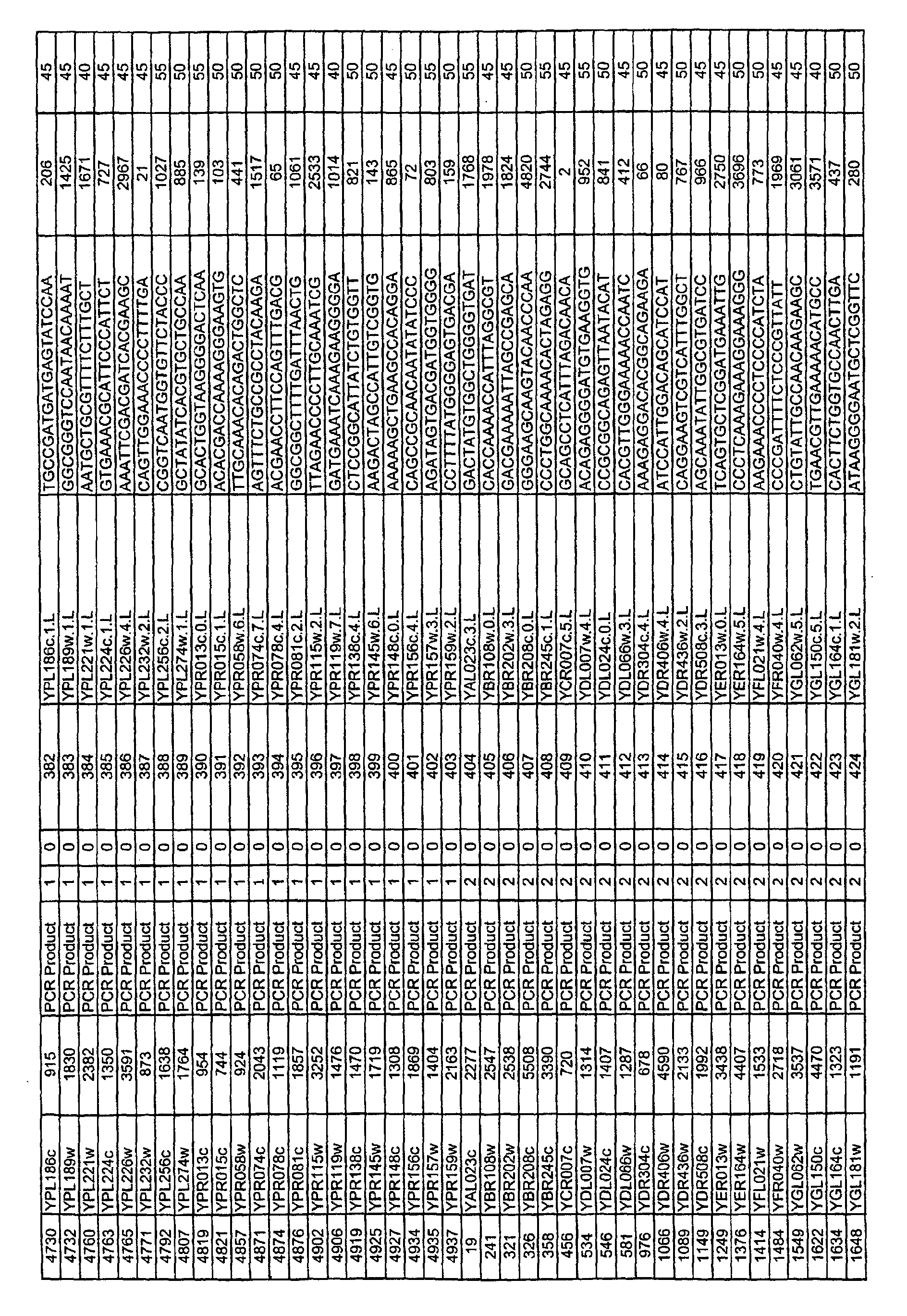

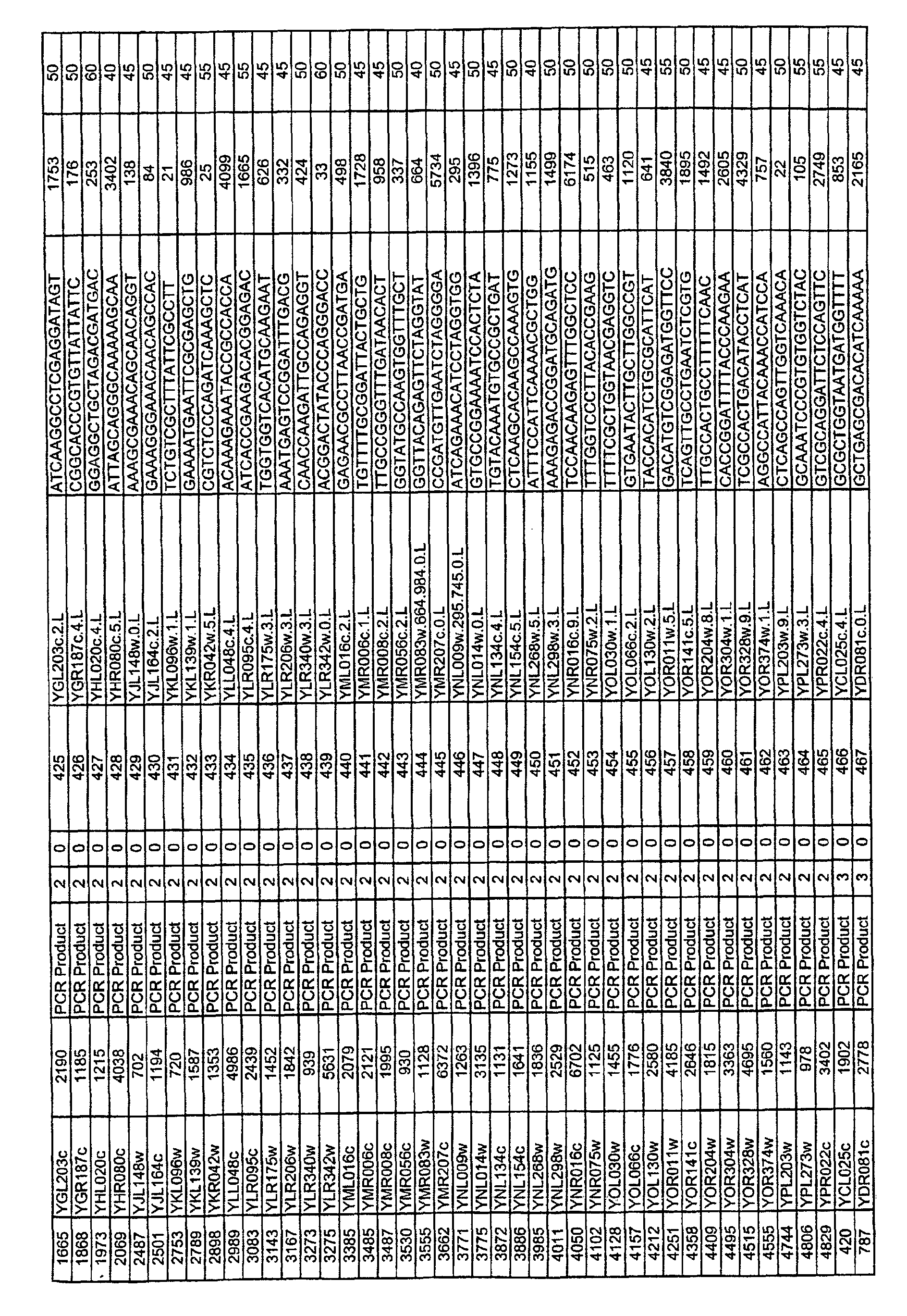

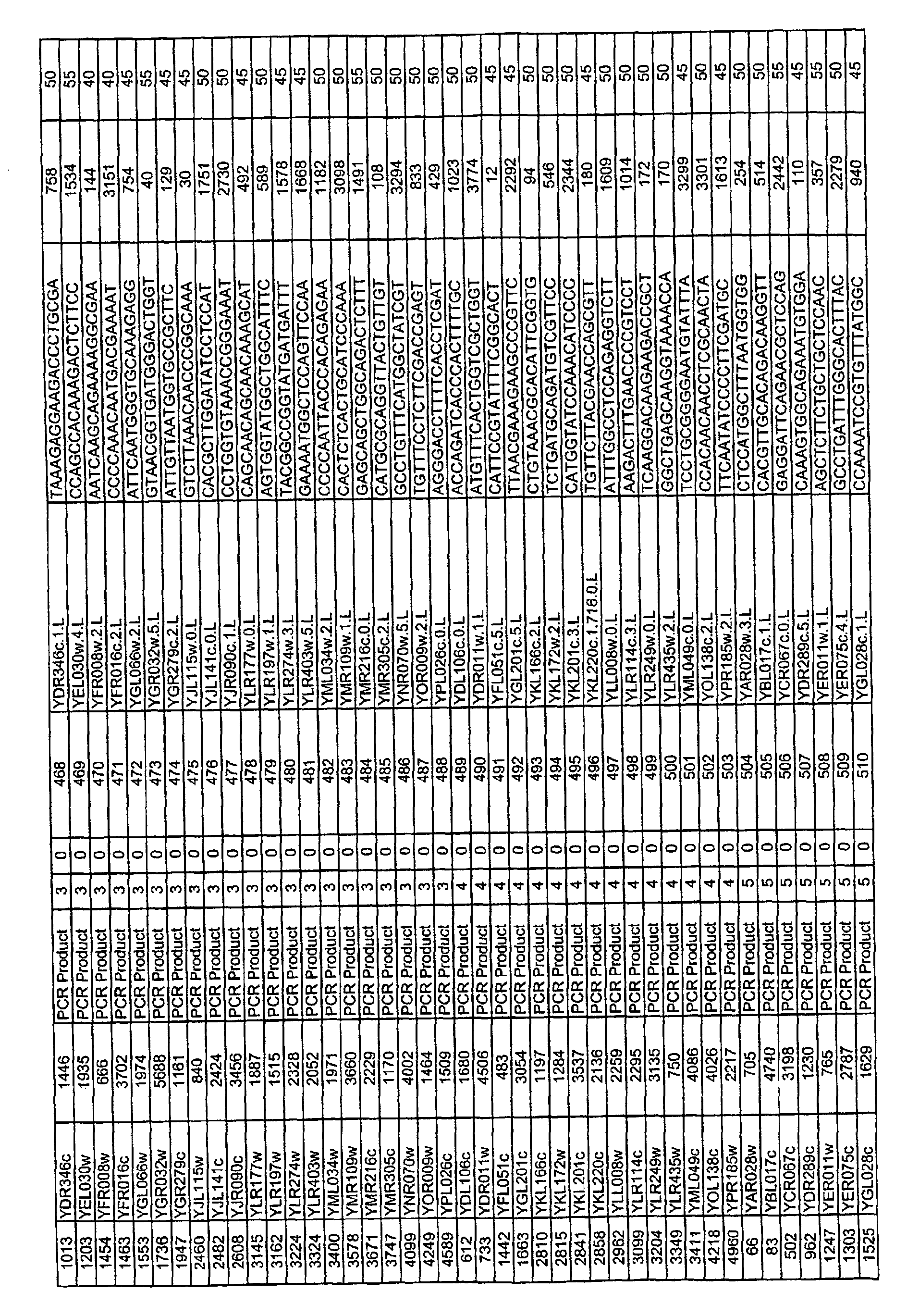

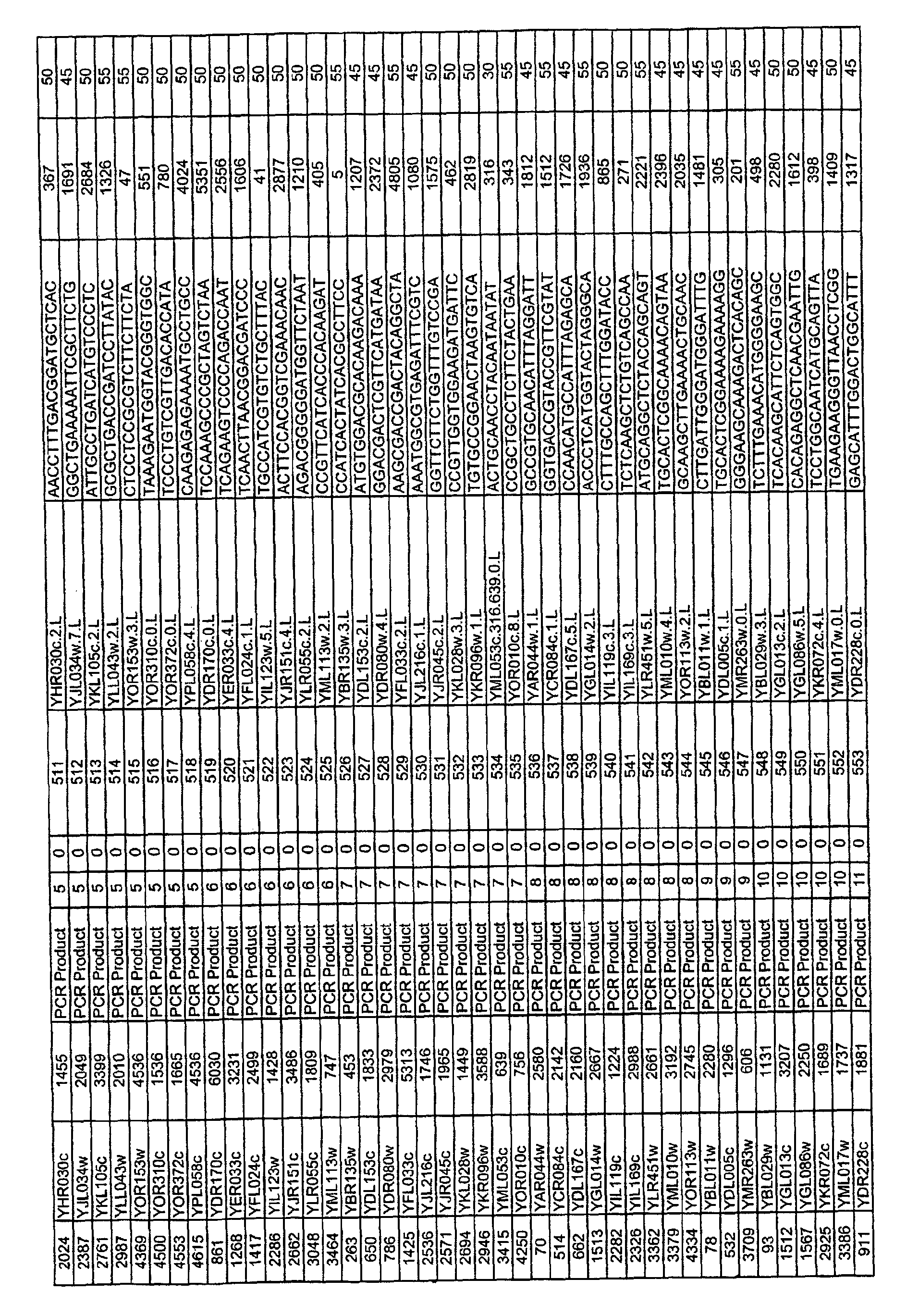

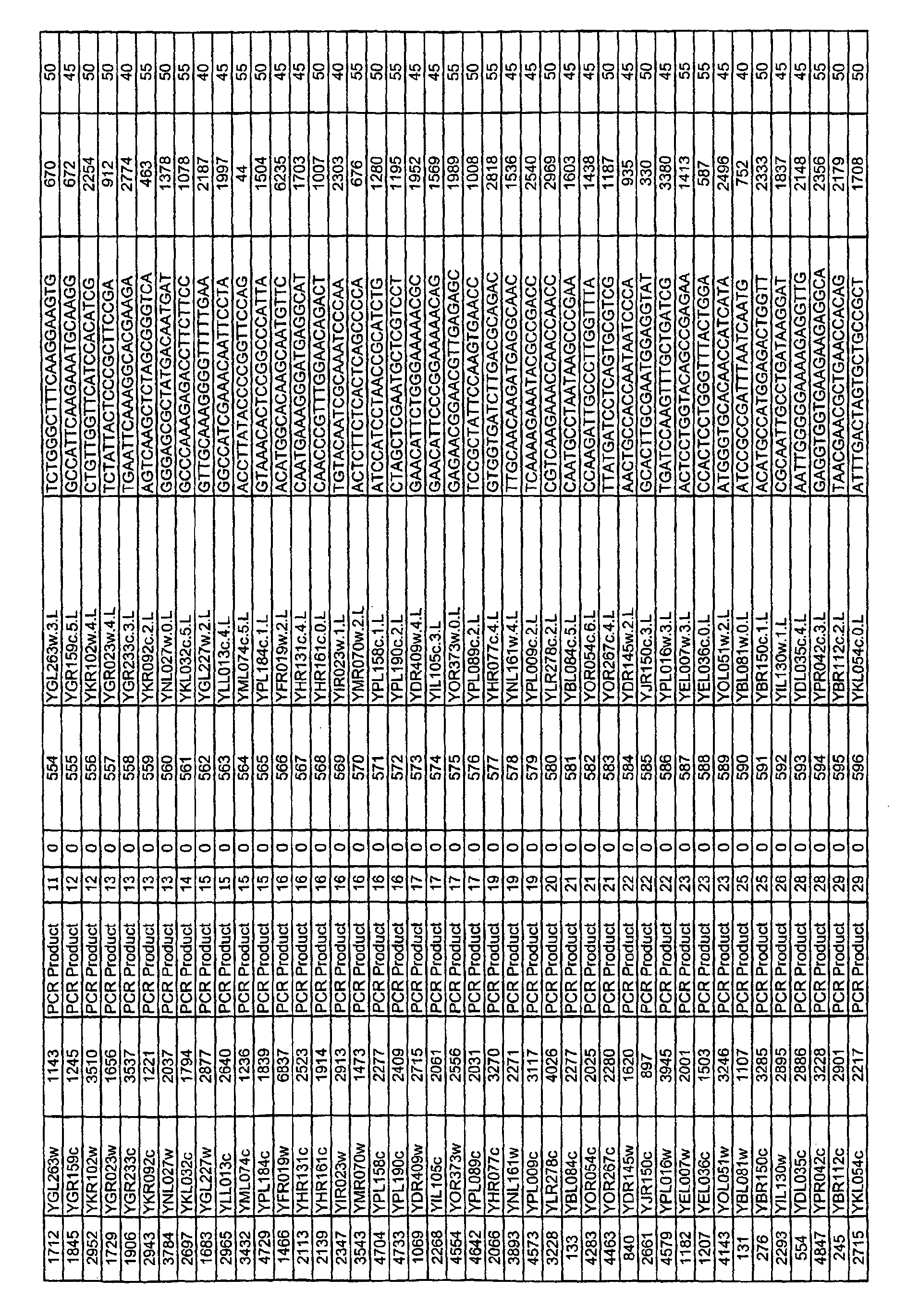

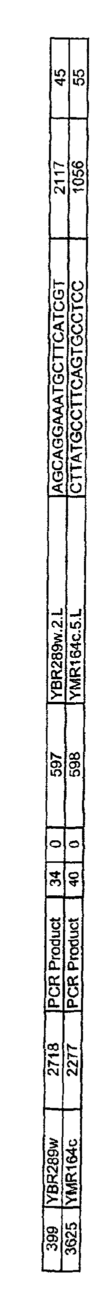

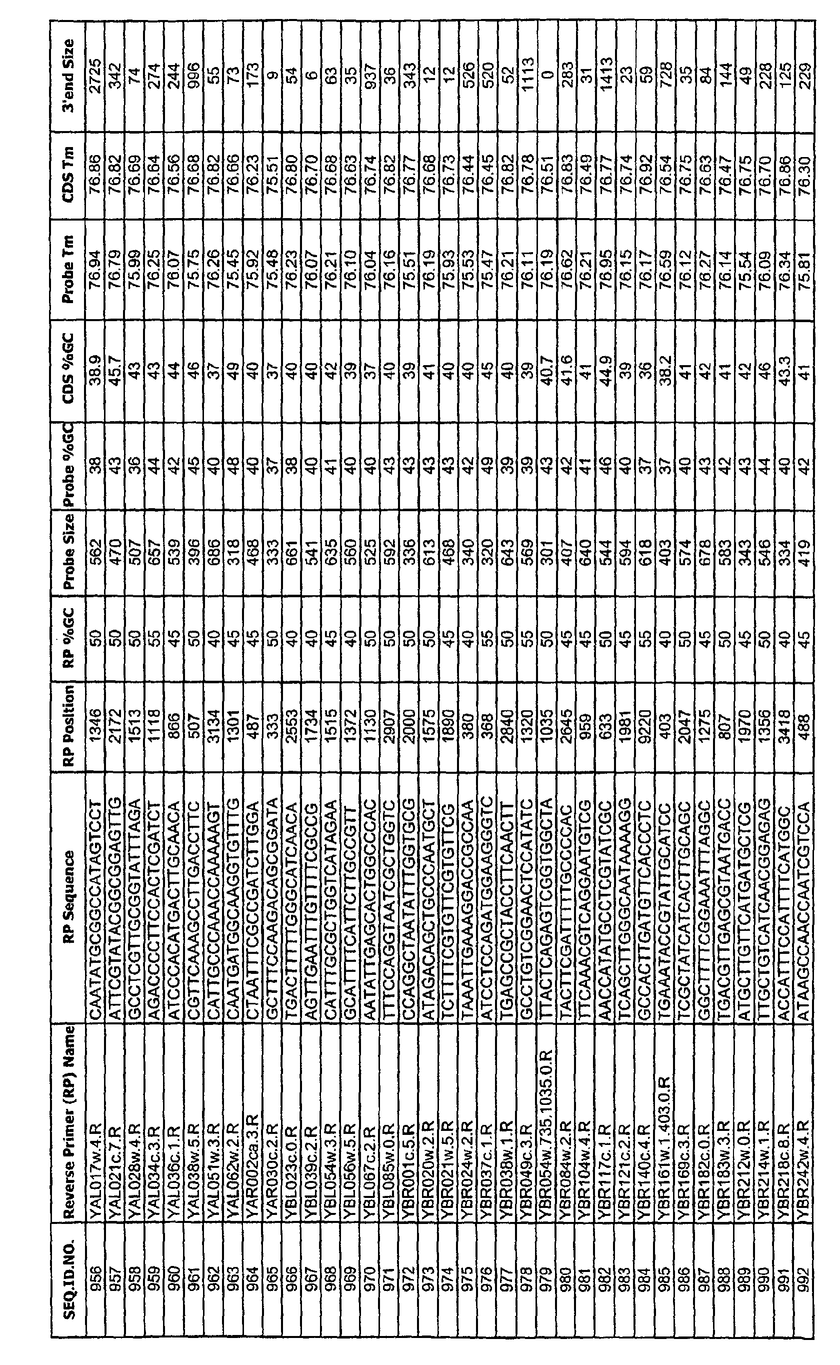

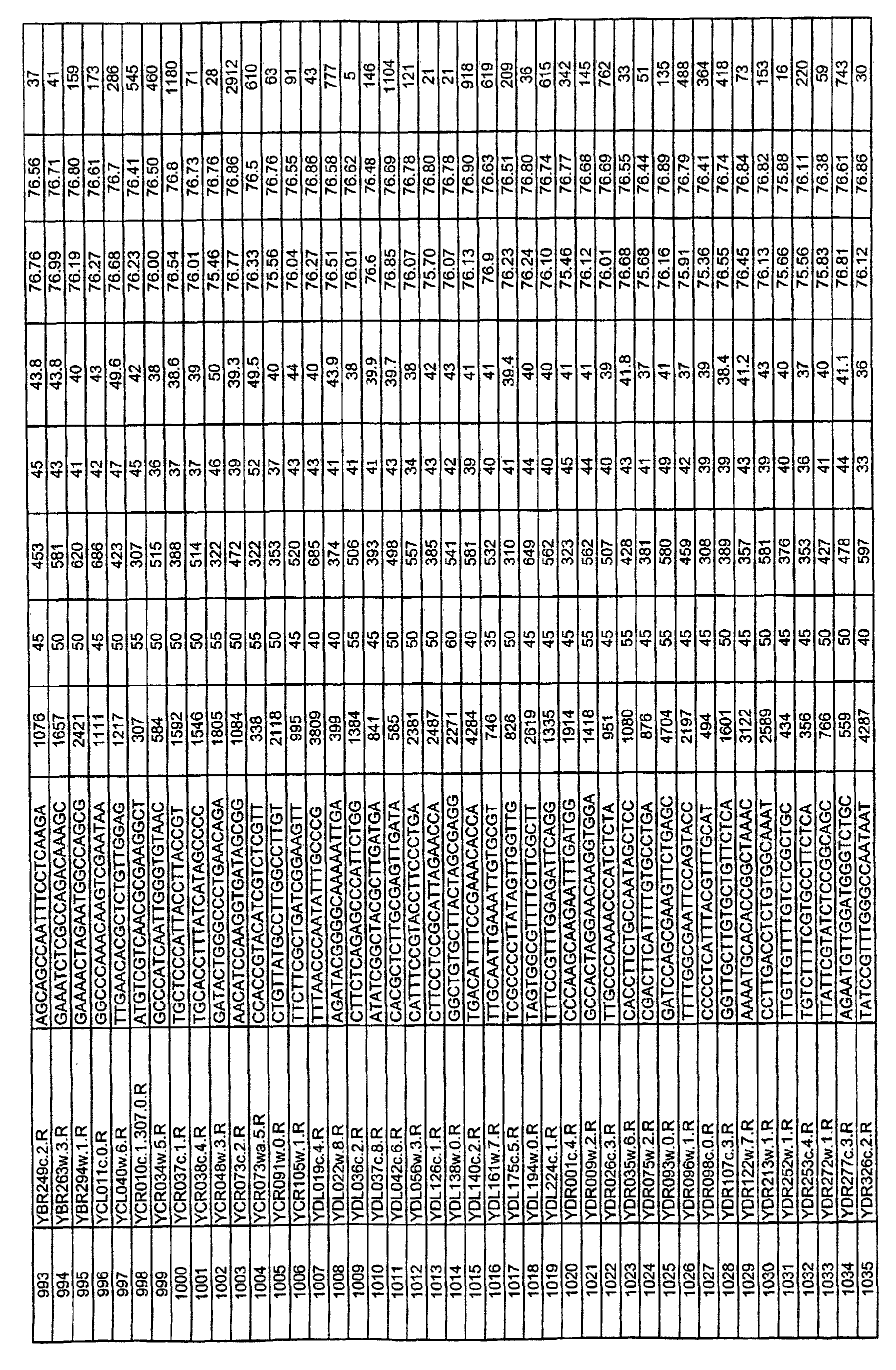

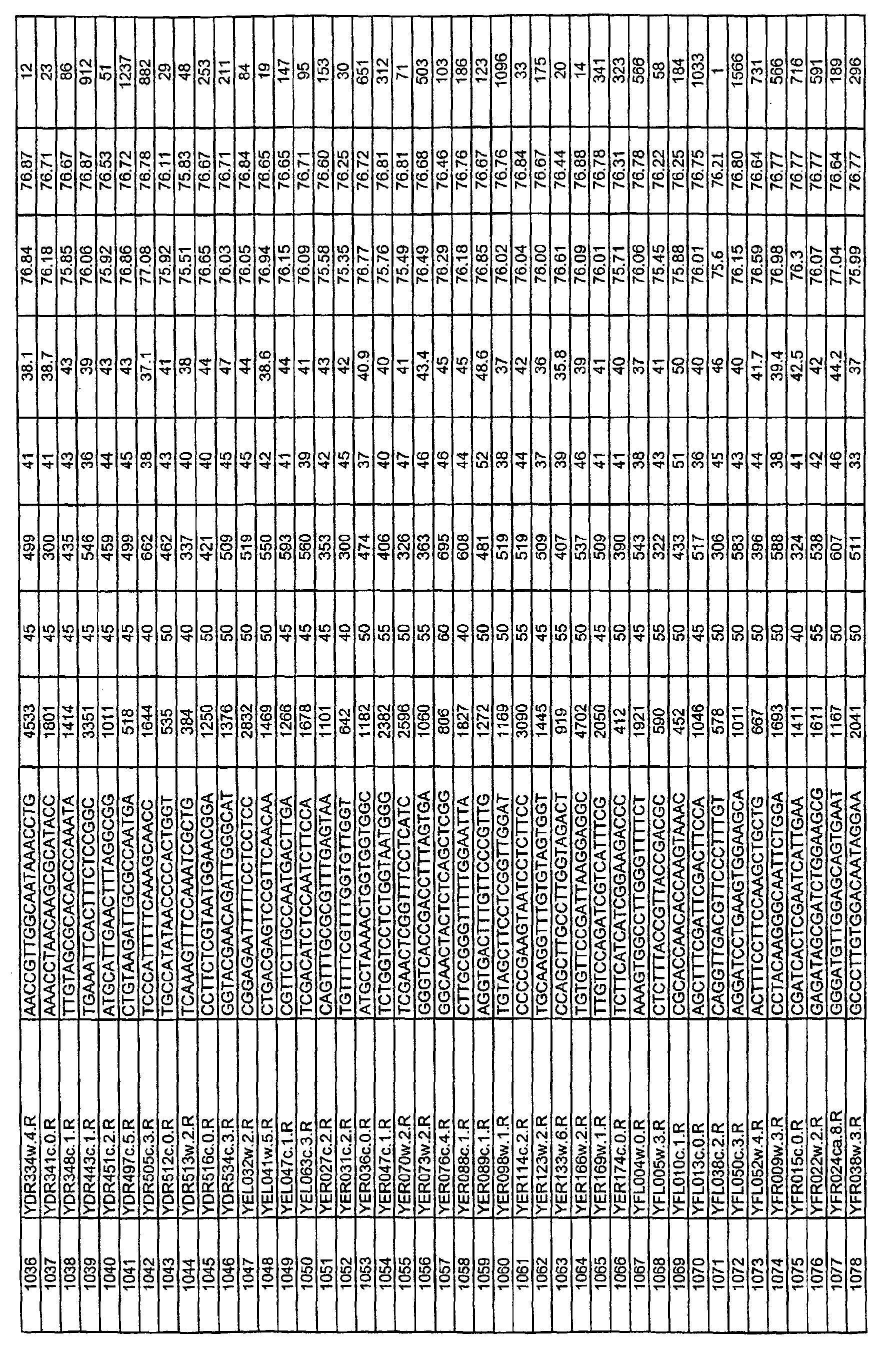

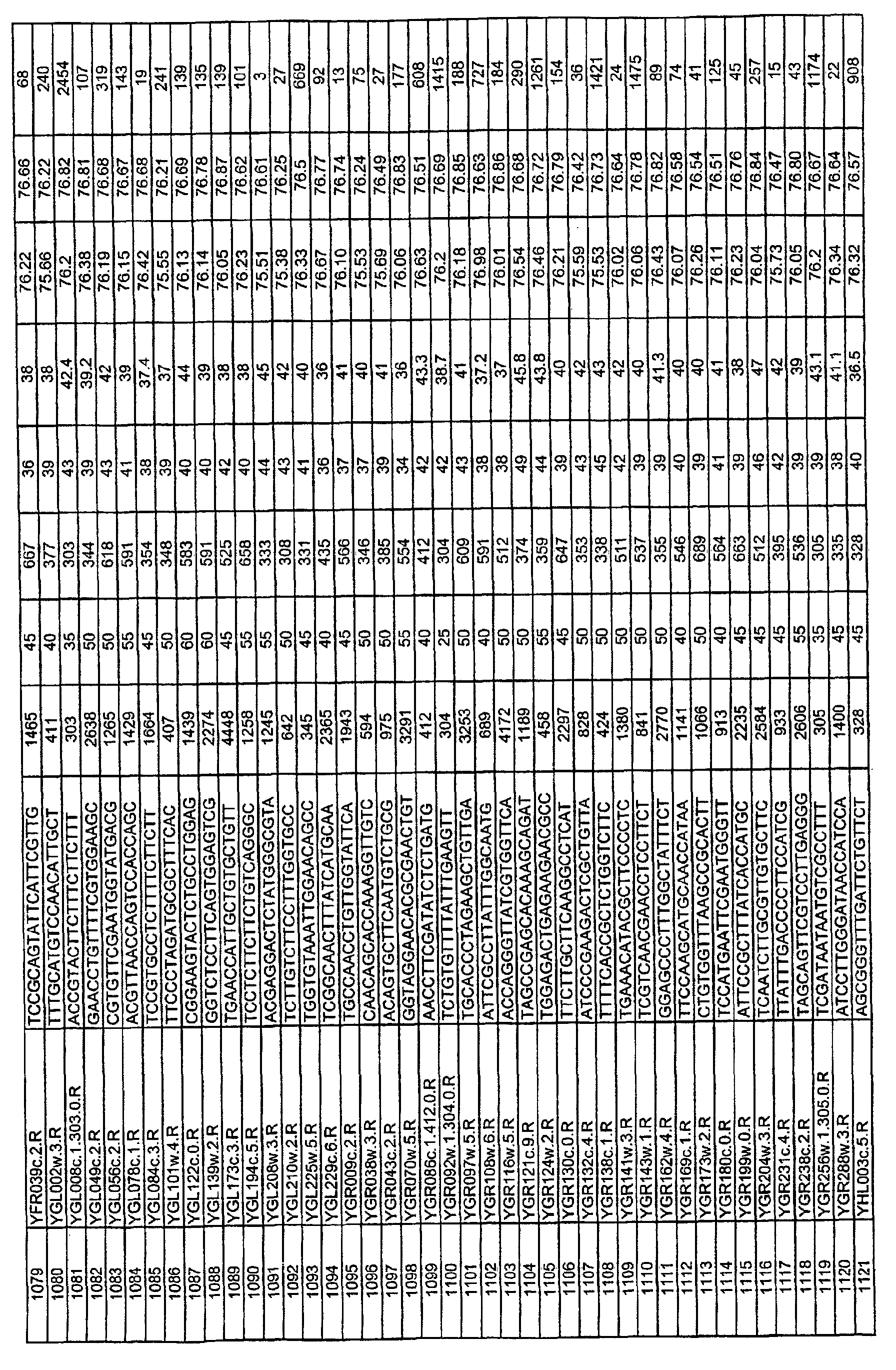

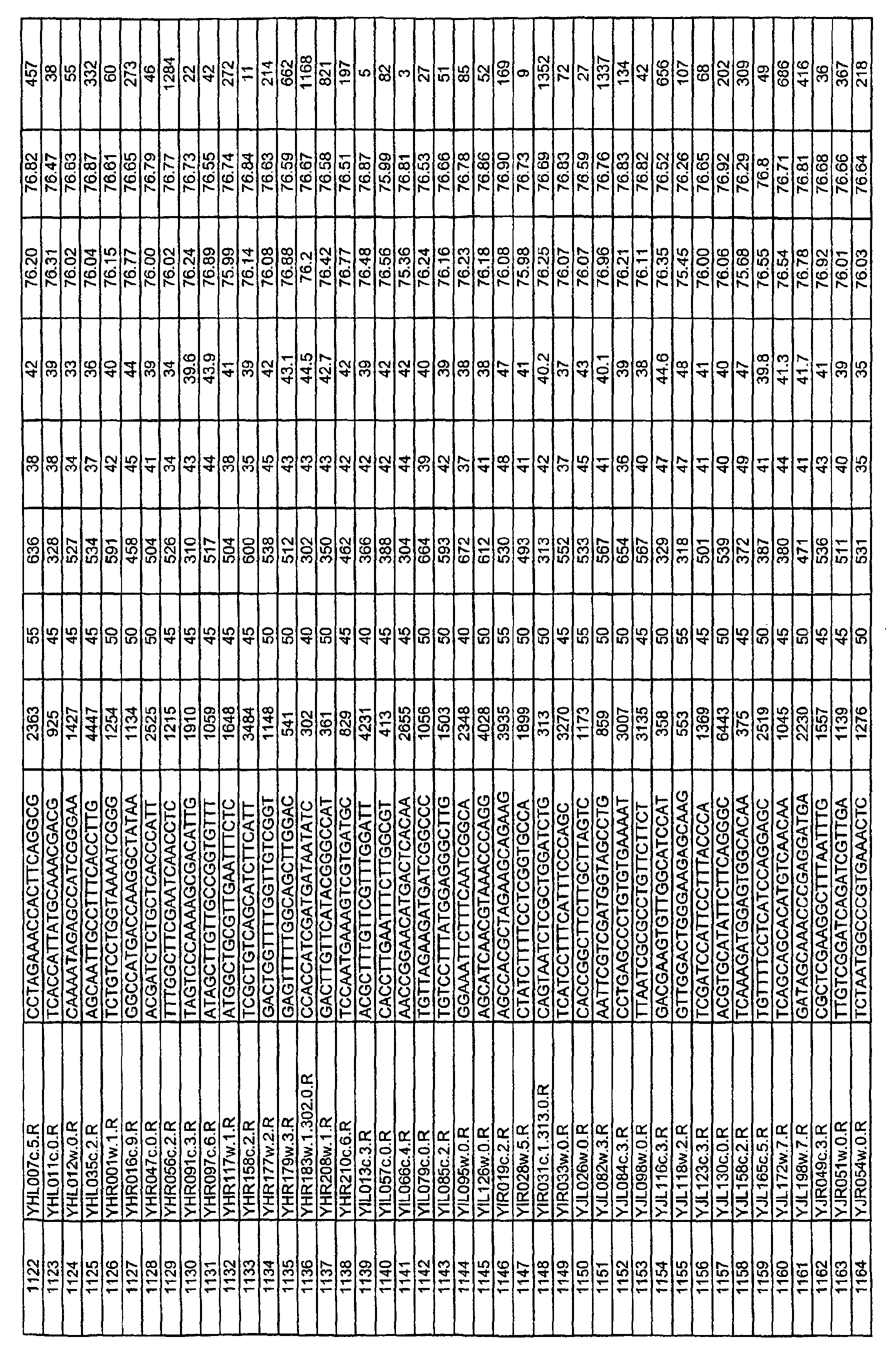

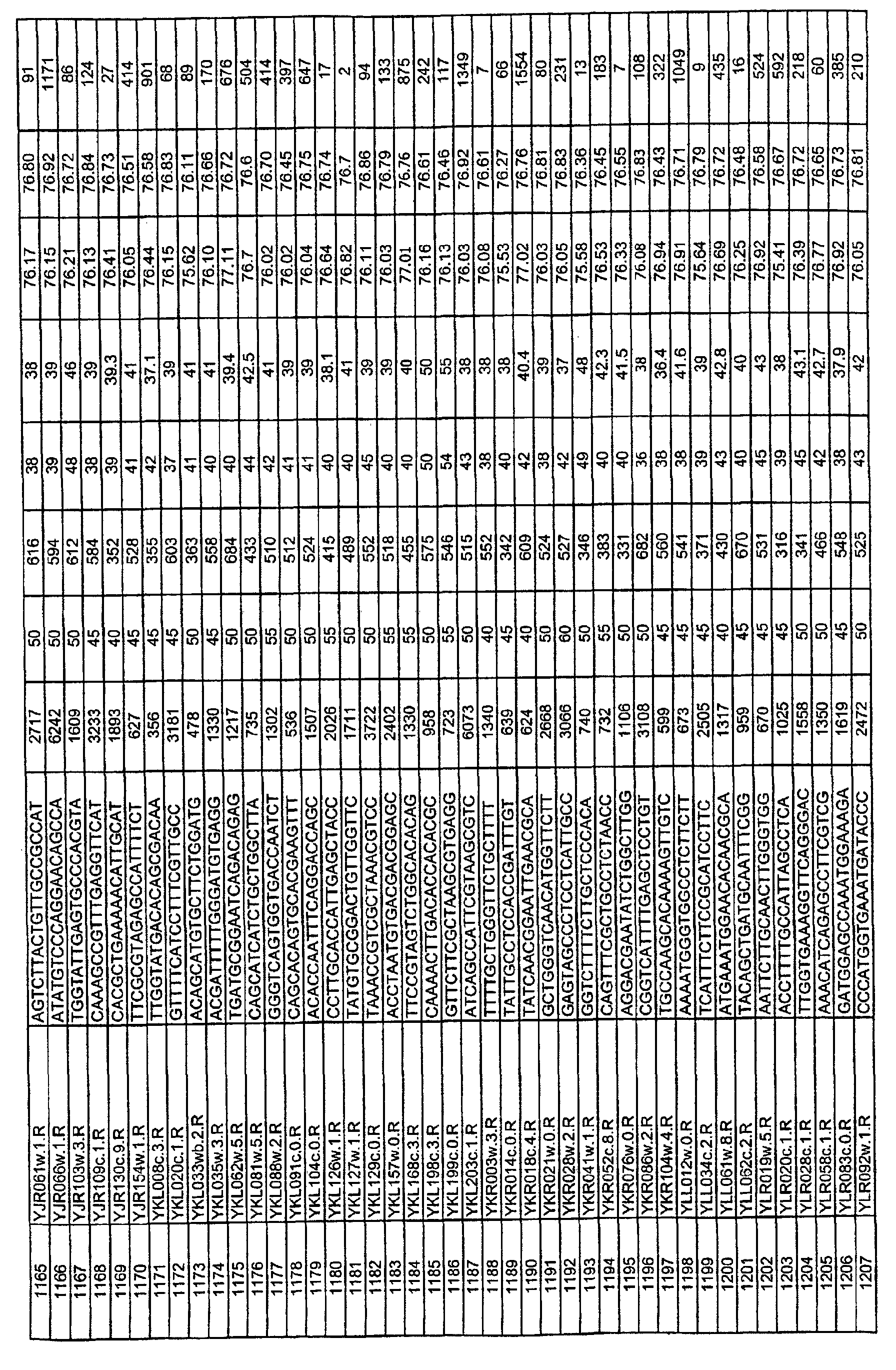

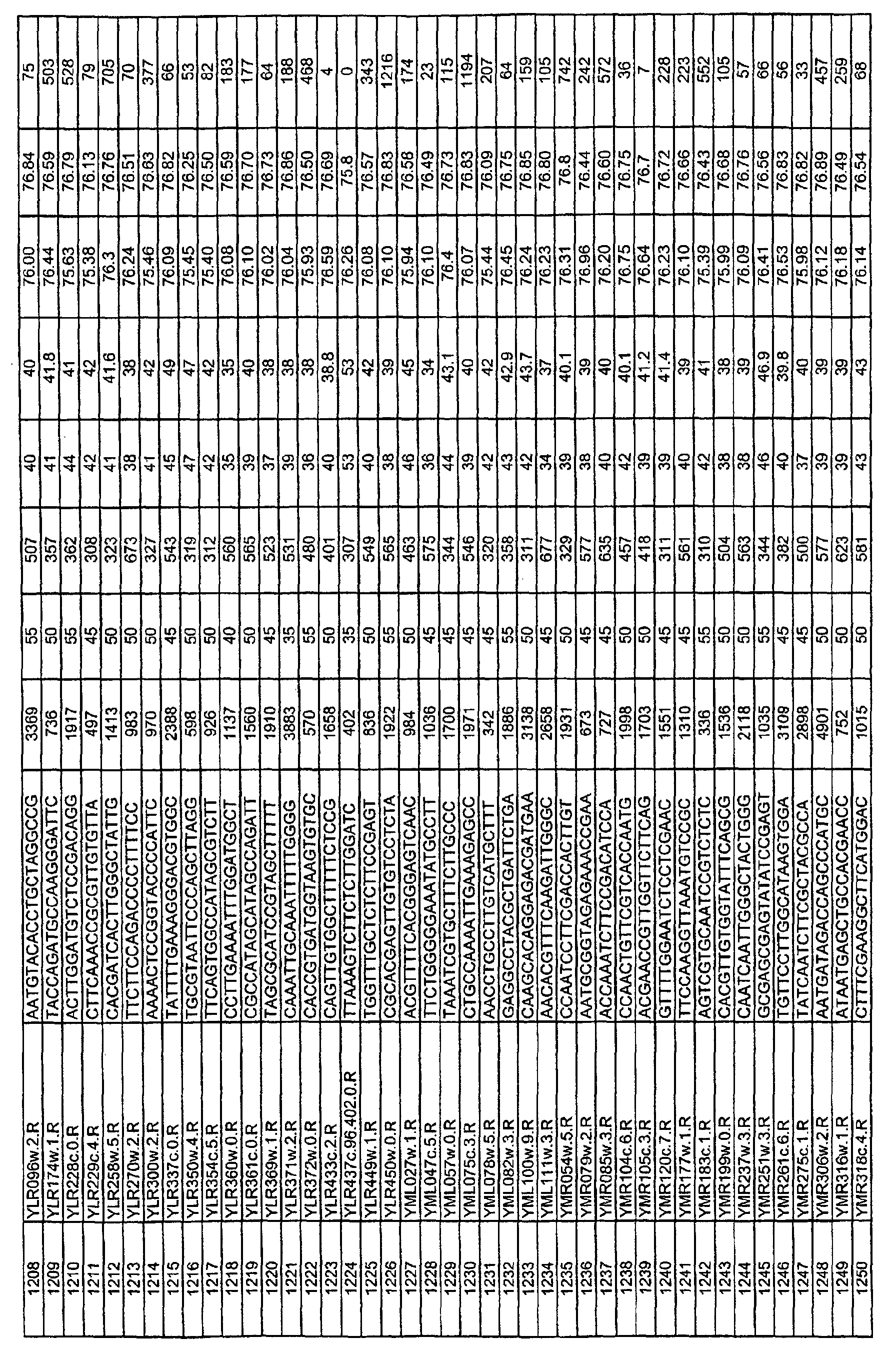

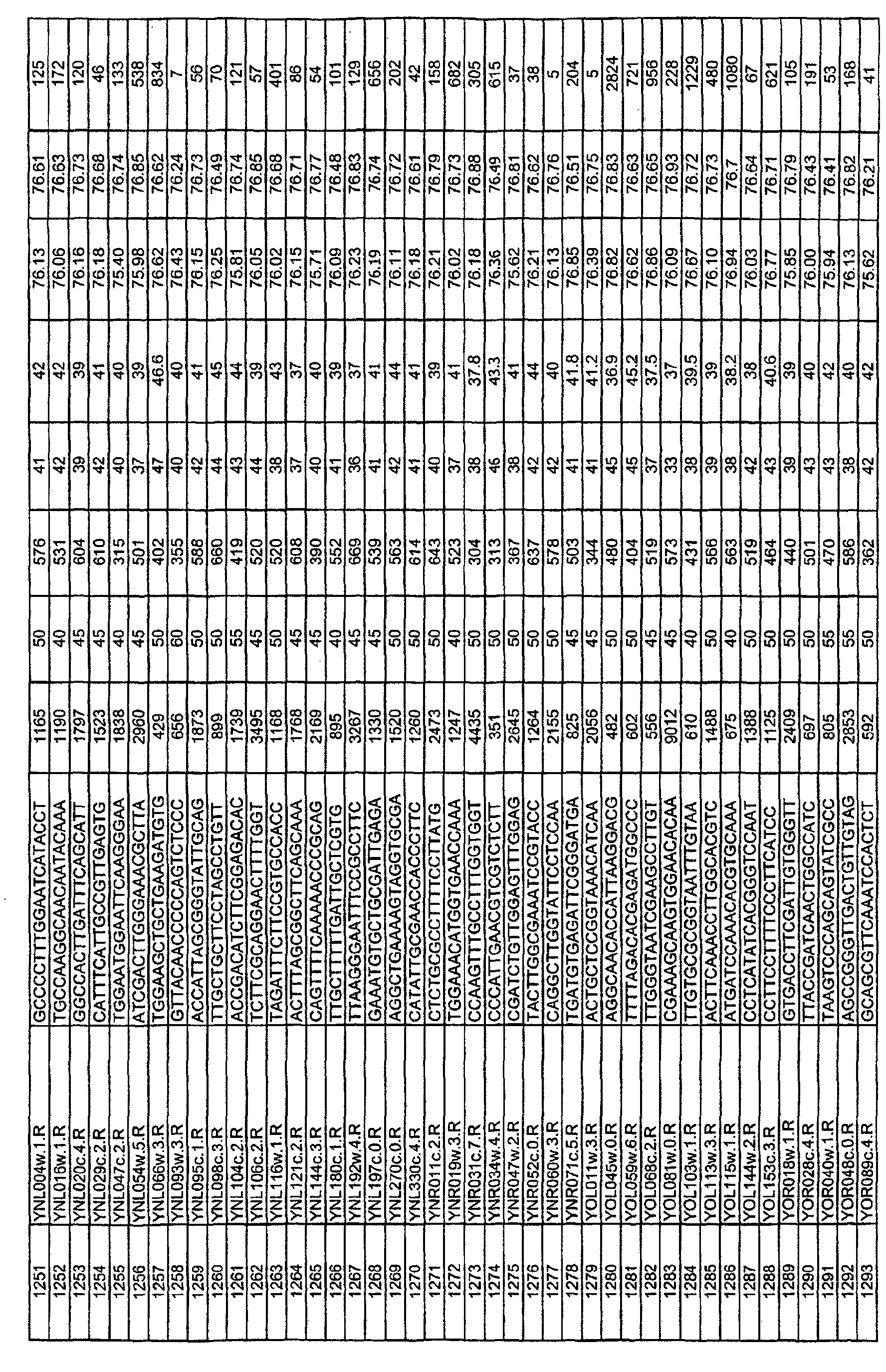

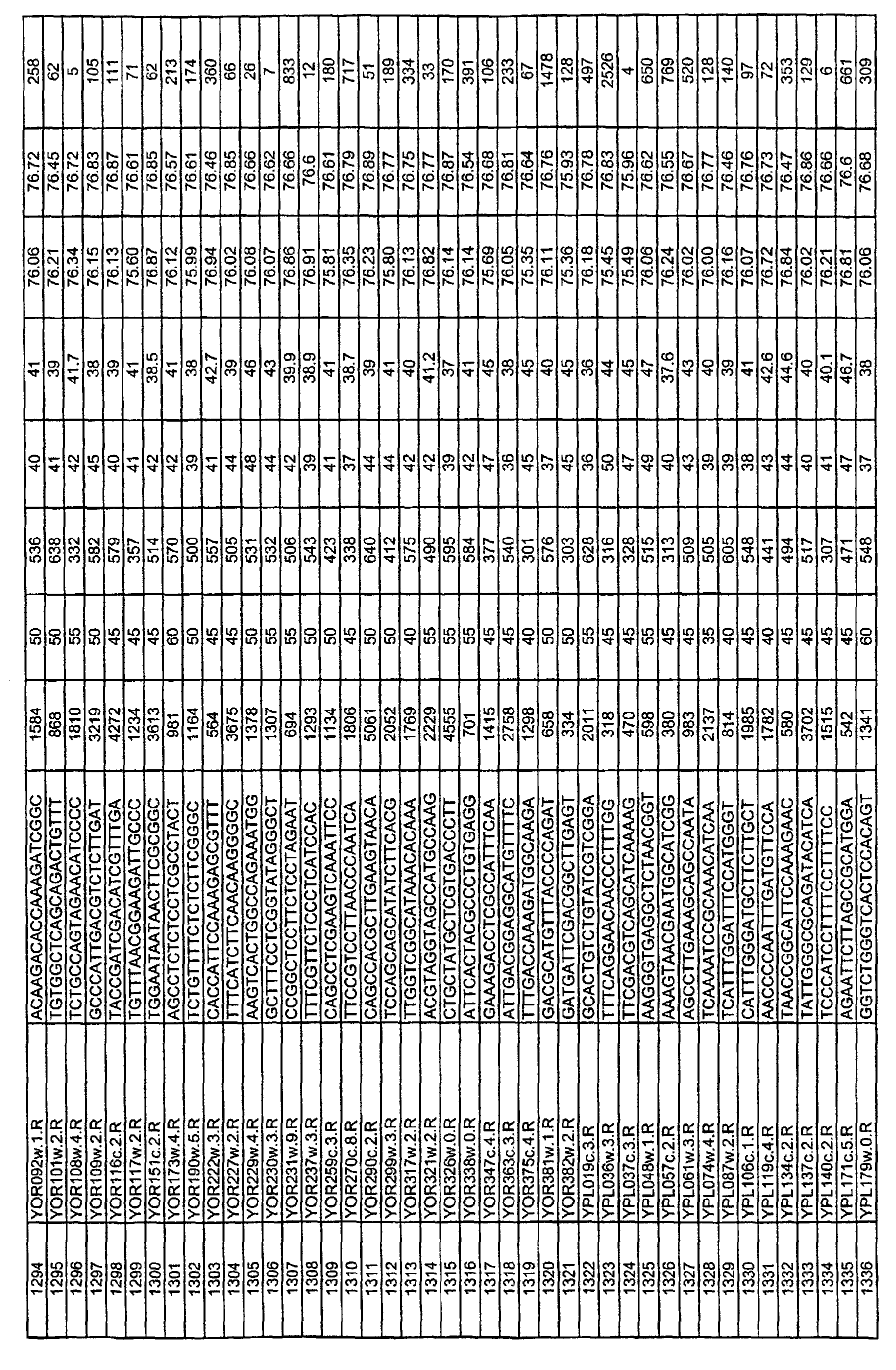

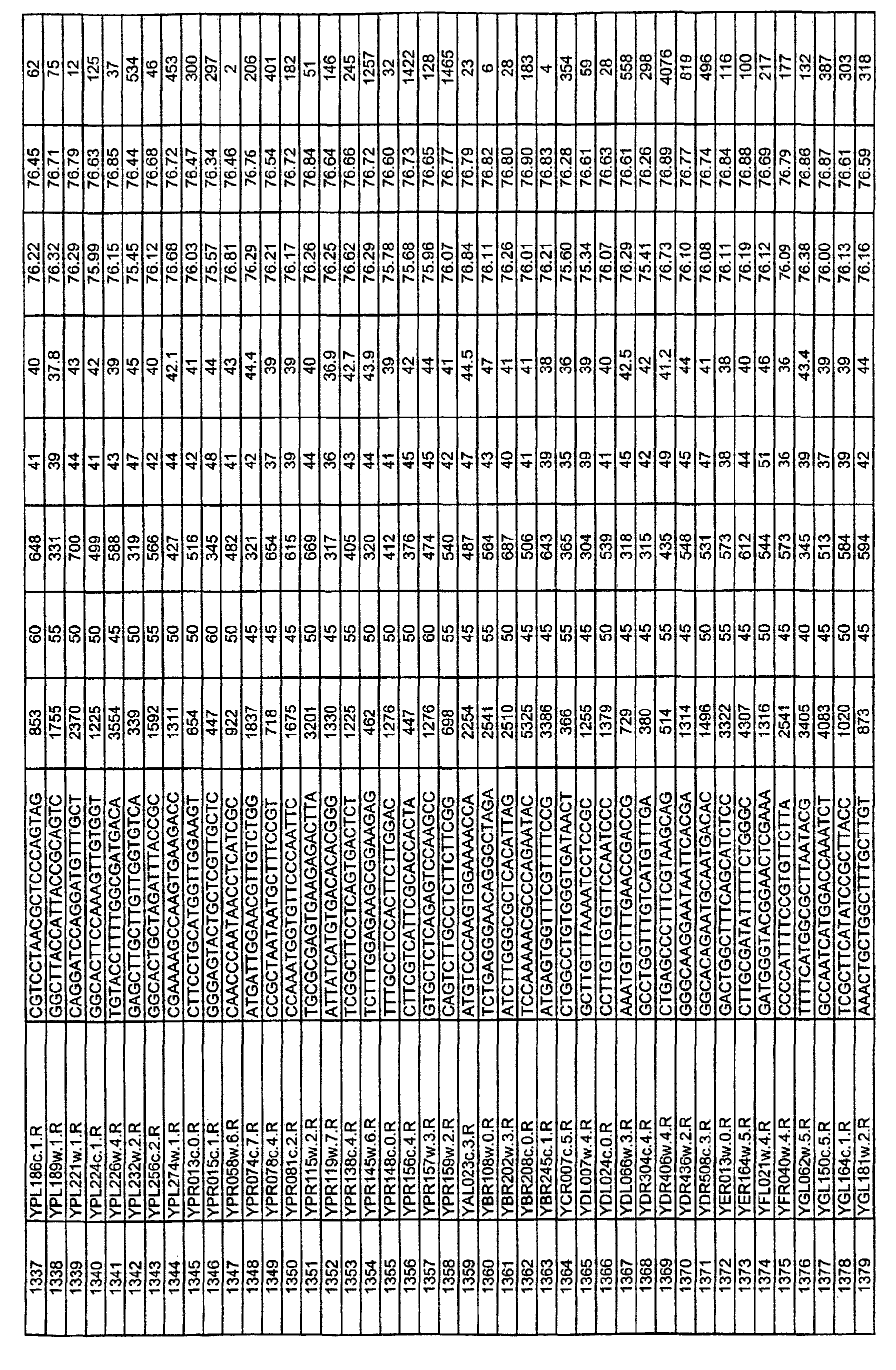

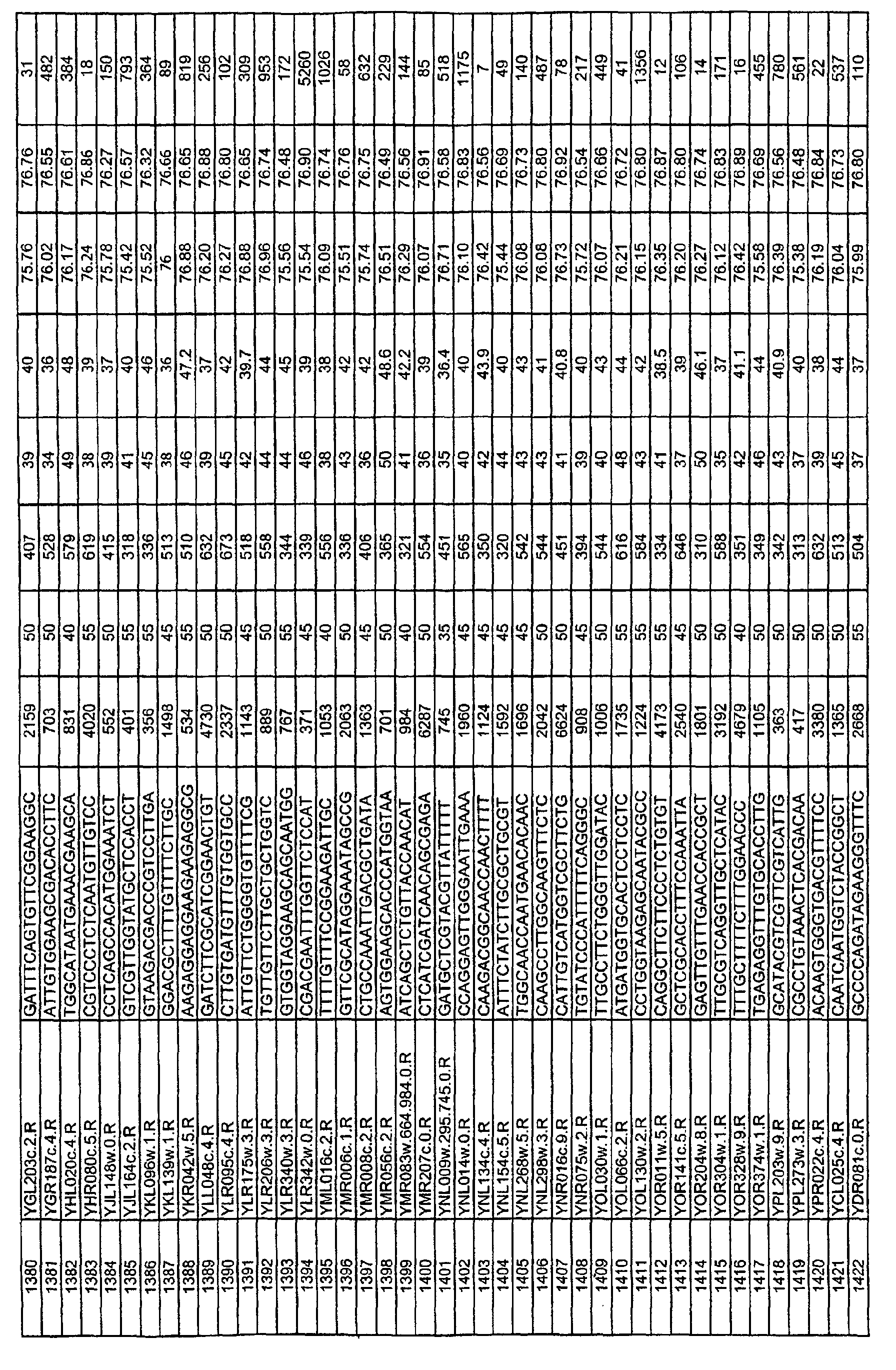

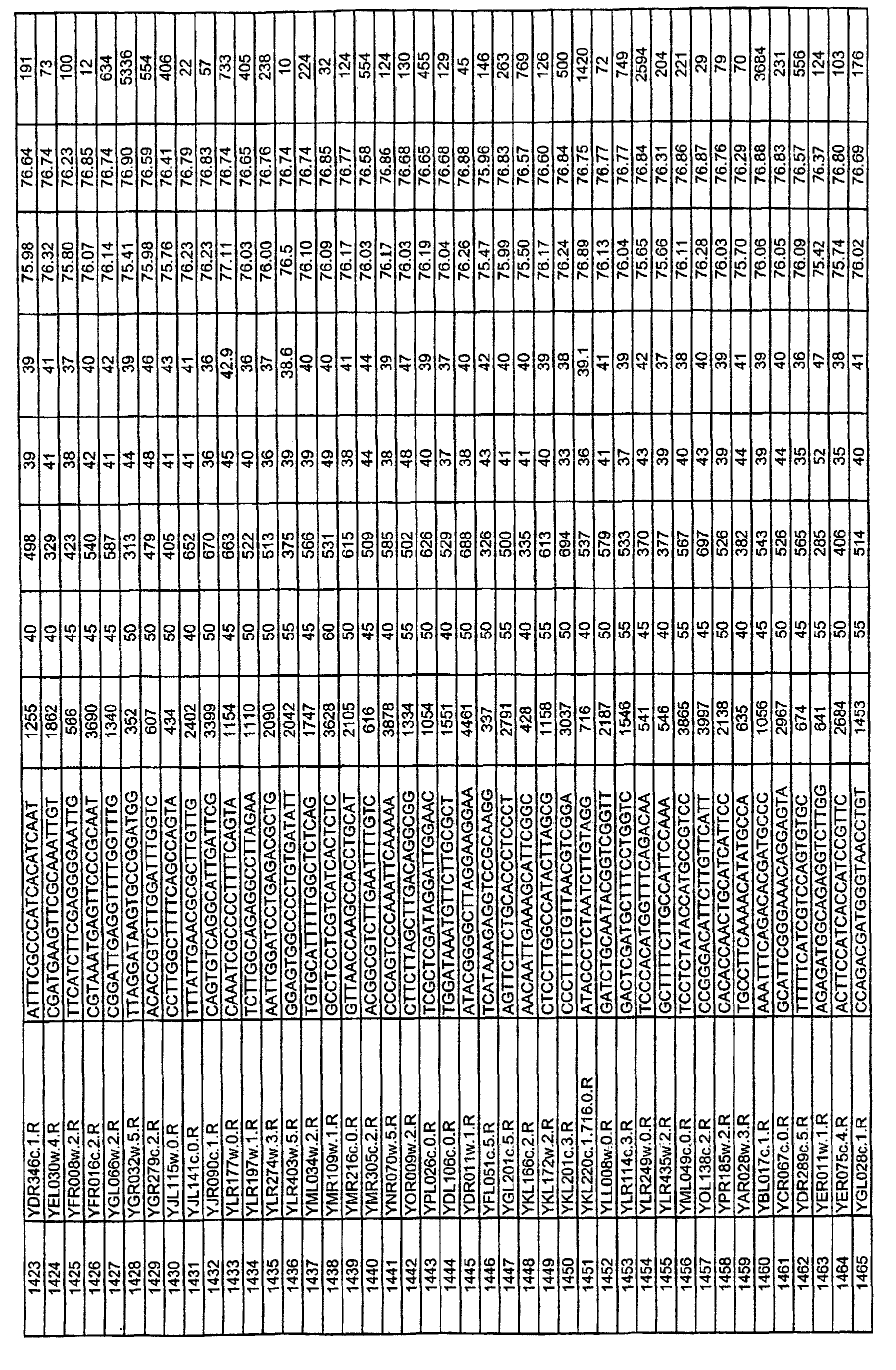

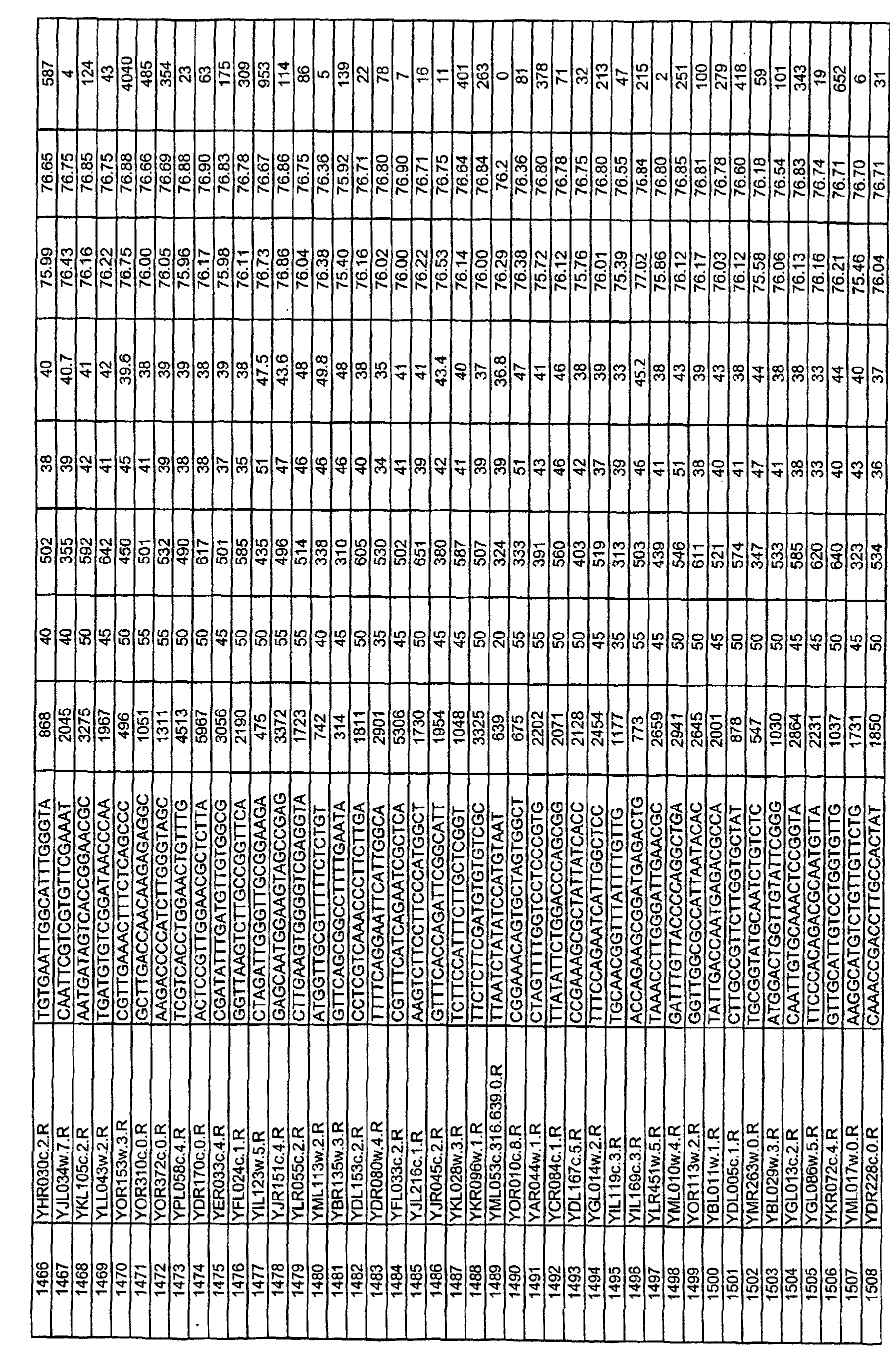

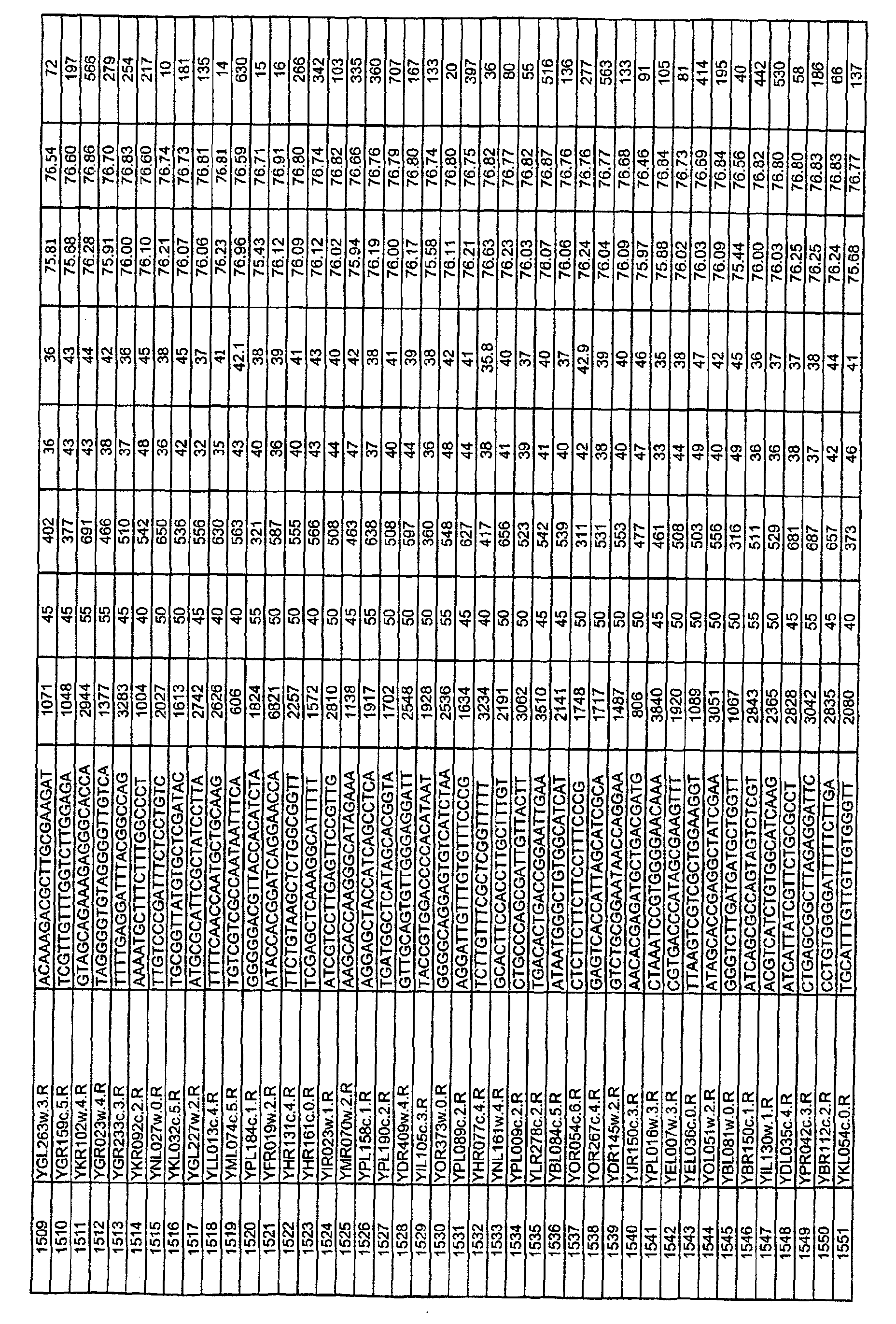

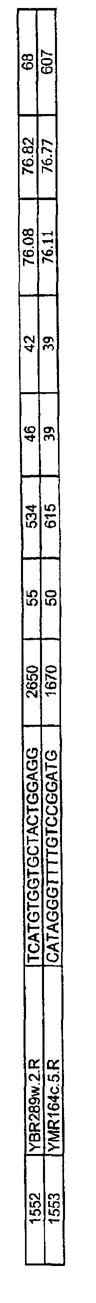

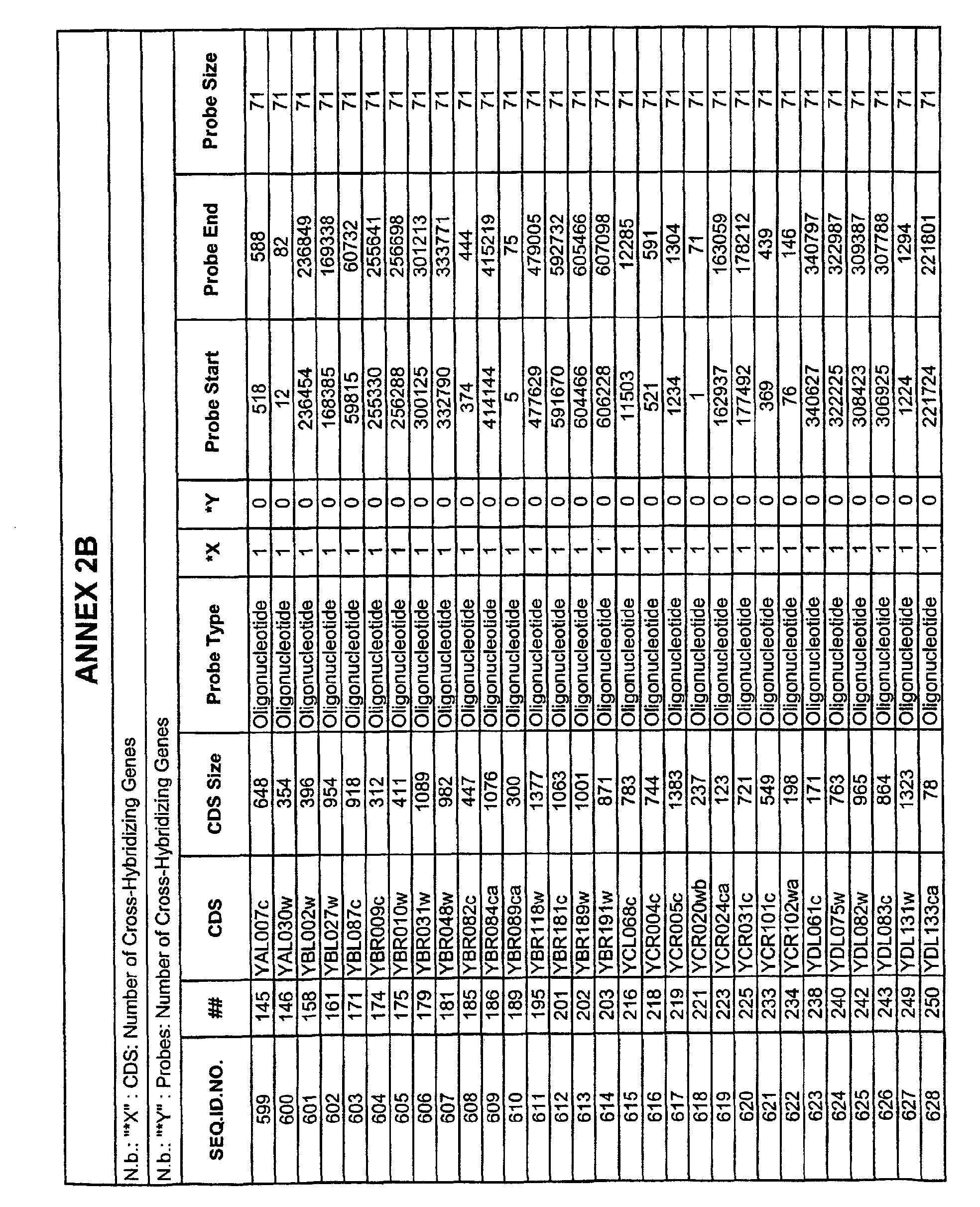

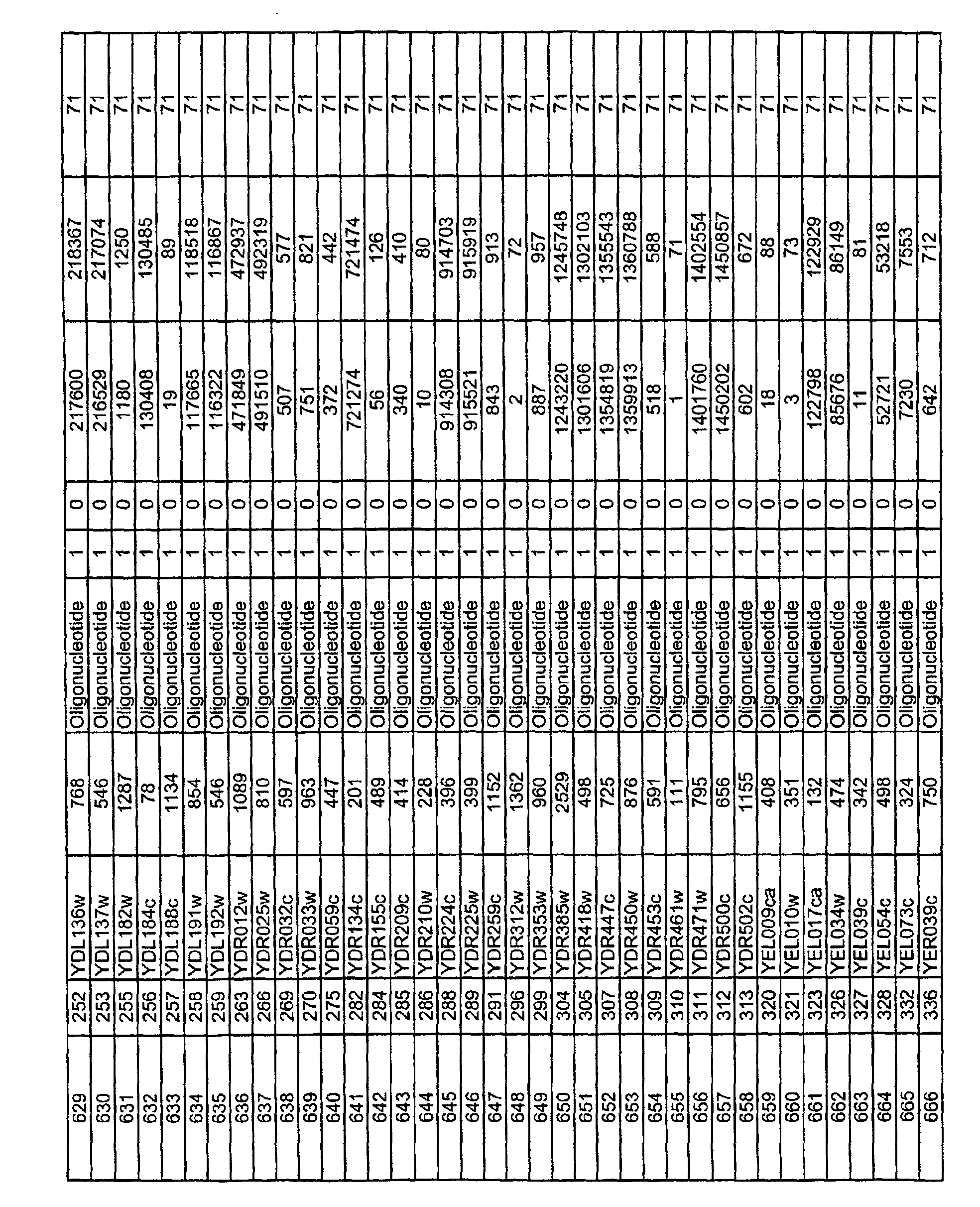

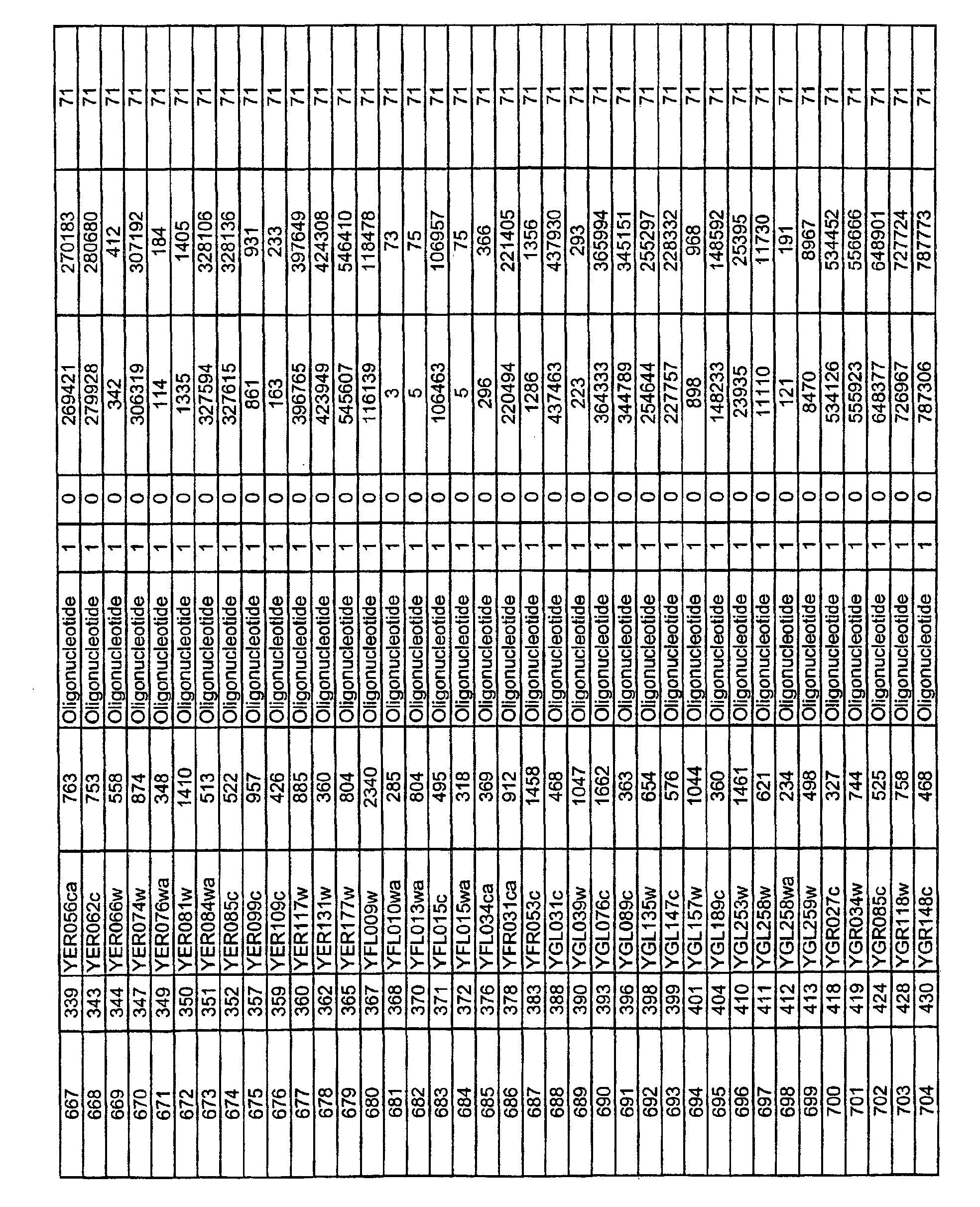

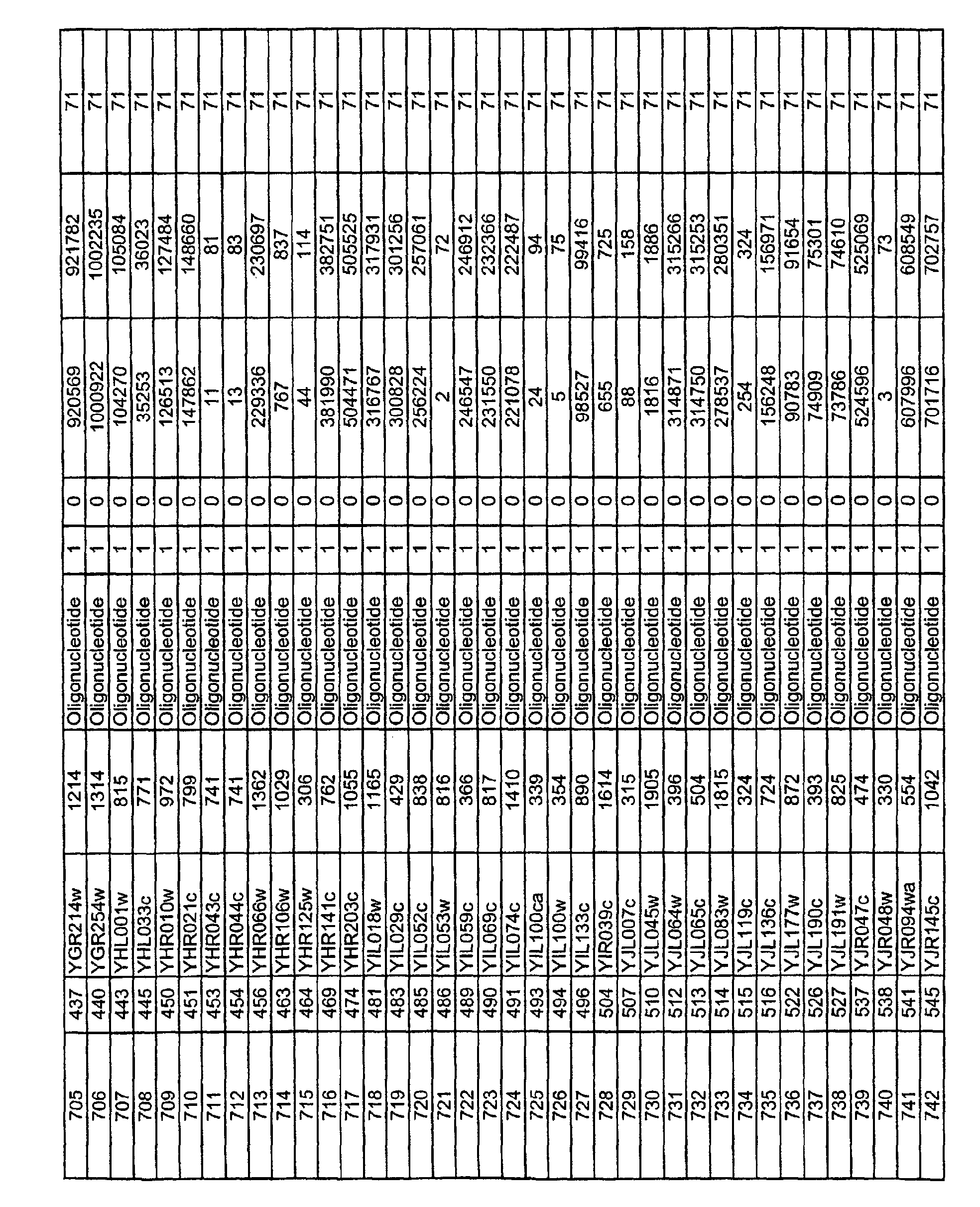

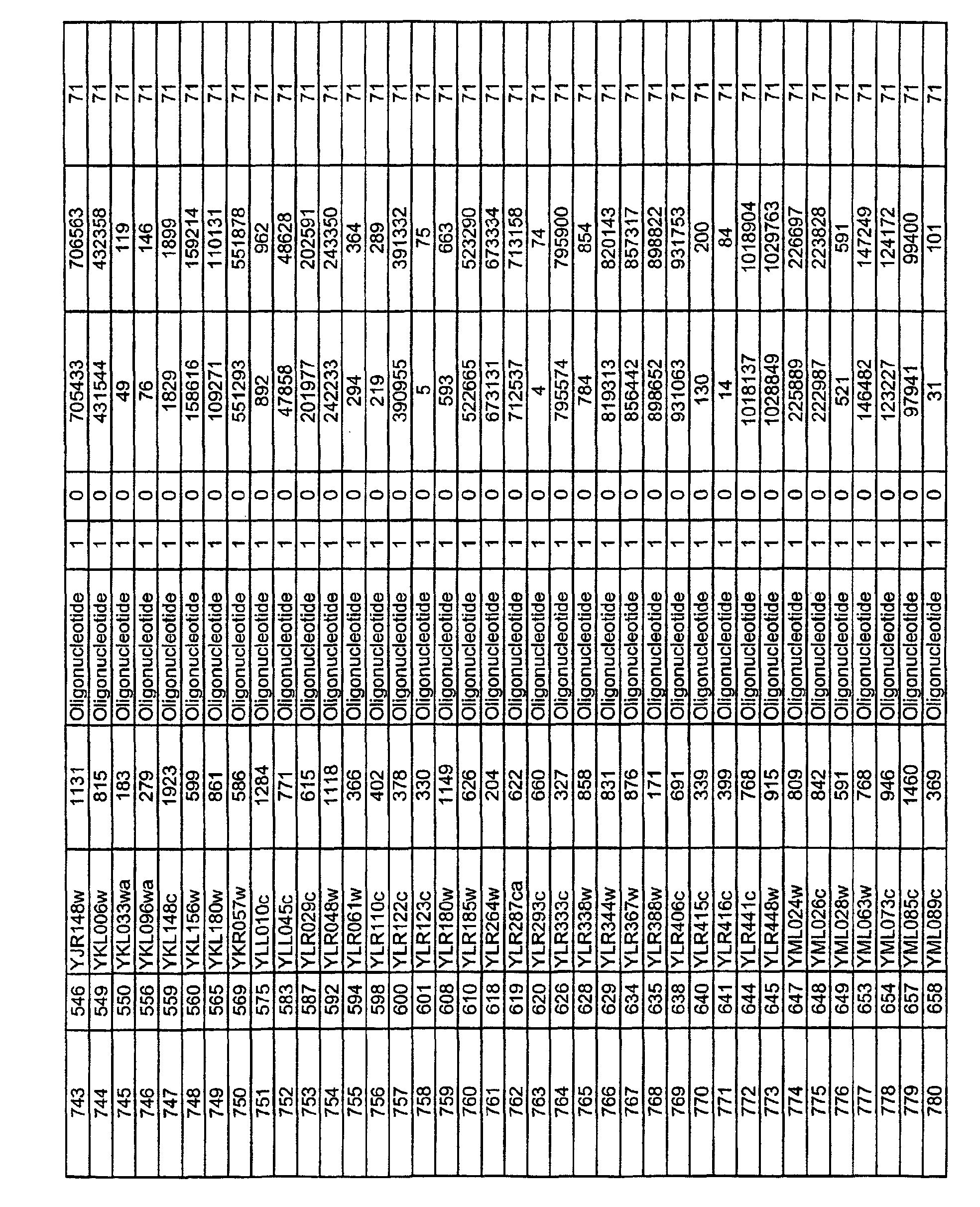

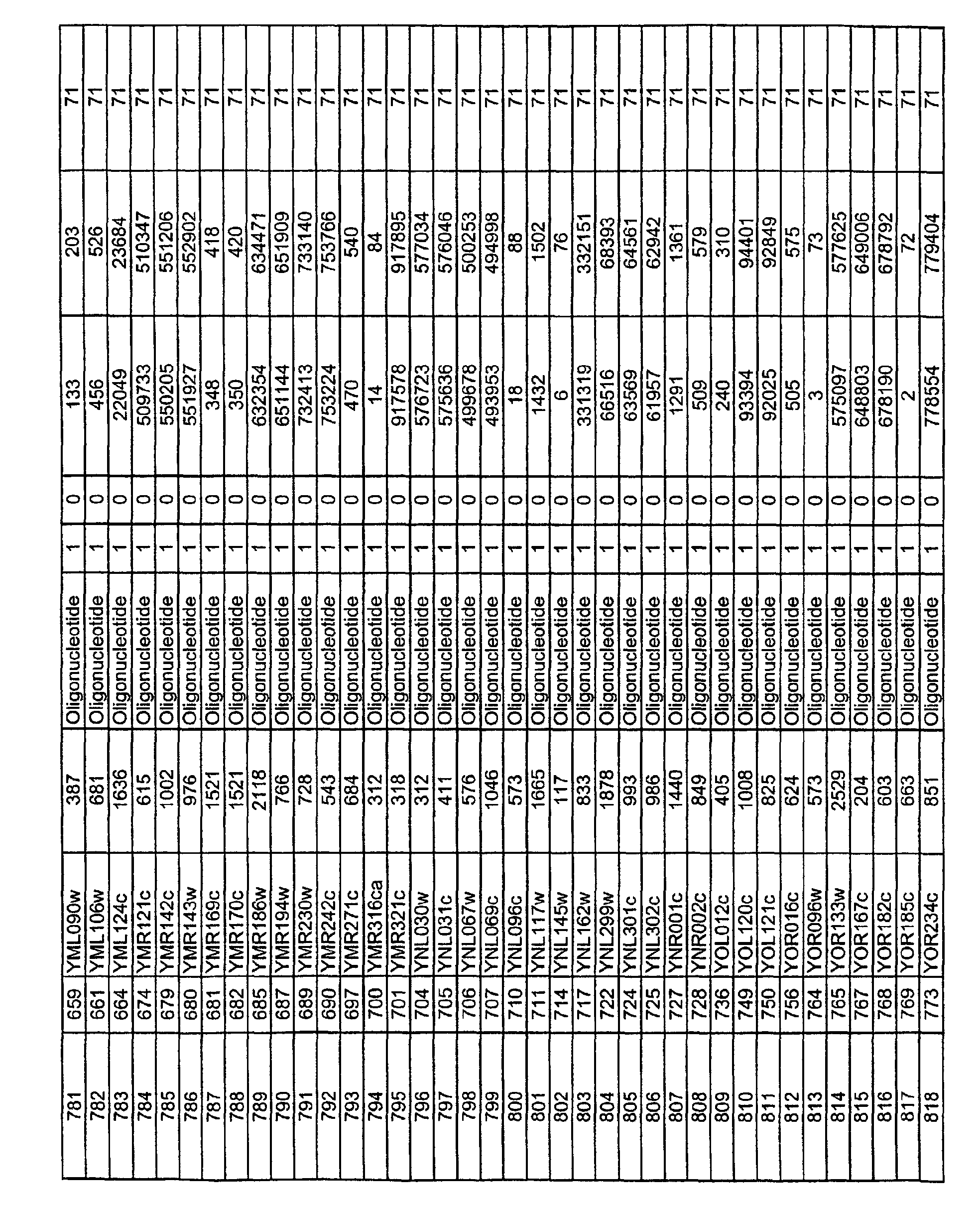

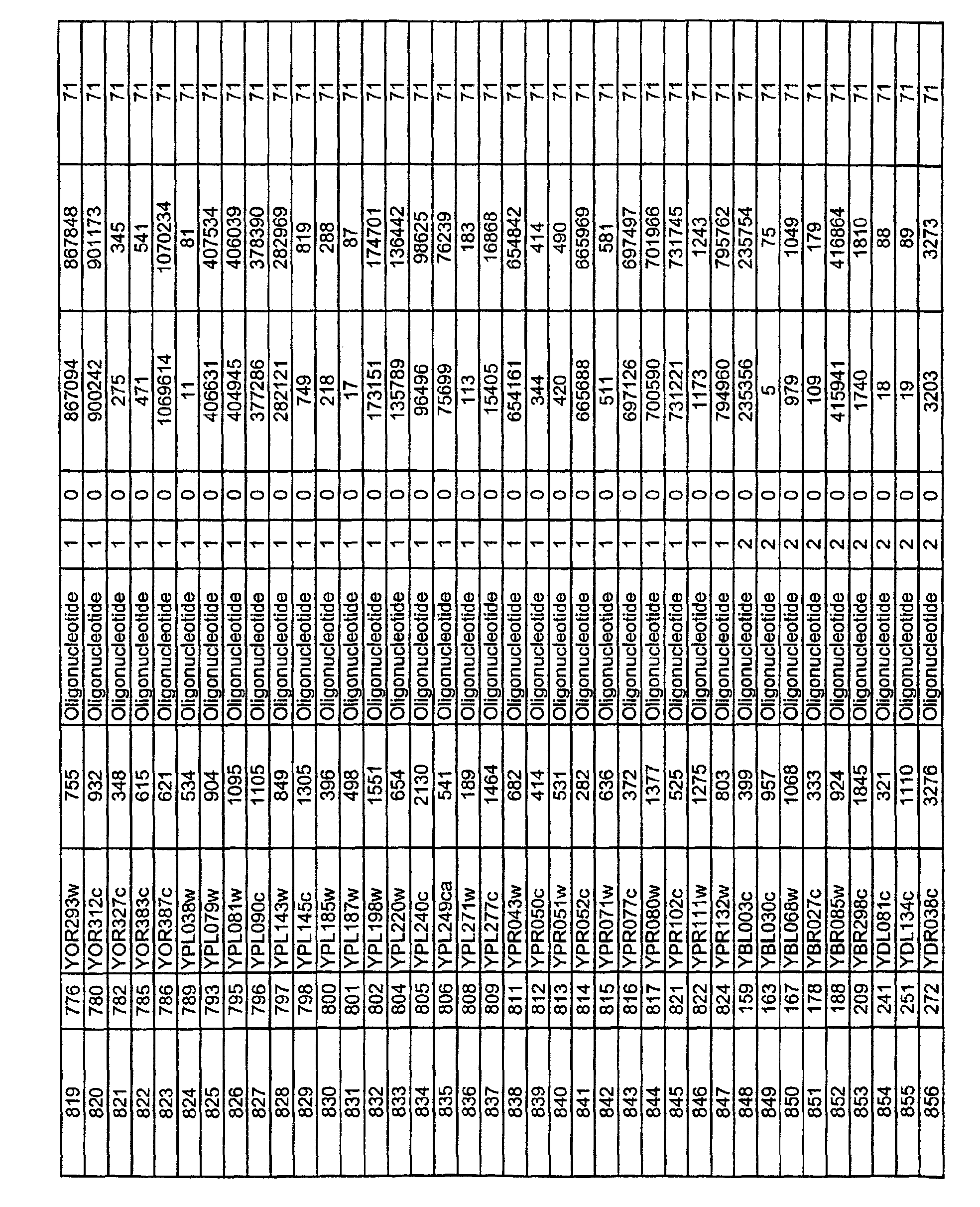

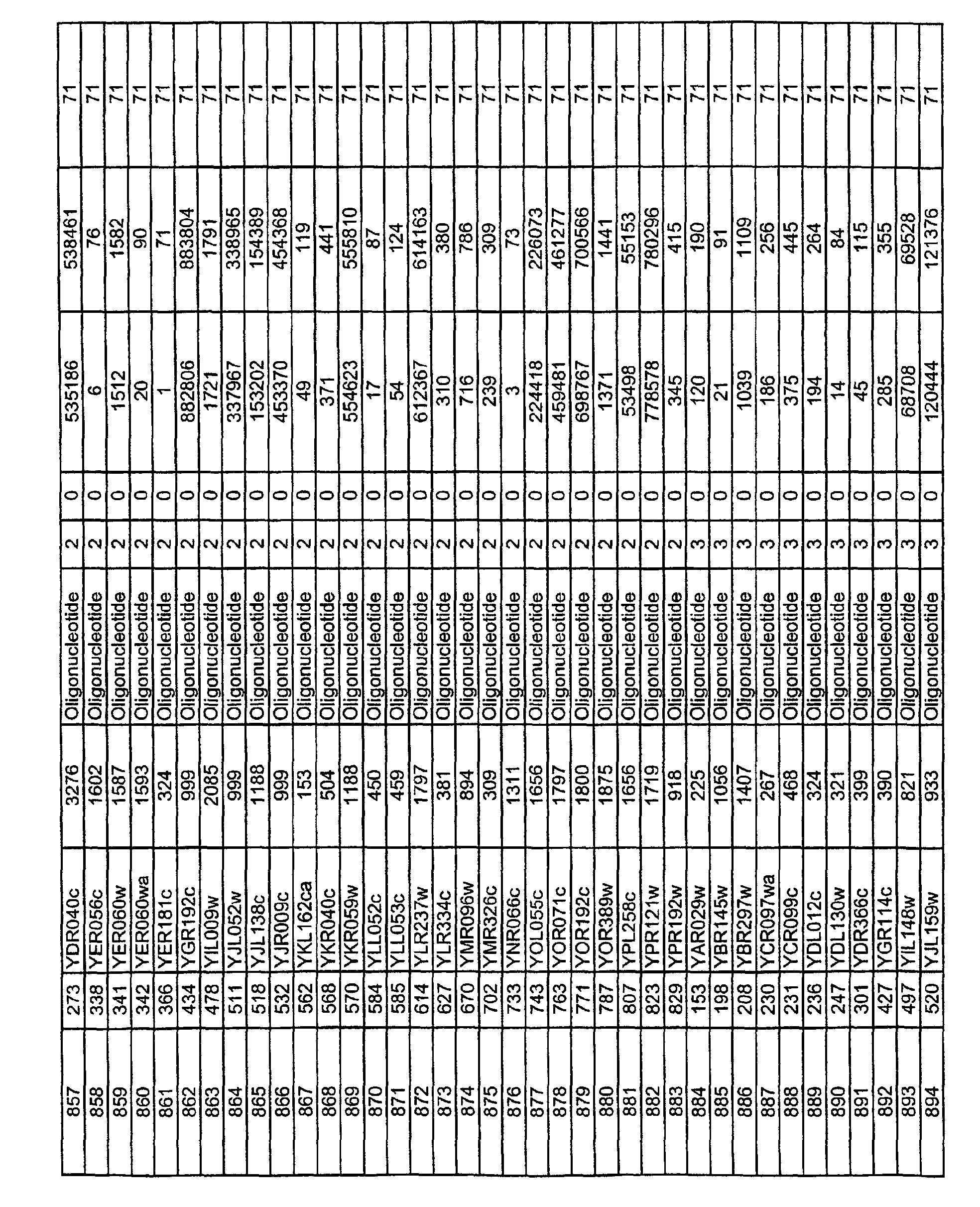

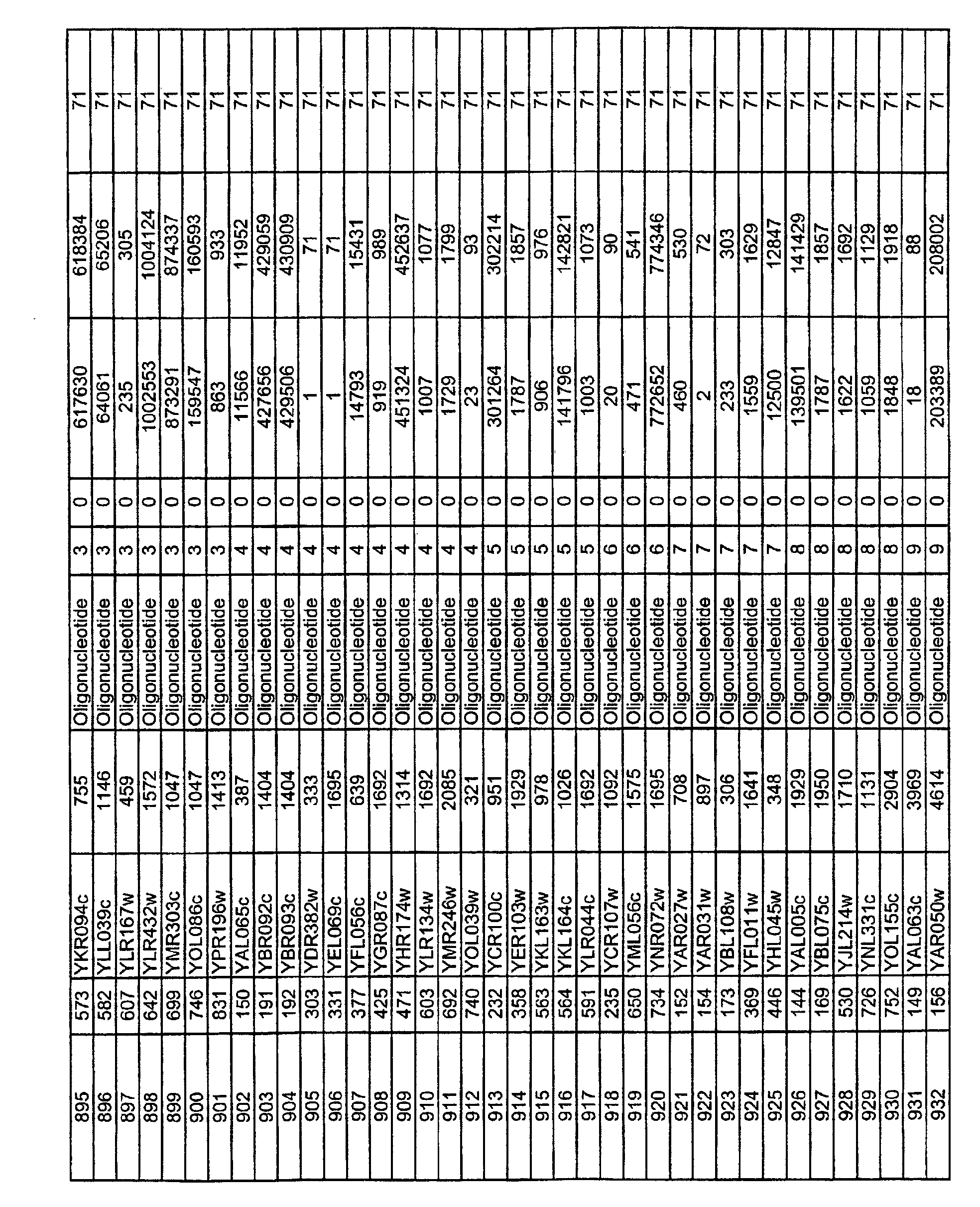

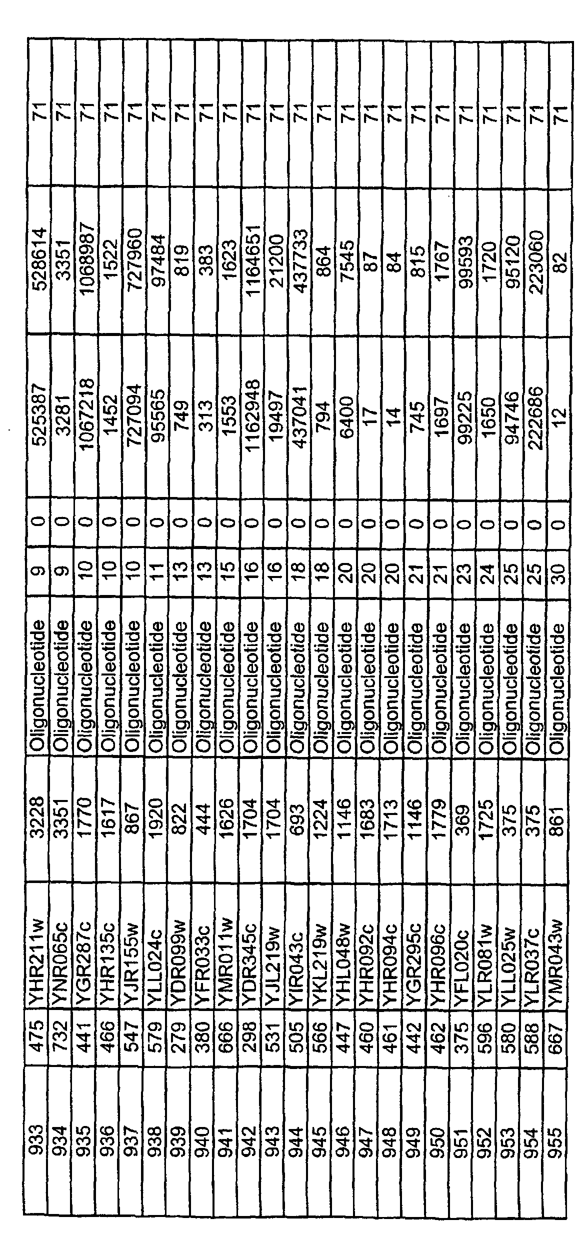

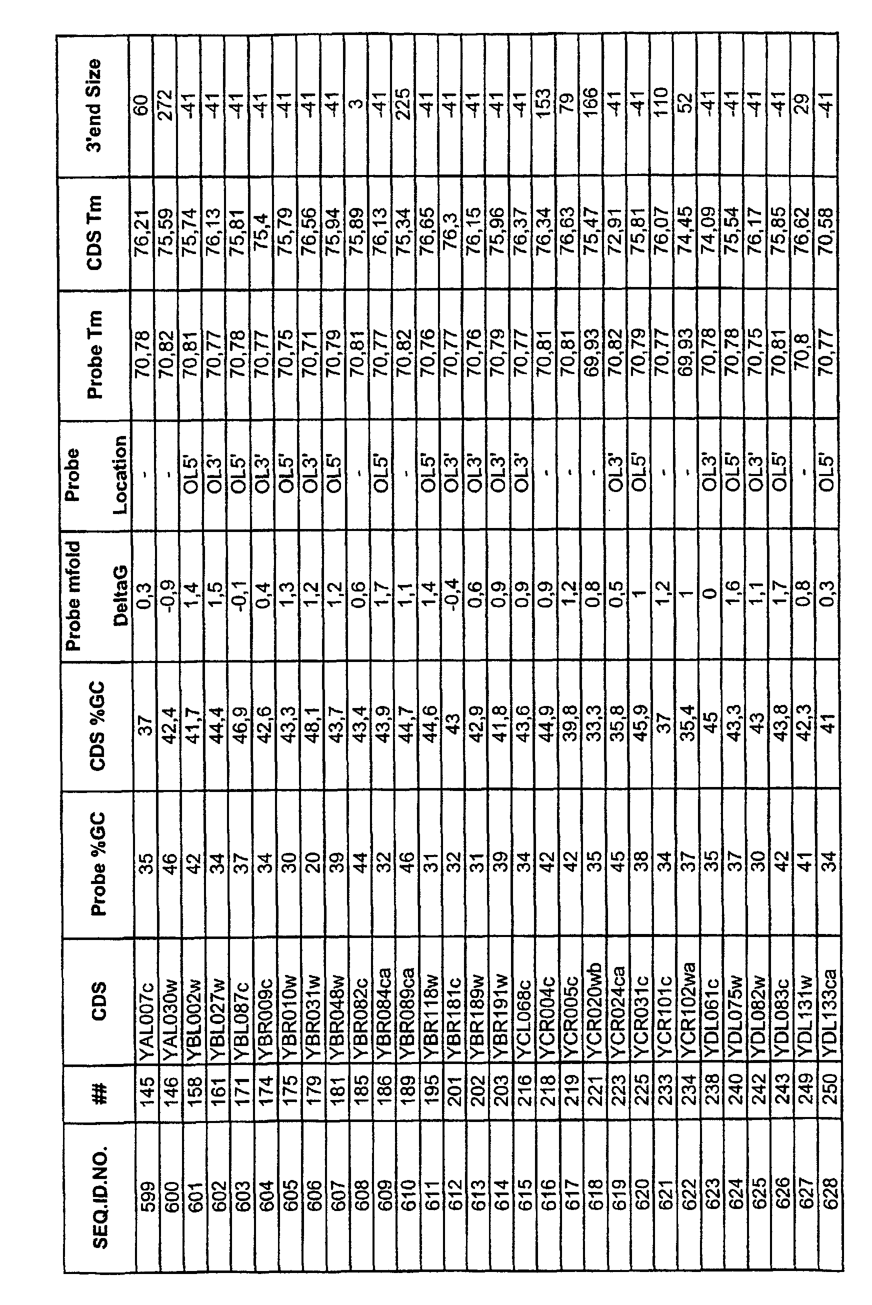

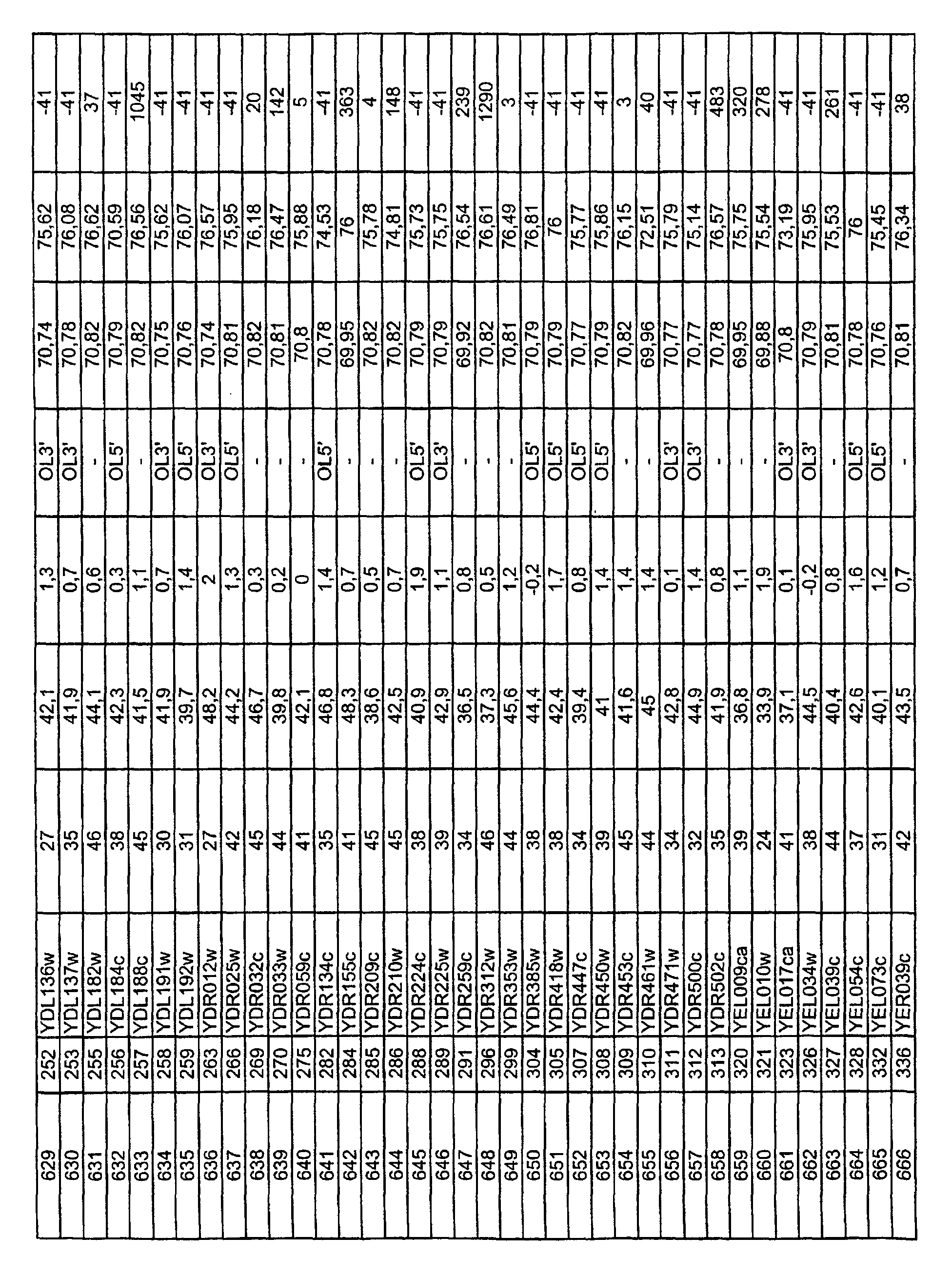

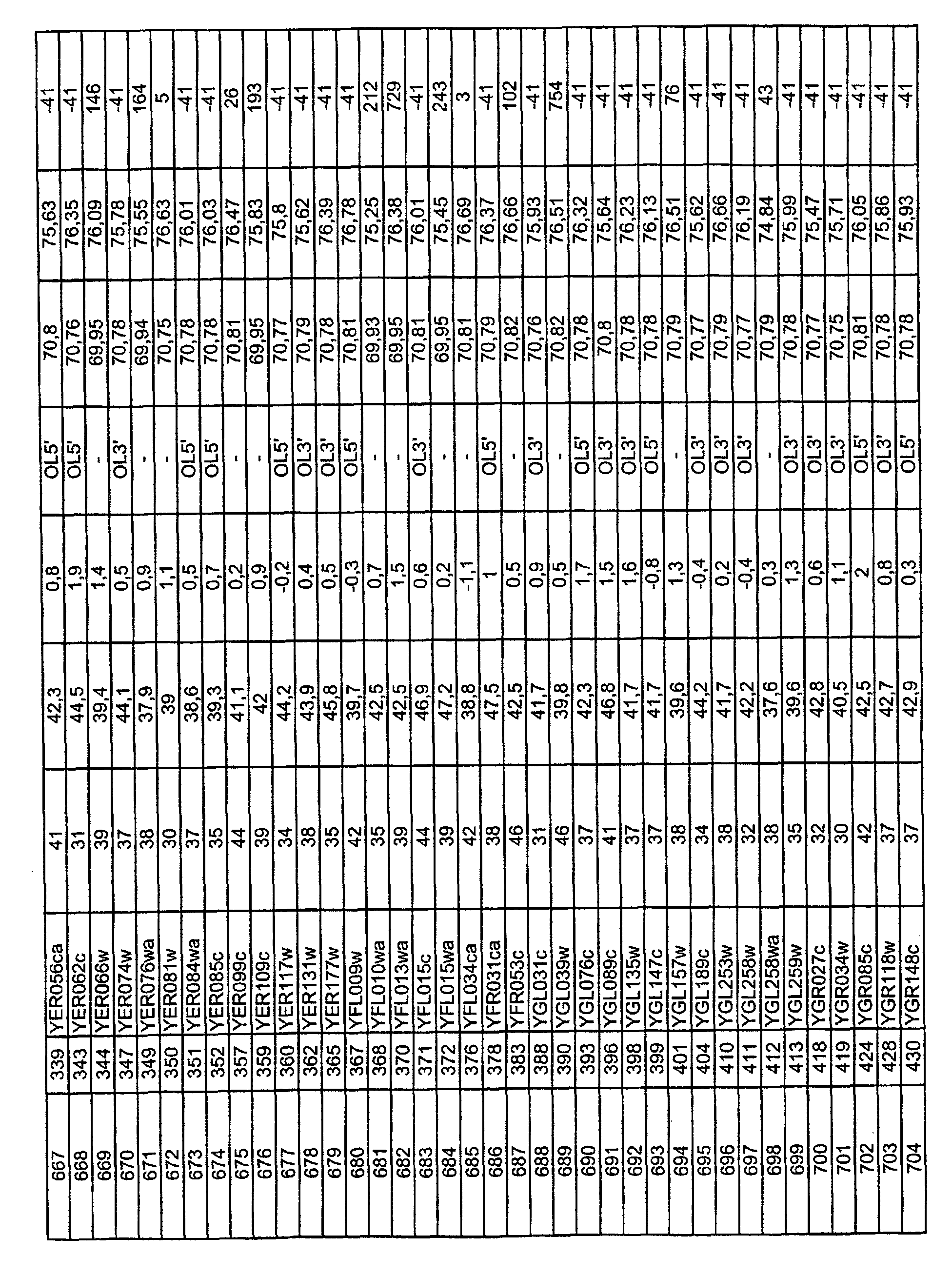

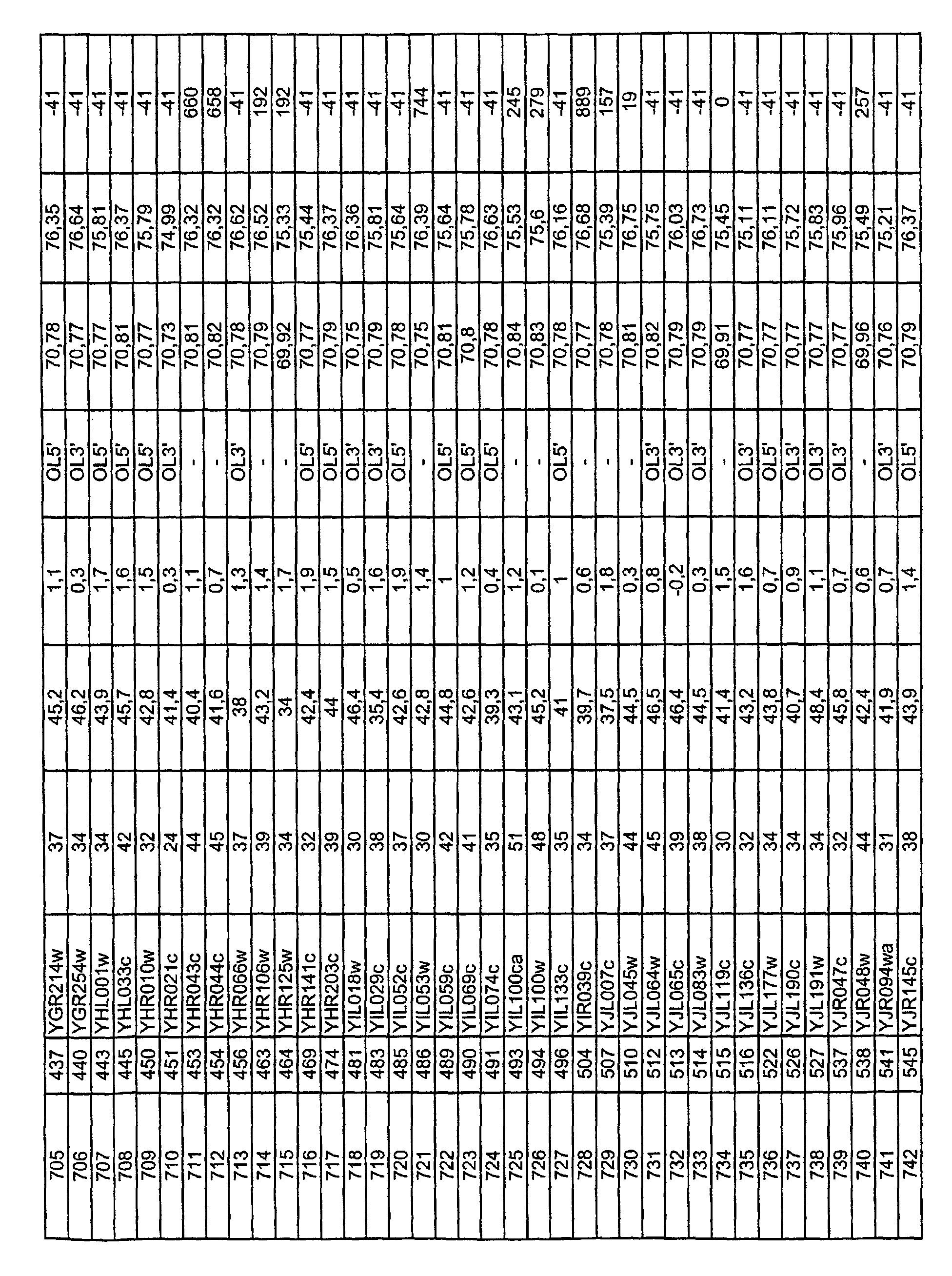

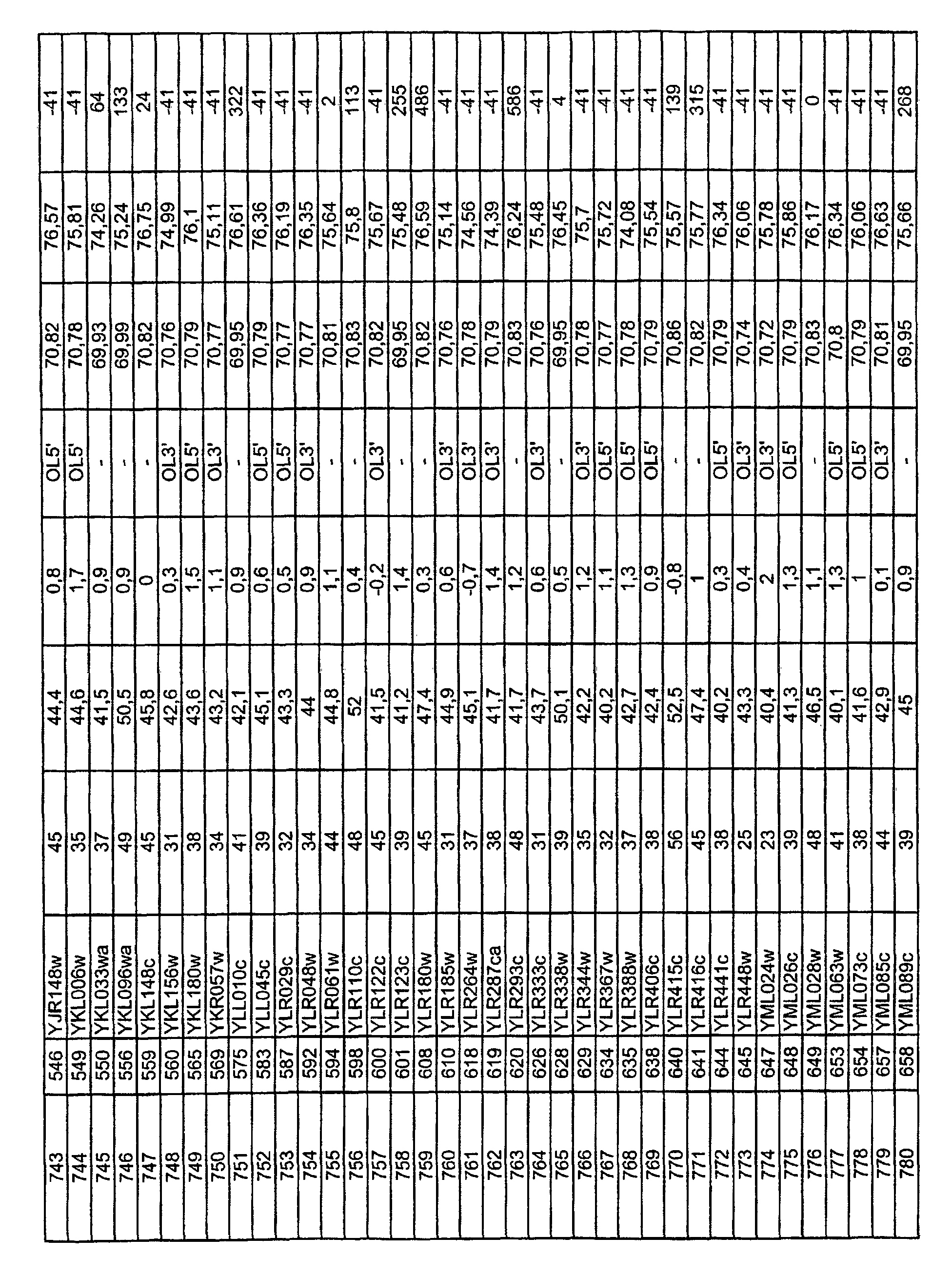

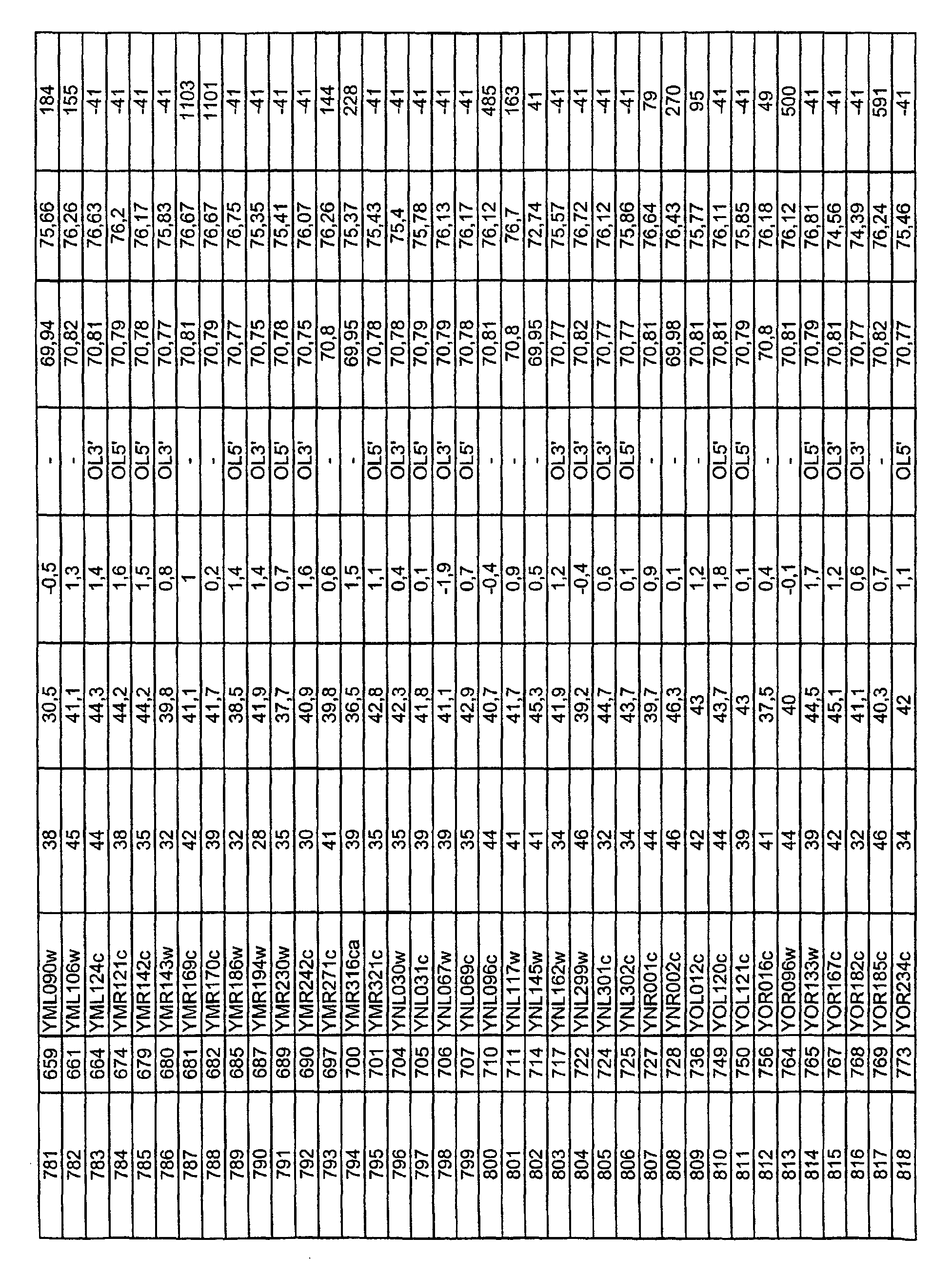

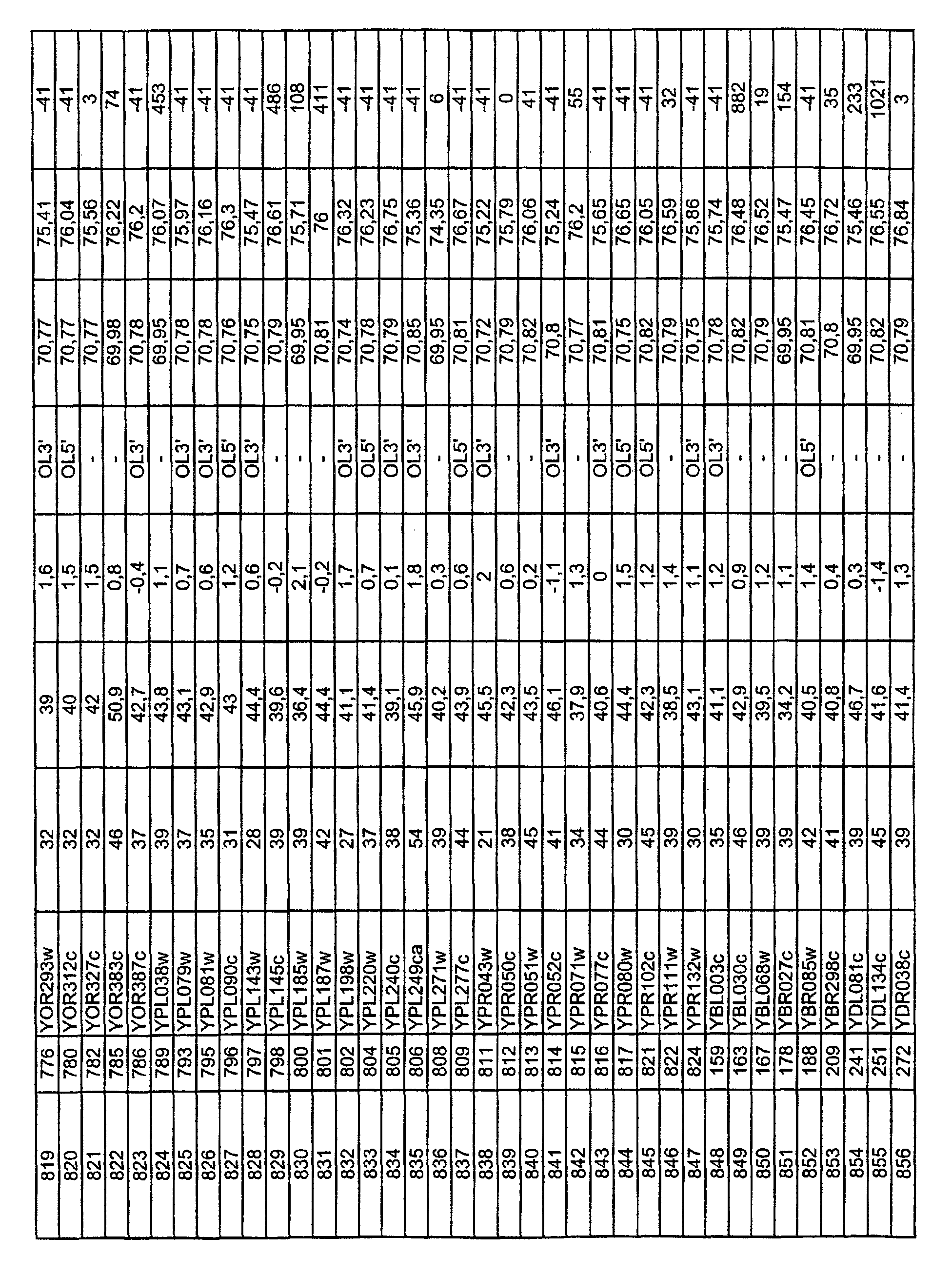

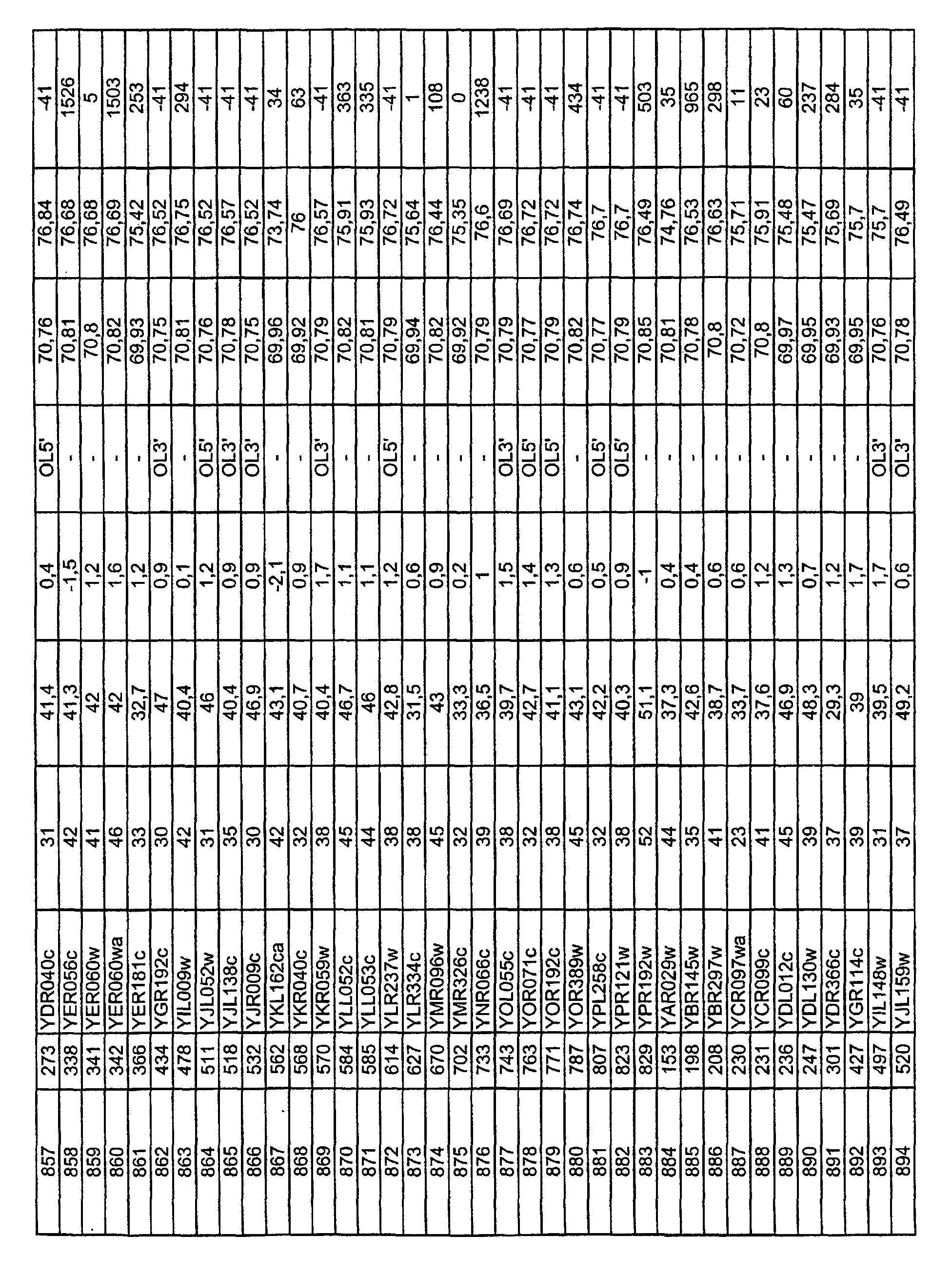

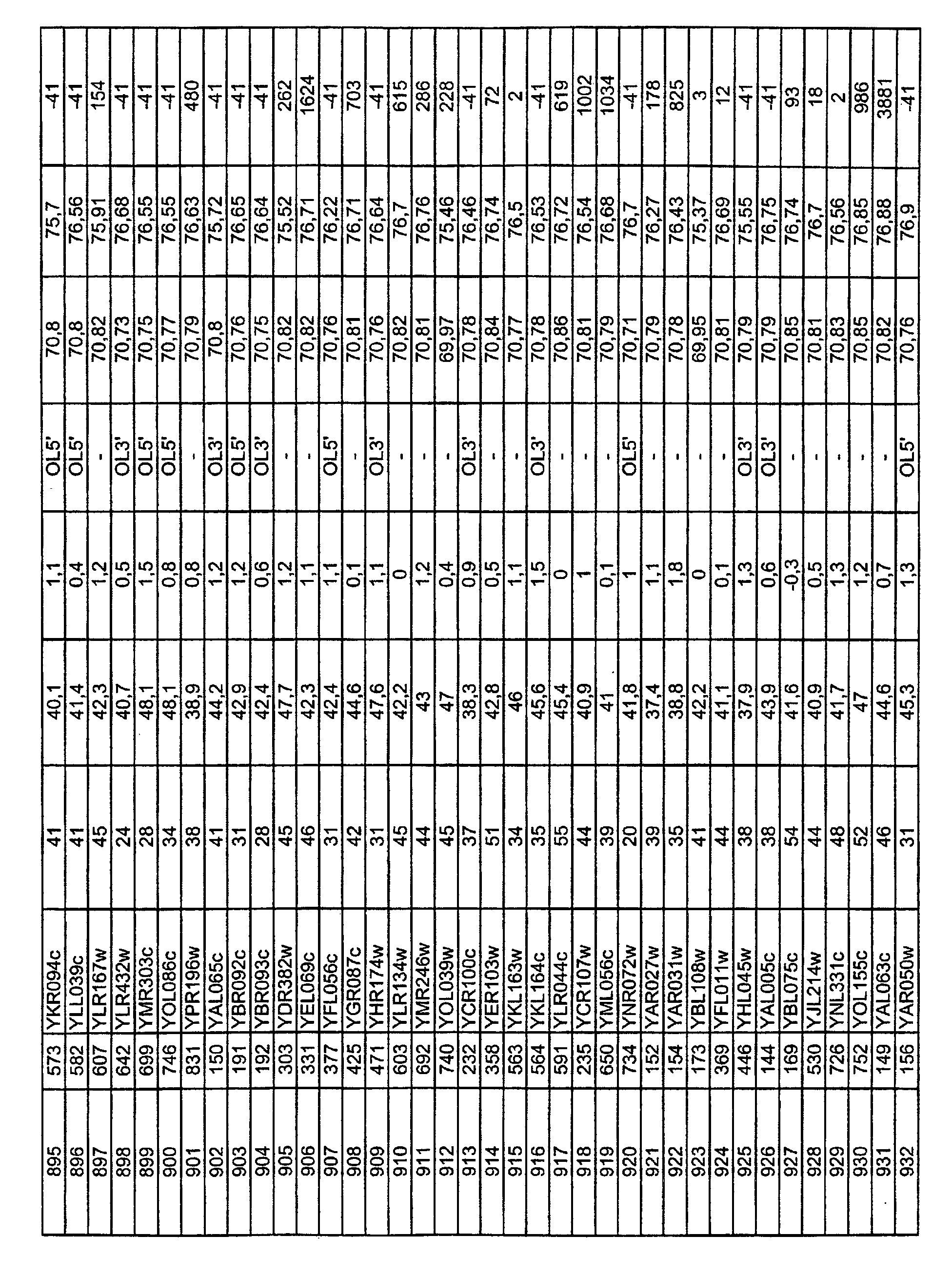

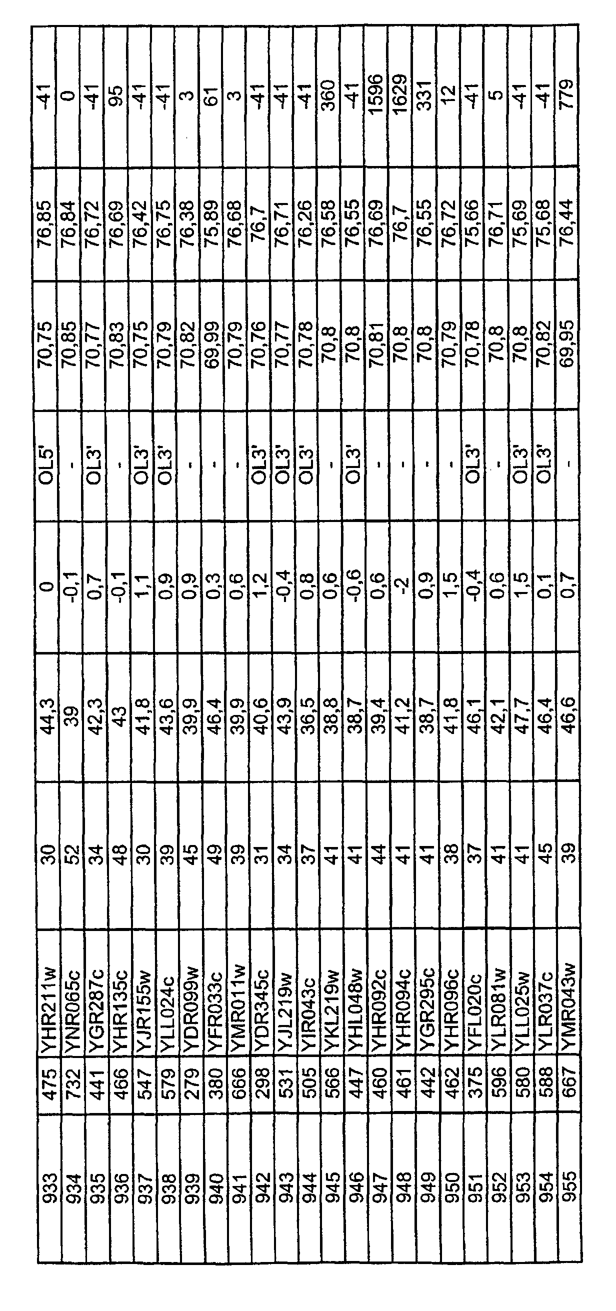

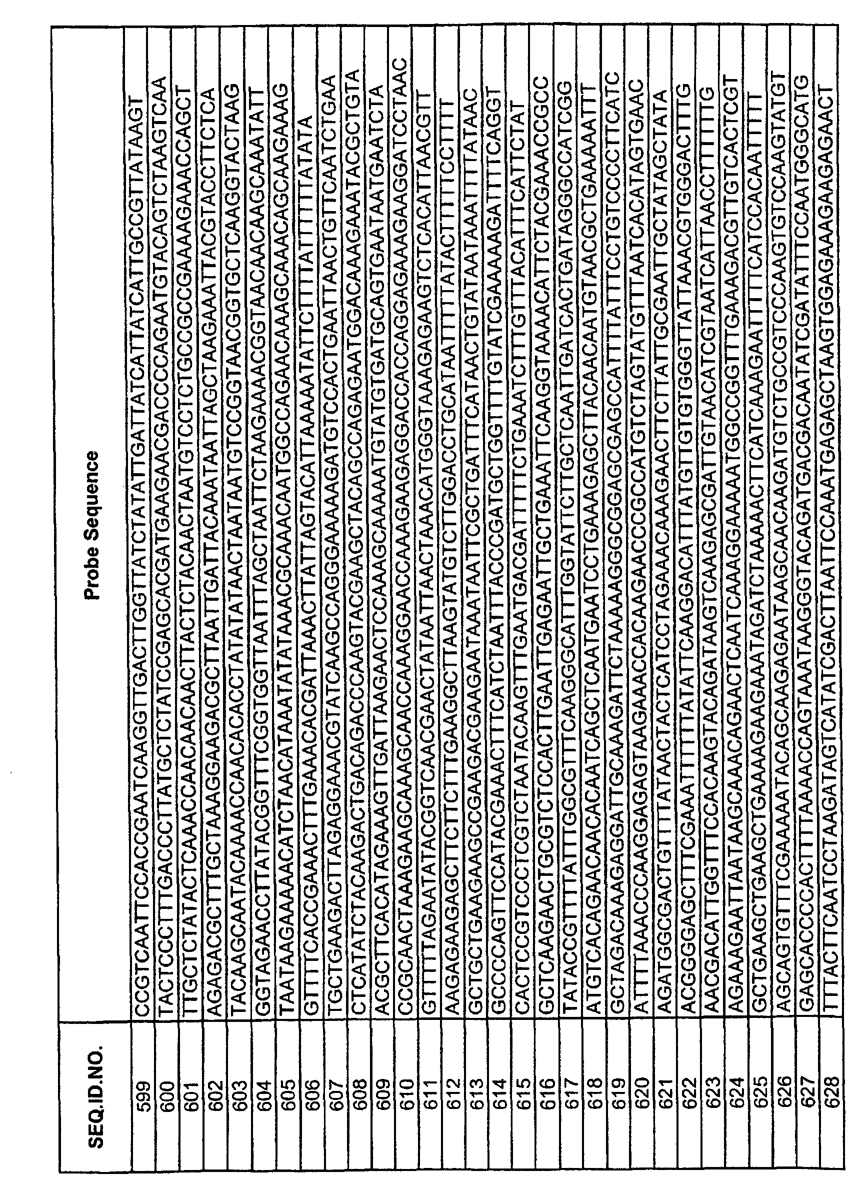

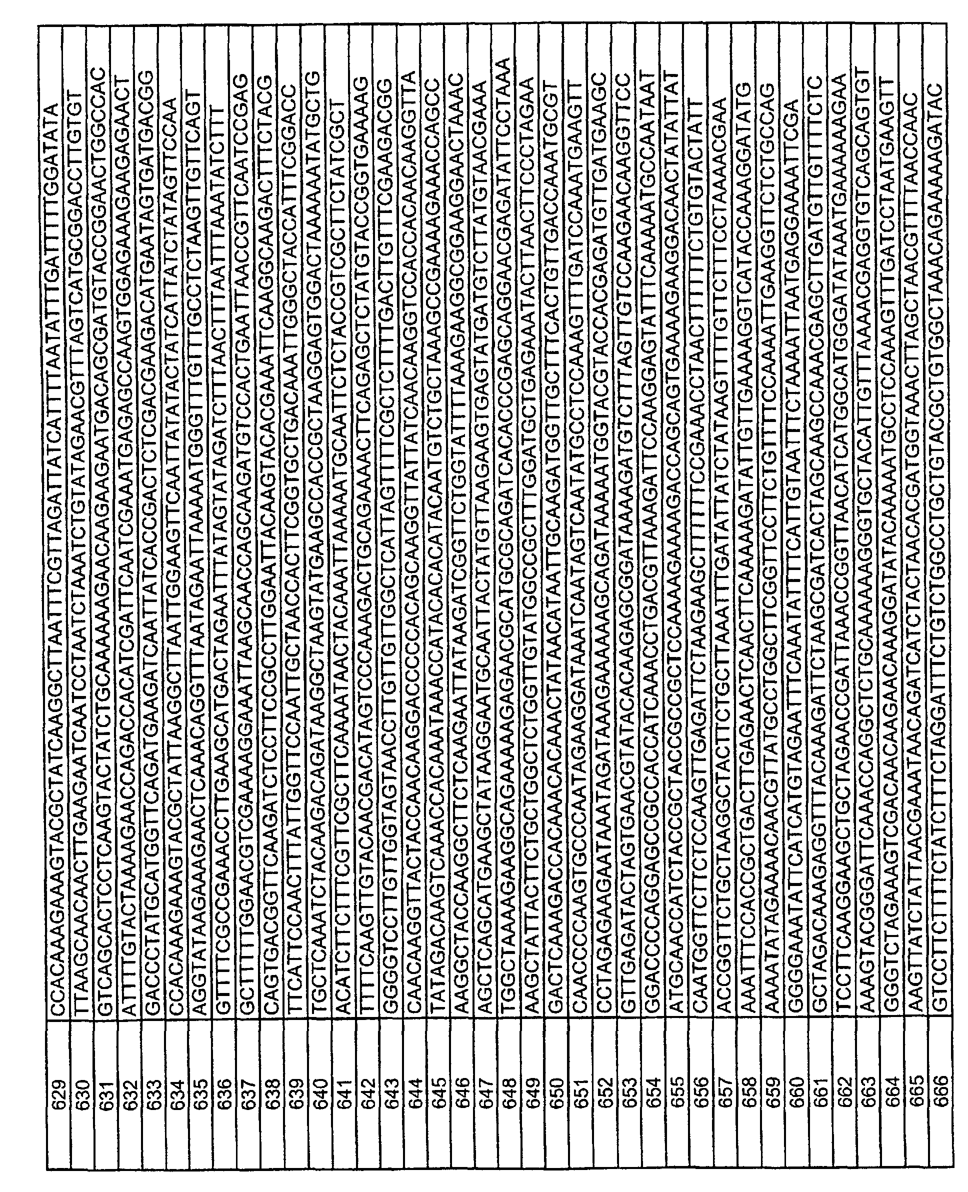

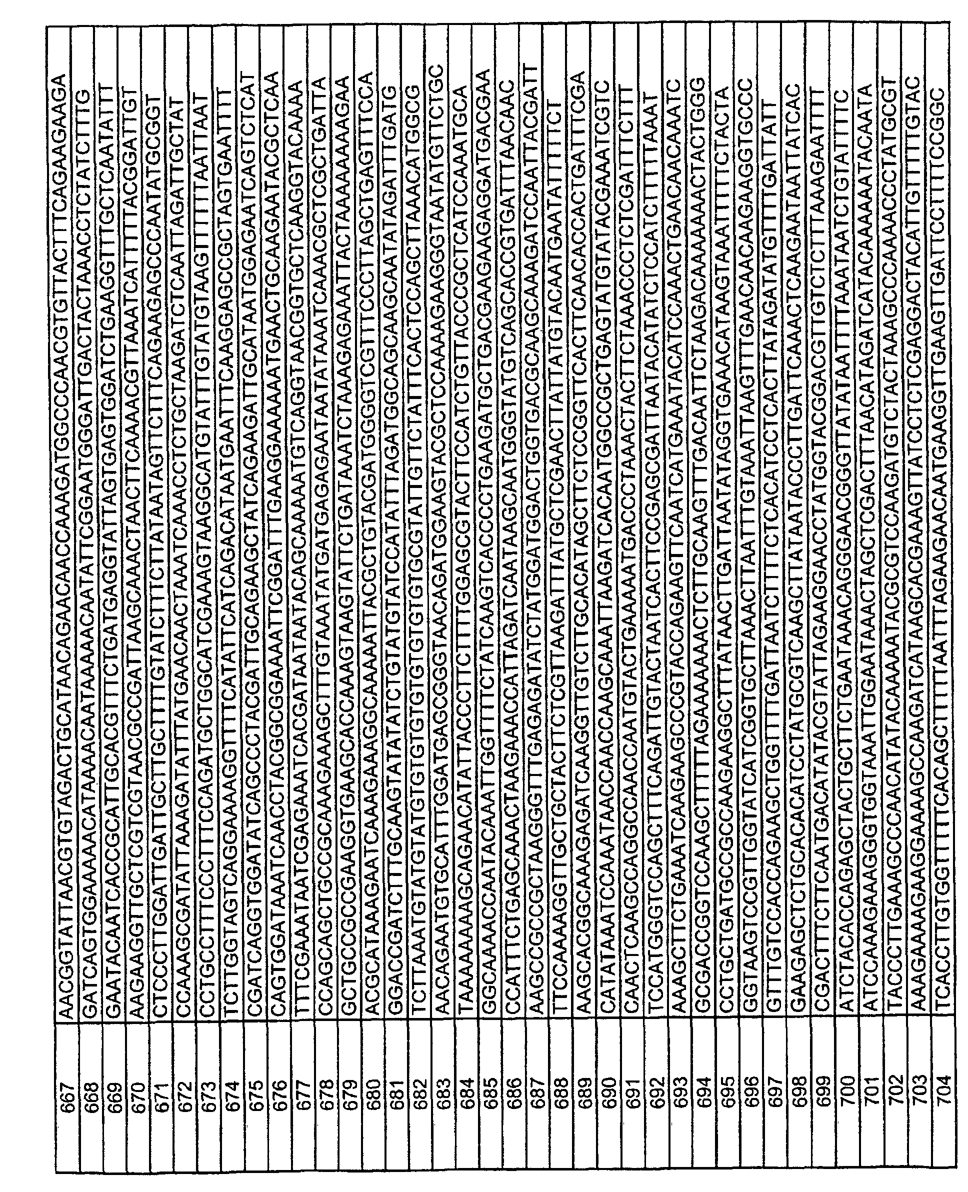

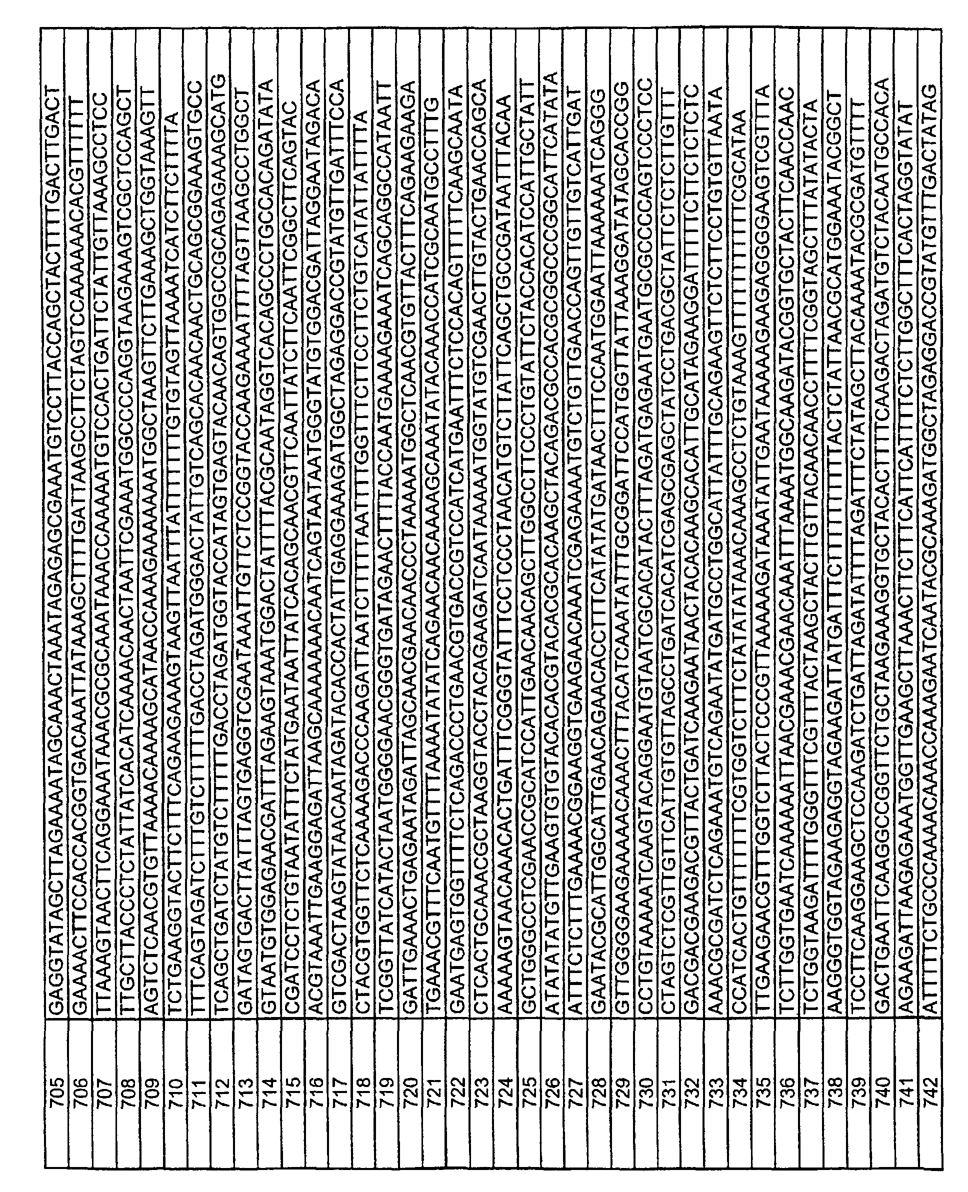

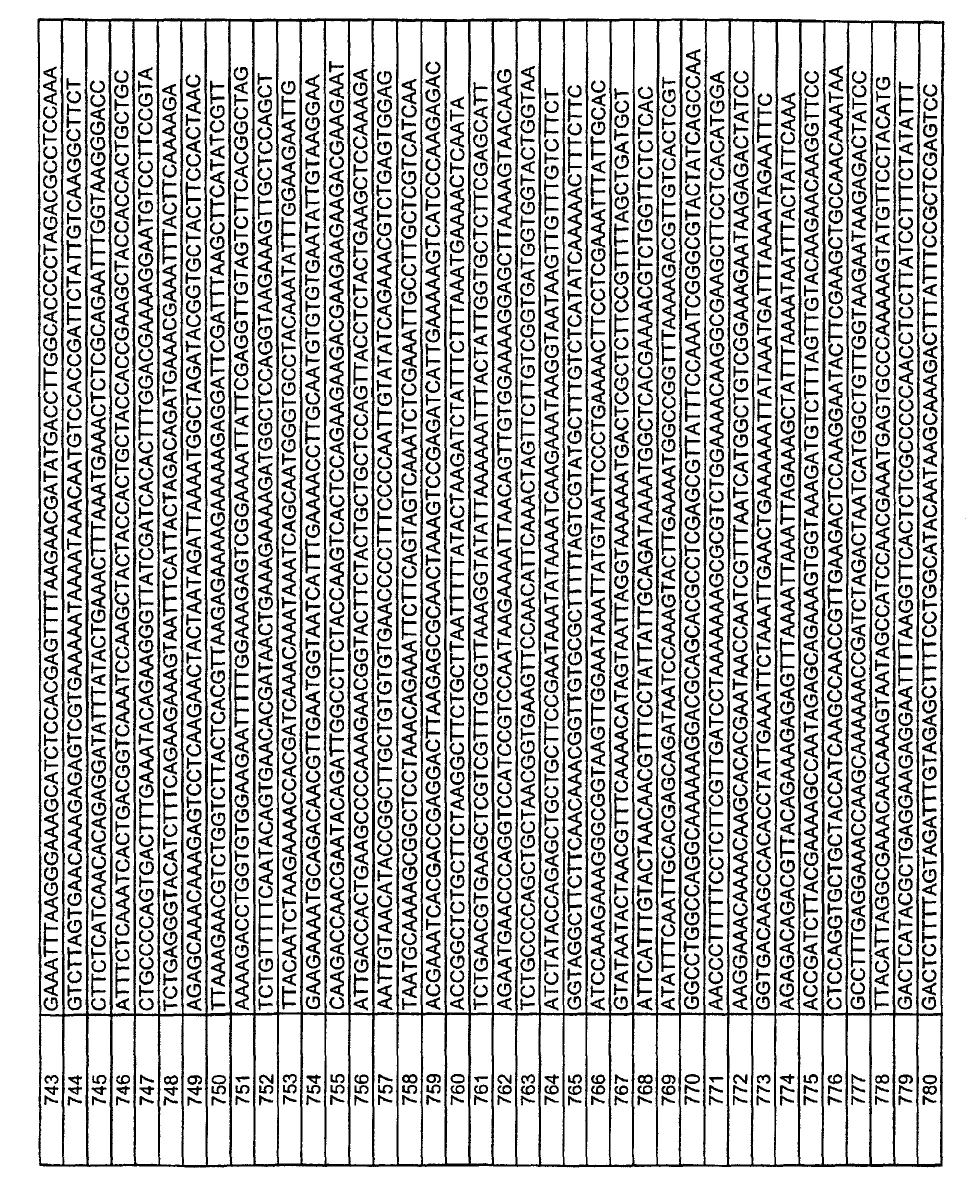

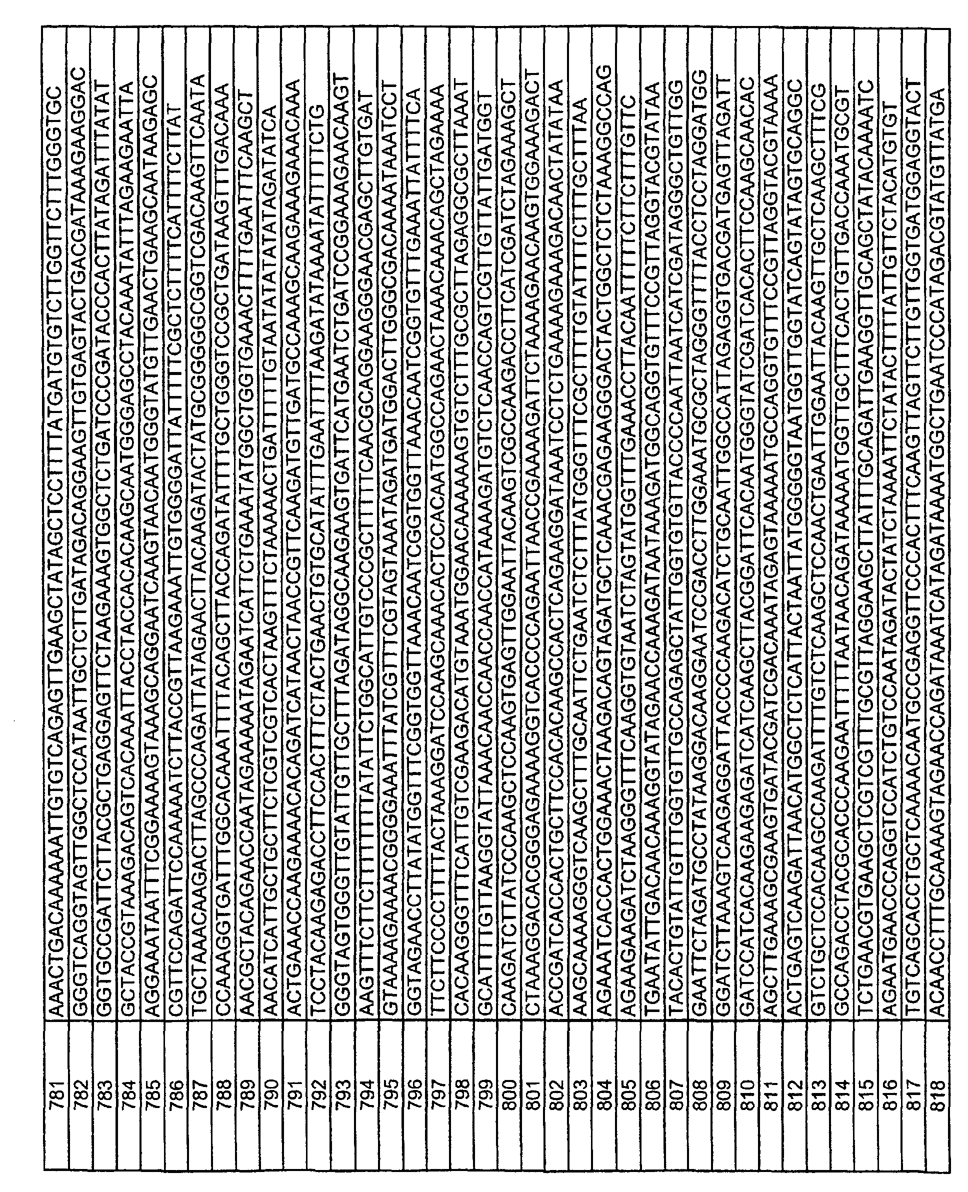

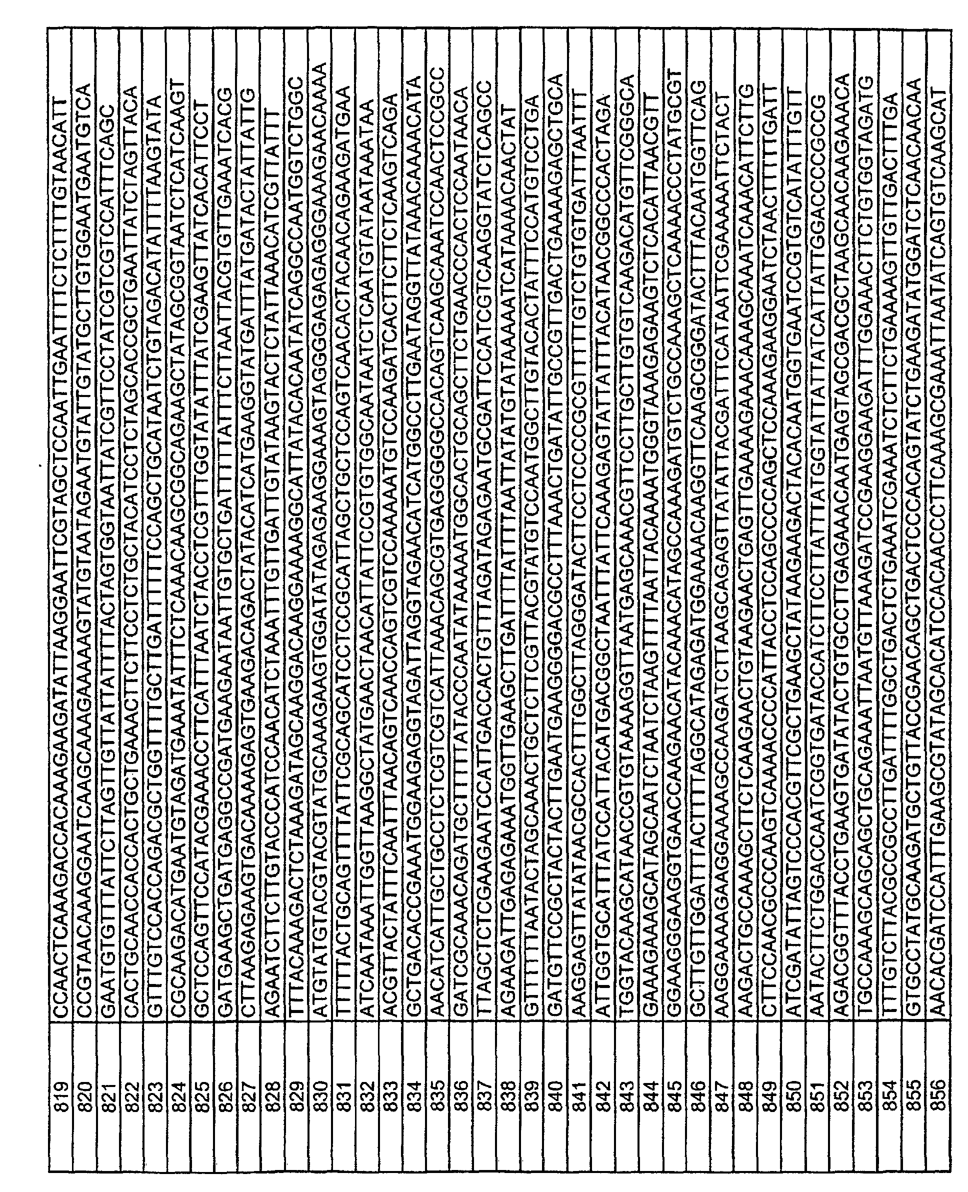

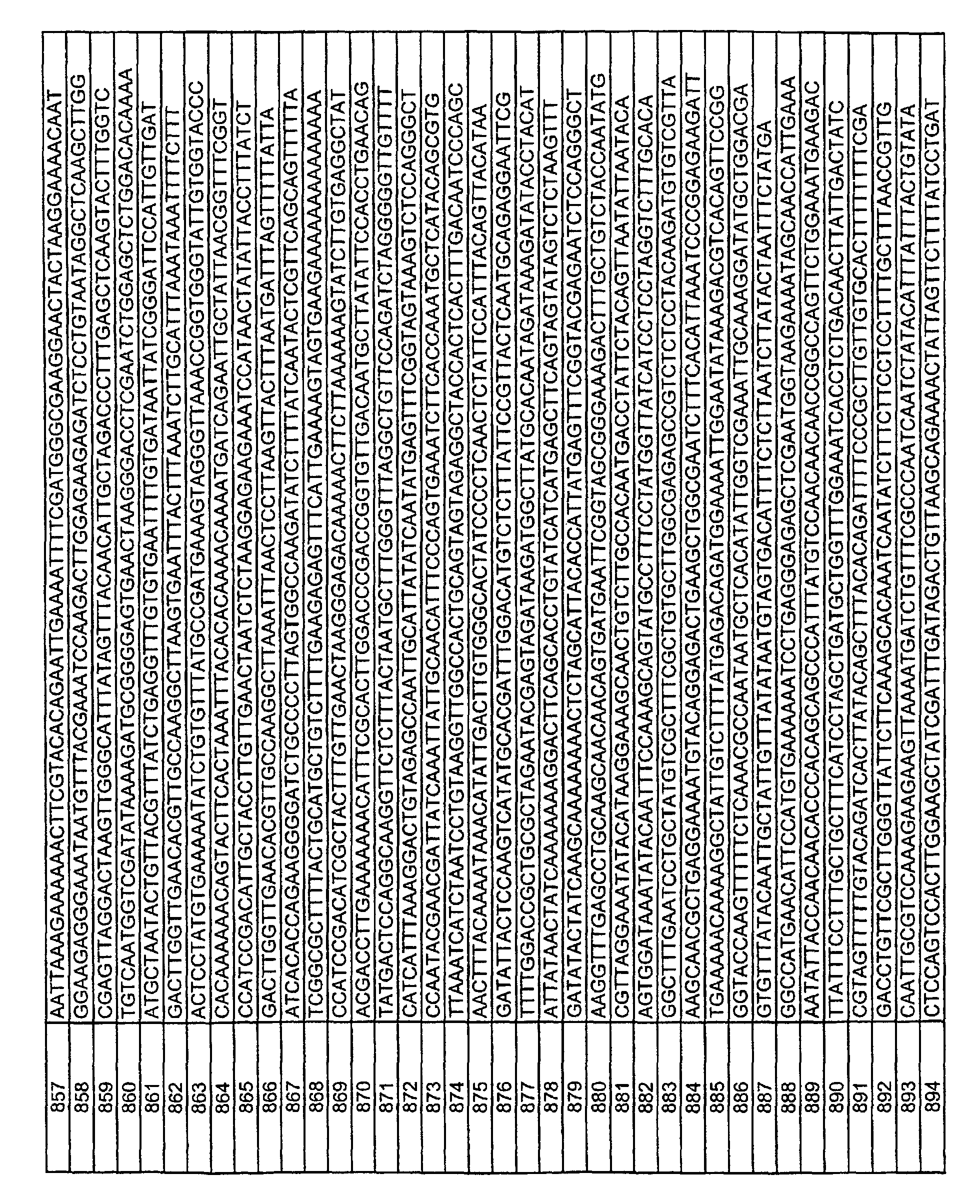

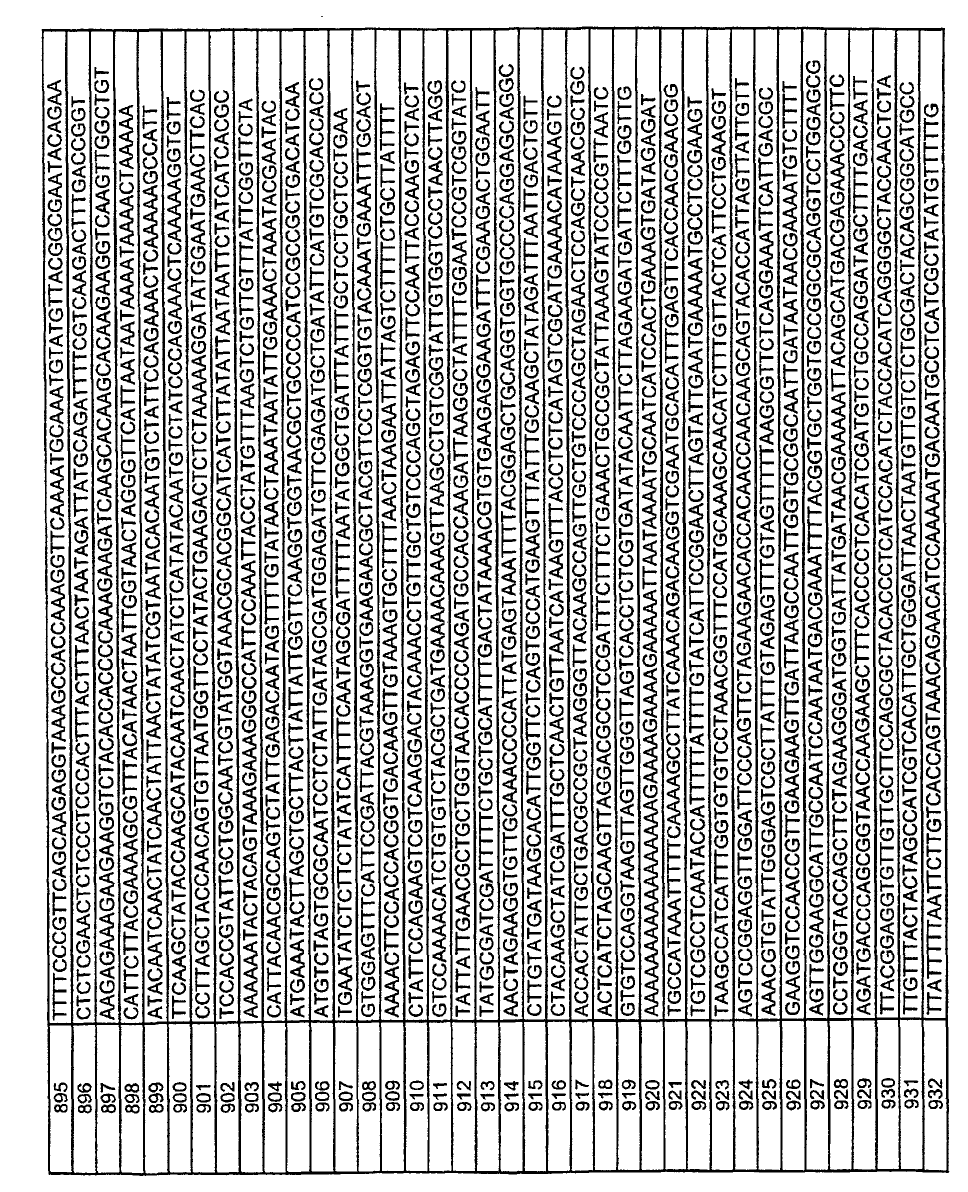

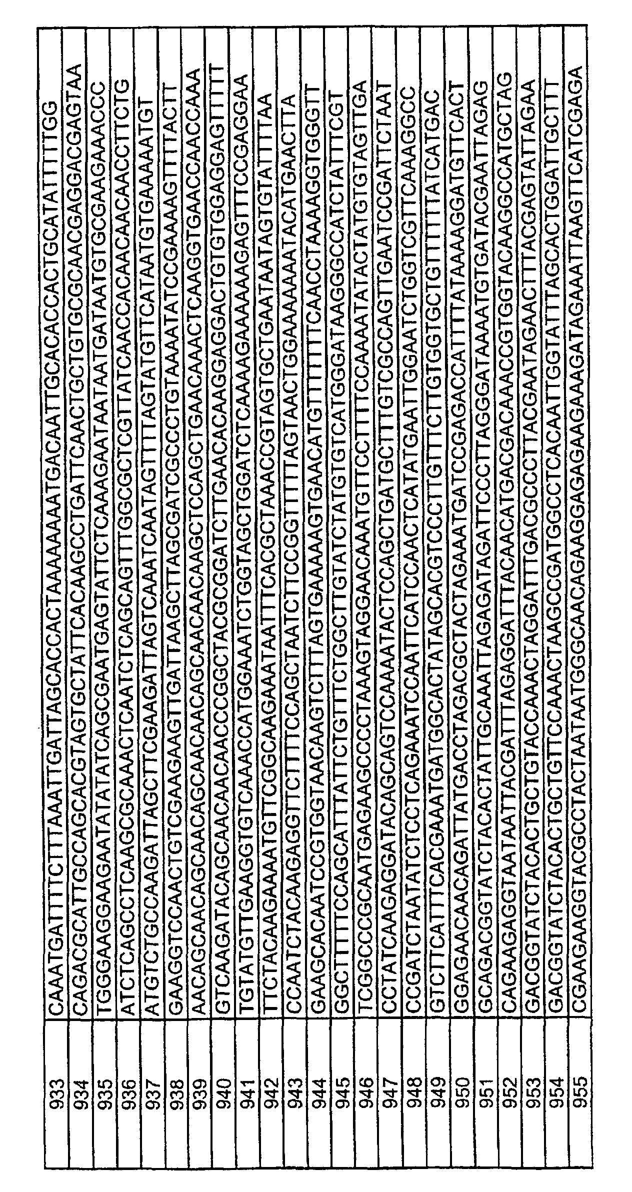

### REFERENCES

Altschul, S. F. et al., " Nucleic Acids Res **25** (17): 3389-402 (1997).
Ausubel, F. et al., Current protocols in molecular biology, K.Janssen ed (1993).
Bisson, L. F. et al., "Yeast sugar transporters." Crit Rev Biochem Mol Biol **28** (4): 259-308 (1993).
Blandin, G. et al., FEBS Lett **487** (1): 31-6 (2000).
Bolton, E. T. and B. J. McCarthy (1962). Proc. Natl. Acad. Sci. **48:** 1390 (1962).
Brachmann, C. B. et al., " Yeast **14** (2): 115-32 (1998).
Bussey, H. et al., Proc Natl Acad Sci U S A **92** (9): 3809-13 (1995).
Cliften, P. F. et al, Genome Res **11** (7): 1175-86 (2001).
DeRisi, J. et al., FEBS Lett **470** (2): 156-60 (200).
DeRisi, J. L. et al, Science **278** (5338): 680-6 (1997).
Dujon, B. et al., Nature **369** (6479): 371-8 (1994).
Girke, T. et al., Plant Physiol **124** (4): 1570-81 (2000).
Goffeau, A. et al., Science **274** (5287): 546, 563-7 (1996).
Goffeau, A. et al., Nature **387**: 1-105 (1997).
Grate, L. and M. Ares, Jr., Methods Enzymol **350** : 380-92 (2002).
Heller, R. A. et al., Proc Natl Acad Sci U S A **94** (6): 2150-5 (1997).
Hughes, T. R. et al., Nat Biotechnol **19** (4): 342-7 (2001).
Johnston, M. et al., Science **265** (5181): 2077-82 (1994).
Le Novere, N., Bioinformatics **17** (12): 1226-7 (2001).
Lipshutz, R. J. et al, Nat Genet **21** (1 Suppl): 20-4 (1999).
Lopez, P. J. and B. Seraphin, Nucleic Acids Res **28** (1): 85-6 (2000).
Lucau-Danila, A. et al., Yeast **16** (6): 547-52 (2000).
Mewes, H. W. et al., Nature **387** (6632 Suppl): 7-65 (1997).
Nielsen, H. B. et al., Bioinformatics **18** (2): 321-2 (2002).
Raddatz, G. et al., Bioinformatics **17** (1): 98-9 (2001).
Richard, G. F. et al., Res Microbiol **148** (9): 731-44 (1997).
Richmond, C. S. et al., Nucleic Acids Res **27** (19): 3821-35 (1999).
Rouillard, J. M. et al., Bioinformatics **18** (3): 486-7 (2002).
Rozen, S. and H. J. Skaletsky (1998). "Primer3. Code available at http://www-genome.wi.mit.edu/genome_software/other/primer3.html."
Sharp, P. M. et al. Nucleic Acids Res **15**(3): 1281-95 (1987).
Souciet, J. et al., FEBS Lett **487** (1): 3-12 (2000).
Walter, A. E. et al., Proc Natl Acad Sci U S A **91** (20): 9218-22 (1994).
Yang, Y. H. et al., Proceedings of SPIE (2001).
Microarrays: Optical Technologies and Informatics. Y. C. M.L. Bittner, A.N. Dorsel, and E.R. Dougherty (eds). **Vol. 4266**: Tech. report #589.

## Claims

1. A nucleotide sequence selected from the group consisting of SEQ.ID.NO.1 to SEQ.ID.NO.955 and their variants.

2. The nucleotide sequence according to claim 1, which is specific for a gene of *Saccharomyces cerevisiae.*

3. The nucleotide sequence according to claim 2, selected from the group consisting of SEQ.ID.NO.1 to SEQ.ID.NO.598.

4. The nucleotide sequence according to claim 2, selected from the group consisting of SEQ.ID.NO.599 to SEQ.ID.NO.955.

5. The nucleotide primer for obtaining by PCR amplification the nucleotide sequence according to any one of claims 1 to 3.

6. The nucleotide primer according to claim 5, selected from the group consisting of SEQ.ID.NO.956 to SEQ.ID.NO.1553.

7. An amplicon nucleotide sequence, amplified by two nucleotide primers selected from the group consisting of primers according to the claim 6.

8. A peptide encoded by the nucleotide sequence according to any one of claims 1 to 4.

9. A kit of parts comprising at least one nucleotide sequence according to any one of claims 1 to 4 or 7 and/or one nucleotide primer according to claim 5 or 6.

10. A diagnostic kit for the identification of *Saccharomyces cerevisiae* genes comprising at least one nucleotide sequence according to any one of claim 1 to 4 or 7 and/or a primer according to the claims 5 or 6.

11. The diagnostic kit according to claim 9, **characterised in that** the nucleotide sequence or the nucleotide primer is bound to the surface of a solid support surface according to a microarray.

12. A microarray comprising at least 400, preferably at least 500, more preferably at least 700, more specifically at least 900 probes, each probe being specific for a unique target gene of *Saccharomyces cerevisiae.*

13. The microarray according to claim 5, **characterised in that** it comprises 955 probes selected from the group consisting of SEQ. ID n°1 to SEQ. ID n° 955.

14. A method for the selection of a nucleotide coding sequence specific for a *Saccharomyces cerevisiae* gene comprising the steps of:
a) comparing the CDS, which is the test nucleotide sequence, to all the hit sequences of the *Saccharomyces cerevisiae genome* so as to obtain sequence alignments;
b) classifying the alignments obtained in step a) into two classes, a class(i), also referred as "Type4 alignment", and class (ii), also referred as "Types 1,2,3", class(i) corresponding to the alignments wherein two or more test sequence regions arranged in a given order within the entire test sequence have matches against two or several hit sequence regions in the same order along a unique hit sequence, and class (ii) corresponding to the other test sequence alignments;
c) identifying for each of said alignments the potential cross-hybridising regions by calculating the values of at least three parameters, which are the size AS, the percentage of identity percent and the longest contiguous perfect match segment and considering the existence of cross-hybridizing regions when alignments meet one of the two following conditions (i) the size AS is greater than 50 and the identity is greater than 70 for Type4 alignments or (ii) the longest contiguous perfect match segment LC is greater than 30 for Types 1-3 alignments;
d) comparing any nucleotide sequence longer than 72 nucleotides and devoid of potential cross-hybridising regions, introns and trinucleotide repeats with intergenic sequences of *Saccharomyces cerevisiae* genome; wherein the remaining non-cross-hybridising regions are sequences specific for *Saccharomyces cerevisiae* CDS;
e) selecting said remaining sequences
f) and possibly fixing said nucleotide sequences at dedicated locations upon the surface of a solid support according to a micro-array.

15. A method for designing an oligonucleotide probe specific for a *Saccharomyces cerevisiae* CDS comprising the steps of:
a) providing a sequence specific for a *Saccharomyces cerevisiae* CDS selected by the method according to claim 14;
b) designing a synthetic oligonucleotide that meets the criteria of having a GC content in percent between 34-46 and of being devoid of monotonous hexanucleotides and of secondary structures.

16. A method for designing a primer pair which can be used for the amplification of a sequence specific for a *Saccharomyces cerevisiae* CDS comprising the steps of:
a) providing a sequence specific for a *Saccharomyces cerevisiae* CDS selected by the method of claim 14;
b) designing a primer pair with the following criteria: a primer length about 20 pb, a primer melting temperature range of about 56-63°C and an optimum PCR product size about 300-700 pb.

17. A method for detecting and/or possibly quantifying a target nucleotide sequence of *Saccharomyces cerevisiae* and/or its expression in a biological sample, which comprises the steps of:
a) possibly purifying said sample in order to obtain a sufficient amount of said target sequence;
b) putting said target sequence in contact with the set of other sequences selected from the group consisting of SEQ.ID.NO.1 to SEQ.ID.NO.955, or with the primer pairs consisting of SEQ.ID.NO.956 to SEQ.ID.NO.1553;
c) and identifying if said target sequence corresponds to a sequence specific *for Saccharomyces cerevisiae* by testing the possibility of having a hybridisation between said sequence with at least one of the said other sequences or an amplification of the target sequence by complementary base pairing with the primer pairs.
